# EUROPEAN PATENT SPECIFICATION

(11) **EP 4 210 694 B1**
(45) Date of publication and mention of the grant of the patent: **15.10.2025**
(21) Application number: 21867551.0
(22) Date of filing: 09.09.2021
(51) Int. Cl.: C07D 401/14, C07D 403/14, C07D 471/04, C07D 487/04, C07D 487/08, C07D 491/048, A61P 3/10, A61P 9/00, A61P 27/02, A61P 29/00, A61P 35/00, A61P 37/00, A61K 31/497, A61K 31/506, A61K 45/06

(54) **PLASMA KALLIKREIN INHIBITORS**
PLASMAKALLIKREINHEMMER
INHIBITEURS DE LA KALLICRÉINE PLASMATIQUE

(30) Priority: 10.09.2020 US 202063076592 P
(43) Date of publication of application: 19.07.2023
(73) Proprietor: Merck Sharp & Dohme LLC, Rahway, New Jersey 07065 (US)
(72) Inventor: OGAWA, Anthony Ken, San Francisco, CA 94080 (US); SINZ, Christopher, J., Walnut Creek, CA 94598 (US); HICKS, Jacqueline, D., Kenilworth, NJ 07033 (US); CHENG, Alan, C., San Francisco, CA 94080 (US); YANG, Song, San Francisco, CA 94080 (US); BAO, Jianming, Princeton, NJ 08540 (US); HAYES, Donna, A. A. W., San Francisco, CA 94080 (US); LANG, Simon, B., San Francisco, CA 94080 (US); TIAN, Maoqun, Forster, CA 94404 (US); JABRI, Salman, San Francisco, CA 94080 (US); SHEARN-NANCE, Galen Paul, Tiburon, CA 94920 (US); KUANG, Rongze, Kenilworth, NJ 07033 (US); ZHAO, Zhiqiang, Kenilworth, NJ 07033 (US); WU, Zhicai, Kenilworth, NJ 07033 (US)
(74) Representative: Merck Sharp & Dohme LLC
(86) International application number: PCT/US2021/049545
(87) International publication number: WO 2022/056051

(56) References cited:
- WO-A1-2012/017020
- WO-A1-2017/207983
- WO-A1-2021/257353
- US-A1- 2010 190 980
- US-A1- 2012 035 168
- US-B2- 9 284 298
- DATABASE Pubchem Subtances 1 November 2019 (2019-11-01), "SUBSTANCE RECORD SID 386554046", XP055917341, retrieved from ncbi Database accession no. SID 386554046

## Description

### BACKGROUND OF THE INVENTION

Plasma kallikrein is a zymogen of a trypsin-like serine protease and is present in plasma. The gene structure is similar to that of factor XI. Overall, the amino acid sequence of plasma kallikrein has 58% homology to factor XI. Proteolyticactivation by factor XIIa at an internal I 389-R390 bond yields a heavy chain (371 amino acids) and a light chain (248 amino acids). The active site of plasma kallikrein is contained in the light chain. The light chain of plasma kallikrein reacts with protease inhibitors, including alpha 2 macroglobulin and Cl-inhibitor. Interestingly, heparin significantly accelerates the inhibition of plasma kallikrein by antithrombin III in the presence of high molecular weight kininogen (HMWK). In blood, the majority of plasma kallikrein circulates in complex with HMWK. Plasma kallikrein cleaves HMWK to liberate bradykinin. Bradykinin release results in increase of vascular permeability and vasodilation (for review, Coleman, R., "Contact Activation Pathway", Hemostasis and Thrombosis, pp. 103-122, Lippincott Williams & Wilkins (2001); Schmaier A.H., "Contact Activation", Thrombosis and Hemorrhage, pp. 105-128 (1998)).

Patients presenting genetic deficiency on C1-esterase inhibitor suffer from hereditary angioedema (HAE), a lifelong disease that results in intermittent swelling throughout the body, including the hands, feet, face, throat, genitals and gastrointestinal tract. Analysis of blisters arising from acute episodes have been shown to contain high levels of plasma kallikrein, and treatment with a protein-based reversible plasma kallikrein inhibitor, Ecallantide (Kalbitor), has been approved by the FDA for the treatment of acute attacks of HAE (Schneider, L, et al., J.Allergy Clin.Immunol., 120: p.416 (2007)).

Additionally, the plasma kallikrein-kinin system is abnormally abundant in patients diagnosed with advanced diabetic macular edema (DME). Recent publications have shown that plasma kallikrein contributes to observed retinal vascular leakage and dysfunction in diabetic rodent models (A. Clermont, et al., Diabetes, 60:1590 (2011)), and that treatment with a small molecule plasma kallikrein inhibitor ameliorated the observed retinal vascular permeability and other abnormalities related to retinal blood flow.

It would be desirable in the art to develop plasma kallikrein inhibitors having utility to treat a wide range of disorders, including hereditary angioedema, diabetic macular edema and diabetic retinopathy. WO 2017/207983 A1 and WO 2012/017020 A1 describe plasma kallikrein inhibitor compounds.

### SUMMARY OF THE INVENTION

The present invention relates to compounds of Formula I as defined in the appended claims and pharmaceutically acceptable salts thereof. The compounds of Formula I are inhibitors of plasma kallikrein, and as such may be useful in the treatment, inhibition or amelioration of one or more disease states that could benefit from inhibition of plasma kallikrein, including hereditary angioedema, uveitis, posterior uveitis, wet age related macular edema, diabetic macular edema, diabetic retinopathy and retinal vein occlusion. The compounds of this invention could further be used in combination with other therapeutically effective agents, including but not limited to, other drugs useful for the treatment of hereditary angioedema, uveitis, posterior uveitis, wet age related macular edema, diabetic macular edema, diabetic retinopathy and retinal vein occlusion. The invention furthermore relates to processes for preparing compounds of Formula I, and pharmaceutical compositions which comprise compounds of Formula I and pharmaceutically acceptable salts thereof.

Any references to methods of treatment in the subsequent paragraphs of this description are to be interpreted as references to the compounds, pharmaceutical compositions and medicaments of the present invention for use in a method for treatment of the human (or animal) body by therapy (or for diagnosis).

### DETAILED DESCRIPTION OF THE INVENTION

The present invention relates to compounds of Formula I: 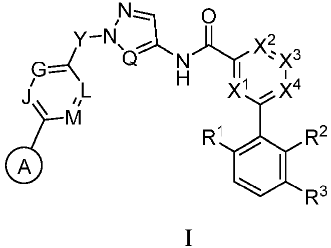 wherein 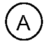 is selected from 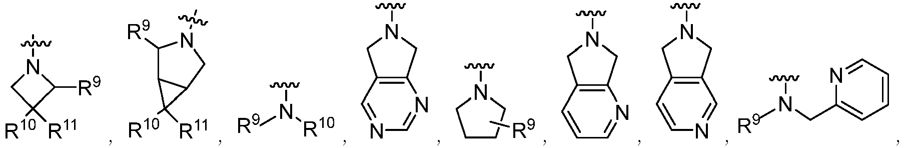 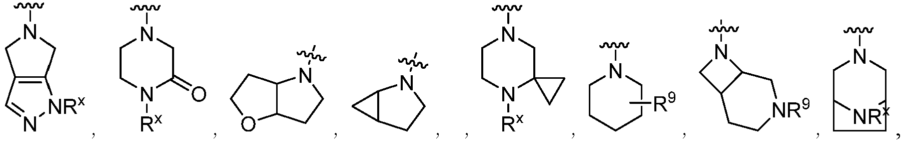 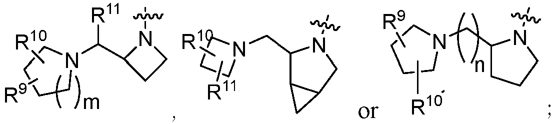
X¹ is N;
X² is CR⁴;
X³ is N;
X⁴ is CH;
Y is CR⁵R⁶ or SO₂;
Q is CH;
G is N or CR⁷;
J is N or CR⁷;
L is N or CR⁷;
M is N or CR⁷;
R¹ is selected from the group consisting of bromo, cyano, tetrazolyl, CHF₂, CF₃, OCHF₂ and SO₂CH₃;
R² is hydrogen or halo;
R³ is hydrogen, halo or OR^{x};
R⁴ is hydrogen, R^{x}, OR^{x}, (C=O)OR^{x}, (C=O)NR^{x}R^{y}, C₁₋₃ alkyl-OR^{x} or NR^{x}R^{y};
R⁵ is hydrogen or C₁₋₃ alkyl, which is optionally substituted with one to three substituents selected from deuterium, halo and hydroxy;
R⁶ is hydrogen, deuterium or C₁₋₃ alkyl;
or R⁵ and R⁶ can be taken together with the carbon atom between them to form a C₃₋₆ cycloalkyl group;
each R⁷ is independently selected from the group consisting of halo, cyano, R^{x}, OR^{x}, cyclopropyl, (C=O)NR^{x}R^{y} and NR^{x}R^{y};
or R⁵ and R⁷ can be taken together with the atoms between them to form a C₃₋₆ cycloalkyl group;
each R⁹ is independently selected from the group consisting of hydrogen, halo, hydroxy, cyano, triazolyl, cyclopropyl, (C=O)NR^{x}R^{y}, (C=O)R^{x} and C₁₋₃ alkyl, wherein said triazolyl group is optionally substituted with R^{x}, said cyclopropyl group is optionally substituted with R^{x} or OR^{x}, and said alkyl group is optionally substituted with one to four substituents selected from halo, hydroxy, heteroaryl, heterocyclyl, C₃₋₆ cycloalkyl, C₃₋₆ cycloalkyl(R¹⁰), OR^{x}, (C=O)NR^{x}R^{y} and NR^{x}R^{y};
R¹⁰ is hydrogen, halo, hydroxy or C₁₋₃ alkyl, wherein said alkyl is optionally substituted with one to four substituents selected from halo and hydroxy;
R¹¹ is hydrogen, halo, hydroxy or R^{x};
or R¹⁰ and R¹¹ can be taken together with the atoms between them to form a C₃₋₆ cycloalkyl group;
each R^{x} is independently hydrogen or C₁₋₆ alkyl, which is optionally substituted with one to four substituents selected from halo and hydroxy;
each R^{y} is independently selected from the group consisting of hydrogen, C₁₋₆ alkyl, C₃₋₆ cycloalkyl, CH₂-C₃₋₆ cycloalkyl and heterocyclyl, wherein said alkyl is optionally substituted with one to four substituents selected from halo and hydroxy, said cycloalkyl is optionally substituted with one or two R^{x} or OR^{x} and said heterocyclyl is optionally substituted with one or two R¹⁰;
m is an integer from zero to two;
n is an integer from zero to two;
or a pharmaceutically acceptable salt thereof.

In an embodiment of the invention, 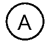 is 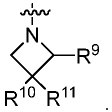 In another embodiment of the invention, 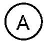 is 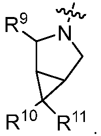 In another embodiment of the invention, 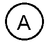 is 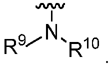 In another embodiment of the invention, 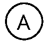 is 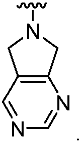 In another embodiment of the invention, 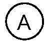 is 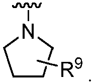 In another embodiment of the invention, 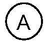 is 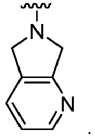 In another embodiment of the invention, 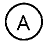 is 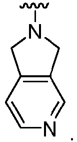 In another embodiment of the invention, 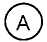 is 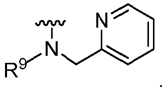 In another embodiment of the invention, 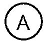 is 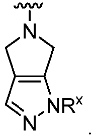 In another embodiment of the invention, 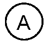 is 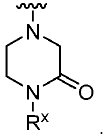 In another embodiment of the invention, 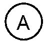 is 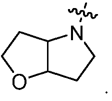 In another embodiment of the invention, 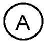 is 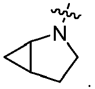 In another embodiment of the invention, 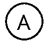 is 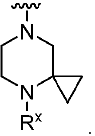 In another embodiment of the invention, 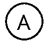 is 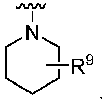 In another embodiment of the invention, 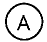 is 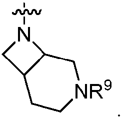 In another embodiment of the invention, 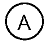 is 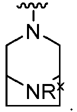 In another embodiment of the invention, 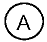 is 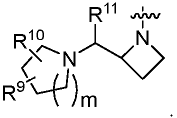 In another embodiment of the invention, 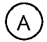 is 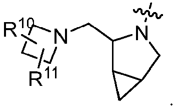 In another embodiment of the invention, 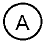 is

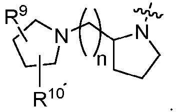

In an embodiment of the invention, Y is CR⁵R⁶. In a class of the invention, Y is CH₂. In another embodiment of the invention, Y is SO₂.

In an embodiment of the invention, G is N. In another embodiment of the invention, G is CR⁷. In a class of the invention, G is CH.

In an embodiment of the invention, J is N. In another embodiment of the invention, J is CR⁷. In a class of the invention, J is CH.

In an embodiment of the invention, L is N. In another embodiment of the invention, L is CR⁷. In a class of the invention, L is CH.

In an embodiment of the invention, M is N. In another embodiment of the invention, M is CR⁷. In a class of the invention, M is CH.

In an embodiment of the invention, J is N; M is N; G is CR⁷; L is CR⁷.

In an embodiment of the invention, R¹ is bromo. In another embodiment of the invention, R¹ is cyano. In another embodiment of the invention, R¹ is tetrazolyl. In another embodiment of the invention, R¹ is CHF₂. In another embodiment of the invention, R¹ is CF₃. In another embodiment of the invention, R¹ is OCHF₂. In another embodiment of the invention, R¹ is SO₂CH₃.

In an embodiment of the invention, R² is hydrogen. In another embodiment of the invention, R² is halo. In a class of the invention, R² is fluoro.

In an embodiment of the invention, R³ is hydrogen. In another embodiment of the invention, R³ is halo. In a class of the invention, R³ is chloro. In another embodiment of the invention, R³ is OCH₃.

In an embodiment of the invention, R⁴ is hydrogen, CH₃, CHF₂, OCH₃, NH₂, N(CH₃)₂, (C=O)OH and (C=O)NH₂.

In an embodiment of the invention, R⁵ is hydrogen, CH₃, CHF₂ or CH₂OH. In another class of the invention, R⁵ is CH₃. In another class of the invention, R⁵ is CHF₂. In another class of the invention, R⁵ is CH₂OH.

In an embodiment of the invention, R⁶ is hydrogen.

In an embodiment of the invention, m is zero. In another embodiment of the invention, m is one. In another embodiment of the invention, m is two.

In an embodiment of the invention, n is zero. In another embodiment of the invention, n is one. In another embodiment of the invention, n is two.

Reference to the preferred classes and subclasses set forth above is meant to include all combinations of particular and preferred groups unless stated otherwise.

Specific embodiments of the present invention include, but are not limited to the compounds identified herein as Examples 1 to 208, or pharmaceutically acceptable salts thereof.

Also included within the scope of the present invention is a pharmaceutical composition which is comprised of a compound of Formula I as described above and a pharmaceutically acceptable carrier. The invention is also contemplated to encompass a pharmaceutical composition which is comprised of a pharmaceutically acceptable carrier and any of the compounds specifically disclosed in the present application. These and other aspects of the invention will be apparent from the teachings contained herein.

The invention includes compositions for treating diseases or condition in which plasma kallikrein activity is implicated. Accordingly the invention includes compositions for treating impaired visual activity, diabetic retinopathy, diabetic macular edema, retinal vein occlusion, hereditary angioedema, diabetes, pancreatitis, cerebral hemorrhage, nephropathy, cardiomyopathy, neuropathy, inflammatory bowel disease, arthritis, inflammation, septic shock, hypotension, cancer, adult respiratory distress syndrome, disseminated intravascular coagulation, blood coagulation during cardiopulmonary bypass surgery, and bleeding from postoperative surgery in a mammal, comprising a compound of the invention in a pharmaceutically acceptable carrier. A class of the invention includes compositions for treating hereditary angioedema, uveitis, posterior uveitis, wet age related macular edema, diabetic macular edema, diabetic retinopathy and retinal vein occlusion. These compositions may optionally include anti-inflammatory agents, anti-VEGF agents, immunosuppressive agents, anticoagulants, antiplatelet agents, and thrombolytic agents. The compositions can be added to blood, blood products, or mammalian organs in order to effect the desired inhibitions.

The invention also includes compositions for preventing or treating retinal vascular permeability associated with diabetic retinopathy and diabetic macular edema in a mammal, comprising a compound of the invention in a pharmaceutically acceptable carrier. These compositions may optionally include anti-inflammatory agents, anti-VEGF agents, immunosuppressive agents, anticoagulants, antiplatelet agents, and thrombolytic agents.

The invention also includes compositions for treating inflammatory conditions of the eye, which includes, but is not limited to, uveitis, posterior uveitis, macular edema, acute macular degeneration, wet age related macular edema, retinal detachments, retinal vein occlusion, ocular tumors, fungal infections, viral infections, multifocal choroiditis, diabetic uveitis, diabetic macular edema, diabetic retinopathy, proliferative vitreoretinopathy, sympathetic opthalmia, Vogt Koyanagi-Harada syndrome, histoplasmosis and uveal diffusion. These compositions may optionally include anti-inflammatory agents, anti-VEGF agents, immunosuppressive agents, anticoagulants, antiplatelet agents, and thrombolytic agents.

The invention also includes compositions treating posterior eye disease, which includes, but is not limited to, uveitis, posterior uveitis, wet age related macular edema, diabetic macular edema, diabetic retinopathy and retinal vein occlusion. These compositions may optionally include anti-inflammatory agents, anti-VEGF agents, immunosuppressive agents, anticoagulants, antiplatelet agents, and thrombolytic agents.

It will be understood that the invention is directed to the compounds of structural Formula I described herein, as well as the pharmaceutically acceptable salts of the compounds of structural Formula I and also salts that are not pharmaceutically acceptable when they are used as precursors to the free compounds or their pharmaceutically acceptable salts or in other synthetic manipulations.

The compounds of the present invention may be administered in the form of a pharmaceutically acceptable salt. The term "pharmaceutically acceptable salt" refers to salts prepared from pharmaceutically acceptable non-toxic bases or acids including inorganic or organic bases and inorganic or organic acids. Salts of basic compounds encompassed within the term "pharmaceutically acceptable salt" refer to non-toxic salts of the compounds of this invention which are generally prepared by reacting the free base with a suitable organic or inorganic acid. Representative salts of basic compounds of the present invention include, but are not limited to, the following: acetate, ascorbate, adipate, alginate, aspirate, benzenesulfonate, benzoate, bicarbonate, bisulfate, bitartrate, borate, bromide, butyrate, camphorate, camphorsulfonate, camsylate, carbonate, chloride, clavulanate, citrate, cyclopentane propionate, diethylacetic, digluconate, dihydrochloride, dodecylsulfanate, edetate, edisylate, estolate, esylate, ethanesulfonate, formic, fumarate, gluceptate, glucoheptanoate, gluconate, glutamate, glycerophosphate, glycollylarsanilate, hemisulfate, heptanoate, hexanoate, hexylresorcinate, hydrabamine, hydrobromide, hydrochloride, 2-hydroxyethanesulfonate, hydroxynaphthoate, iodide, isonicotinic, isothionate, lactate, lactobionate, laurate, malate, maleate, mandelate, mesylate, methylbromide, methylnitrate, methylsulfate, methanesulfonate, mucate, 2-naphthalenesulfonate, napsylate, nicotinate, nitrate, N-methylglucamine ammonium salt, oleate, oxalate, pamoate (embonate), palmitate, pantothenate, pectinate, persulfate, phosphate/diphosphate, pimelic, phenylpropionic, polygalacturonate, propionate, salicylate, stearate, sulfate, subacetate, succinate, tannate, tartrate, teoclate, thiocyanate, tosylate, triethiodide, trifluoroacetate, undeconate, valerate and the like. Furthermore, where the compounds of the invention carry an acidic moiety, suitable pharmaceutically acceptable salts thereof include, but are not limited to, salts derived from inorganic bases including aluminum, ammonium, calcium, copper, ferric, ferrous, lithium, magnesium, manganic, mangamous, potassium, sodium, zinc, and the like. Salts derived from pharmaceutically acceptable organic non-toxic bases include salts of primary, secondary, and tertiary amines, cyclic amines, dicyclohexyl amines and basic ion-exchange resins, such as arginine, betaine, caffeine, choline, N,N-dibenzylethylenediamine, diethylamine, 2-diethylaminoethanol, 2-dimethylaminoethanol, ethanolamine, ethylamine, ethylenediamine, N-ethylmorpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like. Also, included are the basic nitrogen-containing groups may be quaternized with such agents as lower alkyl halides, such as methyl, ethyl, propyl, and butyl chloride, bromides and iodides; dialkyl sulfates like dimethyl, diethyl, dibutyl; and diamyl sulfates, long chain halides such as decyl, lauryl, myristyl and stearyl chlorides, bromides and iodides, aralkyl halides like benzyl and phenethyl bromides and others.

These salts can be obtained by known methods, for example, by mixing a compound of the present invention with an equivalent amount and a solution containing a desired acid, base, or the like, and then collecting the desired salt by filtering the salt or distilling off the solvent. The compounds of the present invention and salts thereof may form solvates with a solvent such as water, ethanol, or glycerol. The compounds of the present invention may form an acid addition salt and a salt with a base at the same time according to the type of substituent of the side chain.

If the compounds of Formula I simultaneously contain acidic and basic groups in the molecule the invention also includes, in addition to the salt forms mentioned, inner salts or betaines (zwitterions).

The present invention encompasses all stereoisomeric forms of the compounds of Formula I. Unless a specific stereochemistry is indicated, the present invention is meant to comprehend all such stereoisomeric forms of these compounds. Centers of asymmetry that are present in the compounds of Formula I can all independently of one another have (R) configuration or (S) configuration. When bonds to the chiral carbon are depicted as straight lines in the structural Formulas of the invention, it is understood that both the (R) and (S) configurations of the chiral carbon, and hence both each individual enantiomer and mixtures thereof, are embraced within the Formula. When a particular configuration is depicted, that enantiomer (either (R) or (S), at that center) is intended. Similarly, when a compound name is recited without a chiral designation for a chiral carbon, it is understood that both the (R) and (S) configurations of the chiral carbon, and hence individual enantiomers and mixtures thereof, are embraced by the name. The production of specific stereoisomers or mixtures thereof may be identified in the Examples where such stereoisomers or mixtures were obtained, but this in no way limits the inclusion of all stereoisomers and mixtures thereof from being within the scope of this invention.

Unless a specific enantiomer or diastereomer is indicated, the invention includes all possible enantiomers and diastereomers and mixtures of two or more stereoisomers, for example mixtures of enantiomers and/or diastereomers, in all ratios. Thus, enantiomers are a subject of the invention in enantiomerically pure form, both as levorotatory and as dextrorotatory antipodes, in the form of racemates and in the form of mixtures of the two enantiomers in all ratios. In the case of a cis/trans isomerism the invention includes both the cis form and the trans form as well as mixtures of these forms in all ratios. The preparation of individual stereoisomers can be carried out, if desired, by separation of a mixture by customary methods, for example by chromatography or crystallization, by the use of stereochemically uniform starting materials for the synthesis or by stereoselective synthesis. Optionally a derivatization can be carried out before a separation of stereoisomers. The separation of a mixture of stereoisomers can be carried out at an intermediate step during the synthesis of a compound of Formula I or it can be done on a final racemic product. Absolute stereochemistry may be determined by X-ray crystallography of crystalline products or crystalline intermediates which are derivatized, if necessary, with a reagent containing a stereogenic center of known configuration. Where compounds of this invention are capable of tautomerization, all individual tautomers as well as mixtures thereof are included in the scope of this invention. The present invention includes all such stereoisomers and tautomers, as well as salts, solvates (including hydrates) and solvated salts of such racemates, enantiomers, diastereomers and tautomers and mixtures thereof.

In the compounds of the invention, the atoms may exhibit their natural isotopic abundances, or one or more of the atoms may be artificially enriched in a particular isotope having the same atomic number, but an atomic mass or mass number different from the atomic mass or mass number predominantly found in nature. The present invention is meant to include all suitable isotopic variations of the specifically and generically described compounds. For example, different isotopic forms of hydrogen (H) include protium (1_{H}) and deuterium (2_{H}). Protium is the predominant hydrogen isotope found in nature. Enriching for deuterium may afford certain therapeutic advantages, such as increasing *in vivo* half-life or reducing dosage requirements, or may provide a compound useful as a standard for characterization of biological samples. Isotopically-enriched compounds can be prepared without undue experimentation by conventional techniques well known to those skilled in the art or by processes analogous to those described in the general process schemes and examples herein using appropriate isotopically-enriched reagents and/or intermediates.

When any variable (e.g. R^{x}, etc.) occurs more than one time in any constituent, its definition on each occurrence is independent at every other occurrence. Also, combinations of substituents and variables are permissible only if such combinations result in stable compounds. Lines drawn into the ring systems from substituents represent that the indicated bond may be attached to any of the substitutable ring atoms. If the ring system is bicyclic, it is intended that the bond be attached to any of the suitable atoms on either ring of the bicyclic moiety.

It is understood that substituents and substitution patterns on the compounds of the instant invention can be selected by one of ordinary skill in the art to provide compounds that are chemically stable and that can be readily synthesized by techniques known in the art, as well as those methods set forth below, from readily available starting materials. If a substituent is itself substituted with more than one group, it is understood that these multiple groups may be on the same carbon or on different carbons, so long as a stable structure results. The phrase "optionally substituted" (with one or more substituents) should be understood as meaning that the group in question is either unsubstituted or may be substituted with one or more substituents.

Furthermore, compounds of the present invention may exist in amorphous form and/or one or more crystalline forms, and as such all amorphous and crystalline forms and mixtures thereof of the compounds of Formula I are intended to be included within the scope of the present invention. In addition, some of the compounds of the instant invention may form solvates with water (i.e., a hydrate) or common organic solvents. Such solvates and hydrates, particularly the pharmaceutically acceptable solvates and hydrates, of the instant compounds are likewise encompassed within the scope of this invention, along with un-solvated and anhydrous forms.

Except where noted herein, the terms "alkyl" and "alkylene" are intended to include both branched- and straight-chain saturated aliphatic hydrocarbon groups having the specified number of carbon atoms. Commonly used abbreviations for alkyl groups are used throughout the specification, e.g. methyl, may be represented by conventional abbreviations including "Me" or CH₃ or a symbol that is an extended bond as the terminal group, e.g. " 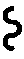 -", ethyl may be represented by "Et" or CH₂CH₃, propyl may be represented by "Pr" or CH₂CH₂CH₃, butyl may be represented by "Bu" or CH₂CH₂CH₂CH₃, etc. "C₁₋₄ alkyl" (or "C₁-C₄ alkyl") for example, means linear or branched chain alkyl groups, including all isomers, having the specified number of carbon atoms. For example, the structures 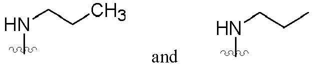 have equivalent meanings. C₁₋₄ alkyl includes n-, iso-, sec- and t-butyl, n- and isopropyl, ethyl and methyl. If no number is specified, 1-4 carbon atoms are intended for linear or branched alkyl groups.

Except where noted, the term "cycloalkyl" means a monocyclic or bicyclic saturated aliphatic hydrocarbon group having the specified number of carbon atoms. For example, "cycloalkyl" includes cyclopropyl, cyclobutyl, cyclopentyl, cyclohexyl, and so on.

Except where noted, the term "aryl", as used herein, represents a stable monocyclic or bicyclic ring system of up to 10 carbon atoms in each ring, wherein at least one ring is aromatic. Bicyclic aryl ring systems include fused ring systems, where two rings share two atoms, and spiro ring systems, where two rings share one atom. Aryl groups within the scope of this definition include, but are not limited to: phenyl, indene, isoindene, naphthalene, and tetralin.

Except where noted, the term "heteroaryl", as used herein, represents a stable monocyclic or bicyclic ring system of up to 10 atoms in each ring, wherein at least one ring is aromatic, and at least one ring contains from 1 to 4 heteroatoms selected from the group consisting of O, N and S. Bicyclic heteroaryl ring systems include fused ring systems, where two rings share two atoms, and spiro ring systems, where two rings share one atom. Heteroaryl groups within the scope of this definition include but are not limited to: azaindolyl, benzoimidazolyl, benzisoxazolyl, benzofuranyl, benzofurazanyl, benzopyrazolyl, benzotriazolyl, benzothiophenyl, benzoxazolyl, carbazolyl, carbolinyl, cinnolinyl, dihydroindenyl, furanyl, indolinyl, indolyl, indolazinyl, indazolyl, isobenzofuranyl, isoindolyl, isoquinolyl, isothiazolyl, isoxazolyl, naphthalenyl, naphthpyridinyl, oxadiazolyl, oxazolyl, oxazoline, isoxazoline, pyranyl, pyrazinyl, pyrazolyl, pyrazolopyrimidinyl, pyridazinyl, pyridopyridinyl, pyridyl, pyrimidinyl, pyrrolyl, quinazolinyl, quinolyl, quinoxalinyl, tetrazolyl, tetrazolopyridyl, thiadiazolyl, thiazolyl, thienyl, triazolyl, dihydrobenzoimidazolyl, dihydrobenzofuranyl, dihydrobenzothiophenyl, dihydrobenzoxazolyl, dihydroindolyl, dihydroquinolinyl, dihydrobenzodioxinyl, dihydropyrazoloxazinyl, dihydropyrazolyothiazinedioxidyl, methylenedioxybenzene, benzothiazolyl, benzothienyl, quinolinyl, isoquinolinyl, oxazolyl, tetrahydroquinoline and 3-oxo-3,4dihydro-2N-benzo[b][1,4]thiazine. If the heteroaryl contains nitrogen atoms, it is understood that the corresponding N-oxides thereof are also encompassed by this definition.

The term "heterocycle" or "heterocyclyl" as used herein is intended to mean a stable nonaromatic monocyclic or bicyclic ring system of up to 10 atoms in each ring, unless otherwise specified, containing from 1 to 4 heteroatoms selected from the group consisting of O, N, S, SO, or SO₂. Bicyclic heterocyclic ring systems include fused ring systems, where two rings share two atoms, and spiro ring systems, where two rings share one atom. "Heterocyclyl" therefore includes, but is not limited to the following: azaspirononanyl, azaspirooctanyl, azetidinyl, dioxanyl, oxadiazaspirodecenyl, oxaspirooctanyl, oxazolidinonyl, piperazinyl, piperidinyl, pyrrolidinyl, morpholinyl, thiomorpholinyl, tetrahydrofurnayl, tetrahydropyranyl, dihydropiperidinyl, tetrahydrothiophenyl and the like. If the heterocycle contains a nitrogen, it is understood that the corresponding N-oxides thereof are also encompassed by this definition.

Except where noted, the term "halogen" or "halo" means fluorine, chlorine, bromine or iodine.

"Celite^{®}" (Fluka) diatomite is diatomaceous earth, and can be referred to as "celite".

Except where noted herein, structures containing substituent variables such as variable "R" below: 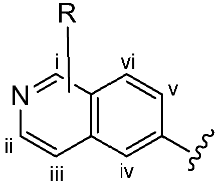 which are depicted as not being attached to any one particular bicyclic ring carbon atom, represent structures in which the variable can be optionally attached to any bicyclic ring carbon atom. For example, variable R shown in the above structure can be attached to any one of 6 bicyclic ring carbon atoms i, ii, iii, iv, v or vi.

Except where noted herein, bicyclic ring systems include fused ring systems, where two rings share two atoms, and spiro ring systems, where two rings share one atom.

The invention also relates to medicaments containing at least one compound of the Formula I and/or of a pharmaceutically acceptable salt of the compound of the Formula I and/or an optionally stereoisomeric form of the compound of the Formula I or a pharmaceutically acceptable salt of the stereoisomeric form of the compound of Formula I, together with a pharmaceutically suitable and pharmaceutically acceptable vehicle, additive and/or other active substances and auxiliaries.

The term "patient" used herein is taken to mean mammals such as primates, humans, sheep, horses, cattle, pigs, dogs, cats, rats, and mice.

The medicaments according to the invention can be administered by oral, inhalative, rectal or transdermal administration or by subcutaneous, intraarticular, intraperitoneal or intravenous injection. Oral administration is preferred. Coating of stents with compounds of the Formulas I and other surfaces which come into contact with blood in the body is possible.

Suitable solid or galenical preparation forms are, for example, granules, powders, coated tablets, tablets, (micro)capsules, suppositories, syrups, juices, suspensions, emulsions, drops or injectable solutions and preparations having prolonged release of active substance, in whose preparation customary excipients such as vehicles, disintegrants, binders, coating agents, swelling agents, glidants or lubricants, flavorings, sweeteners and solubilizers are used. Frequently used auxiliaries which may be mentioned are magnesium carbonate, titanium dioxide, lactose, mannitol and other sugars, talc, lactose, gelatin, starch, cellulose and its derivatives, animal and plant oils such as cod liver oil, sunflower, peanut or sesame oil, polyethylene glycol and solvents such as, for example, sterile water and mono- or polyhydric alcohols such as glycerol.

The dosage regimen utilizing the plasma kallikrein inhibitors is selected in accordance with a variety of factors including type, species, age, weight, sex and medical condition of the patient; the severity of the condition to be treated; the route of administration; the renal and hepatic function of the patient; and the particular compound or salt thereof employed. An ordinarily skilled physician or veterinarian can readily determine and prescribe the effective amount of the drug required to prevent, counter, or arrest the progress of the condition.

Oral dosages of the plasma kallikrein inhibitors, when used for the indicated effects, will range between about 0.01 mg per kg of body weight per day (mg/kg/day) to about 30 mg/kg/day, preferably 0.025-7.5 mg/kg/day, more preferably 0.1-2.5 mg/kg/day, and most preferably 0.1-0.5 mg/kg/day (unless specificed otherwise, amounts of active ingredients are on free base basis). For example, an 80 kg patient would receive between about 0.8 mg/day and 2.4 g/day, preferably 2-600 mg/day, more preferably 8-200 mg/day, and most preferably 8-40 mg/kg/day. A suitably prepared medicament for once a day administration would thus contain between 0.8 mg and 2.4 g, preferably between 2 mg and 600 mg, more preferably between 8 mg and 200 mg, and most preferably 8 mg and 40 mg, e.g., 8 mg, 10 mg, 20 mg and 40 mg. Advantageously, the plasma kallikrein inhibitors may be administered in divided doses of two, three, or four times daily. For administration twice a day, a suitably prepared medicament would contain between 0.4 mg and 4 g, preferably between 1 mg and 300 mg, more preferably between 4 mg and 100 mg, and most preferably 4 mg and 20 mg, e.g., 4 mg, 5 mg, 10 mg and 20 mg.

Intravenously, the patient would receive the active ingredient in quantities sufficient to deliver between 0.025-7.5 mg/kg/day, preferably 0.1-2.5 mg/kg/day, and more preferably 0.1-0.5 mg/kg/day. Such quantities may be administered in a number of suitable ways, e.g. large volumes of low concentrations of active ingredient during one extended period of time or several times a day, low volumes of high concentrations of active ingredient during a short period of time, e.g. once a day. Typically, a conventional intravenous formulation may be prepared which contains a concentration of active ingredient of between about 0.01-1.0 mg/mL, e.g. 0.1 mg/mL, 0.3 mg/mL, and 0.6 mg/mL, and administered in amounts per day of between 0.01 mL/kg patient weight and 10.0 mL/kg patient weight, e.g. 0.1 mL/kg, 0.2 mL/kg, 0.5 mL/kg. In one example, an 80 kg patient, receiving 8 mL twice a day of an intravenous formulation having a concentration of active ingredient of 0.5 mg/mL, receives 8 mg of active ingredient per day. Glucuronic acid, L-lactic acid, acetic acid, citric acid or any pharmaceutically acceptable acid/conjugate base with reasonable buffering capacity in the pH range acceptable for intravenous administration may be used as buffers. The choice of appropriate buffer and pH of a formulation, depending on solubility of the drug to be administered, is readily made by a person having ordinary skill in the art.

Compounds of Formula I can be administered both as a monotherapy and in combination with other therapeutic agents, including but not limited to anti-inflammatory agents, anti-VEGF agents, immunosuppressive agents, anticoagulants, antiplatelet agents, and thrombolytic agents.

An "anti-inflammatory agent" is any agent which is directly or indirectly effective in the reduction of inflammation when administered at a therapeutically effective level. "Anti-inflammatory agent" includes, but is not limited to steroidal anti-inflammatory agents and glucocorticoids. Suitable anti-inflammatory agents include, but are not limited to, cortisone, dexamethasone, hydrocortisone, methylprednisolone, prednisolone, prednisone and triamcinolone.

An "anti-VEGF agent" is any agent which is directly or indirectly effective in inhibiting the activity of VEGF (Vascular Endothelial Growth Factor). Suitable anti-VEGF agents include, but are not limited to, bevacizumab, ranibizumab and aflibercept.

An "immunosuppressant agent" is any agent which is directly or indirectly effective in suppressing, or reducing, the strength of the body's immune system. Suitable immunosuppressant agents include, but are not limited to, corticosteroids (for example, prednisone, budesonide, prednisolone), janus kinase inhibitors (for example, tofacitinib), calcineurin inhibitors (for example, cyclosporin, tacrolimus), mTOR inhibitors (for example, sirolimus, everolimus), IMDH inhibitors (for example, azathioprine, leflunomide, mycophenolate), biologics (for example, abatacept, adalimumab, anakinra, certolizumab, etanercept, golimumab, infliximab, ixekizumab, natalizumab, rituximab, secukinumab, tocilizumab, ustekinumab, vedolizumab), and monoclonal antibodies (for example, basiliximab, daclizumab).

Suitable anticoagulants include, but are not limited to, factor XIa inhibitors, thrombin inhibitors, thrombin receptor antagonists, factor VIIa inhibitors, factor Xa inhibitors, factor IXa inhibitors, factor XIIa inhibitors, adenosine diphosphate antiplatelet agents (e.g., P2Y12 antagonists), fibrinogen receptor antagonists (e.g. to treat or prevent unstable angina or to prevent reocclusion after angioplasty and restenosis), other anticoagulants such as aspirin, and thrombolytic agents such as plasminogen activators or streptokinase to achieve synergistic effects in the treatment of various vascular pathologies. Such anticoagulants include, for example, apixaban, dabigatran, cangrelor, ticagrelor, vorapaxar, clopidogrel, edoxaban, mipomersen, prasugrel, rivaroxaban, and semuloparin. For example, patients suffering from coronary artery disease, and patients subjected to angioplasty procedures, would benefit from coadministration of fibrinogen receptor antagonists and thrombin inhibitors.

In certain embodiments the anti-inflammatory agents, anti-VEGF agents, immunosuppressant agents, anticoagulants, antiplatelet agents, and thrombolytic agents described herein are employed in their conventional dosage ranges and regimens as reported in the art, including, for example, the dosages described in editions of the Physicians' Desk Reference, such as the 70th edition (2016) and earlier editions. In other embodiments, the anti-inflammatory agents, anti-VEGF agents, immunosuppressant agents, anticoagulants, antiplatelet agents, and thrombolytic agents described herein are employed in lower than their conventional dosage ranges.

Alternatively or additionally, one or more additional pharmacologically active agents may be administered in combination with a compound of the invention. The additional active agent (or agents) is intended to mean a pharmaceutically active agent (or agents) that is active in the body, including pro-drugs that convert to pharmaceutically active form after administration, which is different from the compound of the invention, and also includes free-acid, free-base and pharmaceutically acceptable salts of said additional active agents when such forms are sold commercially or are otherwise chemically possible. Generally, any suitable additional active agent or agents, including but not limited to anti-hypertensive agents, additional diuretics, anti-atherosclerotic agents such as a lipid modifying compound, anti-diabetic agents and/or anti-obesity agents may be used in any combination with the compound of the invention in a single dosage formulation (a fixed dose drug combination), or may be administered to the patient in one or more separate dosage formulations which allows for concurrent or sequential administration of the active agents (co-administration of the separate active agents). Examples of additional active agents which may be employed include but are not limited to angiotensin converting enzyme inhibitors (e.g, alacepril, benazepril, captopril, ceronapril, cilazapril, delapril, enalapril, enalaprilat, fosinopril, imidapril, lisinopril, moveltipril, perindopril, quinapril, ramipril, spirapril, temocapril, or trandolapril); angiotensin II receptor antagonists also known as angiotensin receptor blockers or ARBs, which may be in free-base, free-acid, salt or pro-drug form, such as azilsartan, e.g., azilsartan medoxomil potassium (EDARBI^{®}), candesartan, e.g., candesartan cilexetil (ATACAND^{®}), eprosartan, e.g., eprosartan mesylate (TEVETAN^{®}), irbesartan (AVAPRO^{®}), losartan, e.g., losartan potassium (COZAAR^{®}), olmesartan, e.g, olmesartan medoximil (BENICAR^{®}), telmisartan (MICARDIS^{®}), valsartan (DIOVAN^{®}), and any of these drugs used in combination with a thiazide-like diuretic such as hydrochlorothiazide (e.g., HYZAAR^{®}, DIOVAN HCT^{®}, ATACAND HCT^{®}), etc.); potassium sparing diuretics such as amiloride HCl, spironolactone, epleranone, triamterene, each with or without HCTZ; neutral endopeptidase inhibitors (e.g., thiorphan and phosphoramidon); aldosterone antagonists; aldosterone synthase inhibitors; renin inhibitors; enalkrein; RO 42-5892; A 65317; CP 80794; ES 1005; ES 8891; SQ 34017; aliskiren (2(S),4(S),5(S),7(S)-N-(2-carbamoyl-2-methylpropyl)-5-amino-4-hydroxy-2,7-diisopropyl-8-[4-methoxy-3-(3-methoxypropoxy)-phenyl]-octanamid hemifumarate) SPP600, SPP630 and SPP635); endothelin receptor antagonists; vasodilators (e.g. nitroprusside); calcium channel blockers (e.g., amlodipine, nifedipine, verapamil, diltiazem, felodipine, gallopamil, niludipine, nimodipine, nicardipine); potassium channel activators (e.g., nicorandil, pinacidil, cromakalim, minoxidil, aprilkalim, loprazolam); sympatholitics; beta-adrenergic blocking drugs (e.g., acebutolol, atenolol, betaxolol, bisoprolol, carvedilol, metoprolol, metoprolol tartate, nadolol, propranolol, sotalol, timolol); alpha adrenergic blocking drugs (e.g., doxazosin, prazosin or alpha methyldopa); central alpha adrenergic agonists; peripheral vasodilators (e.g. hydralazine); lipid lowering agents, e.g., HMG-CoA reductase inhibitors such as simvastatin and lovastatin which are marketed as ZOCOR^{®} and MEVACOR^{®} in lactone pro-drug form and function as inhibitors after administration, and pharmaceutically acceptable salts of dihydroxy open ring acid HMG-CoA reductase inhibitors such as atorvastatin (particularly the calcium salt sold in LIPITOR^{®}), rosuvastatin (particularly the calcium salt sold in CRESTOR^{®}), pravastatin (particularly the sodium salt sold in PRAVACHOL^{®}), and fluvastatin (particularly the sodium salt sold in LESCOL^{®}); a cholesterol absorption inhibitor such as ezetimibe (ZETIA^{®}), and ezetimibe in combination with any other lipid lowering agents such as the HMG-CoA reductase inhibitors noted above and particularly with simvastatin (VYTORIN^{®}) or with atorvastatin calcium; niacin in immediate-release or controlled release forms, and particularly niacin in combination with a DP antagonist such as laropiprant and/or with an HMG-CoA reductase inhibitor; niacin receptor agonists such as acipimox and acifran, as well as niacin receptor partial agonists; metabolic altering agents including insulin sensitizing agents and related compounds for the treatment of diabetes such as biguanides (e.g., metformin), meglitinides (e.g., repaglinide, nateglinide), sulfonylureas (e.g., chlorpropamide, glimepiride, glipizide, glyburide, tolazamide, tolbutamide), thiazolidinediones also referred to as glitazones (e.g., pioglitazone, rosiglitazone), alpha glucosidase inhibitors (e.g., acarbose, miglitol), dipeptidyl peptidase inhibitors, (e.g., sitagliptin (JANUVIA^{®}), alogliptin, vildagliptin, saxagliptin, linagliptin, dutogliptin, gemigliptin), ergot alkaloids (e.g., bromocriptine), combination medications such as JANUMET^{®} (sitagliptin with metformin), and injectable diabetes medications such as exenatide and pramlintide acetate; inhibitors of glucose uptake, such as sodium-glucose transporter (SGLT) inhibitors and its various isoforms, such as SGLT-1, SGLT-2 (e.g., ASP-1941, TS-071, BI-10773, tofogliflozin, LX-4211, canagliflozin, dapagliflozin, ertugliflozin, ipragliflozin, remogliflozin and sotagliflozin), and SGLT-3; or with other drugs beneficial for the prevention or the treatment of the above-mentioned diseases including but not limited to diazoxide; and including the free-acid, free-base, and pharmaceutically acceptable salt forms, pro-drug forms, e.g., esters, and salts of pro-drugs of the above medicinal agents, where chemically possible. Trademark names of pharmaceutical drugs noted above are provided for exemplification of the marketed form of the active agent(s); such pharmaceutical drugs could be used in a separate dosage form for concurrent or sequential administration with a compound of the invention, or the active agent(s) therein could be used in a fixed dose drug combination including a compound of the invention.

Typical doses of the plasma kallikrein inhibitors of the invention in combination with other suitable agents may be the same as those doses of plasma kallikrein inhibitors administered without coadministration of additional agents, or may be substantially less that those doses of plasma kallikrein inhibitors administered without coadministration of additional agents, depending on a patient's therapeutic needs.

The compounds are administered to a mammal in a therapeutically effective amount. By "therapeutically effective amount" it is meant an amount of a compound of the present invention that, when administered alone or in combination with an additional therapeutic agent to a mammal, is effective to treat (i.e., prevent, inhibit or ameliorate) the disease condition or treat the progression of the disease in a host.

The compounds of the invention are preferably administered alone to a mammal in a therapeutically effective amount. However, the compounds of the invention can also be administered in combination with an additional therapeutic agent, as defined below, to a mammal in a therapeutically effective amount. When administered in a combination, the combination of compounds is preferably, but not necessarily, a synergistic combination. Synergy, as described for example by Chou and Talalay, Adv. Enzyme Regul. 1984, 22, 27-55, occurs when the effect (in this case, inhibition of the desired target) of the compounds when administered in combination is greater than the additive effect of each of the compounds when administered individually as a single agent. In general, a synergistic effect is most clearly demonstrated at suboptimal concentrations of the compounds. Synergy can be in terms of lower cytotoxicity, increased anticoagulant effect, or some other beneficial effect of the combination compared with the individual components.

By "administered in combination" or "combination therapy" it is meant that the compound of the present invention and one or more additional therapeutic agents are administered concurrently to the mammal being treated. When administered in combination each component may be administered at the same time or sequentially in any order at different points in time. Thus, each component may be administered separately but sufficiently closely in time so as to provide the desired therapeutic effect. The administration of each component does not need to be via the same route of administration; for example, one component can be administered orally, and another can be delivered into the vitreous of the eye.

### GENERAL METHODS

Compounds of the present invention may be prepared using conventional techniques or according to the methodology outlined in the following general synthetic schemes. One skilled in the art can vary the procedures and reagents shown to arrive at similar intermediates and/or final compounds.

NMR spectra were measured on VARIAN or Bruker NMR Systems (400, 500 or 600 MHz). Chemical shifts are reported in ppm downfield and up field from tetramethylsilane (TMS) and referenced to either internal TMS or solvent resonances (¹H NMR: δ 7.27 for C*D*Cl₃, δ 2.50 for (CD₃)(C*H*D₂)SO, and ¹³C NMR: δ 77.02 for *C*DCl₃, δ 39.51 for (*C*D₃)₂SO. Coupling constants (*J*) are expressed in hertz (Hz), and spin multiplicities are given as s (singlet), d (doublet), dd (double doublet), t (triplet), m (multiplet), and br (broad). Chiral resolutions were performed on either Waters Thar 80 SFC or Berger MG II preparative SFC systems. LC-MS data were recorded on SHIMADAZU LC-MS-2020, SHIMADAZU LC-MS-2010, or Agilent 1100 series LC-MS, Agilent Prime-1260, or Waters Acquity LC-MS instruments using C18 columns employing a MeCN gradient in water containing 0.02 to 0.1% TFA. UV detections were at 220 and/or 254 nm and ESI ionization was used for MS detection.

When chiral resolution was achieved by chromatography using chiral columns, the chiral columns used for SFC chiral resolutions are listed in tables. Some of the chiral columns used were CHIRALPAK AD, CHIRALCEL OJ, CHIRALPAK AS, CHIRALPAK AY, CHIRALPAK IA, CHIRALPAK AD-H, and CHIRALPAK AS-H. Henceforth, they will be referred by their two or three letter abbreviations. As a convention, the fast-eluting isomer from a chiral resolution is always listed first in this table followed immediately by the slower-eluting isomer from the same resolution. If more than two isomers were separated, they will be always listed in the tables in order they were eluted, such as Peak 1 followed by Peak 2, Peak 3 and so on. A * symbol near a chiral center in a structure denotes that this chiral center was resolved by chiral resolution without its stereochemical configuration unambiguously determined.

Also, TLC is thin layer chromatography; UV is ultraviolet; W is watts; wt. % is percentage by weight; x g is times gravity; α_{D} is the specific rotation of polarized light at 589 nm; °C is degrees Celsius; % w/v is percentage in weight of the former agent relative to the volume of the latter agent; Hz is hertz; cpm is counts per minute; δ_{H} is chemical shift; d is doublet; dd is doublet of doublets; MHz is megahertz; MS is mass spectrum, and a mass spectrum obtained by ES-MS may be denoted herein by "LC-MS"; *m*/*z* is mass to charge ratio; *n* is normal; N is normal; nm is nanometer; nM is nanomolar.

Several catalysts and ligands are used in the following procedures. "Me₄^{t}Bu XPhos Pd G3" is methanesulfonato (2-di-*tert*-butylphosphino-3,4,5,6-tetramethyl-2',4',6'-triisopropyl-1,1-biphenyl)(2'-amino-1,1'-biphenyl-2-yl) palladium(II). "[Ir{dFCF₃ppy}₂(bpy)]PF₆" is bis[2-(2,4-difluorophenyl)-5-trifluoromethylpyridine] [2-2'-bipyridyl] iridium hexafluorophosphate. These catalysts and ligands are available from Millipore Sigma.
For purposes of this specification, the following abbreviations have the indicated meanings:
- Ac: acetyl
- ACN: acetonitrile
- AcOH HOAc: oracetic acid
- aq.: aqueous
- Ar: aryl
- Boc or BOC: *tert*-butoxycarbonyl
- Bpin: bis(pinacolato)diboron
- br: broad
- calcd.: calculated
- CPME: cyclopentylmethyl ether
- d: doublet
- DAST: (diethylamino)sulfur trifluoride
- DBAD: Di-*tert*-butyl azodicarboxylate
- DCE: 1,2-dichloroethane
- DCM: dichloromethane
- dd: doublet of doublets
- DEA: diethanolamine
- DIAD: diisopropyl azodicarboxylate
- DIBAL-H Dibal-H: ordiisobutylaluminum hydride
- DIEA Hünig's base: or*N,N*-diisopropylethylamine
- DMA: 1,2-dimethylacetamide
- DMAP: 4-dimethylaminopyridine
- DMF: dimethylformamide
- DMP: Dess-Martin periodinane (1,1,1-triacetoxy)-1,1-dihydro-1,2-benziodoxol-3(1H)-one
- DMPU: 3,4,5,6-tetrahydro-2(1H)-pyrimidinone
- DMS: dimethylsulfide
- DMSO: dimethyl sulfoxide
- dpm: 2,2,6,6-tetramethylheptane-3,5-dione
- DTT: dithiothreitol
- EDC or EDCI: 1-ethyl-3-(3-dimethylaminopropyl)carbodiimide
- EDTA: ethylenediamine tetraacetic acid
- equiv: equivalents
- ESI: electrospray ionization
- Et: ethyl
- EtOH: ethanol
- EtOAc: ethyl acetate
- g: grams
- GST: glutathione S-transferase
- h: hour
- HATU: N,N,N,N-tetramethyl-O-(7-azabenzotriazol-1-yl)uronium hexafluorophosphate
- HOBt: 1-hydroxybenzotriazole
- HPLC: high-performance liquid chromatography
- IC₅₀: concentration at which 50% inhibition exists
- IPA: isopropanol
- iPr: isopropyl
- *J*: coupling constant
- L: liters
- LAH: lithium aluminum hydride
- LC: liquid chromatography
- LCMS: liquid chromatography mass spectrometry
- LDA: lithium diisopropylamide
- LED: light emitting diode
- LG: leaving group
- M: mass
- M: molar
- m: multiplet
- mCPBA: 3-chloroperoxybenzoic acid
- Me: methyl
- MeOH: methanol
- mg: milligrams
- min: minute
- µL: microliters
- mL: milliliters
- mmol: millimoles
- MPLC: medium pressure liquid chromatography
- Ms: methanesulfonyl (mesyl)
- MTBE: methyl *tert*-butyl ether
- nm: nanometer
- NMP: 1-methylpyrrolidinone
- NMR: nuclear magnetic resonance spectroscopy
- p: pentet
- pH: measure of acidity or basicity of aq. or other solutions
- Ph: phenyl
- pin: pinacol
- PMB: 4-methoxybenzyl
- PMBOH: 4-methoxybenzyl alcohol
- Pr: propyl
- psi: pounds per square inch
- q: quartet
- quint: quintet
- rac: racemic mixture
- RT or rt: room temperature (ambient, about 25 °C)
- s: singlet
- satd.: saturated
- SEM: 2-(trimethylsilyl)ethoxymethyl
- SFC: supercritical fluid chromatography
- S_{N}Ar: nucleophilic aromatic substitution
- t: triplet
- T3P: propylphosphonic anhydride
- TBAF: *tert-*butyl ammonium fluoride
- TBS TBDMS: or *tert-*butyldimethyl silyl
- TBDPS: *tert*-butyldiphenylsilyl
- TBDPSCl: *tert*-butyldiphenylsilyl chloride
- TBSCl: *tert*-butyldimethylsilyl chloride
- tBu: *tert*-butyl
- TEA: triethylamine (Et₃N)
- Tf: triflate
- TFA: trifluoroacetic acid
- TFAA: trifluoroacetic anhydride
- THF: tetrahydrofuran
- TMS: trimethylsilyl
- TMSOTf: trimethylsilyl trifluoromethanesulfonate
- Tris: tris(hydroxymethyl)aminomethane
- UPLC: ultra performance liquid chromatography

### General

Starting materials used were obtained from commercial sources, prepared in other examples, or prepared as known in the literature, unless otherwise noted.

The methods used for the preparation of the compounds of this invention are illustrated by the following schemes.

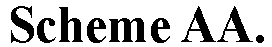

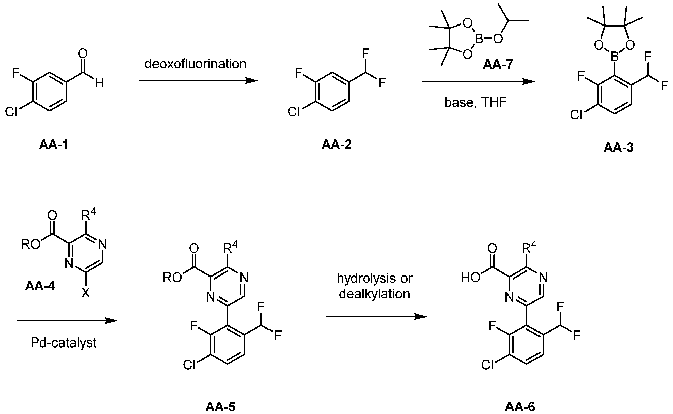

Scheme **AA** illustrates a synthetic sequence for the preparation of bi-heteroaryl derivatives such as **AA-6** from arene **AA-1** and alkyl pyrazine-2-carboxylates such as **AA-4.** Deoxofluorination of aldehyde **AA-1** provides **AA-2.** Borylation of **AA-2** by treatment with a trialkoxyborane such as **AA-7** and a base such as LDA furnishes boronate ester **AA-3.** Palladium-catalyzed cross-coupling of **AA-3** with halide **AA-4** affords **AA-5.** Base-mediated hydrolysis or dealkylation of the ester of **AA-5** by treatment with reagents such as LiOH, NaOH or LiI furnishes carboxylic acid **AA-6** that can be carried on to compounds of this invention.

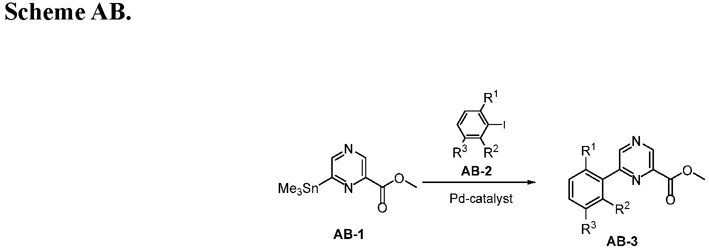

Scheme **AB** illustrates the synthetic sequence for preparation of ester derivatives such as **AB-3** from a stannane **AB-1.** Pd-catalyzed Stille coupling. of **AB-1** with aryl iodide **AB-2** gives the biaryl ester derivative **AB-3** that can be carried on to compounds of this invention.

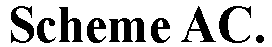

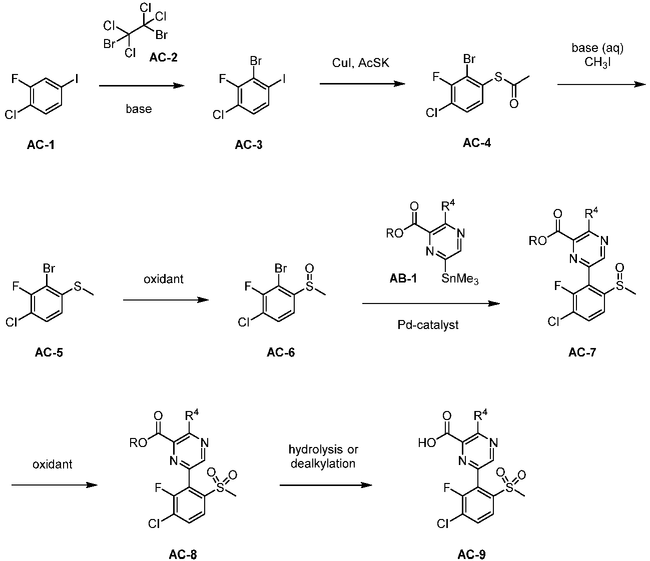

Scheme **AC** illustrates a synthetic sequence for the preparation of bi-heteroaryl derivatives such as **AC-9** from arene **AC-1** and pyrazine **AB-1.** Bromination of **AC-1** by treament with a base such as LDA and a brominating agent such as 1,2-dibromo-1,1,2,2-tetrachloroethane **AC-2** affords **AC-3.** Copper-catalyzed cross-coupling of **AC-3** with potassium thioacetate gives thioester **AC-4.** Treatment with an aqueous base such as NaOH and an alkylation agent such as methyliodide converts thioester **AC-4** to thioether **AC-5,** which is subsequently oxidized to sulfoxide **AC-6** by use of an oxidant such as mCPBA. Palladium-catalyzed cross-coupling of bromide **AC-6** with stannane **AB-1** affords **AC-7.** Oxidation of the suloxide of **AC-7** using an oxidant such as oxone^{®} gives sulfone **AC-8,** and base-mediated hydrolysis or dealkylation of the ester of **AC-8** furnishes carboxylic acid **AC-9** that can be carried on to compounds of this invention.

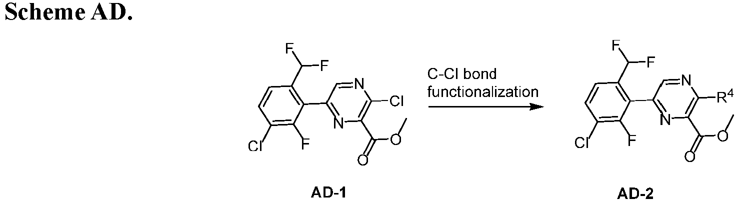

Scheme **AD** illustrates the synthetic sequence for preparation of biaryl derivatives such as **AD-2** by C-Cl functionalization of the aryl chloride **AD-1.**

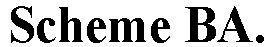

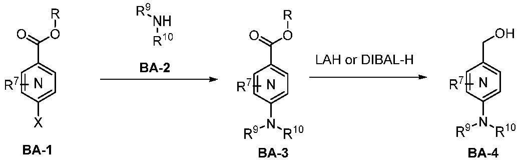

Scheme **BA** illustrates the synthetic sequence for preparation of primary alcohols such as **BA-4.** S_{N}Ar reaction of **BA-1** with aliphatic amines **BA-2** gives anilines **BA-3.** The subsequent reduction of ester group in **BA-3** using LAH or DIBAL-H, for example, gives the alcohols **BA-4** that can be carried on to compounds of this invention.

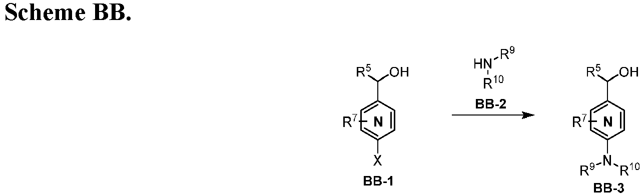

Scheme **BB** illustrates the synthetic sequence for preparation of alcohol derivatives such as **BB-3.** S_{N}Ar reaction of aryl halides **BB-1** with aliphatic amines **BB-2** gives the anilines **BB-3** that can be carried on to compounds of this invention.

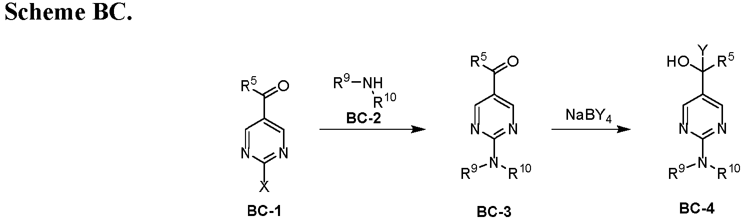

Scheme **BC** illustrates the synthetic sequence for preparation of alcohols such as **BC-4** from ketones such as **BC-1** by S_{N}Ar of aryl halides such as **BC-2,** as described above, followed by reduction of ketones, such as **BC-3,** to afford alcohols **(BC-4)** that can be carried on to compounds of this invention.

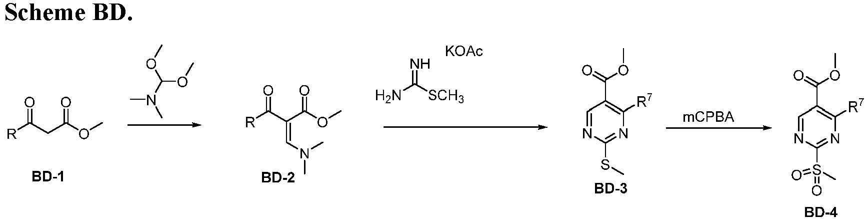

Scheme **BD** illustrates the synthetic sequence for preparation of sulfone **BD-4** from condensation between 1,3-dicarbonyl derivatives **(BD-1)** and N,N-dimethylformamide dimethyl acetal, followed by cyclization with thiopseudourea to afford a 2-mercaptopyrimidine **(BD-3)** and oxidation to afford sulfones **(BD-4)** that can be carried on to compounds of this invention.

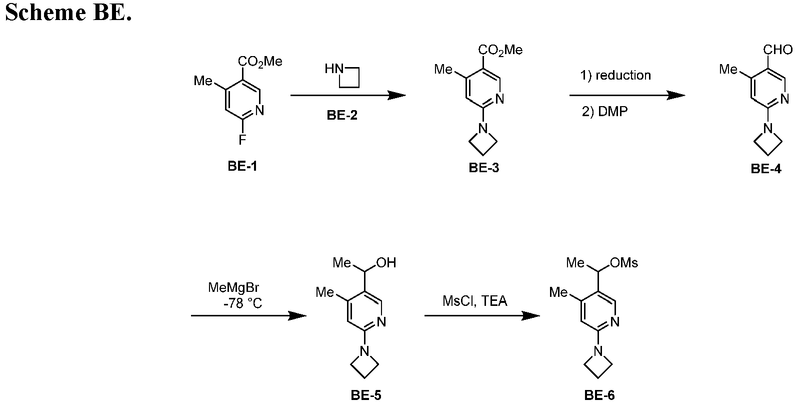

Scheme **BE** illustrates the synthetic sequence for preparation of mesylate **BE-6** from ester **BE-1.** S_{N}Ar of **BE-1** with azetidine gives **BE-2** affords an ester **(BE-3)** which can undergo reduction, followed by oxidation to give an aldehyde **(BE-4).** Grignard addition to **BE-4** to afford a secondary alcohol **BE-5,** followed by mesylation can yield intermediate **BE-6** that can be carried on to compounds of this invention.

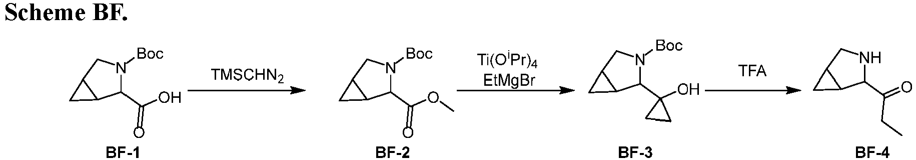

Scheme **BF** illustrates the synthetic sequence for preparation of ketone **BF-4** from the acid derivative **BF-1.** Esterification of **BF-1,** followed by cyclopropanation can afford alcohol **BF-3,** which can undergo dual Boc-deprotection/rearrangement under acidic conditions to give ketone **BF-4** that can be carried on to compounds of this invention.

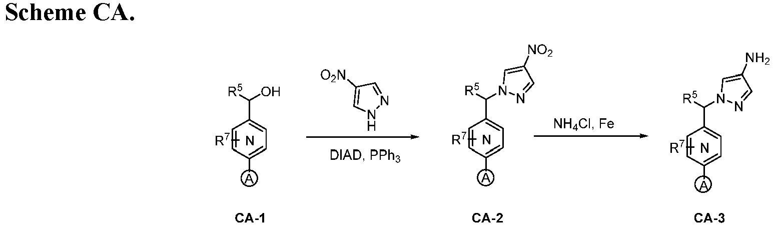

Scheme **CA** illustrates the synthetic sequence for preparation of 4-aminopyrazole derivatives such as **CA-3** from alcohols such as **CA-1.** Mitsunobu reaction of 4-nitropyrazole with alcohols **CA-2** followed by reduction of nitro group **(CA-2)** can afford the aminopyrazole derivatives **CA-3.**

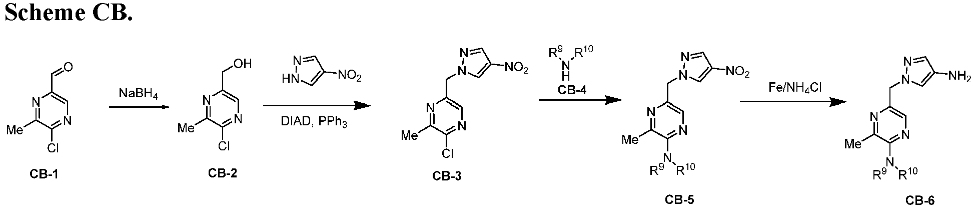

Scheme **CB** illustrates the synthetic sequence for preparation of aminopyrazole derivative **CB-6** from aldehyde **CB-1.** Reduction of **CB-1,** followed by Mitsunobu displacement of the alcohol moiety with nitropyrazole can give chloropyrazine **CB-3.** S_{N}Ar of **CB-3** with an amine **(CB-4),** followed by reduction of nitro group in **CB-5** can yield an aminopyrazole **CB-6** that can be carried on to compounds of this invention.

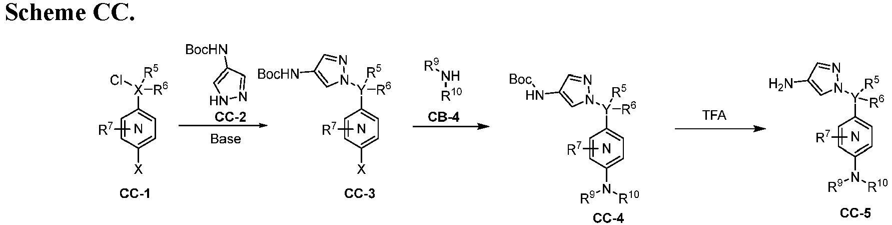

Scheme **CC** illustrates the synthetic sequence for preparation of aminopyrazole derivatives such as **CC-5** from compounds such as **CC-1.** Nucleophilic displacement of Cl from **CC-1** with Boc-protected aminopyrazole **CC-2** can yield an aryl halide **(CC-3),** which can undergo S_{N}Ar reaction with aliphatic amines **CB-4** to give anilines **CC-4.** Boc-deprotection under acidic conditions can give the free aminopyrazole **CC-5** that can be carried on to compounds of this invention.

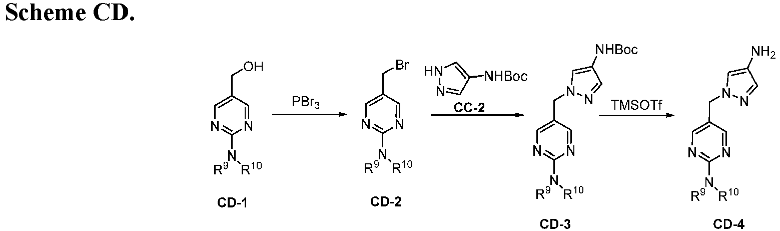

Scheme **CD** illustrates the synthetic sequence for preparation of amino pyrazole derivative **CD-4** from alcohol such as **CD-1.** Bromine displacment of the alcohol moiety from **CD-1,** followed by substitution with Boc-amino pyrazole **(CD-2)** and deprotection **(CD-3)** gives amino pyrazole **CD-4** that can be carried on to compounds of this invention.

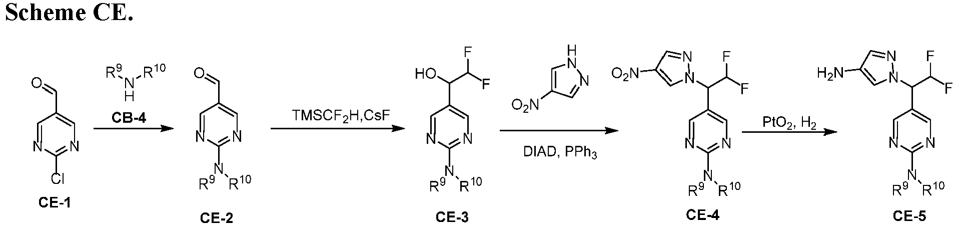

Scheme **CE** illustrates the synthetic sequence for preparation of aminopyrazole derivative such as **CE-5** from aldehyde **CE-1.** S_{N}Ar with an amine **(CB-4),** followed by reaction with a difluoromethyl nucleophile to the aldehyde moiety can give alcohol **CE-3.** Mitsunobu reaction with nitropyrazole, followed by nitro group reduction as described previously can afford an aminopyrazole derivative **CE-5** that can be carried on to compounds of this invention.

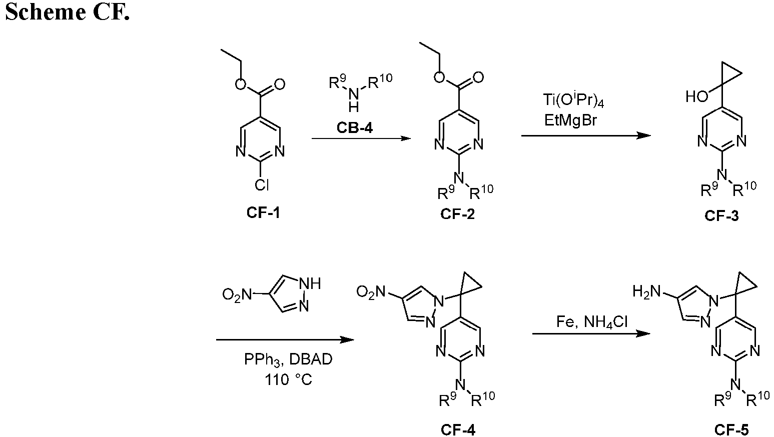

Scheme CF illustrates the synthetic sequence for preparation of aminopyrazole **CF-5** from ester **CF-1.** S_{N}Ar reaction of an amine **(CB-4)** with **CF-1,** followed by titanium-mediated cylcopropanation can yield alcohol **CF-3.** Mitsunobu reaction at elevated reaction temperature, and subsequent reduction of the nitro group, as previously described, can afford the aminopyrazole derivative **CF-5** that can be carried on to compounds of this invention.

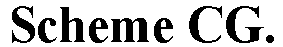

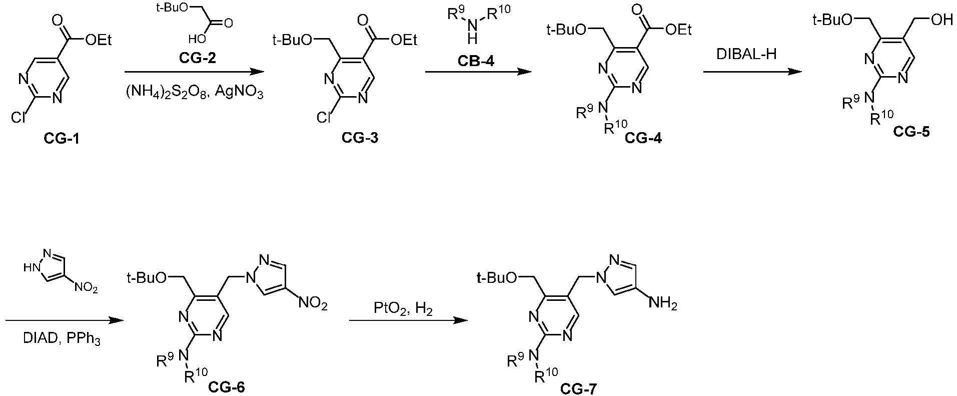

Scheme **CG** illustrates the synthetic sequence for preparation of an aminopyrazole **CG-6** from pyrimidine ester **CG-1.** Minisci reaction of **CG-1** with an alkoxyacetic acid **(CG-2)** can give an ether derivative **CG-3.** S_{N}Ar reaction with an amine **(CB-4),** followed by reduction of the ester moiety can yield a benzylic alcohol **CG-5.** Mitsunobu reaction, followed by nitro group reduction, as described above, can give an aminopyrazole **CG-7** that can be carried on to compounds of this invention.

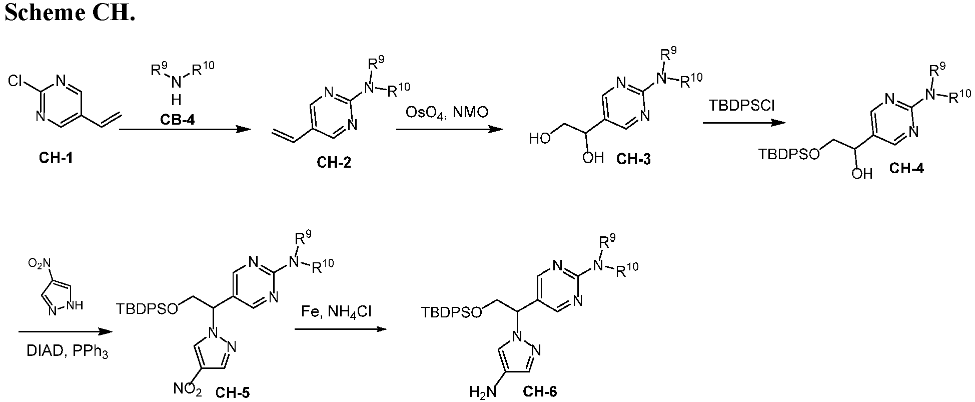

Scheme **CH** illustrates the synthetic sequence for preparation of an aminopyrazole **CH-7** from olefin **CH-1.** S_{N}Ar reaction of **CH-1** with an amine **(CB-4)** can give aminopyrimidine **CH-2,** which can undergo dihydroxylation to yield afford a diol such as **CH-3.** Selective protection of the primary alcohol gives CH-4, which enables Mitsunobu reaction of nitropyrazole at the secondary hydroxyl group to afford adduct **CH-5.** Reduction can yield an aminopyrazole **(CH-6)** that can be carried on to compounds of this invention.

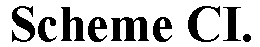

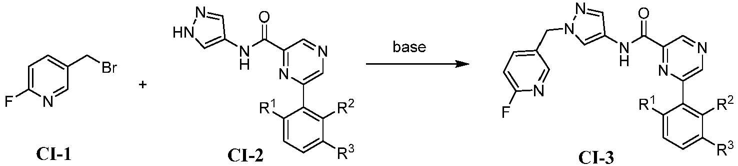

Scheme **CI** illustrates the synthetic sequence for preparation of tetracyclic intermediate **CI-3** by reaction of benzylic bromide **CI-1** with an aminopyrazole nucleophile **CI-2** in the presence of a suitable base, such as Cs₂CO₃ or the like. The reaction is typically performed at room temperature, but can require elevated reaction temperatures to produce **CI-3,** which can be carried on to compounds of this invention.

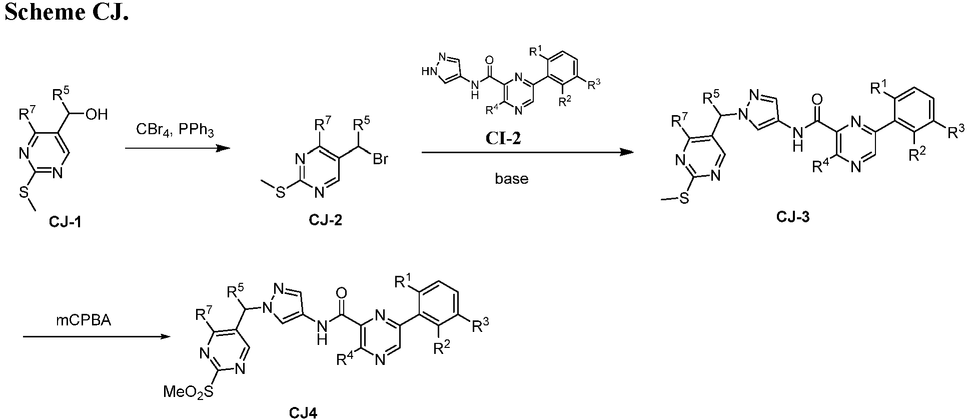

Scheme **CJ** illustrates the synthetic sequence for preparation of the sulfone derivatives such as **CJ-4** from benzylic alcohols such as **CJ-1.** Bromination of **CJ-1,** followed by reaction with an aminopyrazole **(CI-2)** in the presence of a suitable base, such as Cs₂CO₃ or the like, can afford a sulfide intermediate **(CJ-3)** that can undergo oxidation to sulfones **(CJ-4)** that can be carried on to compounds of this invention.

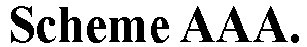

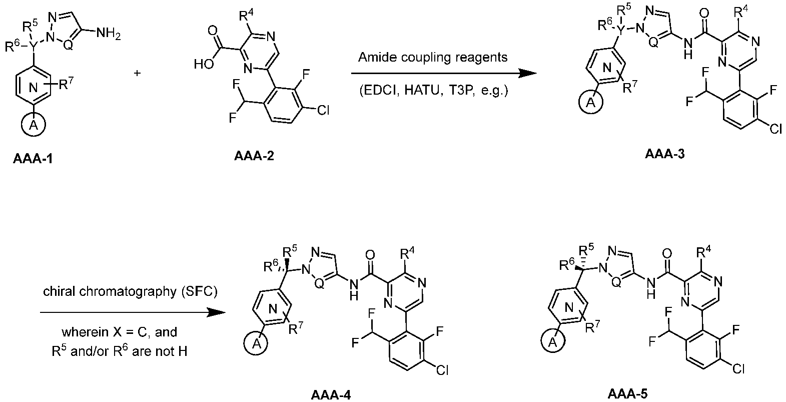

Scheme **AAA** illustrates the synthetic sequence for preparing amide compounds of this invention, such as **AAA-3** employing amide couplings between amino pyrazole derivatives such as **AAA-1** and carboxylic acid derivatives such as **AAA-2.** When compounds **AAA-3** exist as mixtures of stereoisomers, specifically as racemic mixture, such compounds can be separated by chiral chromatography, preferably SFC, when appropriate (wherein Y=C, and R⁵ and/or R⁶ are not H) to yield individual stereoisomers **AAA-4** and **AAA-5.**

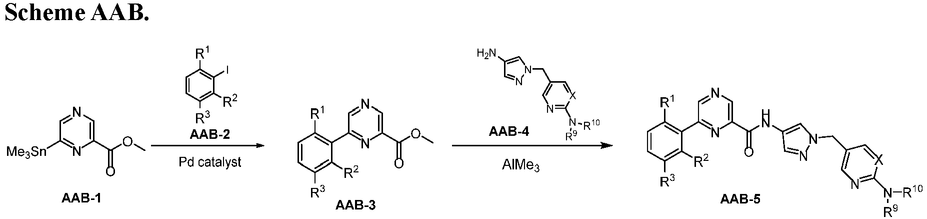

Scheme **AAB** illustrates a synthetic sequence for preparing amide compounds of this invention, such as **AAA-5.** The sequence is initiated from cross-coupling of a stanylated heteroaryl ester (**AAB-1**) with an aryl iodide partner **(AAB-2)** using a suitable palladium catalyst and ligand. The biaryl product **(AAB-3)** can be hydrolyzed to the corresponding carboxylic acid and coupled as described in Scheme **AAA** (not shown) or alternatively reacted in the presence of an amine donor like aminopyrazole **AAB-4** and a carboxylate activating reagent, such as trimethylaluminum, to afford compounds of this invention **(AAB-5).**

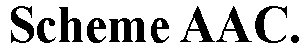

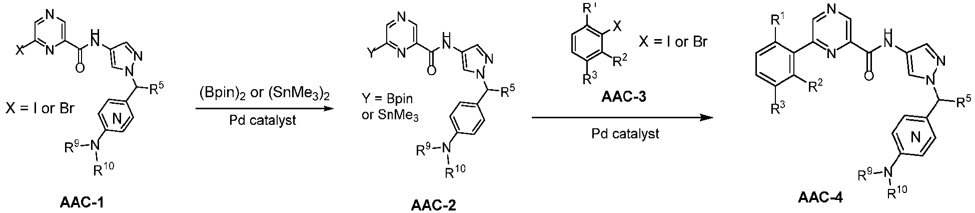

Scheme **AAC** illustrates the synthetic sequence for an alternate preparation of biaryl compounds of this invention **(AAC-4)** from tricyclic heteroaryl halides **(AAC-1)** and aryl halide **(AAC-3)** inputs. Initial palladium catalyzed borylation or stannylation of **AAC-1** yields an activated coupling partner **(AAC-2)** for **AAC-3,** which can be cross-coupled with a subsequent palladium catalyzed coupling reaction to give the desired biaryl compounds of this invention **(AAC-4).**

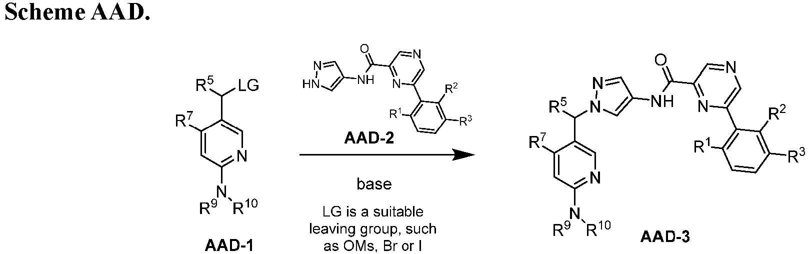

Scheme **AAD** illustrates an alternate synthetic sequence for preparing compounds of this invention **(AAD-3).** Alkylation of activated intermediate **AAD-1** with a suitable heteroaryl nucleophile, such as **AAD-2,** in the presence of a base (Cs₂CO₃, or the like), can afford the desired compounds of this invention **AAD-3.** It should be noted that the alkylation can require elevated reaction temperatures to enable complete reaction of **AAD-1** and **AAD-2.**

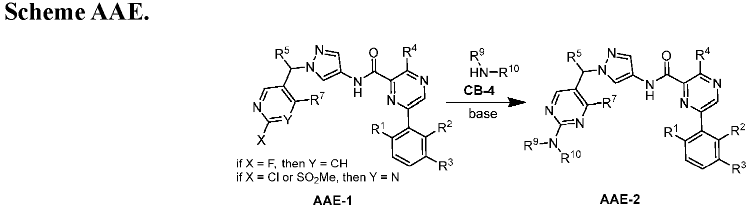

Scheme **AAE** illustrates a synthetic sequence for preparing amide compounds of this invention **AAB-2.** S_{N}Ar reaction of an activated heterocyclic intermediate **(AAE-1)** with a suitable amine nucleophile **(CB-4),** under reaction conditions that can require elevated temperatures to facilitate completion, can afford **AAE-2.**

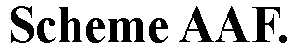

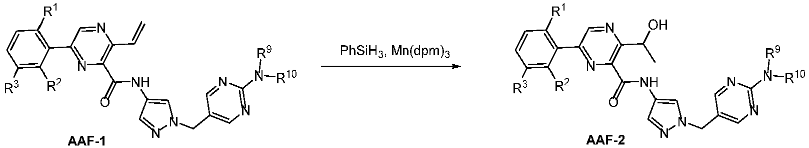

Scheme **AAF** illustrates the synthetic sequence for preparing secondary alcohols **AAF-2** from Mn-catalyzed hydration of the olefin **AAF-1.**

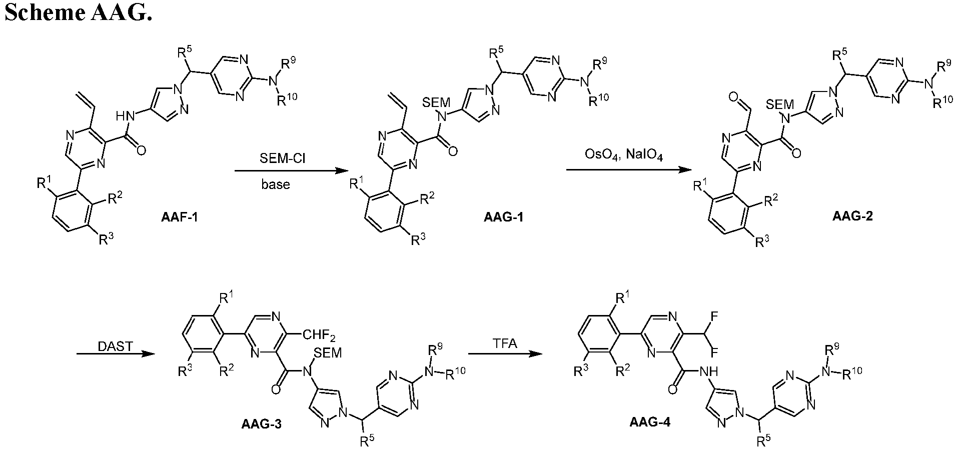

Scheme **AAG** illustrates an alternate synthetic sequence for modifying olefin intermediate **AAF-1** to afford compounds of this invention. Amide protection can yield a SEM-protected intermediate **(AAG-1)** that can be reacted under oxidizing conditions to an aldehyde intermediate **(AAG-2).** Difluorination to **AAG-3,** followed by SEM-deprotection under acidic conditions can afford **AAG-4.**

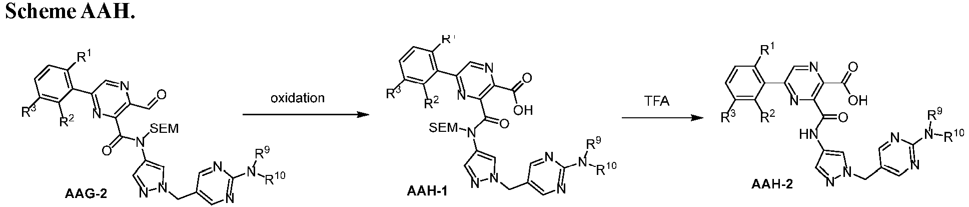

Scheme **AAH** illustrates an alternate synthetic sequence for modifying aldehyde intermediate **AAG-2** to compounds of this invention. Oxidation of the aldehyde to the corresponding carboxylic acid moiety **(AAH-1)** can be performed using a variety of mild reaction conditions, an example of which is a Pinnick oxidation involving sodium chlorite as oxidant. SEM-deprotection as described above, can afford the desired carboxylic acid product **AAH-2.**

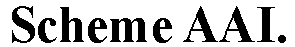

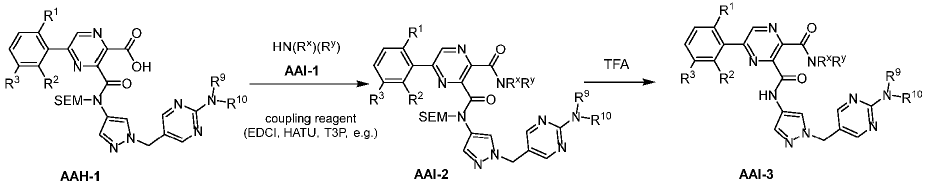

Scheme **AAI** illustrates the synthetic sequence for preparing amide derivatives **(AAI-3)** from carboxylic acid intermediate **AAH-1.** Reaction of carboxylic acid **AAH-1** with an amine **(AAI-1)** under reaction conditions previously described can afford SEM-protected pyrazinyl carboxamides **(AAI-2)** that can undergo SEM-deprotection as described above to afford carboxamides **(AAI-3)** of this invention.

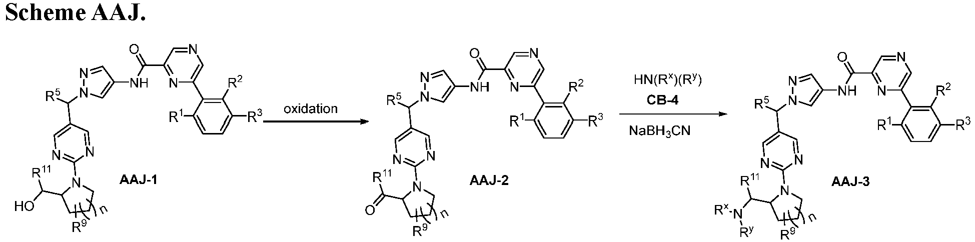

Scheme **AAJ** illustrates a synthetic sequence for preparing of amine derivatives **AAJ-3** as compounds of this invention. Oxidation of an alcohol such as **AAJ-1** which can be performed using a variety of reagents, including DMP or chromium reagents, such as PDC, or under conditions of the Swern oxdation to afford a ketone or aldehyde intermediate **(AAJ-2).** Reductive amination in the presence of an amine **(CB-4)** and suitable reducing agent, such as NaBH₃CN, NaHB(OAc)₃ or the like, can afford compounds of this invention.

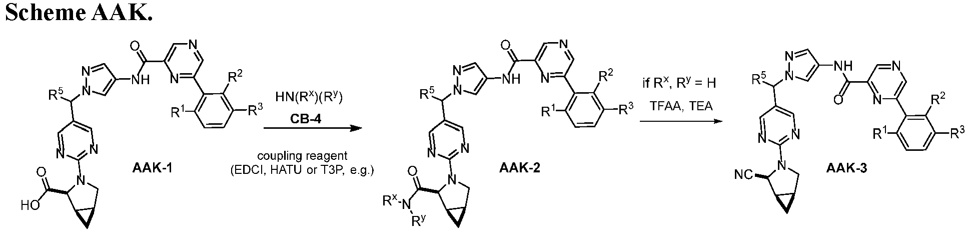

Scheme **AAK** illustrates a synthetic sequence that can be used to prepare amide compounds (**AAK-2**) or nitrile compounds **(AAK-3)** of this invention from a common carboxylic acid intermediate, **AAK-1.** Reaction of carboxylic acid **AAK-1** with an amine **(CB-4)** in the presence of a suitable coupling agent, such as EDCI, HATU, T3P or the like, can afford amide compounds of this invention. In the case wherein R^{x} and R^{y} = H, the primary amide represented by **AAK-2** can be submitted to dehydration conditions to yield a nitrile compound **(AAK-3)** of this invention.

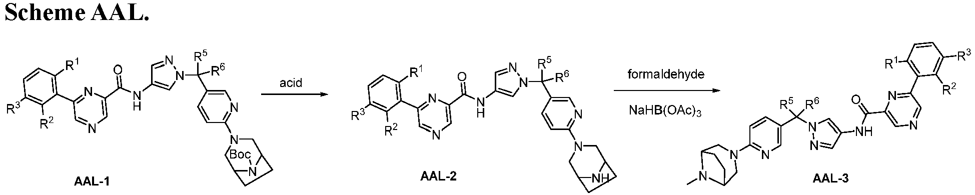

Scheme **AAL** illustrates a synthetic sequence for preparing tertiary amine compounds **(AAL-3)** of this invention. Deprotection of **AAL-1** in the presence of strong acid, such as TFA, HCl or the like, can give a secondary amine **(AAL-2)** that can be further reacted with formaldehyde in the presence of a suitable reducing reagent as described above to give a tertiary amine compound (**AAL-3**) of this invention.

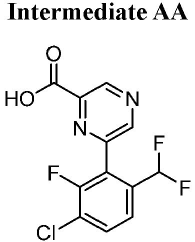

### 6-(3-Chloro-6-(difluoromethyl)-2-fluorophenyl)pyrazine-2-carboxylic acid (Scheme AA)

Step 1. 1-Chloro-4-(difluoromethyl)-2-fluorobenzene: To a solution of 4-chloro-3-fluorobenzaldehyde (300 g, 1.89 mmol) in DCM (1.5 L) under a nitrogen atmosphere, cooled to 0 °C was added bis(2-methoxyethyl)aminosulfur trifluoride (456 mL, 2.08 mol) drop-wise into the mixture. The reaction mixture was stirred at rt for 12 h. The resulting mixture was poured slowly into an ice-water solution, then extracted with DCM. The organic layers were combined and washed with brine, dried over Na₂SO₄ and concentrated under reduced pressure to afford the title compound. ¹H NMR (400 MHz, CDCl₃) δ 7.53 (t, *J* = 8.0 Hz, 1H), 7.34 (d, *J* = 9.6 Hz, 1H), 7.27 (d, *J=* 9.2 Hz, 1H), 6.64 (t, *J* = 56 Hz, 1H).

Step 2. 2-(3-Chloro-6-(difluoromethyl)-2-fluorophenyl)-4,4,5,5-tetramethyl-1,3,2-dioxaborolane: To a solution of 1-chloro-4-(difluoromethyl)-2-fluorobenzene (300 g, 1.66 mol) in THF (1.8 L) under a nitrogen atmosphere, cooled to -78 °C was added LDA (997 mL, 1.99 mol, 2M in THF) drop-wise into the mixture. The reaction mixture was stirred at -78 °C for 30 min, then 2-isopropoxy-4,4,5,5-tetramethyl-1,3,2-dioxaborolane (508 mL, 2.49 mol) was added dropwise into the mixture. The reaction mixture was stirred at -78 °C for 30 min, then poured into a satd. aq. NH₄Cl solution. The resulting mixture was filtered, then extracted with EtOAc. The organic layers were combined and washed with brine, dried over Na₂SO₄ and concentrated under reduced pressure to afford the title compound. ¹H NMR (400 MHz, CDCl₃) δ 7.53 (t, *J* = 8.0 Hz, 1H), 7.37 (d, *J* = 8.4 Hz, 1H), 7.02 (t, *J* = 56 Hz, 1H), 1.39 (s , 12H).

### Step 3. Methyl 6-(3-chloro-6-(difluoromethyl)-2-fluorophenyl)pyrazine-2-carboxylate:

The reaction was conducted in two parallel vessels on the equivalent scale as specified. To a solution of 2-(3-chloro-6-(difluoromethyl)-2-fluorophenyl)-4,4,5,5-tetramethyl-1,3,2-dioxaborolane (127 g, 415 mmol), methyl 6-bromopyrazine-2-carboxylate (60.0 g, 276 mmol) and potassium fluoride (48.2 g, 829 mmol, 19.4 mL) in a mixture of dioxane (720 mL) and water (120 mL) under a nitrogen atmosphere was added 1,1'-bis(di-*tert*-butylphosphino)ferrocene palladium dichloride (11.3 g, 13.8 mmol). The reaction mixture was stirred at 120 °C for 12 h. The reaction mixtures from both reactions conducted in parallel were cooled to rt, combined, then concentrated under reduced pressure. The crude residue was suspended in EtOAc and the resulting mixture was filtered. The filtrate was washed with water, brine, dried over Na₂SO₄ and concentrated under reduced pressure to afford the title compound. ¹H NMR (400 MHz, CDCl₃) δ 9.36 (s, 1H), 8.96 (d, *J=* 2.8 Hz, 1H), 7.65 (t, *J* = 8.4 Hz, 1H), 8.56 (d, *J=* 8.4 Hz, 1H), 6.99 (t, *J* = 56 Hz, 1H), 4.06 (s, 3H).

Step 4. 6-(3-Chloro-6-(difluoromethyl)-2-fluorophenyl)pyrazine-2-carboxylic acid: To a solution of methyl 6-(3-chloro-6-(difluoromethyl)-2-fluorophenyl)pyrazine-2-carboxylate (350 g, 1.11 mol) in EtOH (1.75 L) at 10-20 °C was added 1M NaOH (2.10 L, 2.10 mol). The reaction mixture was stirred at rt for 30 min, then concentrated to afford a crude residue that was extracted with MTBE. The organic layers were discarded, and the pH of the aqueous layer was adjusted to 2-3 by addition of 2M HCl. The resulting mixture was filtered, and the filter cake was dried to afford the title compound. ¹H NMR (400 MHz, DMSO-*d*₆) δ 13.9 (br s, 1H), 9.28 (s, 1H), 9.14 (s, 1H), 7.98 (t, *J=* 8.0 Hz, 1H), 7.70 (d, *J* = 8.4 Hz, 1H), 7.02 (t, *J* = 56 Hz, 1H). MS = 302.9 [M + 1]⁺.

**Table AA. The following compounds were prepared using procedures similar to those described above using the appropriate starting materials.**

| **Intermediate No.** | **Structure** | **Name** | **[M+H]⁺ or [M+Na]⁺** |
|---|---|---|---|
| AA-1 | 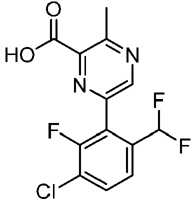 | 6-(3-Chloro-6-(difluoromethyl)-2-fluorophenyl)-3-methylpyrazine-2-carboxylic acid | Calcd.: 317.0 |
| | | | Found: 316.9 |
| AA-2 | 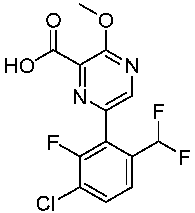 | 6-(3-Chloro-6-(difluoromethyl)-2-fluorophenyl)-3-methoxypyrazine-2-carboxylic acid | Calcd.: 333.0 |
| | | | Found: 333.1 |
| AA-3 | 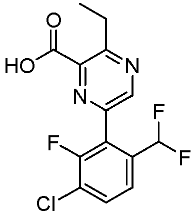 | 6-(3-Chloro-6-(difluoromethyl)-2-fluorophenyl)-3-ethylpyrazine-2-carboxylic acid | Calcd.: 331.0 |
| | | | Found: 331.0 |
| AA-4 | 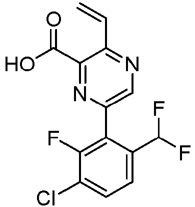 | 6-(3-Chloro-6-(difluoromethyl)-2-fluorophenyl)-3-vinylpyrazine-2-carboxylic acid | Calcd.: 329.0 |
| | | | Found: 328.9 |
| AA-5 | 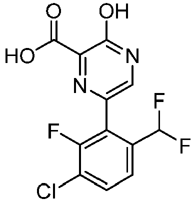 | 6-(3-Chloro-6-(difluoromethyl)-2-fluorophenyl)-3-hydroxypyrazine-2-carboxylic acid | Calcd.: 319.0 |
| | | | Found: 319.1 |
| AA-6 | 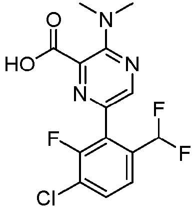 | 6-(3-Chloro-6-(difluoromethyl)-2-fluorophenyl)-3-(dimethylamino)pyrazine-2-carboxylic acid | Calcd.: 346.0 |
| | | | Found: 346.2 |
| AA-7 | 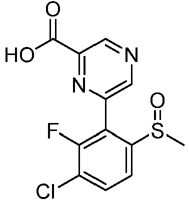 | 6-(3-Chloro-2-fluoro-6-(methylsulfinyl)phenyl)pyrazine -2-carboxylic acid | Calcd.: 315.0 |
| | | | Found: 315.2 |
| AA-8 | 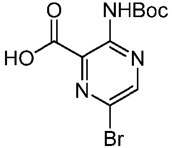 | 6-Bromo-3-((tert-butoxycarbonyl)amino)pyrazine -2-carboxylic acid | Calcd.: 318.0 |
| | | | Found: 318.2 |
| AA-9 | 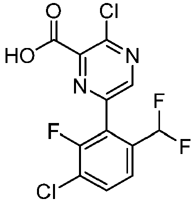 | 3-Chloro-6-(3-chloro-6-(difluoromethyl)-2-fluorophenyl)pyrazine-2-carboxylic acid | Calcd. [M-18]⁺: 319.0 |
| | | | Found: 319.0 |

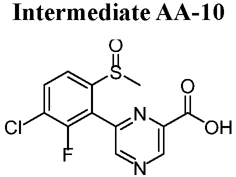

### Methyl 6-(3-chloro-2-fluoro-6-(methylsulfinyl)phenyl)pyrazine-2-carboxylate (Scheme AD)

Step 1: 2-Bromo-4-chloro-3-fluoro-1-iodobenzene: To a mixture of 1-chloro-2-fluoro-4-iodobenzene (5.00 g, 19.5 mmol) in THF (50 mL) was added LDA (10.7 mL, 21.4 mmol, 2M in THF) dropwise at -70 °C under N₂, and the mixture was stirred at -70 °C for 0.5 h. Then 1,2-dibromo-1,1,2,2-tetrachloroethane (7.62 g, 23.4 mmol) in THF (10 mL) was added dropwise, and the mixture was stirred at -70 °C for 20 min, then warmed to 10 °C for another 1 h. The reaction was quenched by the addition of satd. aq. NH₄Cl and extracted with EtOAc. The combined organic layers were dried over Na₂SO₄, filtered and concentrated to give crude product, which was purified by silica gel chromatography (petroleum ether) to give the title compound. ¹H NMR (400 MHz, CDCl₃) δ 7.52 - 7.65 (m, 1H), 7.00 - 7.13 (m, 1H).

Step 2: S-(2-Bromo-4-chloro-3-fluorophenyl) ethanethioate: To a solution of 2-bromo-4-chloro-3-fluoro-1-iodobenzene (3.00 g, 8.95 mmol) in toluene (30 mL) was added potassium thioacetate (1.53 g, 13.4 mmol), copper(I) iodide (0.170 g, 0.895 mmol) and 1,10-phenanthroline (161 mg, 0.895 mmol), and the resulting mixture was heated to 90 °C for 16 h. The reaction was cooled to rt, filtered, diluted with water and extracted with EtOAc. The combined organic layers were washed with brine, dried over Na₂SO₄, filtered and concentrated to give a crude residue that was purified by silica gel chromatography (EtOAc/petroleum ether) to give the title compound. ¹H NMR (400 MHz, CDCl₃) δ 7.38-7.45 (m, 1H), 7.27-7.33 (m, 1H), 2.48 (d, *J* = 1.2 Hz, 3H).

Step 3: 2-Bromo-4-chloro-3-fluoro-1-(methylsulfinyl)benzene: To a solution of *S*-(2-bromo-4-chloro-3-fluorophenyl) ethanethioate (1.30 g, 4.58 mmol) in EtOH (10 mL) was added NaOH (0.550 g, 13.8 mmol) (dissolved into 2.5 mL of water) and iodomethane (0.781 g, 5.50 mmol), and the resulting mixture was stirred at 15 °C for 10 min. The reaction was concentrated, diluted with water, and extracted with EtOAc. The combined organic layers were washed with brine, dried over Na₂SO₄ and concentrated to give a crude product that was immediately dissolved in DCM (10 mL). mCPBA (1.58 g, 9.17 mmol) was added, and the resulting mixture was stirred at 15 °C for 1 h. The reaction mixture was quenched by the addition of satd. aq. Na₂SO₃ and allowed to stir for 30 min. The mixture was filtered and extracted with DCM. The organic layer was separated, dried over Na₂SO₄, filtered and concentrated to give a crude residue that was purified by silica gel chromatography (EtOAc/petroleum ether) to give the title compound. ¹H NMR (400 MHz, CDCl₃) δ 7.70 (s, 1H), 7.24 (s, 1H), 2.81 (d, *J* = 1.5 Hz, 3H).

Step 4: Methyl 6-(3-chloro-2-fluoro-6-(methylsulfinyl)phenyl)pyrazine-2-carboxylate: To a solution of 2-bromo-4-chloro-3-fluoro-1-(methylsulfinyl)benzene (200 mg, 0.737 mmol) in 1,4-dioxane (3 mL) were added methyl 6-(trimethylstannyl)pyrazine-2-carboxylate (288 mg, 0.958 mmol), tris(dibenzylideneacetone)dipalladium(0) (67 mg, 0.074 mmol) and 1,1'-bis(di-*tert*-butylphosphino)ferrocene (35 mg, 0.074 mmol), and the resulting mixture was heated to 100 °C for 15 h. The reaction was cooled to rt, filtered, and concentrated to give crude product that purified by prep-TLC (1:1 EtOAc/petroleum ether) to give the title compound. MS = 329.2 (M+H).

Step 5: 6-(3-Chloro-2-fluoro-6-(methylsulfinyl)phenyl)pyrazine-2-carboxylic acid: To a solution of methyl 6-(3-chloro-2-fluoro-6-(methylsulfinyl)phenyl)pyrazine-2-carboxylate (180 mg, 0.548 mmol) in THF (0.5 mL) and water (0.5 mL) was added lithium hydroxide (39 mg, 1.64 mmol), and the mixture was stirred at 20 °C for 1 h. The reaction was diluted with water and extracted with DCM. The aq. layer was adjusted to pH = 3 with 2M HCl then concentrated to give the crude title compound that was carried on without further purification. MS = 315.2 (M+H).

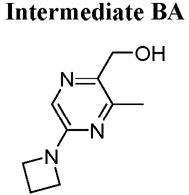

### (5-(azetidin-1-yl)-3-methylpyrazin-2-yl)methanol (Scheme BA)

Step 1. (Methyl 5-(azetidin-1-yl)-3-methylpyrazine-2-carboxylate: Methyl 5-bromo-3-methylpyrazine-2-carboxylate (1.0 g, 4.33 mmol) was added to a vial with NMP (10.82 ml) at room temperature. Azetidine (1.0 g, 17.3 mmol) was added and the reaction mixture was stirred at 100 °C for 4 h. The solution was cooled to room temperature before aq NaHCO₃ was added. The resulting mixture was extracted with DCM, and the combined organic fractions were dried (MgSO₄) and concentrated in vacuo to provide the title compound. MS = 208.1 [M + 1]⁺.

Step 2. (5-(azetidin-1-yl)-3-methylpyrazin-2-yl)methanol: The solution of methyl 5-(azetidin-1-yl)-3-methyl-pyrazine-2-carboxylate (767 mg, 3.7 mmol) in DCM (15 ml) was cooled to -78 °. DIBAL-H (11.1 ml, 11.1 mmol, 1M in THF) was added dropwise and the reaction was stirred at -78C for 1 h before it was warmed to 0 °C and stirred for 1 h at 0 °C. The reaction was quenched with aq NaOH and MgSO₄ was added. The resulting suspension was filtered, and the filtrate was concentrated. The residue was purified by silica gel column chromatography eluting with DCM/MeOH to provide the title product. MS = 180.0 [M + 1]⁺.

**Table BA. The following compounds were prepared using procedures similar to those described above using the appropriate starting materials.**

| **Intermediate No.** | **Structure** | **Name** | **[M+H]⁺ or [M+Na]⁺** |
|---|---|---|---|
| BA-1 | 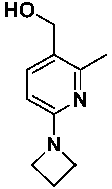 | (6-(azetidin-1-yl)-2-methylpyridin-3-yl)methanol | Calcd.: 179.1 |
| | | | Found: 179.1 |
| BA-2 (GSN) | 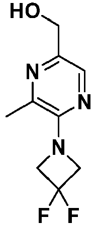 | (5-(3,3-difluoroazetidin-1-yl)-6-methylpyrazin-2-yl)methanol | Calcd.: 216.1 |
| | | | Found:216.0 |
| BA-3 | 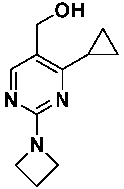 | (2-(azetidin-1-yl)-4-cyclopropylpyrimidin-5-yl)methanol | Calcd.: 206.1 |
| | | | Found: 206.1 |
| BA-4 | 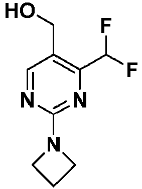 | (2-(azetidin-1-yl)-4-(difluoromethyl)pyrimidin-5-yl)methanol | Calcd.: 216.1 |
| | | | Found: 216.0 |

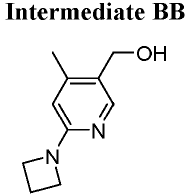

### (6-(azetidin-1-yl)-4-methylpyridin-3-yl)methanol (Scheme BB)

Step 1. (6-(azetidin-1-yl)-4-methylpyridin-3-yl)methanol: (6-Fluoro-4-methylpyridin-3-yl)-methanol (460 mg, 3.26 mmol) was added to a vial with NMP (5 mL). Azetidine (744 mg, 13.0 mmol) was added, and the reaction mixture was heated to 80 °C for 2 h. The reaction was cooled to rt, and purified by silica gel column chromatography (MeOH/DCM) to provide the title compound. MS = 179.1 [M + 1]⁺.

**Table BB. The following compounds were prepared using procedures similar to those described above using the appropriate starting materials.**

| **Intermediate No.** | **Structure** | **Name** | **[M+H]⁺ or [M+Na]⁺** |
|---|---|---|---|
| BB-1 | 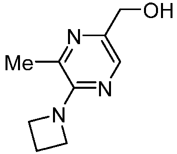 | (5-(azetidin-1-yl)-6-methylpyrazin-2-yl)methanol | Calcd.: 180.1 |
| | | | Found: 180.0 |
| BB-2 | 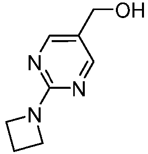 | (2-(azetidin-1-yl)pyrimidin-5-yl)methanol | Calcd.: 166.1 |
| | | | Found: 166.1 |

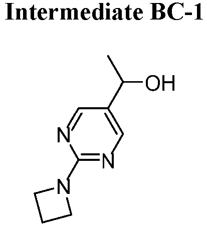

### 1-(2-(Azetidin-1-yl)pyrimidin-5-yl)ethanol (Scheme BC)

Step 1. 1-(2-(azetidin-1-yl)pyrimidin-5-yl)ethanone: A solution of 1-(2-chloropyrimidin-5-yl)ethanone (400 mg, 2.55 mmol), azetidine hydrochloride (239 mg, 2.55 mmol) and TEA (0.89 mL, 6.4 mmol) in EtOH (5 mL) was heated to 80 °C for 1 h. The reaction was cooled to rt, quenched with water, and extracted with EtOAc. The combined organic extracts were washed with brine, dried over Na₂SO₄, filtered and concentrated to give the title compound. MS = 178.1 [M+H]⁺.

Step 2. 1-(2-(azetidin-1-yl)pyrimidin-5-yl)ethanol: To a solution of 1-(2-(azetidin-1-yl)pyrimidin-5-yl)ethanone (200 mg, 1.13 mmol) in THF (3 mL) at 0 °C was added sodium borohydride (55.5 mg, 1.47 mmol), followed by dropwise addition of MeOH (0.5 mL), and the resulting mixture was stirred at rt for 0.5 h. The reaction was quenched with satd. aq. NH₄Cl and extracted with EtOAc. The combined organic extracts were washed with brine, dried over Na₂SO₄, filtered and concentrated to give the title compound. MS = 180.0 [M+H]⁺.

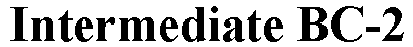

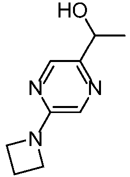

### 1-(5-(azetidin-1-yl)pyrazin-2-yl)ethan-1-ol (Scheme BC)

Step 1. 1-(5-(Azetidin-1-yl)pyrazin-2-yl)ethan-1-one: A mixture of 1-(5-chloropyrazin-2-yl)ethanone (130 mg, 0.830 mmol), azetidine hydrochloride (93 mg, 1.0 mmol) and TEA (0.29 mL, 2.1 mmol) in 1,4-dioxane (1.5 mL) was heated to 40 °C for 12 h. The reaction was cooled to rt, diluted with water and extracted with DCM. The combined organic layers were washed with water and brine, dried over Na₂SO₄, filtered and concentrated. The crude product was purified by silica gel chromatography (EtOAc/petroleum ether) to give the title compound. MS = 178.1 [M + 1]⁺.

Step 2. 1-(5-(Azetidin-1-yl)pyrazin-2-yl)ethan-1-ol: To a solution of 1-(5-(azetidin-1-yl)pyrazin-2-yl)ethanone (120 mg, 0.677 mmol) in THF (1.5 mL) was added NaBH₄ (33 mg, 0.88 mmol) at 0 °C, followed by MeOH (0.5 mL). The reaction mixture was stirred at 0 °C for 1 h, warmed to rt and continued stirring at rt for 1 h. The reaction was quenched with satd. aq. NH₄Cl and extracted with EtOAc. The combined organic layers were washed with water and brine, dried over Na₂SO₄, filtered and concentrated to the title compound. MS = 179.9 [M + 1]⁺.

**Table BC. The following compounds were prepared using procedures similar to those described above using the appropriate starting materials.**

| **Intermediate No.** | **Structure** | **Name** | **[M+H]⁺** |
|---|---|---|---|
| BC-3 | 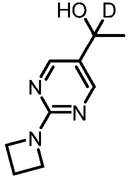 | 1-(2-(azetidin-1-yl)pyrimidin-5-yl)ethan-1-d-1-ol | Calcd.: 181.1 |
| | | | Found: 181.1 |
| BC-4 | 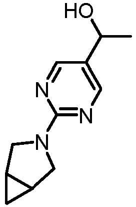 | 1-(2-(3-azabicyclo[3.1.0]hexan-3-yl)pyrimidin-5-yl)ethan-1-ol | Calcd.: 206.1 |
| | | | Found: 206.1 |
| BC-5 | 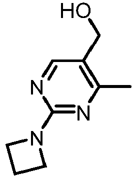 | (2-(azetidin-1-yl)-4-methylpyrimidin-5-yl)methanol | Calcd.: 180.1 |
| | | | Found: 180.1 |
| BC-6 | 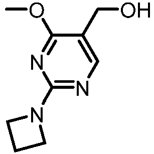 | (2-(azetidin-1-yl)-4-methoxypyrimidin-5-yl)methanol | Calcd.: 196.1 |
| | | | Found: 196.1 |
| BC-7 | 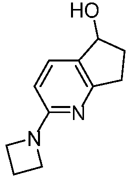 | 2-(azetidin-1-yl)-6,7-dihydro-5H-cyclopenta[b]pyridin-5-ol | Calcd.: 191.1 |
| | | | Found: 191.0 |

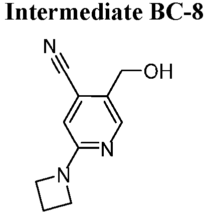

### 2-(azetidin-1-yl)-5-(hydroxymethyl)isonicotinonitrile (Scheme BC)

Step 1. 2-(Azetidin-1-yl)-5-chloroisonicotinonitrile: To a solution of 5-chloro-2-fluoroisonicotinonitrile (1.30 g, 8.30 mmol) in DMF (20 mL) was added K₂CO₃ (3.44 g, 24.9 mmol) and azetidine hydrochloride (855 mg, 9.13 mmol), and the resulting mixture was heated to 60 °C for 1 h. The reaction was cooled to rt, diluted with water and extracted with EtOAc. The combined organic layers were washed with, dried over anhydrous Na₂SO₄, filtered and concentrated to afford a crude residue that was purified by silica gel chromatography (EtOAc/petroleum ether) to give the title compound. MS = 194.0 [M+H]⁺.

Step 2. 2-(Azetidin-1-yl)-5-vinylisonicotinonitrile: A stirred solution of 2-(azetidin-1-yl)-5-chloroisonicotinonitrile (1.40 g, 7.23 mmol), methanesulfonato(2-dicyclohexylphosphino-2',6'-di-isopropoxy-1,1'-biphenyl)(2'-amino-1,1'-biphenyl-2-yl)palladium(II) (605 mg, 0.723 mmol), potassium vinyltrifluoroborate (1.94 g, 14.5 mmol) and Cs₂CO₃ (7.07 g, 21.7 mmol) in CPME (15 mL) and water (5 mL) was heated at 100 °C for 12 h. The reaction was cooled to rt, diluted with water and extracted with EtOAc. The combined organic layers were washed with brine, dried over anhydrous Na₂SO₄, filtered and concentrated to afford a crude residue that was purified by silica gel chromatography (EtOAc/petroleum ether) to give the title compound. MS = 186.0 [M+H]⁺.

Step 3. 2-(Azetidin-1-yl)-5-formylisonicotinonitrile: To a solution of 2-(azetidin-1-yl)-5-vinylisonicotinonitrile (1.00 g, 5.40 mmol) in THF (20 mL) and water (10 mL) was added osmium tetroxide (27.5 mL, 1.080 mmol, 10 mg/mL in water) and sodium periodate (4.62 g, 21.6 mmol), and the resulting mixture was stirred at 15 °C for 12 h. The reaction was quenched with satd. aq. Na₂S₂O₃ and stirred for 0.5 h, at which time, the mixture was extracted with EtOAc. The combined organic layers were washed with brine, dried over anhydrous Na₂SO₄, filtered and concentrated to give the title compound, which was used in next step directly. MS = 187.9 [M+H]⁺.

Step 4. 2-(Azetidin-1-yl)-5-(hydroxymethyl)isonicotinonitrile: To a solution of 2-(azetidin-1-yl)-5-formylisonicotinonitrile (1.00 g, 5.34 mmol) in MeOH (15 mL) was added NaBH₄ (404 mg, 10.7 mmol), and the resulting mixture was stirred at 15 °C for 1.5 h. The reaction was quenched with acetone, partially concentrated and diluted with water. The mixture was extracted with EtOAc, and the combined organic layers were washed with brine, dried over anhydrous Na₂SO₄, filtered and concentrated to give the title compound, which was used in next step directly. MS = 190.0 [M+H]⁺.

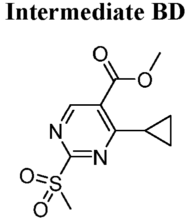

### Methyl 4-cyclopropyl-2-(methylsulfonyl)pyrimidine-5-carboxylate (Scheme BD)

Step 1. Methyl 2-(cyclopropanecarbonyl)-3-(dimethylamino)acrylate: To a 250 mL round-bottom flask under N₂ was added methyl 3-cyclopropyl-3-oxopropanoate (5.00 g, 35.2 mmol) and dioxane (50 ml). N,N-dimethylformamide dimethyl acetal (4.7 mL, 35 mmol) was added at rt, and the resulting mixture was heated to 100 °C for 3 h. The mixture was cooled to rt, concentrated, and used in the next step without purification. MS: 198.1 [M+H]⁺.

Step 2. Methyl 4-cyclopropyl-2-(methylthio)pyrimidine-5-carboxylate: Methyl 2-(cyclopropanecarbonyl)-3-(dimethylamino)acrylate (4.50 g, 22.8 mmol) was added to a 250 mL round-bottom flask with potassium acetate (11.2 g, 114 mmol), 2-methyl-2-thiopseudourea hemisulfate hemihydrate (3.90 g, 13.2 mmol) and DMF (50 mL). The resulting mixture was heated to 80 °C for 3 h, then cooled to rt and quenched with water. The aq. layer was extracted with EtOAc, and the combined organic layers were washed with satd. aq. NaHCO₃, dried with MgSO₄, and concentrated. The crude material was purified on a silica gel column chromatography (EtOAc/hexanes) to provide the title compound. MS: 225.0 [M+H]⁺.

Step 3. Methyl 4-cyclopropyl-2-(methylsulfonyl)pyrimidine-5-carboxylate: Methyl 4-cyclopropyl-2-(methylthio)pyrimidine-5-carboxylate (3.00 g, 13.4 mmol) was added to a flask with DCM (34 mL). mCPBA (6.95 g, 40.3 mmol) was added in multiple portions at rt, and the reaction mixture was stirred at rt for 2 h. The mixture was poured into satd. aq. NaHCO₃ and extracted with DCM. The combined organic layers were dried with MgSO₄ and concentrated to provide the title compound. MS: 257.0 [M+H]⁺.

**Table BD. The following compounds were prepared using procedures similar to those described for above using the appropriate starting materials.**

| **Intermediate No.** | **Structure** | **Name** | **[M+H]⁺** |
|---|---|---|---|
| BD-1 | 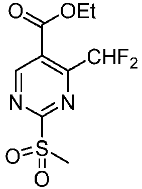 | Ethyl 4-(difluoromethyl)-2-(methylsulfonyl)pyrimidine-5-carboxylate | Calcd.: 281.0 |
| | | | Found: 280.9 |

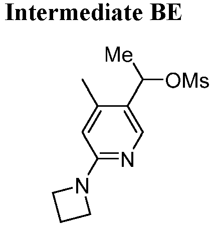

### 1-(6-(Azetidin-1-yl)-4-methylpyridin-3-yl)ethyl methanesulfonate (Scheme BE)

Step 1. Methyl 6-(azetidin-1-yl)-4-methylnicotinate: Azetidine (1.7 mL, 25.3 mmol) was added to a solution of methyl 6-fluoro-4-methylnicotinate (1.1 g, 6.3 mmol) in ACN (16 mL), and the resulting mixture was heated to 50 °C for 2 h. The reaction was cooled to rt, and concentrated to afford a crude residue that was purified by silica gel chromatography (MeOH/DCM) to give the title compound.

Step 2. (6-(Azetidin-1-yl)-4-methylpyridin-3-yl)methanol: Methyl 6-(azetidin-1-yl)-4-methylnicotinate (1.0 g, 4.6 mmol) was added to THF (2 mL), and the solution was cooled to - 78 °C. LiAlH₄ (100 mg, 2.70 mmol) was added in several portions, and the resulting mixture was stirred at - 78 °C for 1 h before warming to rt and stirring at rt for 5 h. The reaction was quenched with water, dried (MgSO₄), filtered, and concentrated to give the title compound that was carried on without purification. MS: 179 [M+H]⁺

Step 3. 6-(Azetidin-1-yl)-4-methylnicotinaldehyde: A solution of DMP (2.97 g, 7.00 mmol) in DCM (15 mL) was added to a stirred solution of (6-(azetidin-1-yl)-4-methylpyridin-3-yl)methanol (830 mg, 4.60 mmol) in DCM (2 mL), and the resulting mixture was stirred at rt for 1 h. The reaction was quenched with satd. aq. NaHCO₃ and satd. aq. Na₂S₂O₃, and the mixture was extracted with DCM. The combined organic layers were washed with brine, dried (MgSO₄), filtered, and concentrated to give the title compound that was carried on without purification. MS: 177 [M+H]⁺

Step 4. 1-(6-(Azetidin-1-yl)-4-methylpyridin-3-yl)ethan-1-ol: Methylmagnesium iodide (2.3 mL, 7.0 mmol, 3M in diethyl ether) was added dropwise to a stirred solution of 6-(azetidin-1-yl)-4-methylnicotinaldehyde (0.82 g, 4.6 mmol) in THF (15 mL) at - 78 °C, and the resulting mixture was stirred at - 78 °C for 30 min. The reaction was warmed to rt and allowed to stir at rt for 3 h. The reaction was quenched with satd. aq. NH₄Cl, and after continuing to stir at rt for 2 h, was extracted with EtOAc. The combined organics were dried (MgSO₄), filtered and concentrated to provide the give the title compound that was carried on without purification. MS: 193 [M+H]⁺

### Step 5. 1-(6-(Azetidin-1-yl)-4-methylpyridin-3-yl)ethyl methanesulfonate:

Methanesulfonyl chloride (0.26 mL, 3.2 mmol) was added to a stirred solution of 1-(6-(azetidin-1-yl)-4-methylpyridin-3-yl)ethan-1-ol (414 mg, 2.20 mmol) and TEA (0.90 mL, 6.5 mmol) in DCM (22 mL) at 0 °C. The resulting mixture was stirred at 0 °C for 2 h, at which time, the reaction was diluted with water and extracted with DCM. The combined organic layers were dried (Na₂SO₄), filtered, and concentrated to give the title compound that was carried on without purification. MS: 271 [M+H]⁺

**Table BE. The following compounds were prepared using procedures similar to those described above using the appropriate starting materials.**

| **Intermediate No.** | **Structure** | **Name** | **[M+H]⁺ or [M+Na]⁺** |
|---|---|---|---|
| BE-1 | 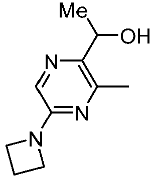 | 1-(5-(Azetidin-1-yl)-3-methylpyrazin-2-yl)ethan-1-ol | Calcd.: 194.1 |
| | | | Found: 194.0 |
| BE-2 | 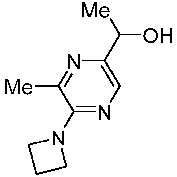 | 1-(5-(Azetidin-1-yl)-6-methylpyrazin-2-yl)ethan-1-ol | Calcd.: 194.1 |
| | | | Found: 194.0 |

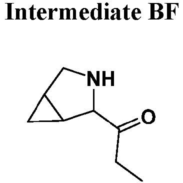

### 1-(3-Azabicyclo[3.1.0]hexan-2-yl)propan-1-one (Scheme BF)

Step 1: 3-*tert*-Butyl 2-methyl 3-azabicyclo[3.1.0]hexane-2,3-dicarboxylate: To a solution of 3-(*tert*-butoxycarbonyl)-3-azabicyclo[3.1.0]hexane-2-carboxylic acid (200 mg, 0.880 mmol) in DCM (10 mL) and MeOH (1 mL) was added TMS-diazomethane (0.66 mL, 1.32 mmol) at 0 °C. The reaction mixture was stirred at 0 °C for 2 h, at which time, the reaction was warmed to rt, diluted with water, and extracted with DCM. The combined organic layers were dried over Na₂SO₄, filtered and concentrated to give the title compound. MS = 142.1 [M - Boc + 1]⁺.

Step 2: *tert*-Butyl 2-(1-hydroxycyclopropyl)-3-azabicyclo[3.1.0]hexane-3-carboxylate: To a solution of 3-*tert*-butyl 2-methyl 3-azabicyclo[3.1.0]hexane-2,3-dicarboxylate (150 mg, 0.622 mmol) in THF (1 mL) was added titanium(IV) isopropoxide (0.55 mL, 1.9 mmol), and the resulting mixture was stirred at rt for 30 min. Ethylmagnesium bromide (2.07 mL, 6.22 mmol, 3M in THF) was added, and the reaction was stirred at rt for 12 h. The reaction was cooled to 0 °C, quenched with water and extracted with DCM. The combined organic layers were dried over Na₂SO₄, filtered and concentrated to give the title compound. MS = 140.1 [M - Boc + 1]⁺.

Step 3: 1-(3-Azabicyclo[3.1.0]hexan-2-yl)propan-1-one: To a solution of *tert*-butyl 2-(1-hydroxycyclopropyl)-3-azabicyclo[3.1.0]hexane-3-carboxylate (70 mg, 0.29 mmol) in DCM (1.5 mL) was added TFA (0.70 mL, 9.1 mmol), and the resulting mixture was stirred at rt for 2 h. The reaction was concentrated to afford a crude residue that was purified by reversed-phase preparatory-HPLC (C18 stationary phase, water(0.1%TFA)-ACN) to afford the title compound. MS = 140.0 [M+H]⁺

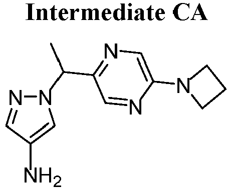

### 1-(1-(5-(Azetidin-1-yl)pyrazin-2-yl)ethyl)-1H-pyrazol-4-amine (Scheme CA)

Step 1. 2-(Azetidin-1-yl)-5-(1-(4-nitro-1*H*-pyrazol-1-yl)ethyl)pyrazine: To a solution of 1-(5-(azetidin-1-yl)pyrazin-2-yl)ethanol (100 mg, 0.558 mmol), triphenylphosphine (220 mg, 0.837 mmol) and 4-nitro-1H-pyrazole (69 mg, 0.61 mmol) in toluene (5 mL) was added DIAD (0.13 mL, 0.67 mmol) at 0 °C, and the resulting mixture was warmed to rt and allowed to stir for 2 h. The reaction was diluted with water and extracted with EtOAc. The combined organic layers were washed with water and brine, dried over Na₂SO₄, filtered and concentrated to afford a crude residue that was purified by preparatory-TLC (1:1 petroleum ether/EtOAc) to afford the title compound. MS = 275.0 [M + 1]⁺.

Step 2. 1-(1-(5-(Azetidin-1-yl)pyrazin-2-yl)ethyl)-1*H*-pyrazol-4-amine: To a mixture of 2-(azetidin-1-yl)-5-(1-(4-nitro-1*H*-pyrazol-1-yl)ethyl)pyrazine (78 mg, 0.28 mmol) and ammonium chloride (76 mg, 1.4 mmol) in EtOH (5 mL) and water (5 mL) was added iron (79 mg, 1.4 mmol), and the resulting mixture was heated to 60 °C for 2 h. The reaction was cooled to rt and diluted with DCM and water. The resulting suspension was filtered through a pad of Celite^{®}, the filtrate layers were separated, and the aq. layer was extracted with DCM. The combined organic layers were washed with water and brine, dried over Na₂SO₄, filtered and concentrated to give the title compound which was carried to next step without further purification. MS = 245.1 [M + 1]⁺.

**Table CA. The following compounds were prepared using procedures similar to those described above using the appropriate starting materials.**

| **Intermediate No.** | **Structure** | **Name** | **[M+H]⁺ or [M+Na]⁺** |
|---|---|---|---|
| CA-1 | 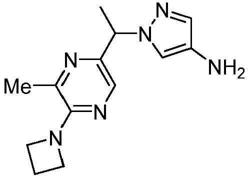 | 1-(1-(5-(Azetidin-1-yl)-6-methylpyrazin-2-yl)ethyl)-1*H-*pyrazol-4-amine | Calcd.: 259.2 |
| | | | Found: 259 |
| CA-2 | 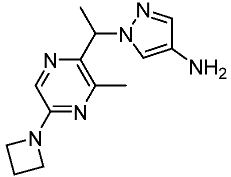 | 1-(1-(5-(Azetidin-1-yl)-3-methylpyrazin-2-yl)ethyl)-1*H-*pyrazol-4-amine | Calcd.: 259.2 |
| | | | Found: 259 |
| CA-3 | 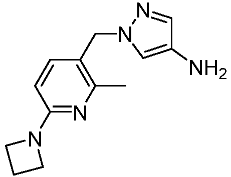 | 1-((6-(Azetidin-1-yl)-2-methylpyridin-3-yl)methyl)-1*H-*pyrazol-4-amine | Calcd.: 244.2 |
| | | | Found: 244.1 |
| CA-4 | 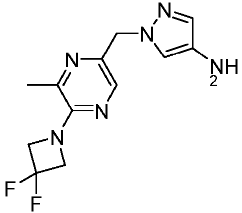 | 1-((5-(3,3-Difluoroazetidin-1-yl)-6-methylpyrazin-2-yl)methyl)-1*H*-pyrazol-4-amine | Calcd.: 281.1 |
| | | | Found: 281 |
| CA-5 | 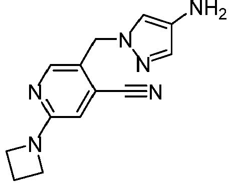 | 5-((4-Amino-1*H*-pyrazol-1-yl)methyl)-2-(azetidin-1-yl)isonicotinonitrile | Calcd.: 255.1 |
| | | | Found: 255.0 |
| CA-6 | 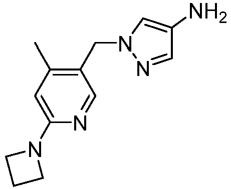 | 1-((6-(Azetidin-1-yl)-4-methylpyridin-3-yl)methyl)-1*H-*pyrazol-4-amine | Calcd.: 244.2 |
| | | | Found: 244.1 |
| CA-7 | 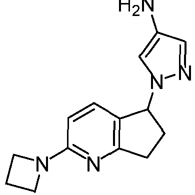 | 1-(2-(Azetidin-1-yl)-6,7-dihydro-5*H-*cyclopenta[b]pyridin-5-yl)-1*H-*pyrazol-4-amine | Calcd.: 256.1 |
| | | | Found: 256.1 |
| CA-8 | 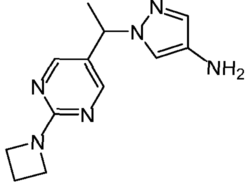 | 1-(1-(2-(Azetidin-1-yl)pyrimidin-5-yl)ethyl)-1*H-*pyrazol-4-amine | Calcd.: 245.2 |
| | | | Found: 245.0 |
| CA-9 | 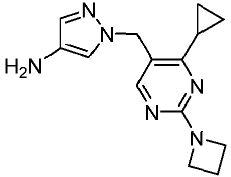 | 1-((2-(Azetidin-1-yl)-4-cyclopropylpyrimidin-5-yl)methyl)-1*H*-pyrazol-4-amine | Calcd.: 271.2 |
| | | | Found: 271.1 |
| CA-10 | 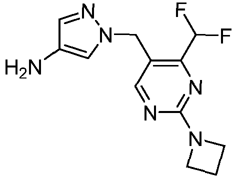 | 1-((2-(Azetidin-1-yl)-4-(difluoromethyl)pyrimidin-5-yl)methyl)-1*H*-pyrazol-4-amine | Calcd.: 281.1 |
| | | | Found: 281.0 |
| CA-11 | 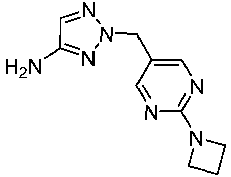 | 2-((2-(Azetidin-1-yl)pyrimidin-5-yl)methyl)-2*H*-1,2,3-triazol-4-amine | Calcd.: 232.1 |
| | | | Found: 232.0 |
| CA-12 | 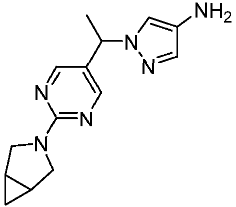 | 1-(1-(2-(3-Azabicyclo[3.1.0]hexan-3-yl)pyrimidin-5-yl)ethyl)-1*H-*pyrazol-4-amine | Calcd.: 271.6 |
| | | | Found: 271.1 |
| CA-13 | 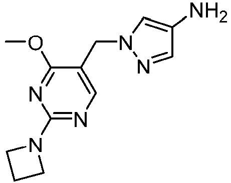 | 1-((2-(Azetidin-1-yl)-4-methoxypyrimidin-5-yl)methyl)-1*H*-pyrazol-4-amine | Calcd.: 261.1 |
| | | | Found: 261.0 |
| CA-14 | 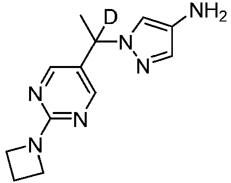 | 1-(1-(2-(Azetidin-1-yl)pyrimidin-5-yl)ethyl-1-*d*)-1*H*-pyrazol-4-amine | Calcd.: 246.3 |
| | | | Found: 246.1 |
| CA-15 | 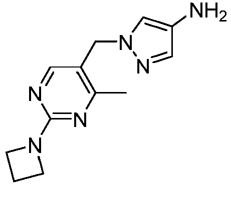 | 1-((2-(Azetidin-1-yl)-4-methylpyrimidin-5-yl)methyl)-1*H*-pyrazol-4-amine | Calcd.: 245.3 |
| | | | Found: 245.1 |
| CA-16 | 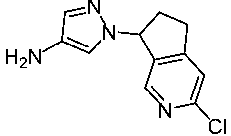 | 1-(3-Chloro-6,7-dihydro-5*H-*cyclopenta[c]pyridin-7-yl)-1*H-*pyrazol-4-amine | Calcd.: 235.1 |
| | | | Found: 235.1 |

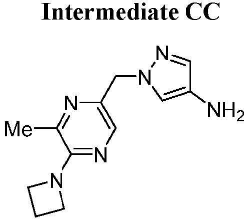

### 1-((5-(Azetidin-1-yl)-6-methylpyrazin-2-yl)methyl)-1H-pyrazol-4-amine (Scheme CC)

Step 1. (5-Chloro-6-methylpyrazin-2-yl)methanol: A solution of 5-chloro-6-methylpyrazine-2-carbaldehyde (950 mg, 6.07 mmol) in MeOH (8.3 mL) was cooled to 0 °C, at which time NaBH₄ (241 mg, 6.37 mmol) was added. The resulting mixture was warmed to rt and allowed to stir for 3 h. The reaction was quenched with satd. aq. NH₄Cl and concentrated. The resulting crude residue was diluted in EtOAc and washed with satd. aq. NaHCO₃ and brine. The combined organic extracts were dried (Na₂SO₄), filtered and concentrated to provide the title compound. MS = 159.1 [M + 1]⁺

Step 2. 2-Chloro-3-methyl-5-((4-nitro-1*H*-pyrazol-1-yl)methyl)pyrazine: To a vial containing (5-chloro-6-methylpyrazin-2-yl)methanol (552 mg, 3.48 mmol), 4-nitro-1H-pyrazole (590 mg, 5.22 mmol) and triphenyl phosphine (1.37 g, 5.22 mmol) in THF (17 mL) was added DIAD (1.02 ml, 5.22 mmol), and the resulting mixture was heated to 50 °C overnight. The reaction was cooled to rt and concentrated to afford a crude residue that was purified by silica gel chromatography (MeOH/DCM) to give the title compound. MS = 254 [M + 1]⁺

Step 3. 2-(Azetidin-1-yl)-3-methyl-5-((4-nitro-1*H*-pyrazol-1-yl)methyl)pyrazine: To a solution of 2-chloro-3-methyl-5-((4-nitro-1H-pyrazol-1-yl)methyl)pyrazine (310 mg, 0.550 mmol) in ACN (1.4 ml) was added azetidine (0.15 mL, 2.2 mmol), and the resulting mixture was heated to 50 °C for 2 h. The reaction was cooled to rt and concentrated to afford a crude residue that was purified by silica gel chromatography (MeOH/DCM) to give the title compound. MS = 275 [M + 1]⁺

Step 4. 1-((5-(Azetidin-1-yl)-6-methylpyrazin-2-yl)methyl)-1*H*-pyrazol-4-amine: 2-(Azetidin-1-yl)-3-methyl-5-((4-nitro-1*H*-pyrazol-1-yl)methyl)pyrazine (325 mg, 1.18 mmol) was added to a vial with iron powder (0.53 g, 9.5 mmol) and ammonium chloride (634 mg, 11.8 mmol). EtOH (4.7 ml) and water (1.2 ml) were added, and the reaction mixture was heated to 80 °C for 4 h. The reaction was cooled to rt, filtered through a pad of Celite^{®}, and diluted with satd. aq. NaHCO₃. The mixture was extracted with DCM, and the combined organic fractions were dried (MgSO₄) and concentrated to provide the title compound that was used without further purification. MS = 245 [M + 1]⁺

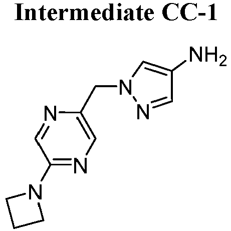

### 1-((5-(azetidin-1-yl)pyrazin-2-yl)methyl)-1H-pyrazol-4-amine (Scheme CC)

Step 1. *tert*-Butyl (1-((5-chloropyrazin-2-yl)methyl)-1*H*-pyrazol-4-yl)carbamate: A mixture of *tert*-butyl (1*H*-pyrazol-4-yl)carbamate (247 mg, 1.35 mmol), 2-chloro-5-(chloromethyl)pyrazine (200 mg, 1.23 mmol) and cesium carbonate (800 mg, 2.45 mmol) in DMF (6.1 mL) was stirred at 50 °C for 3 h. The reaction was cooled to rt, diluted with EtOAc and washed with brine. The organic layer was dried over Na₂SO₄, filtered and concentrated to afford a crude residue that was purified by silica gel chromatography (EtOAc/hexanes) to give the title compound. MS = 310.1 [M + 1]⁺

Step 2. *tert*-Butyl (1-((5-(azetidin-1-yl)pyrazin-2-yl)methyl)-1*H*-pyrazol-4-yl)carbamate: A mixture of *tert*-butyl (1-((5-chloropyrazin-2-yl)methyl)-1*H*-pyrazol-4-yl)carbamate (80 mg, 0.26 mmol), azetidine (52 µL, 0.78 mmol), and potassium carbonate (107 mg, 0.78 mmol) in DMF (2.6 mL) was stirred at 90 °C for 3 h. The reaction was cooled to rt, diluted with EtOAc, and washed with brine. The organic layer was dried over Na₂SO₄, filtered and concentrated to afford a crude residue that was purified by silica gel chromatography (MeOH/DCM) to give the title compound. MS = 331.1 [M + 1]⁺

Step 3. 1-((5-(Azetidin-1-yl)pyrazin-2-yl)methyl)-1*H*-pyrazol-4-amine: The title compound was prepared from *tert*-butyl (1-((5-(azetidin-1-yl)pyrazin-2-yl)methyl)-1*H*-pyrazol-4-yl)carbamate following procedures similar to those described above for Intermediate BF and was carried on without purification. MS = 231.1 [M + 1]⁺

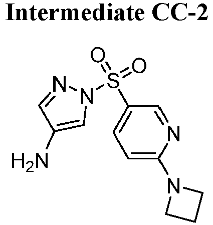

### 1-((6-(Azetidin-1-yl)pyridin-3-yl)sulfonyl)-1H-pyrazol-4-amine (Scheme CC)

Step 1. *tert*-Butyl (1-((6-chloropyridin-3-yl)sulfonyl)-1*H*-pyrazol-4-yl)carbamate: Sodium hydride (72 mg, 1.80 mmol, 60% w/w dispersion in mineral spirits) was added to a stirred solution of tert-butyl (1H-pyrazol-4-yl)carbamate (300 mg, 1.64 mmol) in THF (8 mL) at 0 °C. The resulting mixture was warmed to rt for 15 min and recooled to 0 °C. 6-Chloropyridine-3-sulfonyl chloride (417 mg, 1.96 mmol) was added, and the reaction mixture was stirred at rt for 1 h. The reaction was concentrated, and the crude residue was purified by silica gel chromatography (EtOAc/hexanes) to give the title compound. ¹H NMR (400 MHz, CDCl₃; mixture of rotational isomers) δ 8.87, 8.58 (br s, 1H), 8.27 (br s, 1H), 8.20, 7.96 (br d, 1H), 7.77, 7.69 (br s, 1H), 6.89, 6.81 (br s, 1H), 1.50, 1.46 (br s, 9H). Steps 2-3 1-((6-(Azetidin-1-yl)pyridin-3-yl)sulfonyl)-1*H*-pyrazol-4-amine: Steps 2-3 were performed using the procedures similar to steps 2-3 described for **Intermediate CC-1.** MS = 280.0 [M + 1]⁺

**Table CC. The following compounds were prepared using procedures similar to those described above using the appropriate starting materials.**

| **Intermediate No.** | **Structure** | **Name** | **[M+H]⁺ or [M+Na]⁺** |
|---|---|---|---|
| CC-3 | 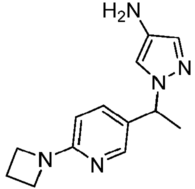 | 1-(1-(6-(Azetidin-1-yl)pyridin-3-yl)ethyl)-1H-pyrazol-4-amine | Calcd.: 244.2 |
| | | | Found: 244.2 |
| CC-4 | 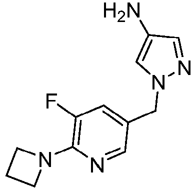 | 1-((6-(Azetidin-1-yl)-5-fluoropyridin-3-yl)methyl)-1H-pyrazol-4-amine | Calcd.: 248.1 |
| | | | Found: 248.1 |
| CC-5 | 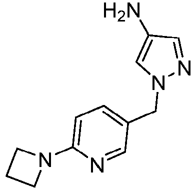 | 1-((6-(Azetidin-1-yl)pyridin-3-yl)methyl)-1H-pyrazol-4-amine | Calcd.: 230.1 |
| | | | Found: 230.1 |

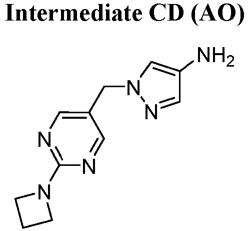

### 1-((2-(Azetidin-1-yl)pyrimidin-5-yl)methyl)-1H-pyrazol-4-amine (Scheme CD)

Steps 1 and 2. *tert*-Butyl (1-((2-(azetidin-1-yl)pyrimidin-5-yl)methyl)-1*H*-pyrazol-4-yl)carbamate: Into a 3-L 3-necked round-bottom flask purged and maintained with an inert atmosphere of nitrogen, was placed **BB-2** (80.0 g, 484 mmol). This was followed by the addition of DCM (1.60 L). The resulting mixture was cooled to 0 °C, and tribromophosphane (144.2 g, 532.7 mmol) was added dropwise. The resulting solution was stirred at rt for 2 h. The solids were collected by filtration to afford a crude product that was charged into a 2-L 3-necked round-bottom flask purged and maintained with an inert atmosphere of nitrogen and dissolved in DMF (800 mL). K₂CO₃ (96.95 g, 701.4 mmol), and tert-butyl N-(1H-pyrazol-4-yl)carbamate (77.11 g, 420.9 mmol) were added, and the resulting solution was stirred overnight at 50 °C. The reaction was then quenched by the addition of water, and the resulting solution was extracted with EtOAc. The combined organic layers were washed with brine and concentrated to afford a crude residue that was purified by silica gel chromatography (EtOAc/petroleum ether) to give the title compound. MS = 331.1 [M+H]⁺.

Step 3. 1-((2-(Azetidin-1-yl)pyrimidin-5-yl)methyl)-1*H*-pyrazol-4-amine: Into a 2-L 3-necked round-bottom flask purged and maintained with an inert atmosphere of nitrogen, was placed *tert-*butyl *N*-(1-[[2-(azetidin-1-yl)pyrimidin-5-yl]methyl]-1*H*-pyrazol-4-yl)carbamate (60.00 g, 181.6 mmol). This was followed by the addition of DCM (1.20 L). To this mixture was added TMSOTf (44.40 g, 199.8 mmol) dropwise with stirring at 0 °C. The resulting solution was warmed to rt and allowed to stir for 1.5 h. The reaction was adjusted to pH = 9-10 with 2M aq. K₂CO₃. The resulting mixture was extracted with DCM, and the organic layers were combined and concentrated to afford a crude residue that was subsequently washed with DCM/petroleum ether to yield a solid filter cake as the title compound. MS = 231.0 [M+H]⁺.

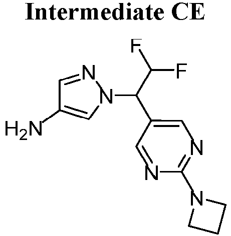

### 1-(1-(2-(Azetidin-1-yl)pyrimidin-5-yl)-2,2-difluoroethyl)-1H-pyrazol-4-amine (Scheme CE)

Step 1. 2-(Azetidin-1-yl)pyrimidine-5-carbaldehyde: 2-Chloropyrimidine-5-carbaldehyde (2.00 g, 14.0 mmol) and TEA (2.35 mL, 16.8 mmol) were dissolved in dioxane (30 mL), and azetidine hydrochloride (1.44 g, 15.4 mmol) was added. The reaction mixture was stirred at rt for 2 h, at which time, the mixture was concentrated and resuspended in water. The mixture was extracted with EtOAc, and the combined organic layers were dried over Na₂SO₄, filtered and concentrated to afford a crude residue that was purified by silica gel chromatography (EtOAc/petroleum ether) to give the title compound. MS = 164.0 [M+H]⁺.

Step 2. 1-(2-(Azetidin-1-yl)pyrimidin-5-yl)-2,2-difluoroethanol: To a solution of 2-(azetidin-1-yl)pyrimidine-5-carbaldehyde (600 mg, 3.68 mmol) in DMF (10 mL) was added (difluoromethyl)trimethylsilane (2.28 g, 18.4 mmol) and cesium fluoride (1.12 g, 7.35 mmol), and the resulting mixture was stirred at rt for 12 h. TBAF (7.35 mL, 7.35 mmol, 1M in THF) was added, and the mixture continued stirring at rt for 1 h. The reaction was partitioned between water and EtOAc, the layers were separated, and the aq. layer was extracted with EtOAc. The combined organic layers were washed with brine, dried over anhydrous Na₂SO₄, filtered and concentrated to afford a crude residue that was purified by silica gel chromatography (EtOAc/petroleum ether) to give the title compound. MS = 216.0 [M+H]⁺.

Step 3. 2-(Azetidin-1-yl)-5-(2,2-difluoro-1-(4-nitro-1*H*-pyrazol-1-yl)ethyl)pyrimidine: A solution of 4-nitro-1*H*-pyrazole (420 mg, 3.72 mmol), 1-(2-(azetidin-1-yl)pyrimidin-5-yl)-2,2-difluoroethanol (400 mg, 1.86 mmol), (*E*)-diisopropyl diazene-1,2-dicarboxylate (752 mg, 3.72 mmol) and Ph₃P (975 mg, 3.72 mmol) in THF (20 mL) was stirred at rt for 1 h. The mixture was concentrated to afford a crude residue that was purified by silica gel chromatography (EtOAc/petroleum ether) to give the title compound. MS = 311.0 [M+H]⁺.

Step 4. 1-(1-(2-(Azetidin-1-yl)pyrimidin-5-yl)-2,2-difluoroethyl)-1*H*-pyrazol-4-amine: To a solution of 2-(azetidin-1-yl)-5-(2,2-difluoro-1-(4-nitro-1*H*-pyrazol-1-yl)ethyl)pyrimidine (600 mg, 1.93 mmol) in EtOAc (9 mL) and MeOH (3 mL) was added platinum(IV) oxide (43.9 mg, 0.193 mmol) under N₂ atmosphere. The mixture was degassed and backfilled with H₂ (three times), and the resulting mixture was stirred under H₂ (15 psi) at rt for 1.5 h. The reaction was filtered through a pad of Celite^{®}, and filtrate was concentrated to afford a crude residue that was purified by silica gel chromatography (MeOH/DCM) to give the title compound. MS = 281.1 [M+H]⁺.

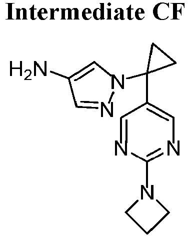

### 1-(1-(2-(Azetidin-1-yl)pyrimidin-5-yl)cyclopropyl)-1H-pyrazol-4-amine (Scheme CF)

Step 1. Ethyl 2-fazetidin-1-yl)pyrimidine-5-carboxylate: To a solution of ethyl 2-chloropyrimidine-5-carboxylate (5.00 g, 26.8 mmol) and TEA (11.2 mL, 80.0 mmol) in dioxane (50 mL) was added azetidine hydrochloride (3.01 g, 32.2 mmol). The reaction mixture was stirred at rt for 16 h, at which time, the reaction was poured into water and extracted with EtOAc. The combined organic layers were dried over Na₂SO₄ and concentrated to afford a crude residue that was purified by silica gel chromatography (MeOH/DCM) to give the title compound. MS = 280.0 [M+H]⁺.

Step 2. 1-(2-(Azetidin-1-yl)pyrimidin-5-yl)cyclopropanol: To a solution of titanium(IV) isopropoxide (686 mg, 2.41 mmol) and ethyl 2-(azetidin-1-yl)pyrimidine-5-carboxylate (1.00 g, 4.83 mmol) in THF (4 mL) was added ethylmagnesium bromide (4.83 mL, 14.5 mmol, 3M in diethyl ether) at rt over 30 min, and the resulting mixture was stirred at rt for 3 h. The reaction was quenched with satd. aq. NH₄Cl and extracted with EtOAc. The combined organic layers were washed with water and brine, dried over Na₂SO₄, filtered and concentrated to afford a crude residue that was purified by reversed-phase preparatory-HPLC (C18 stationary phase, water(0.1%TFA)-ACN to give the title compound. MS = 192.1 [M+H]⁺.

Step 3. 2-(Azetidin-1-yl)-5-(1-(4-nitro-1*H*-pyrazol-1-yl)cyclopropyl)pyrimidine: To a solution of 1-(2-(azetidin-1-yl)pyrimidin-5-yl)cyclopropanol (120 mg, 0.628 mmol), triphenylphosphine (329 mg, 1.26 mmol) and 4-nitro-1H-pyrazole (71 mg, 0.63 mmol) in toluene (3 mL) was added (E)-di-tert-butyl diazene-1,2-dicarboxylate (217 mg, 0.941 mmol), and the resulting mixture was heated to 110 °C for 12 h. The reaction was cooled to rt and concentrated to afford a crude residue that was purified by reversed-phase preparatory-HPLC (C18 stationary phase, water(0.1%TFA)-ACN to give the title compound. MS = 287.0 [M+H]⁺.

Step 4. 1-(1-(2-(Azetidin-1-yl)pyrimidin-5-yl)cyclopropyl)-1*H*-pyrazol-4-amine: To a mixture of 2-(azetidin-1-yl)-5-(1-(4-nitro-1H-pyrazol-1-yl)cyclopropyl)pyrimidine (67 mg, 0.23 mmol) and ammonium chloride (62.6 mg, 1.17 mmol) in EtOH (5 mL) and water (5 mL) was added iron (65.3 mg, 1.17 mmol), and the resulting mixture was heated to 60 °C for 2 h. The reaction was cooled to rt, diluted with DCM and water, and filtered through a pad of Celite^{®}. The layers were separated, and the aq. layer was extracted with DCM. The combined organic layers were washed with water and brine, dried over Na₂SO₄, filtered and concentrated to give the title compound which was carried to next step without further purification. MS = 257.0 [M+H]⁺.

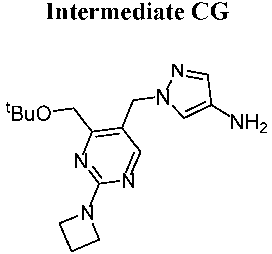

### 1-((2-(Azetidin-1-yl)-4-(tert-butoxymethyl)pyrimidin-5-yl)methyl)-1H-pyrazol-4-amine

Step 1. Ethyl 4-(*tert*-butoxymethyl)-2-chloropyrimidine-5-carboxylate: To a solution of ethyl 2-chloropyrimidine-5-carboxylate (10.0 g, 53.6 mmol) in ACN (30 mL) and water (30 mL) was added 2-(tert-butoxy)acetic acid (21.3 g, 161 mmol), silver nitrate (36.4 g, 214 mmol) and ammonium persulfate (61.1 g, 268 mmol), and the resulting mixture was heated to 60 °C for 2 h. The reaction was poured into water and extracted with EtOAc. The combined organic layers were dried over Na₂SO₄, filtered, and concentrated to give a crude residue that was purified by silica gel chromatography (EtOAc/petroleum ether) to give the title compound. MS = 273.0 [M+H]⁺.

Step 2. Ethyl 2-(azetidin-1-yl)-4-(*tert*-butoxymethyl)pyrimidine-5-carboxylate: TEA (5.1 mL, 37 mmol) was added to a stirred solution of ethyl 4-(tert-butoxymethyl)-2-chloropyrimidine-5-carboxylate (4.00 g, 14.7 mmol) and azetidine hydrochloride (2.74 g, 29.3 mmol) in EtOH (40 mL), and the resulting mixture was heated to 70 °C for 2 h. The reaction was diluted with EtOAc and washed by water followed by brine. The organics were dried over Na₂SO₄, filtered and concentrated to give the crude title compound which was used in next step directly. MS = 294.2 [M+H]⁺.

Step 3. (2-(Azetidin-1-yl)-4-(tert-butoxymethyl)pyrimidin-5-yl)methanol: To solution of ethyl 2-(azetidin-1-yl)-4-(*tert*-butoxymethyl)pyrimidine-5-carboxylate (1.40 g, 4.77 mmol) in THF (30 mL) at 0 °C was added DIBAL-H (9.5 mL, 9.5 mmol, 1M in toluene) dropwise, and the resulting mixture was stirred at 0 °C for 2 h. The reaction was quenched by slow addition of 15% aq. NaOH at 0 °C. The mixture was filtered, and the filtrate was concentrated to give a crude residue that was purified by silica gel chromatography (EtOAc/petroleum ether) to give the title compound. MS = 252.1 [M+H]⁺.

Step 4. 2-(Azetidin-1-yl)-4-(*tert*-butoxymethyl)-5-((4-nitro-1*H*-pyrazol-1-yl)methyl)pyrimidine: To a mixture of (2-(azetidin-1-yl)-4-(*tert*-butoxymethyl)pyrimidin-5-yl)methanol (600 mg, 2.39 mmol), 4-nitro-1*H*-pyrazole (324 mg, 2.86 mmol) and triphenylphosphine (939 mg, 3.58 mmol) in THF (15 mL) at 0 °C was added (*E*)-di-*tert*-butyl diazene-1,2-dicarboxylate (825 mg, 3.58 mmol), and the resulting mixture was warmed to rt and allowed to stir for 3 h. The reaction was concentrated to give a crude residue that was purified by silica gel chromatography (EtOAc/petroleum ether) to give the title compound. MS = 347.1 [M+H]⁺.

Step 5. 1-((2-(Azetidin-1-yl)-4-(*tert*-butoxymethyl)pyrimidin-5-yl)methyl)-1*H*-pyrazol-4-amine: To a solution of 2-(azetidin-1-yl)-4-(*tert*-butoxymethyl)-5-((4-nitro-1H-pyrazol-1-yl)methyl)pyrimidine (800 mg, 2.31 mmol) in EtOAc (9 mL) and MeOH (3 mL) was added platinum(IV) oxide (52 mg, 0.23 mmol), and the resulting mixture was degassed and backfilled with H₂. The mixture was stirred under H₂ (15 psi) at rt for 1 h, at which time, the reaction was filtered, and the filtrate was concentrated to give crude residue that was purified by preparatory-TLC (EtOAc) to give the title compound. MS = 317.2 [M+H]⁺.

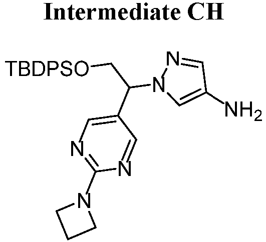

### 1-(1-(2-(Azetidin-1-yl)pyrimidin-5-yl)-2-((tert-butyldiphenylsilyl)oxy)ethyl)-1H-pyrazol-4-amine

Step 1. 2-(Azetidin-1-yl)-5-vinylpyrimidine: Azetidine hydrochloride (1.10 g, 11.7 mmol) was added to a stirred solution of 2-chloro-5-vinylpyrimidine (1.50 g, 10.7 mmol) and TEA (2.97 mL, 21.3 mmol) in 1,4-dioxane (15 mL), and the resulting mixture was heated to 35 °C for 3 h. The reaction was poured into water and extracted with EtOAc. The combined organic layers were dried over Na₂SO₄, filtered and concentrated to give a crude residue that was purified by silica gel chromatography (EtOAc/petroleum ether) to give the title compound. MS = 162.1 [M+H]⁺.

Step 2. 1-(2-(Azetidin-1-yl)pyrimidin-5-yl)ethane-1,2-diol: An aq. solution of osmium(VIII) oxide (23 mL, 0.90 mmol, 10 mg/mL) and 4-methylmorpholine 4-oxide (1.05 g, 8.99 mmol) was added to a stirred solution of the 2-(azetidin-1-yl)-5-vinylpyrimidine (1.45 g, 8.99 mmol) in ACN (15 mL) and water (5 mL), and the resulting mixture was heated to 35 °C for 1 h. The reaction was cooled to 0 °C, and quenched with satd. aq. NaS₂O₃ and continued stirring at 0 °C for 0.5 h. The mixture was filtered through a pad of Celite^{®} and concentrated to afford a crude residue that was purified by reversed-phase preparatory-HPLC (C18 stationary phase, water (0.1%TFA) - ACN) to give the title compound. MS = 196.0 [M+H]⁺.

Step 3. 1-(2-(Azetidin-1-yl)pyrimidin-5-yl)-2-((*tert*-butyldiphenylsilyl)oxy)ethanol: To a stirred solution of 1-(2-(azetidin-1-yl)pyrimidin-5-yl)ethane-1,2-diol (2.61 g, 13.4 mmol) in DMF (20 mL) was added 1*H*-imidazole (2.00 g, 29.5 mmol) and tert-butylchlorodiphenylsilane (4.42 g, 16.1 mmol), and the resulting mixture was heated to 50 °C for 6 h. The reaction was diluted with water and extracted with EtOAc. The combined organic layers were washed with brine, dried over Na₂SO₄, filtered and concentrated to give a crude residue that was purified by silica gel chromatography (EtOAc/petroleum ether) to give the title compound. MS = 434.1 [M+H]⁺.

Step 4. 2-(Azetidin-1-yl)-5-(2-((*tert*-butyldiphenylsilyl)oxy)-1-(4-nitro-1*H*-pyrazol-1-yl)ethyl)pyrimidine: The stirred solution of 4-nitro-1H-pyrazole (344 mg, 3.04 mmol), 1-(2-(azetidin-1-yl)pyrimidin-5-yl)-2-((tert-butyldiphenylsilyl)oxy)ethanol (659 mg, 1.52 mmol), DIAD (615 mg, 3.04 mmol) and triphenylphosphine (797 mg, 3.04 mmol) in THF (10 mL) was heated to 50 °C for 2 h. The reaction was concentrated, resuspended in water and extracted with EtOAc. The combined organic layers were washed with brine, dried over Na₂SO₄, filtered and concentrated to give a crude residue that was purified by silica gel chromatography (EtOAc/petroleum ether) to give the title compound. MS = 529.2 [M+H]⁺.

Step 5. 1-(1-(2-(Azetidin-1-yl)pyrimidin-5-yl)-2-((*tert*-butyldiphenylsilyl)oxy)ethyl)-1*H-*pyrazol-4-amine: Iron (1.47 g, 26.3 mmol) and ammonium chloride (1.69 g, 31.6 mmol) were added to a stirred solution of 2-(azetidin-1-yl)-5-(2-((tert-butyldiphenylsilyl)oxy)-1-(4-nitro-1H-pyrazol-1-yl)ethyl)pyrimidine (2.78 g, 5.26 mmol) in EtOH (18 mL) and water (6 mL), and the resulting mixture was heated to 80 °C for 1 h. The reaction was diluted with water, filtered through a pad of Celite^{®}, and the resulting filtrate was diluted further with EtOH and concentrated to afford a crude residue that was purified by reversed-phase preparatory-HPLC (C18 stationary phase, water (0.1%TFA)-ACN to give the title compound. MS = 499.5 [M+H]⁺.

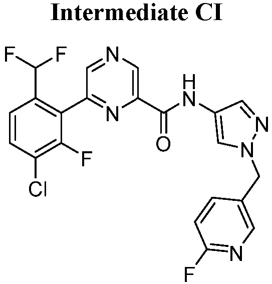

### 6-(3-Chloro-6-(difluoromethyl)-2-fluorophenyl)-N-(1-((6-fluoropyridin-3-yl)methyl)-1H-pyrazol-4-yl)pyrazine-2-carboxamide (Scheme CI)

To a solution of 6-(3-chloro-6-(difluoromethyl)-2-fluorophenyl)-N-(1H-pyrazol-4-yl)pyrazine-2-carboxamide (200 mg, 0.544 mmol) in DMF (2 mL) was added 5-(bromomethyl)-2-fluoropyridine (103 mg, 0.544 mmol) and Cs₂CO₃ (532 mg, 1.63 mmol), and the resulting mixture was stirred at for 6 h. The reaction was diluted with EtOAc and washed with brine. The organics were dried (Na₂SO₄), filtered and concentrated to afford a crude residue that was purified by silica gel chromatography (EtOAc/hexanes) to give the title compound. MS = 477.1 [M + 1]⁺.

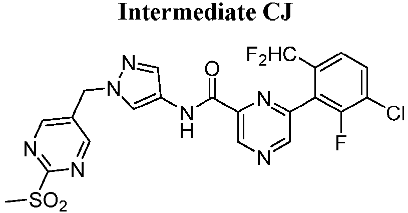

### 6-(3-Chloro-6-(difluoromethyl)-2-fluorophenyl)-N-(1-((2-(methylsulfonyl)pyrimidin-5-yl)methyl)-1H-pyrazol-4-yl)pyrazine-2-carboxamide (Scheme CJ)

Step 1. 5-(Bromomethyl)-2-(methylthio)pyrimidine: To a solution of (2-(methylthio)pyrimidin-5-yl)methanol (1.50 g, 9.60 mmol) in DCM (30 mL) at 0 °C was added carbon tetrabromide (4.14 g, 12.5 mmol) and triphenylphosphine (3.27 mg, 12.5 mmol). The resulting mixture was stirred at 0 °C for 2 h. The reaction mixture was purified directly by silica gel chromatography (EtOAc/hexanes) to give the title compound. MS = 221.0 [M + 1]⁺.

Step 2. 6-(3 -Chloro-6-(difluoromethyl)-2-fluoroi)henyl)-*N*-(1-((2-(methylthio)pyrimidin-5-yl)methyl)-1*H*-pyrazol-4-yl)pyrazine-2-carboxamide: To a solution of 6-(3-chloro-6-(difluoromethyl)-2-fluorophenyl)-N-(1H-pyrazol-4-yl)pyrazine-2-carboxamide and ammonium chloride (545 mg, 1.48 mmol) in DMF (10 mL) was added 5-(bromomethyl)-2-(methylthio)pyrimidine (325 mg, 1.48 mmol) and cesium carbonate (1.45 g, 4.45 mmol). The resulting mixture was stirred at rt for 16 h, at which time it was diluted with EtOAc and washed with water, followed by brine. The organic layer was dried (Na₂SO₄), filtered and concentrated to afford a crude residue that was purified by silica gel chromatography (EtOAc/hexanes) to give the title compound. MS = 506.2 [M + 1]⁺.

Step 3. 6-(3-Chloro-6-(difluoromethyl)-2-fluorophenyl)-*N*-(1-((2-(methylsulfonyl)pyrimidin-5-yl)methyl)-1*H*-pyrazol-4-yl)pyrazine-2-carboxamide: To a suspension of 6-(3-chloro-6-(difluoromethyl)-2-fluorophenyl)-*N*-(1-((2-(methylthio)pyrimidin-5-yl)methyl)-1H-pyrazol-4-yl)pyrazine-2-carboxamide (1.26 g, 2.49 mmol) in DCM (20 mL) was added mCPBA (1.17 g, 5.23 mmol), and the resulting mixture was stirred at rt for 2 h. The reaction was diluted with DCM and washed with 1M aq. Na₂CO₃. The organic layer was dried (Na₂SO₄), filtered and concentrated to afford a crude residue that was purified by silica gel chromatography eluting with (EtOAc:EtOH/hexanes) to give the title compound. MS = 538.2 [M + 1]⁺.

**Table CJ. The following compounds were prepared using procedures similar to those described above using the appropriate starting materials.**

| **Intermedi ate No.** | **Structure** | **Name** | **[M+H]⁺ or [M+Na]⁺** | **Chiral column** |
|---|---|---|---|---|
| CJ-1 | 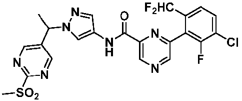 | *rac*-6-(3-Chloro-6-(difluoromethyl)-2-fluorophenyl)-*N*-(1-(1-(2-(methylsulfonyl)pyrimidin -5-yl)ethyl)-1*H*-pyrazol-4-yl)pyrazine-2-carboxamide | Calcd.: 551.9 | |
| | | | Found: 552.0 | |
| CJ-2 | 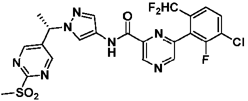 | (*S*)-6-(3-Chloro-6-(difluoromethyl)-2-fluorophenyl)-*N*-(1-(1-(2-(methylsulfonyl)pyrimidin-5-yl)ethyl)-1*H*-pyrazol-4-yl)pyrazine-2-carboxamide | Calcd.: 551.9 | CHIRALPAK AD-3 |
| | | | Found: 552.0 | |
| CJ-3 | 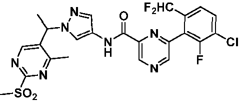 | 6-(3-Chloro-6-(difluoromethyl)-2-fluorophenyl)-*N*-(1-(1-(4-methyl-2-(methylsulfonyl)pyrimidin-5-yl)ethyl)-1*H*-pyrazol-4-yl)pyrazine-2-carboxamide | Calcd.: 565.1 | |
| | | | Found: 565.9 | |

### Examples

### Example 1

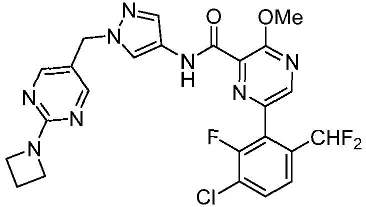

### N-(1-((2-(Azetidin-1-yl)pyrimidin-5-yl)methyl)-1H-pyrazol-4-yl)-6-(3-chloro-6-(difluoromethyl)-2-fluorophenyl)-3-methoxypyrazine-2-carboxamide (Scheme AAA)

Step 1: *N*-(1-((2-(Azetidin-1-yl)pyrimidin-5-yl)methyl)-1*H*-pyrazol-4-yl)-6-(3-chloro-6-(difluoromethyl)-2-fluorophenyl)-3-methoxypyrazine-2-carboxamide: 1-((2-(Azetidin-1-yl)pyrimidin-5-yl)methyl)-1H-pyrazol-4-amine (18 mg, 0.078 mmol) was added to a stirred solution of 6-(3-chloro-6-(difluoromethyl)-2-fluorophenyl)-3-methoxypyrazine-2-carboxylic acid (26 mg, 0.078 mmol), EDC (30 mg, 0.16 mmol) in pyridine (0.5 mL), and the resulting mixture was stirred at rt for 2 h. The reaction was purified by prep-HPLC (Method Column Agela Durashell C18 150*30 5u Condition water (0.04% NH₃H₂O+10mM NH₄HCO₃)-ACN Begin B 37 End B 67 Gradient Time(min) 10 100%B Hold Time(min) 2 FlowRate(ml/min) 25), eluting with Acetonitrile/Water + 0.05% NH₃·H₂O, to give the title compound. ¹H NMR (500 MHz, CD₃OD) δ 8.59 (d, *J= 2.7* Hz, 1H), 8.34 (s, 2H), 8.22 (s, 1H), 7.78-7.84 (m, 1H), 7.71 (s, 1H), 7.66 (d, *J* = 8.2 Hz, 1H), 6.80-7.12 (m, 1H), 5.21 (s, 2H), 4.18 (s, 3H), 4.15 (d, *J=* 7.6 Hz, 4H), 2.36-2.48 (m, 2H). MS = 545.2 (M+H). MS: 545.2 [M+H]⁺

### Example 2

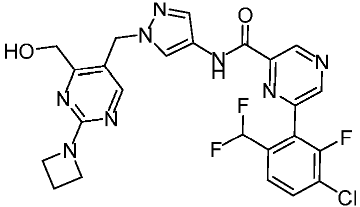

### N-(1-((2-(Azetidin-1-yl)-4-(hydroxymethyl)pyrimidin-5-yl)methyl)-1H-pyrazol-4-yl)-6-(3-chloro-6-(difluoromethyl)-2-fluorophenyl)pyrazine-2-carboxamide (Scheme AAA and deprotection)

Step 1. *N*-(1-((2-(Azetidin-1-yl)-4-(tert-butoxymethyl)pyrimidin-5-yl)methyl)-1*H-*pyrazol-4-yl)-6-(3-chloro-6-(difluoromethyl)-2-fluorophenyl)pyrazine-2-carboxamide: The title compound was prepared following procedures similar to those described above for Example 1. MS = 601.4 [M+H]⁺.

Step 2. *N*-(1-((2-(Azetidin-1-yl)-4-(hydroxymethyl)pyrimidin-5-yl)methyl)-1*H*-pyrazol-4-yl)-6-(3-chloro-6-(difluoromethyl)-2-fluorophenyl)pyrazine-2-carboxamide: The title compound was prepared following procedures similar to those described above for Intermediate BF, Step 3, in which the final compound was purified by reversed-phase preparatory-HPLC (C18 stationary phase, water (0.1%TFA) - ACN). ¹H NMR (400 MHz, CDCl₃) δ 9.44-9.52 (m, 2H), 9.04 (d, *J =* 2.9 Hz, 1H), 8.38 (s, 1H), 8.12 (s, 1H), 7.67-7.76 (m, 1H), 7.58-7.66 (m, 2H), 6.40-6.77 (m, 1H), 5.19 (s, 2H), 4.63 (s, 2H), 4.36 (br t, *J =* 7.6 Hz, 4H), 2.49 (quint, *J =* 7.6 Hz, 2H). MS = 545.1 [M+H]⁺.

### Example 3

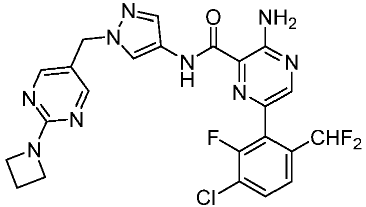

### 3-Amino-N-(1-((2-fazetidin-1-yl)pyrimidin-5-yl)methyl)-1H-pyrazol-4-yl)-6-(3-chloro-6-(difluoromethyl)-2-fluorophenyl)pyrazine-2-carboxamide

The title compound was prepared following procedures similar to those described above for Intermediate BF, Step 3, in which the final compound was purified by reversed-phase preparatory-HPLC (C18 stationary phase, water (0.1%TFA) - ACN). MS = 530.2 [M + H]⁺. ¹H NMR (400 MHz, DMSO-*d*₆) δ 10.48 (s, 1H), 8.43 (d, *J*= 2.4 Hz, 1H), 8.34 (s, 2H), 8.14 (s, 1H), 7.90 - 7.77 (m, 1H), 7.71 - 7.54 (m, 2H), 7.18 (t, *J =* 54.6 Hz, 1H), 5.13 (s, 2H), 3.99 (t, *J =* 7.6, 4H), 2.27 (p, *J =* 7.6 Hz, 2H).

### Examples 4 and 5

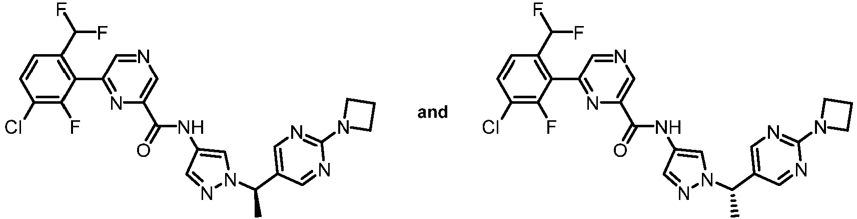

### (R)-N-(1-(1-(2-(Azetidin-1-yl)pyrimidin-5-yl)ethyl)-1H-pyrazol-4-yl)-6-(3-chloro-6-(difluoromethyl)-2-fluorophenyl)pyrazine-2-carboxamide and (S)-N-(1-(1-(2-(Azetidin-1-yl)pyrimidin-5-yl)ethyl)-1H-pyrazol-4-yl)-6-(3-chloro-6-(difluoromethyl)-2-fluorophenyl)pyrazine-2-carboxamide (Scheme AAA)

Step 1: *N*-(1-(1-(2-(Azetidin-1-yl)pyrimidin-5-yl)ethyl)-1*H*-pyrazol-4-yl)-6-(3-chloro-6-(difluoromethyl)-2-fluorophenyl)pyrazine-2-carboxamide: To a solution of 1-(1-(2-(azetidin-1-yl)pyrimidin-5-yl)ethyl)-1*H*-pyrazol-4-amine (160 mg, 0.655 mmol) and 6-(3-chloro-6-(difluoromethyl)-2-fluorophenyl)pyrazine-2-carboxylic acid (198 mg, 0.655 mmol) in pyridine (1.5 mL) was added EDC (251 mg, 1.310 mmol) at 0 °C, and the resulting mixture was warmed to rt for 2 h. The reaction was diluted with water and extracted with EtOAc. The combined organic layers were dried over Na₂SO₄, filtered, and concentrated. The residue was purified by reverse prep-HPLC (Instrument EB Method Column Phenomenex Synergi C18 150*30mm*4um Condition water(0.1%TFA)-ACN Begin B 33 End B 53 Gradient Time(min) 10 100%B Hold Time(min) 2 FlowRate(ml/min) 25 Injections 5 HPLC 3) to the title compound as a mixture of enantiomers, which were separated by SFC (Instrument SFC-80 Method Column YMC CHIRAL Amylose-C (250mm*30mm, 10um) Condition 0.1%NH₃H₂O MeOH Begin B 55% End B 55% Gradient Time(min) 100%B), the first eluted isomer **Example 4:** ¹H NMR (400 MHz, CD₃OD) δ 9.38 (s, 1H), 9.01 (d, *J =* 1.8 Hz, 1H), 8.36 (s, 2H), 8.28 (s, 1H), 7.84 (t, *J =* 7.9 Hz, 1H), 7.77 (s, 1H), 7.67 (d, *J =* 7.9 Hz, 1H), 6.87 (t, *J =* 54.6 Hz, 1H), 5.54 (q, *J =* 6.9 Hz, 1H), 4.20 (t, *J*= 7.5 Hz, 4H), 2.40 - 2.49 (m, 2H), 1.87 (d, *J =* 7.0 Hz, 3H), MS = 529.1 (M+H); the second eluted isomer **Example 5:** ¹H NMR (400 MHz, CD₃OD) δ 9.38 (s, 1H), 9.02 (d, *J* = 1.8 Hz, 1H), 8.36 (s, 2H), 8.28 (s, 1H), 7.84 (t, *J =* 7.9 Hz, 1H), 7.77 (s, 1H), 7.67 (d, *J =* 7.9 Hz, 1H), 6.87 (t, *J =* 54.6 Hz, 1H), 5.54 (q, *J =* 6.9 Hz, 1H), 4.20 (t, *J =* 7.5 Hz, 4H), 2.40 - 2.49 (m, 2H), 1.87 (d, *J =* 7.0 Hz, 3H). MS = 529.1 (M+H).

### Examples 6 and 7

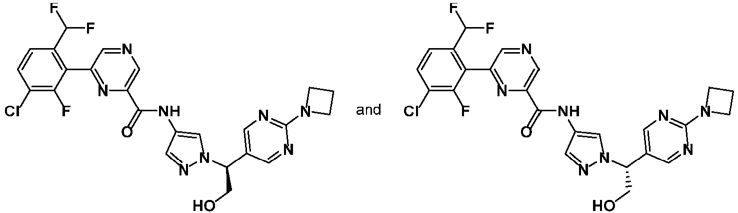

### (R)-N-(1-(1-(2-(Azetidin-1-yl)pyrimidin-5-yl)-2-hydroxyethyl)-1H-pyrazol-4-yl)-6-(3-chloro-6-(difluoromethyl)-2-fluorophenyl)pyrazine-2-carboxamide and (S)-N-(1-(1-(2-(Azetidin-1-yl)pyrimidin-5-yl)-2-hydroxyethyl)-1H-pyrazol-4-yl)-6-(3-chloro-6-(difluoromethyl)-2-fluorophenyl)pyrazine-2-carboxamide (Scheme AAA and deprotection)

Step 1. *N*-(1-(1-(2-(Azetidin-1-yl)pyrimidin-5-yl)-2-((*tert*-butyldiphenylsilyl)oxy)ethyl)-1*H*-pyrazol-4-yl)-6-(3-chloro-6-(difluoromethyl)-2-fluorophenyl)pyrazine-2-carboxamide: Pyridine (0.39 mL, 4.8 mmol) was added to a stirred solution of 1-(1-(2-(azetidin-1-yl)pyrimidin-5-yl)-2-((*tert*-butyldiphenylsilyl)oxy)ethyl)-1*H*-pyrazol-4-amine (600 mg, 1.20 mmol), 6-(3-chloro-6-(difluoromethyl)-2-fluorophenyl)pyrazine-2-carboxylic acid (546 mg, 1.80 mmol) and EDC (692 mg, 3.61 mmol) in DCM (10 mL), and the mixture was stirred at 30 °C for 1 h. The reaction was concentrated to afford a crude residue that was purified by reversed-phase preparatory-HPLC (C18 stationary phase, water (0.1%TFA) - ACN) to give the title compound. MS = 783.3 [M+H]⁺.

Step 2. *N*-(1-(1-(2-(Azetidin-1-yl)pyrimidin-5-yl)-2-hydroxyethyl)-1*H*-pyrazol-4-yl)-6-(3-chloro-6-(difluoromethyl)-2-fluorophenyl)pyrazine-2-carboxamide: TBAF (0.13 mL, 0.13 mmol, 1M in THF) was added to a stirred solution of N-(1-(1-(2-(azetidin-1-yl)pyrimidin-5-yl)-2-((*tert*-butyldiphenylsilyl)oxy)ethyl)-1H-pyrazol-4-yl)-6-(3-chloro-6-(difluoromethyl)-2-fluorophenyl)pyrazine-2-carboxamide (50 mg, 0.064 mmol) in THF (2 mL), and the resulting mixture was heated to 35 °C for 1 h. The reaction was purified by reversed-phase preparatory-HPLC (C18 stationary phase, water (0.1%TFA) - ACN) to give the title compound as a racemic mixture that was separated by chiral SFC chromatography (SFC, DAICEL CHIRALCEL OD; 40% Base-EtOH (contained 0.1% NH₃H₂O) in CO₂) to give the title compounds. The first eluted isomer **Example 6:** ¹H NMR (400 MHz, CDCl₃) δ 9.36-9.50 (m, 2H), 8.98 (br s, 1H), 8.43 (br s, 2H), 8.09 (br s, 1H), 7.54-7.69 (m, 3H), 6.52 (br t, *J =* 54.2 Hz, 1H), 5.22 (br s, 1H), 4.23-4.32 (m, 4H), 4.20 (br s, 2H), 2.42 (br s, 2H), MS = 545.2 (M+H); the second eluted isomer **Example 7:** ¹H NMR (400 MHz, CDCl₃) δ 9.43 (s, 2H), 8.99 (d, *J =* 2.7 Hz, 1H), 8.44 (s, 2H), 8.09 (s, 1H), 7.57-7.69 (m, 3H), 6.38-6.68 (m, 1H), 5.22 (br t, *J =* 4.6 Hz, 1H), 4.28 (t, *J =* 7.7 Hz, 4H), 4.15-4.23 (m, 2H), 2.43 (q, *J =* 7.7 Hz, 2H), MS = 545.1 [M+H]⁺.

**Table AAA. The following compounds were prepared using procedures similar to those described above using the appropriate starting materials.**

| **Example** | **Structure** | **Name** | **[M+H]⁺ or [M+Na]⁺** | **Chiral Column** |
|---|---|---|---|---|
| **8** | 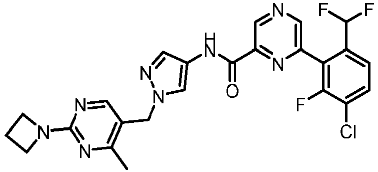 | *N*-(1-((2-(Azetidin-1-yl)-4-methylpyrimidin-5-yl)methyl)-1*H*-pyrazol-4-yl)-6-(3-chloro-6-(difluoromethyl)-2-fluorophenyl)pyrazine-2-carboxamide | Calcd.: 529.1 | |
| | | | Found: 529.2 | |
| **9** | 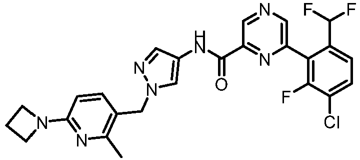 | *N*-(1-((6-(Azetidin-1-yl)-2-methylpyridin-3-yl)methyl)-1*H*-pyrazol-4-yl)-6-(3-chloro-6-(difluoromethyl)-2-fluorophenyl)pyrazine-2-carboxamide | Calcd.: 528.1 | |
| | | | Found: 528.4 | |
| **10** | 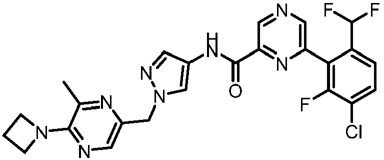 | *N*-(1-((5-(Azetidin-1-yl)-6-methylpyrazin-2-yl)methyl)-1*H*-pyrazol-4-yl)-6-(3-chloro-6-(difluoromethyl)-2-fluorophenyl)pyrazine-2-carboxamide | Calcd.: 529.1 | |
| | | | Found: 529.5 | |
| **11** | 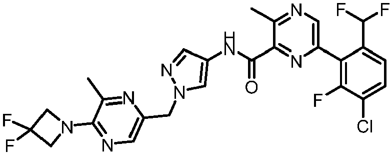 | 6-(3-Chloro-6-(difluoromethyl)-2-fluorophenyl)-*N*-(1-((5-(3,3-difluoroazetidin-1-yl)-6-methylpyrazin-2-yl)methyl)-1*H*-pyrazol-4-yl)-3-methylpyrazine-2-carboxamide | Calcd.: 579.1 | |
| | | | Found: 579.3 | |
| **12** | 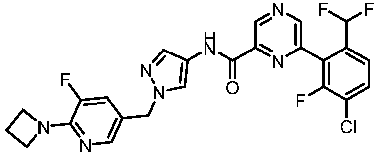 | *N*-(1-((6-(Azetidin-1-yl)-5-fluoropyridin-3-yl)methyl)-1*H*-pyrazol-4-yl)-6-(3-chloro-6-(difluoromethyl)-2-fluorophenyl)pyrazine-2-carboxamide | Calcd.: 532.1 | |
| | | | Found: 532.3 | |
| **13** | 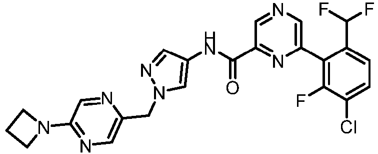 | *N*-(1-((5-(Azetidin-1-yl)pyrazin-2-yl)methyl)-1*H*-pyrazol-4-yl)-6-(3-chloro-6-(difluoromethyl)-2-fluorophenyl)pyrazine-2-carboxamide | Calcd.: 515.1 | |
| | | | Found: 515.3 | |
| **14** | 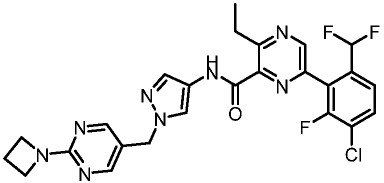 | *N*-(1-((2-(Azetidin-1-yl)pyrimidin-5-yl)methyl)-1*H*-pyrazol-4-yl)-6-(3-chloro-6-(difluoromethyl)-2-fluorophenyl)-3-ethylpyrazine-2-carboxamide | Calcd.: 543.2 | |
| | | | Found: 543.2 | |
| **15** | 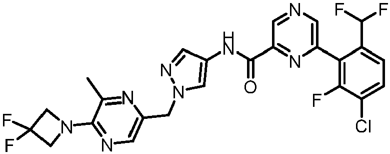 | 6-(3-Chloro-6-(difluoromethyl)-2-fluorophenyl)-*N*-(1-((5-(3,3-difluoroazetidin-1-yl)-6-methylpyrazin-2-yl)methyl)-1*H*-pyrazol-4-yl)pyrazine-2-carboxamide | Calcd.: 565.1 | |
| | | | Found: 565 | |
| **16** | 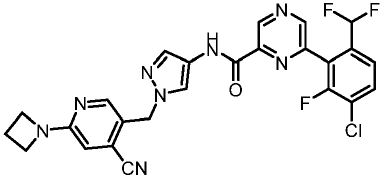 | *N*-(1-((6-(Azetidin-1-yl)-4-cyanopyridin-3-yl)methyl)-1*H*-pyrazol-4-yl)-6-(3-chloro-6-(difluoromethyl)-2-fluorophenyl)pyrazine-2-carboxamide | Calcd.: 539.1 | |
| | | | Found: 539.1 | |
| **17** | 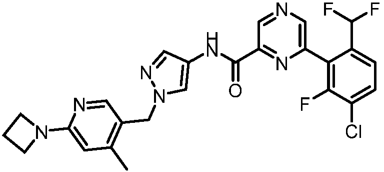 | *N*-(1-((6-(Azetidin-1-yl)-4-methylpyridin-3-yl)methyl)-1*H*-pyrazol-4-yl)-6-(3-chloro-6-(difluoromethyl)-2-fluorophenyl)pyrazine-2-carboxamide | Calcd.: 528.1 | |
| | | | Found: 528.4 | |
| **18** | 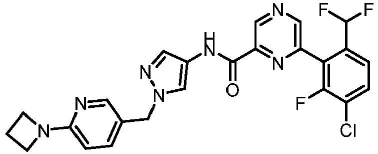 | *N*-(1-((6-(Azetidin-1-yl)pyridin-3-yl)methyl)-1*H*-pyrazol-4-yl)-6-(3-chloro-6-(difluoromethyl)-2-fluorophenyl)pyrazine-2-carboxamide | Calcd.: 514.1 | |
| | | | Found: 514.3 | |
| **19** | 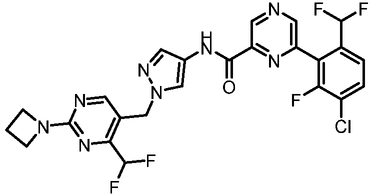 | *N*-(1-((2-(Azetidin-1-yl)-4-(difluoromethyl)pyrimidi n-5-yl)methyl)-1*H-*pyrazol-4-yl)-6-(3-chloro-6-(difluoromethyl)-2-fluorophenyl)pyrazine-2-carboxamide | Calcd.: 565.1 | |
| | | | Found: 565.3 | |
| **20** | 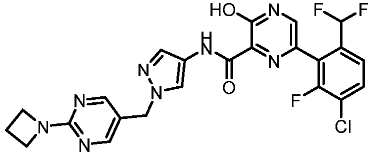 | *N*-(1-((2-(Azetidin-1-yl)pyrimidin-5-yl)methyl)-1*H*-pyrazol-4-yl)-6-(3-chloro-6-(difluoromethyl)-2-fluorophenyl)-3-hydroxypyrazine-2-carboxamide | Calcd.: 531.1 | |
| | | | Found: 531.2 | |
| **21** | 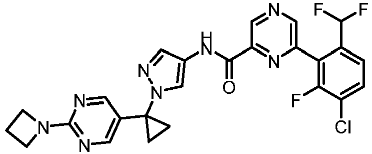 | *N*-(1-(1-(2-(Azetidin-1-yl)pyrimidin-5-yl)cyclopropyl)-1*H-*pyrazol-4-yl)-6-(3-chloro-6-(difluoromethyl)-2-fluorophenyl)pyrazine-2-carboxamide | Calcd.: 541.1 | |
| | | | Found: 541.3 | |
| **22** | 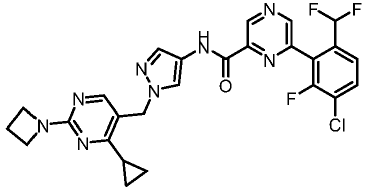 | *N*-(1-((2-(Azetidin-1-yl)-4-cyclopropylpyrimidin-5-yl)methyl)-1*H*-pyrazol-4-yl)-6-(3-chloro-6-(difluoromethyl)-2-fluorophenyl)pyrazine-2-carboxamide | Calcd.: 555.2 | |
| | | | Found: 555.3 | |
| **23** | 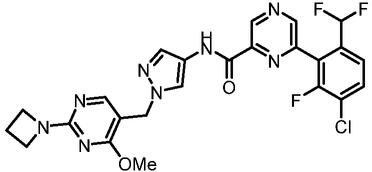 | *N*-(1-((2-(Azetidin-1-yl)-4-methoxypyrimidin-5-yl)methyl)-1*H*-pyrazol-4-yl)-6-(3-chloro-6-(difluoromethyl)-2-fluorophenyl)pyrazine-2-carboxamide | Calcd.: 545.1 | |
| | | | Found: 545.1 | |
| **24** | 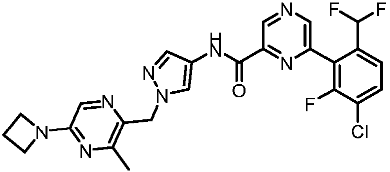 | *N*-(1-((5-(Azetidin-1-yl)-3-methylpyrazin-2-yl)methyl)-1*H*-pyrazol-4-yl)-6-(3-chloro-6-(difluoromethyl)-2-fluorophenyl)pyrazine-2-carboxamide | Calcd.: 529.1 | |
| | | | Found: 529.4 | |
| **25** | 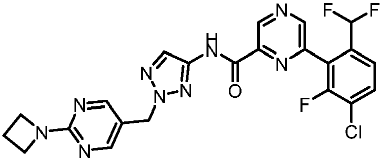 | *N*-(2-((2-(Azetidin-1-yl)pyrimidin-5-yl)methyl)-2*H*-1,2,3-triazol-4-yl)-6-(3-chloro-6-(difluoromethyl)-2-fluorophenyl)pyrazine-2-carboxamide | Calcd.: 516.1 | |
| | | | Found: 516.2 | |
| **26** | 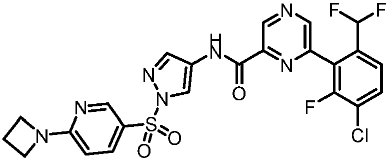 | *N*-(1-((6-(Azetidin-1-yl)pyridin-3-yl)sulfonyl)-1*H*-pyrazol-4-yl)-6-(3-chloro-6-(difluoromethyl)-2-fluorophenyl)pyrazine-2-carboxamide | Calcd.: 564.1 | |
| | | | Found: 564.2 | |
| **27** | 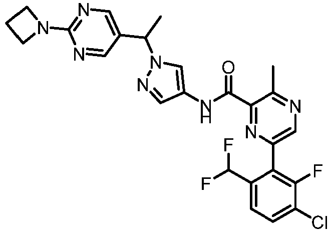 | (*S* or *R*)-N-(1-(1-(2-(azetidin-1-yl)pyrimidin-5-yl)ethyl)-1*H*-pyrazol-4-yl)-6-(3-chloro-6-(difluoromethyl)-2-fluorophenyl)-3-methylpyrazine-2-carboxamide | Calcd.: 543.2 | DAICEL CHIRALCEL OD-H |
| | | | Found: 542.9 | |
| **28** | 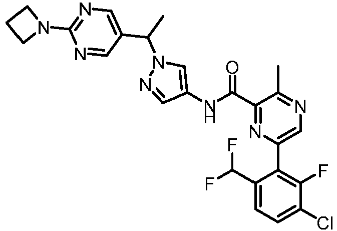 | *(R* or *S)-N-(1-(1-(2-*(azetidin-1-yl)pyrimidin-5-yl)ethyl)-1*H*-pyrazol-4-yl)-6-(3-chloro-6-(difluoromethyl)-2-fluorophenyl)-3-methylpyrazine-2-carboxamide | Calcd.: 543.2 | DAICEL CHIRALCEL OD-H |
| | | | Found: 542.9 | |
| **29** | 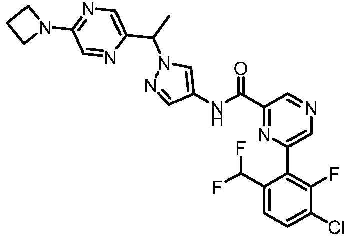 | (*S* or *R*)-N-(1-(1-(5-(azetidin-1-yl)pyrazin-2-yl)ethyl)-1*H-*pyrazol-4-yl)-6-(3-chloro-6-(difluoromethyl)-2-fluorophenyl)pyrazine-2-carboxamide | Calcd.: 529.1 | DAICEL CHIRALCEL OD-H |
| | | | Found: 529.4 | |
| **30** | 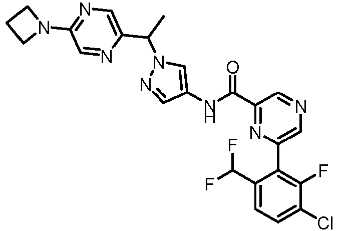 | *(R* or *S)-N-*(1-(1-(5-(azetidin-1-yl)pyrazin-2-yl)ethyl)-1*H*-pyrazol-4-yl)-6-(3-chloro-6-(difluoromethyl)-2-fluorophenyl)pyrazine-2-carboxamide | Calcd.: 529.1 | DAICEL CHIRALCEL OD-H |
| | | | Found: 529.4 | |
| **31** | 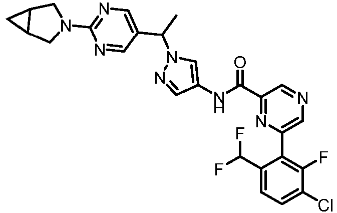 | *N*-(1-((*1S* or *1R*)-1-(2-(3-azabicyclo[3.1.0]hexan-3-yl)pyrimidin-5-yl)ethyl)-1*H-*pyrazol-4-yl)-6-(3-chloro-6-(difluoromethyl)-2-fluorophenyl)pyrazine-2-carboxamide | Calcd.: 555.2 | DAICEL YMC CHIRAL Amylose-C |
| | | | Found: 555.0 | |
| **32** | 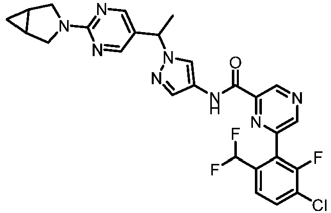 | *N*-(1-((*1R* or *1S*)-1-(2-(3-azabicyclo[3.1.0]hexan-3-yl)pyrimidin-5-yl)ethyl)-1*H-*pyrazol-4-yl)-6-(3-chloro-6-(difluoromethyl)-2-fluorophenyl)pyrazine-2-carboxamide | Calcd.: 555.2 | DAICEL YMC CHIRAL Amylose-C |
| | | | Found: 555.0 | |
| **33** | 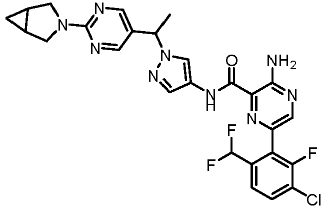 | *N*-(1-((*1S* or *1R*)-1-(2-(3-azabicyclo[3.1.0]hexan-3-yl)pyrimidin-5-yl)ethyl)-1*H*-pyrazol-4-yl)-3-amino-6-(3-chloro-6-(difluoromethyl)-2-fluorophenyl)pyrazine-2-carboxamide | Calcd.: 570.2 | REGIS (s,s) WHELK-O1 |
| | | | Found: 570.4 | |
| **34** | 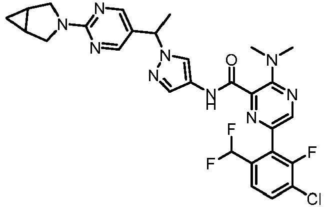 | *N*-(1-((*1R* or *1S*)-1-(2-(3-azabicyclo[3.1.0]hexan-3-yl)pyrimidin-5-yl)ethyl)-1*H*-pyrazol-4-yl)-6-(3-chloro-6-(difluoromethyl)-2-fluorophenyl)-3-(dimethylamino)pyrazine -2-carboxamide | Calcd.: 598.2 | DAICEL CHIRALCEL OD-H |
| | | | Found: 598.4 | |
| **35** | 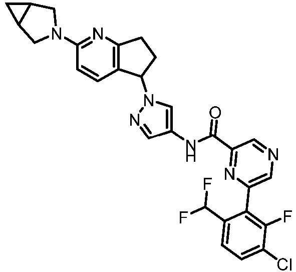 | (*S* or *R*)*-N*-(1-(2-(azetidin-1-yl)-6,7-dihydro-5*H-*cyclopenta[b]pyridin-5-yl)-1*H*-pyrazol-4-yl)-6-(3-chloro-6-(difluoromethyl)-2-fluorophenyl)pyrazine-2-carboxamide | Calcd.: 540.1 | AS-H |
| | | | Found: 540.1 | |
| **36** | 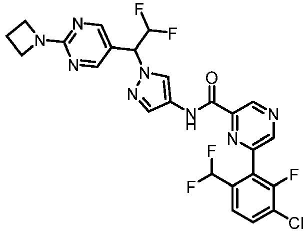 | (*S* or *R*)-*N*-(1-(1-(2-(azetidin-1-yl)pyrimidin-5-yl)-2,2-difluoroethyl)-1*H*-pyrazol-4-yl)-6-(3-chloro-6-(difluoromethyl)-2-fluorophenyl)pyrazine-2-carboxamide | Calcd.: 565.1 | DAICEL CHIRALPAK AD-H |
| | | | Found: 565.1 | |
| **37** | 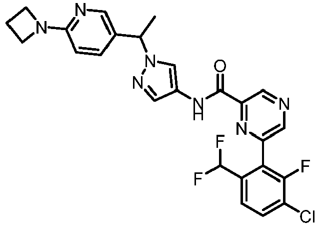 | (*S* or *R*)-*N*-(1-(1-(6-(azetidin-1-yl)pyridin-3-yl)ethyl)-1*H*-pyrazol-4-yl)-6-(3-chloro-6-(difluoromethyl)-2-fluorophenyl)pyrazine-2-carboxamide | Calcd.: 528.1 | DAICEL CHIRALPAK AD-H |
| | | | Found: 528.3 | |
| **38** | 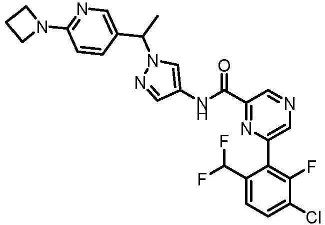 | *(R* or *S*)-*N-*(1-(1-(6-(azetidin-1-yl)pyridin-3-yl)ethyl)-1*H*-pyrazol-4-yl)-6-(3-chloro-6-(difluoromethyl)-2-fluorophenyl)pyrazine-2-carboxamide | Calcd.: 528.1 | DAICEL CHIRALPAK AD-H |
| | | | Found: 528.3 | |
| **39** | 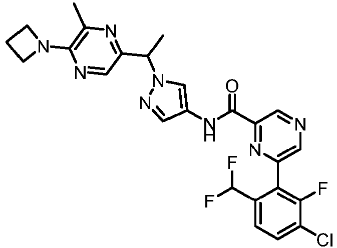 | (*S* or *R*)-*N*-(1-(1-(5-(azetidin-1-yl)-6-methylpyrazin-2-yl)ethyl)-1*H*-pyrazol-4-yl)-6-(3-chloro-6-(difluoromethyl)-2-fluorophenyl)pyrazine-2-carboxamide | Calcd.: 543.2 | WHELK-01 (R,R) |
| | | | Found: 543.0 | |
| **40** | 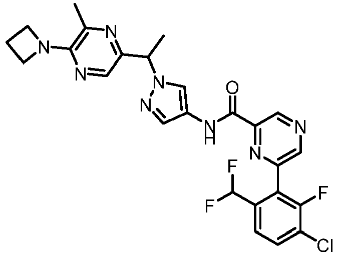 | *(R* or *S*)-*N*-(1-(1-(5-(azetidin-1-yl)-6-methylpyrazin-2-yl)ethyl)-1*H*-pyrazol-4-yl)-6-(3-chloro-6-(difluoromethyl)-2-fluorophenyl)pyrazine-2-carboxamide | Calcd.: 543.2 | WHELK-01 (R,R) |
| | | | Found: 543.0 | |
| **41** | 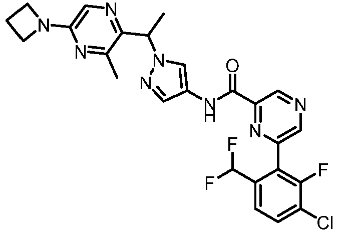 | (*S* or *R*)-*N*-(1-(1-(5-(azetidin-1-yl)-3-methylpyrazin-2-yl)ethyl)-1*H*-pyrazol-4-yl)-6-(3-chloro-6-(difluoromethyl)-2-fluorophenyl)pyrazine-2-carboxamide | Calcd.: 543.2 | OD-H |
| | | | Found: 543 | |
| **42** | 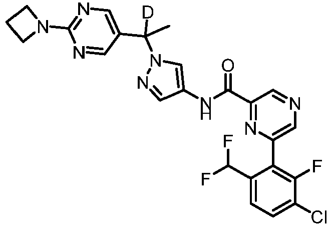 | (*S* or *R*)-*N*-(1-(1-(2-(azetidin-1-yl)pyrimidin-5-yl)ethyl-1-d)-1*H-*pyrazol-4-yl)-6-(3-chloro-6-(difluoromethyl)-2-fluorophenyl)pyrazine-2-carboxamide | Calcd.: 530.2 | WHELK 01 (R,R) |
| | | | Found: 530.0 | |
| **43** | 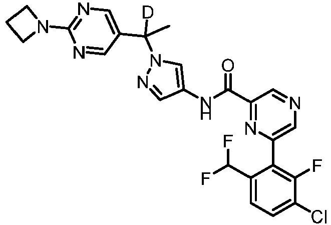 | *(R* or *S*)-*N*-(1-(1-(2-(azetidin-1-yl)pyrimidin-5-yl)ethyl-1-d)-1*H-*pyrazol-4-yl)-6-(3-chloro-6-(difluoromethyl)-2-fluorophenyl)pyrazine-2-carboxamide | Calcd.: 530.2 | WHELK 01 (R,R) |
| | | | Found: 530.0 | |
| **44** | 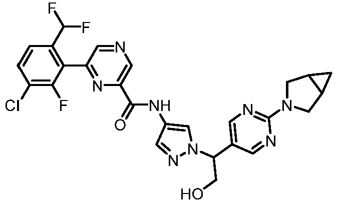 | *N-(1-*((*S* or *R*)-1-(2-(3-Azabicyclo[3.1.0]hexan-3-yl)pyrimidin-5-yl)-2-hydroxyethyl)-1*H-*pyrazol-4-yl)-6-(3-chloro-6-(difluoromethyl)-2-fluorophenyl)pyrazine-2-carboxamide | Calcd.: 571.2 | DAICEL CHIRALPAK IC |
| | | | Found: 571.2 | |
| **45** | 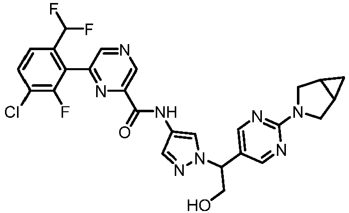 | *N-(1-((R* or *S*)-1-(2-(3-Azabicyclo[3.1.0]hexan-3-yl)pyrimidin-5-yl)-2-hydroxyethyl)-1*H-*pyrazol-4-yl)-6-(3-chloro-6-(difluoromethyl)-2-fluorophenyl)pyrazine-2-carboxamide | Calcd.: 571.2 | DAICEL CHIRALPAK IC |
| | | | Found: 571.2 | |
| **46** | 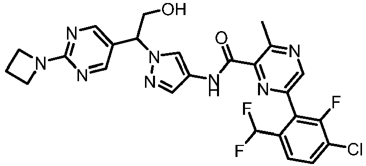 | (*S* or *R*)-*N*-(1-(1-(2-(Azetidin-1-yl)pyrimidin-5-yl)-2-hydroxyethyl)-1*H-*pyrazol-4-yl)-6-(3-chloro-6-(difluoromethyl)-2-fluorophenyl)-3-methylpyrazine-2-carboxamide | Calcd.: 559.2 | DAICEL CHIRALCEL OD-H |
| | | | Found: 559.2 | |
| **47** | 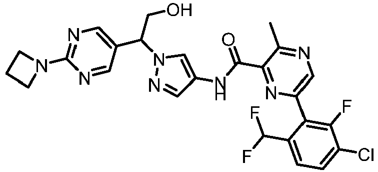 | *(R* or *S*)-*N*-(1-(1-(2-(Azetidin-1-yl)pyrimidin-5-yl)-2-hydroxyethyl)-1*H-*pyrazol-4-yl)-6-(3-chloro-6-(difluoromethyl)-2-fluorophenyl)-3-methylpyrazine-2-carboxamide | Calcd.: 559.2 | DAICEL CHIRALCEL OD-H |
| | | | Found: 559.2 | |
| **48** | 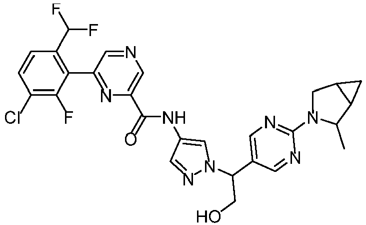 | 6-(3-Chloro-6-(difluoromethyl)-2-fluorophenyl)-*N*-(1-((S or *R*)-2-hydroxy-1-(2-*((1S,2R,5R* or 1*R,* 2S, 5S)-2-methyl-3-azabicyclo[3.1.0]hexan-3-yl)pyrimidin-5-yl)ethyl)-1*H*-pyrazol-4-yl)pyrazine-2-carboxamide | Calcd.: 585.2 | DAICEL CHIRALPAK AD |
| | | | Found: 585.0 | |
| **49** | 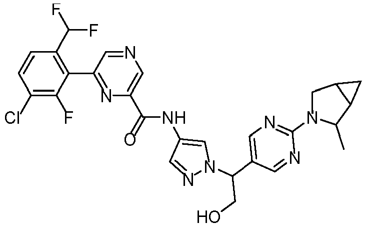 | 6-(3-Chloro-6-(difluoromethyl)-2-fluorophenyl)-*N*-(1-((*R* or S)-2-hydroxy-1-(2-((1*S*,2*R*,*5R* or 1*R,* 2S, 5S)-2-methyl-3-azabicyclo[3.1.0]hexan- | Calcd.: 585.2 | DAICEL CHIRALPAK AD |
| | | | Found: 585.0 | |
| | | 3-yl)pyrimidin-5-yl)ethyl)-1*H*-pyrazol-4-yl)pyrazine-2-carboxamide | | |
| **50** | 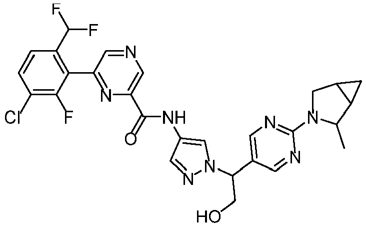 | 6-(3-Chloro-6-(difluoromethyl)-2-fluorophenyl)-*N*-(1-((S or *R*)-2-hydroxy-1-(2-*((1R,2S,5S* or 1S, 2*R,* 5*R*)-2-methyl-3-azabicyclo[3.1.0]hexan-3-yl)pyrimidin-5-yl)ethyl)-1*H*-pyrazol-4-yl)pyrazine-2-carboxamide | Calcd.: 585.2 | DAICEL CHIRALPAK AD |
| | | | Found: 585.2 | |
| **51** | 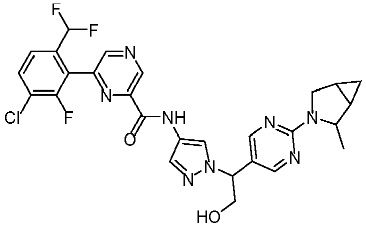 | 6-(3-Chloro-6-(difluoromethyl)-2-fluorophenyl)-*N*-(1-((*R* or *S*)-2-hydroxy-1-(2-*((1R,2S,5S* or 1S, 2*R,* 5*R*)-2-methyl-3-azabicyclo[3.1.0]hexan-3-yl)pyrimidin-5-yl)ethyl)-1*H*-pyrazol-4-yl)pyrazine-2-carboxamide | Calcd.: 585.2 | Phenomenex-Cellulose-2 |
| | | | Found: 585.1 | |

### Example 52

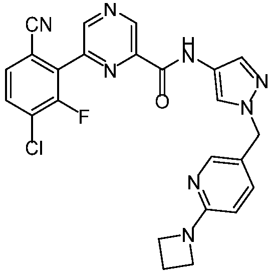

### N-(1-((6-(Azetidin-1-yl)pyridin-3-yl)methyl)-1H-pyrazol-4-yl)-6-(3-chloro-6-cyano-2-fluorophenyl)pyrazine-2-carboxamide (Scheme AAB)

Step 1. Methyl 6-(3-chloro-6-cyano-2-fluorophenyl)pyrazine-2-carboxylate: To a solution of 4-chloro-3-fluoro-2-iodobenzonitrile (500 mg, 1.78 mmol) in 1,4-dioxane (10 mL) were added methyl 6-(trimethylstannyl)pyrazine-2-carboxylate (1.07 g, 3.55 mmol), tris(dibenzylideneacetone)dipalladium(0) (163 mg, 0.178 mmol) and 1,1'-bis(di-*tert-*butylphosphino)ferrocene (84 mg, 0.18 mmol) in a glove box. The resulting mixture was heated at 120 °C for 12 h under N₂. The reaction was cooled to rt, concentrated and purified by silica gel chromatography (EtOAc/hexanes) to give the title compound. MS = 291.9 [M+H]⁺.

Step 2. *N*-(1-((6-(Azetidin-1-yl)pyridin-3-yl)methyl)-1*H*-pyrazol-4-yl)-6-(3-chloro-6-cyano-2-fluorophenyl)pyrazine-2-carboxamide: To a solution of 1-((6-(azetidin-1-yl)pyridin-3-yl)methyl)-1*H*-pyrazol-4-amine (79 mg, 0.34 mmol) in toluene (5 mL) was added trimethylaluminum (0.21 mL, 0.43 mmol, 2M in toluene) at rt. After 10 min, a solution of 1-((6-(azetidin-1-yl)pyridin-3-yl)methyl)-1*H*-pyrazol-4-amine (79 mg, 0.34 mmol) in DCM (2 mL) was added dropwise, and the resulting mixture was heated to 80 °C for 12 h. The reaction was concentrated and the resulting crude residue was purified by reversed-phase preparatory-HPLC (C18 stationary phase, water-ACN (0.05% TFA) to give the title compound. ¹H NMR (500 MHz, CD₃OD) δ 9.45 (s, 1H), 9.28 (d, *J =* 2.9 Hz, 1H), 8.34 (s, 1H), 7.92-7.96 (m, 1H), 7.85-7.90 (m, 3H), 7.80 (s, 1H), 6.82 (d, *J* = 9.3 Hz, 1H), 5.30 (s, 2H), 4.34 (t, *J =* 7.7 Hz, 4H), 2.59 (q, *J =* 7.7 Hz, 2H). MS = 489.1 [M+H]⁺.

**Table AAB. The following compound was prepared using procedures similar to those described above using the appropriate starting materials.**

| **Example** | **Structure** | **Name** | **[M+H]⁺** |
|---|---|---|---|
| **53** | 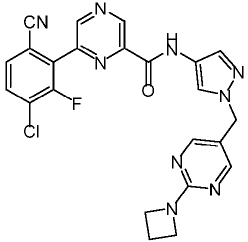 | *N*-(1-((2-(Azetidin-1-yl)pyrimidin-5-yl)methyl)-1*H-*pyrazol-4-yl)-6-(3-chloro-6-cyano-2-fluorophenyl)pyrazi ne-2-carboxamide | Calcd.: 490.1 |
| | | | Found: 490.1 |

### Examples 54 and 55

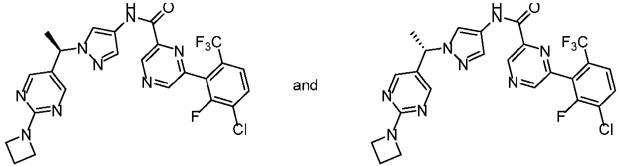

### (R)-N-(1-(1-(2-(Azetidin-1-yl)pyrimidin-5-yl)ethyl)-1H-pyrazol-4-yl)-6-(3-chloro-2-fluoro-6-(trifluoromethyl)phenyl)pyrazine-2-carboxamide and (S)-N-(1-(1-(2-(Azetidin-1-yl)pyrimidin-5-yl)ethyl)-1H-pyrazol-4-yl)-6-(3-chloro-2-fluoro-6-(trifluoromethyl)phenyl)pyrazine-2-carboxamide (Scheme AAC)

Step 1. *N*-(1-(1-(2-(Azetidin-1-yl)pyrimidin-5-yl)ethyl)-1*H*-pyrazol-4-yl)-6-(trimethylstannyl)-pyrazine-2-carboxamide: A solution of N-(1-(1-(2-(azetidin-1-yl)pyrimidin-5-yl)ethyl)-1H-pyrazol-4-yl)-6-bromopyrazine-2-carboxamide (1.00 g, 2.33 mmol), 1,1,1,2,2,2-hexamethyldistannane (1.09 g, 3.33 mmol) and tetrakis(triphenylphosphine)palladium(0) (135 mg, 0.116 mmol) in toluene (20 mL) was heated at 80 °C for 16 h under an N₂ atmosphere. The reaction was cooled to rt and concentrated to afford a crude residue that was purified by silica gel chromatography (petroleum ether) to give the title compound. MS = 515.1 [M+H]⁺.

Step 2. (*R*)-*N*-(1-(1-(2-(Azetidin-1-yl)pyrimidin-5-yl)ethyl)-1*H*-pyrazol-4-yl)-6-(3-chloro-2-fluoro-6-(trifluoromethyl)phenyl)pyrazine-2-carboxamide and (*S*)-*N*-(1-(1-(2-(Azetidin-1-yl)pyrimidin-5-yl)ethyl)-1*H*-pyrazol-4-yl)-6-(3-chloro-2-fluoro-6-(trifluoromethyl)phenyl)pyrazine-2-carboxamide: To a solution of 1-chloro-2-fluoro-3-iodo-4-(trifluoromethyl)benzene (60 mg, 0.19 mmol) in 1,4-dioxane (2 mL) was added N-(1-(1-(2-(azetidin-1-yl)pyrimidin-5-yl)ethyl)-1*H*-pyrazol-4-yl)-6-(trimethyl-stannyl)pyrazine-2-carboxamide (100 mg, 0.175 mmol), tris(dibenzylideneacetone)dipalladium(0) (16 mg, 0.018 mmol) and 1,1'-bis(di-*tert*-butylphosphino)ferrocene (8.3 mg, 0.018 mmol), and the resulting mixture was heated to 110 °C for 15 h under an N₂ atmosphere. The reaction was cooled to rt, filtered, concentrated to afford a crude residue that was purified by reversed-phase preparatory-HPLC (C18 stationary phase, water-ACN (0.05% NH₃) to yield a racemic mixture that was separated by chiral chromatography (SFC, DAICEL CHIRALCEL OD-H; 0.1%NH₃H₂O-EtOH, Begin B 30%, End B 30%) to give the title compounds. The faster-eluting isomer **Example 54:** ¹H NMR (400 MHz, CDCl₃) δ 9.51 (s, 1H), 9.43 (s, 1H), 8.89 (s, 1H), 8.26 (s, 2H), 8.14 (s, 1H), 7.70-7.77 (m, 1H), 7.62-7.68 (m, 1H), 7.58 (s, 1H), 5.36 (q, *J =* 7.1 Hz, 1H), 4.15 (t, *J =* 7.6 Hz, 4H), 2.38 (quint, *J =* 7.5 Hz, 2H), 1.89 (d, *J =* 7.1 Hz, 3H), MS = 547.2 [M+H]⁺; the slower-eluting isomer **Example 55:** ¹H NMR (400 MHz, CDCl₃) δ 9.52 (br s, 1H), 9.41 (br s, 1H), 8.91 (br s, 1H), 8.35 (br s, 2H), 8.12-8.23 (m, 1H), 7.75 (br t, *J =* 7.2 Hz, 1H), 7.57-7.70 (m, 2H), 5.40 (br d, *J =* 6.1 Hz, 1H), 4.16-4.31 (m, 4H), 2.44 (br d, *J =* 6.8 Hz, 2H), 1.91 (br d, *J =* 5.4 Hz, 3H), MS = 547.2 [M+H]⁺.

**Table AAC. The following compounds were prepared using procedures similar to those described above using the appropriate starting materials.**

| **Example** | **Structure** | **Name** | **[M+H]⁺ or [M+Na]⁺** | **Chiral Column** |
|---|---|---|---|---|
| **56** | 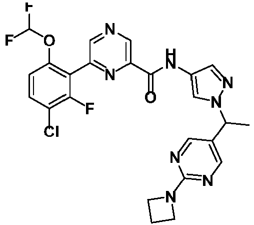 | *(R* or *S*)-*N*-(1-(1-(2-(Azetidin-1-yl)pyrimidin-5-yl)ethyl)-1*H-*pyrazol-4-yl)-6-(3-chloro-6-(difluoromethoxy)-2-fluorophenyl)pyrazine-2-carboxamide | Calcd.: 545.1 | DAICEL CHIRALCEL OD-H |
| | | | Found: 545.2 | |
| **57** | 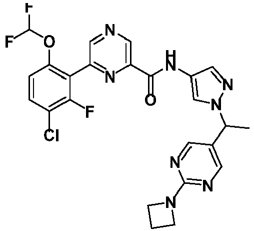 | (*S* or *R*)-*N*-(1-(1-(2-(Azetidin-1-yl)pyrimidin-5-yl)ethyl)-1*H-*pyrazol-4-yl)-6-(3-chloro-6-(difluoromethoxy)-2-fluorophenyl)pyrazine-2-carboxamide | Calcd.: 545.1 | DAICEL CHIRALCEL OD-H |
| | | | Found: 545.2 | |
| **58** | 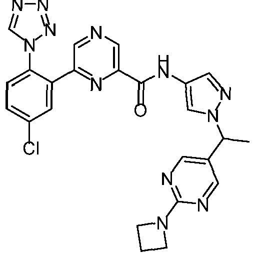 | (*S* or *R*)-*N*-(1-(1-(2-(Azetidin-1-yl)pyrimidin-5-yl)ethyl)-1*H-*pyrazol-4-yl)-6-(5-chloro-2-(1*H-*tetrazol-1-yl)phenyl)pyrazine-2-carboxamide | Calcd.: 529.2 | DAICEL CHIRALCEL OJ-H |
| | | | Found: 529.1 | |
| **59** | 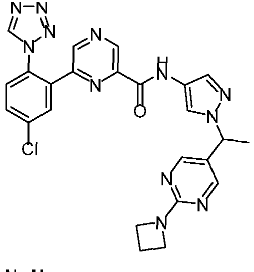 | (*R* or *S*)-*N*-(1-(1-(2-(Azetidin-1-yl)pyrimidin-5-yl)ethyl)-1*H-*pyrazol-4-yl)-6-(5-chloro-2-(1*H-*tetrazol-1-yl)phenyl)pyrazine-2-carboxamide | Calcd.: 529.2 | DAICEL CHIRALCEL OJ-H |
| | | | Found: 529.1 | |
| **60** | 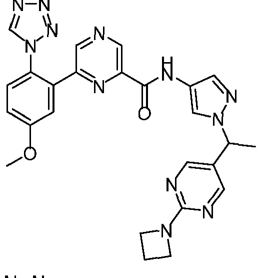 | (*S* or *R*)-*N*-(1-(1-(2-(Azetidin-1-yl)pyrimidin-5-yl)ethyl)-1*H-*pyrazol-4-yl)-6-(5-methoxy-2-(1*H*-tetrazol-1-yl)phenyl)pyrazine-2-carboxamide | Calcd.: 525.2 | DAICEL CHIRALCEL OJ |
| | | | Found: 525.2 | |
| **61** | 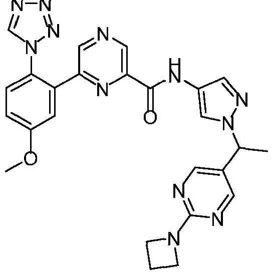 | (*R* or S)-*N*-(1-(1-(2-(Azetidin-1-yl)pyrimidin-5-yl)ethyl)-1*H-*pyrazol-4-yl)-6-(5-methoxy-2-(1*H*-tetrazol-1-yl)phenyl)pyrazine-2-carboxamide | Calcd.: 525.2 | DAICEL CHIRALCEL OJ |
| | | | Found: 525.2 | |
| **62** | 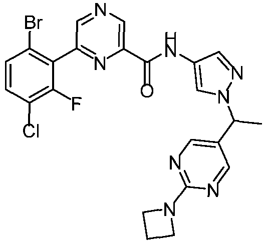 | *N*-(1-(1-(2-(Azetidin-1-yl)pyrimidin-5-yl)ethyl)-1*H-*pyrazol-4-yl)-6-(6-bromo-3-chloro-2-fluorophenyl)pyrazine-2-carboxamide | Calcd.: 557.1 | |
| | | | Found: 557.1 | |
| **63** | 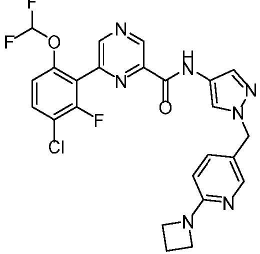 | *N*-(1-((6-(Azetidin-1-yl)pyridin-3-yl)methyl)-1*H*-pyrazol-4-yl)-6-(3-chloro-6-(difluoromethoxy)-2-fluorophenyl)pyrazine-2-carboxamide | Calcd.: 530.1 | |
| | | | Found: 530.2 | |
| **64** | 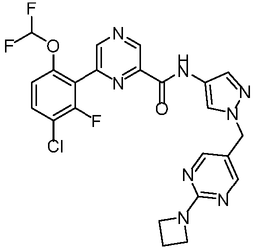 | *N*-(1-((2-(Azetidin-1-yl)pyrimidin-5-yl)methyl)-1*H-*pyrazol-4-yl)-6-(3-chloro-6-(difluoromethoxy)-2-fluorophenyl)pyrazine-2-carboxamide | Calcd.: 531.1 | |
| | | | Found: 531.4 | |
| **65** | 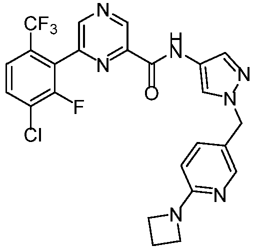 | *N*-(1-((2-(Azetidin-1-yl)pyrimidin-5-yl)methyl)-1*H-*pyrazol-4-yl)-6-(3-chloro-2-fluoro-6-(trifluoromethyl)phenyl)pyrazine-2-carboxamide | Calcd.: 533.8 | |
| | | | Found: 533.3 | |

### Examples 66 and 67

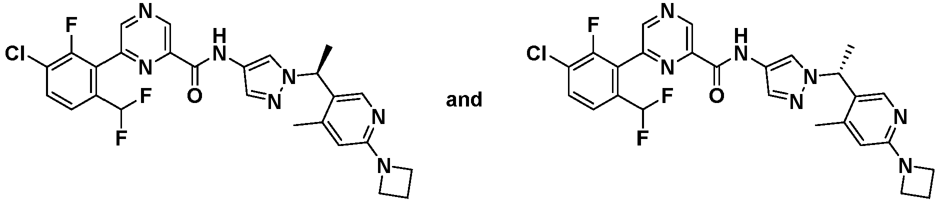

### (S)-N-(1-(1-(6-(Azetidin-1-yl)-4-methylpyridin-3-yl)ethyl)-1H-pyrazol-4-yl)-6-(3-chloro-6-(difluoromethyl)-2-fluorophenyl)pyrazine-2-carboxamide and (R)-N-(1-(1-(6-(Azetidin-1-yl)-4-methylpyridin-3-yl)ethyl)-1H-pyrazol-4-yl)-6-(3-chloro-6-(difluoromethyl)-2-fluorophenyl)pyrazine-2-carboxamide (Scheme AAD)

1-(6-(Azetidin-1-yl)-4-methylpyridin-3-yl)ethyl methanesulfonate (0.15 g, 0.56 mmol) was added to a stirred solution of 6-(3-chloro-6-(difluoromethyl)-2-fluorophenyl)-N-(1H-pyrazol-4-yl)pyrazine-2-carboxamide (0.20 g, 0.56 mmol) and cesium carbonate (0.36 g, 1.1 mmol) in DMF (5.5 mL), and the resulting mixture was heated to 60 °C for 2 h. The reaction was cooled to rt, diluted with brine, and extracted with EtOAc. The combined organic fractions were dried (MgSO₄), filtered, and concentrated to afford a crude residue that was purified with silica gel chromatography (MeOH/DCM) to give the racemic title compound which was separated by chiral SFC (DAICEL CHIRALCEL OD-H 30% methanol (0.1% DEA)/CO₂) to give the first eluted isomer: ¹H NMR (500 MHz, DMSO-*d₆*) δ 10.82 (s, 1H), 9.32 (s, 1H), 9.13 (s, 1H), 8.00 - 7.97 (m, 5H), 7.72 (t, *J =* 3 Hz, 3H), 7.28 - 7.12 (m, 1H), 6.18 (s, 1H), 5.65 (q, *J =* 7.0 Hz, 1H), 3.91 - 3.88 (m, 5H), 2.55 (s, 1H), 2.31 -2.25 (m, 3H), 2.10 (s, 3H), 1.77 - 1.74 (m, 3H). MS = 542.3 [M+H]⁺, and the second eluted isomer: ¹H NMR (500 MHz, DMSO-*d₆*) δ 10.81 (s, 1H), 9.32 (s, 1H), 9.15 (s, 1H), 8.02 - 7.98 (m, 5H), 7.75 - 7.71 (m, 3H), 7.21 (t, *J =* 11 Hz, 1H), 6.18 (s, 1H), 5.67 - 5.65 (m, 1H), 3.93 - 3.89 (m, 5H), 2.55 (s, 1H), 2.32 -2.26 (m, 3H), 2.10 (s, 3H), 1.77 - 1.74 (m, 3H). MS = 542.3 [M+H]⁺.

### Example 68

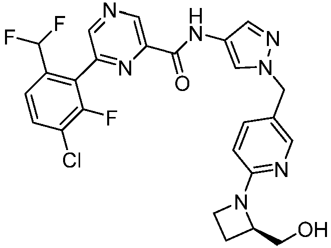

### (R)-6-(3-Chloro-6-(difluoromethyl)-2-fluorophenyl)-N-(1-((6-(2-(hydroxymethyl)azetidin-1-yl)pyridin-3-yl)methyl)-1H-pyrazol-4-yl)pyrazine-2-carboxamide (Scheme AAE)

(*R*)-2-Azetidinemethanol (37 mg, 0.42 mmol) was added to a stirred solution of 6-(3-chloro-6-(difluoromethyl)-2-fluorophenyl)-N-(1-((6-fluoropyridin-3-yl)methyl)-1H-pyrazol-4-yl)pyrazine-2-carboxamide (50 mg, 0.11 mmol) and DIEA (92 µl, 0.52 mmol) in NMP (1.1 mL), and the resulting mixture was heated to 130 °C for 24 h. The reaction was cooled to rt and purified by reversed-phase preparatory-HPLC (C18 stationary phase, water-ACN (0.05% NH₃) to give the title compound. ¹H NMR (600 MHz, CD₃OD) δ 9.38 (s, 1H), 9.01 (d, *J=* 1.9 Hz, 1H), 8.17 (s, 1H), 8.01 (d, *J =* 1.7 Hz, 1H), 7.83 (t, *J =* 7.8 Hz, 1H), 7.74 (s, 1H), 7.66 (d, *J =* 8.5 Hz, 1H), 7.51 (dd, *J* = 8.7, 2.2 Hz, 1H), 7.00 (t, *J =* 54.7 Hz, 1H), 6.49 (d, *J =* 8.6 Hz, 1H), 5.19 (s, 2H), 4.42 - 4.31 (m, 1H), 3.97 - 3.88 (m, 1H), 3.83 (q, *J=* 7.8 Hz, 1H), 3.78 - 3.67 (m, 2H), 2.35 - 2.28 (m, 1H), 2.27 - 2.19 (m, 1H). MS = 544.3 [M+H]⁺.

### Examples 69 and 70

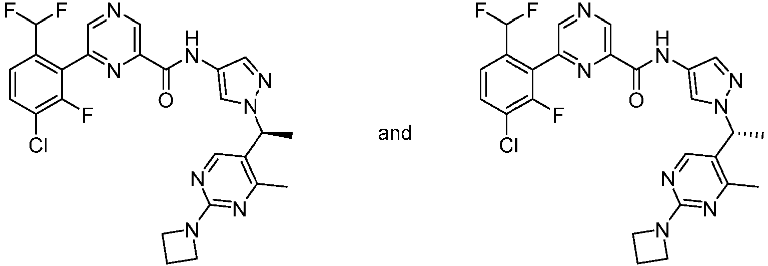

### (S)-N-(1-(1-(2-(Azetidin-1-yl)-4-methylpyrimidin-5-yl)ethyl)-1H-pyrazol-4-yl)-6-(3-chloro-6-(difluoromethyl)-2-fluorophenyl)pyrazine-2-carboxamide and (R)-N-(1-(1-(2-(Azetidin-1-yl)-4-methylpyrimidin-5-yl)ethyl)-1H-pyrazol-4-yl)-6-(3-chloro-6-(difluoromethyl)-2-fluorophenyl)pyrazine-2-carboxamide (Scheme AAE)

Azetidine (24 µL, 0.35 mmol) was added to a stirred solution of 6-(3-chloro-6-(difluoromethyl)-2-fluorophenyl)-*N*-(1-(1-(4-methyl-2-(methylsulfonyl)pyrimidin-5-yl)ethyl)-1*H*-pyrazol-4-yl)pyrazine-2-carboxamide (40 mg, 0.071 mmol) and DIEA (62 µL, 0.35 mmol) in NMP (0.7 mL), and the resulting mixture was heated to 90 °C for 2 h. The mixture was cooled to rt, diluted with brine and extracted with EtOAc. The combined organic fractions were dried (Na₂SO₄), filtered and concentrated to afford a crude residue that was purified by preparative reverse phase HPLC, eluting with acetonitrile/water + 0.1% TFA, to give the title compound as a racemic mixture, which were separated by chiral SFC (DAICEL CHIRALCEL OJ-H 20% ethanol/CO₂) to give the first eluted isomer **Example 69:** ¹H NMR (600 MHz, CD₃OD) δ 9.39 (s, 1H), 9.01 (d, *J =* 2.1 Hz, 1H), 8.15 (s, 1H), 8.14 - 8.13 (m, 1H), 7.88 - 7.79 (m, 1H), 7.76 (d, *J*= 0.6 Hz, 1H), 7.67 (d, *J =* 8.5 Hz, 1H), 7.00 (t, *J =* 54.7 Hz, 1H), 5.68 (q, *J =* 7.0 Hz, 1H), 4.19 - 4.04 (m, 4H), 2.37 (p, *J=* 7.5 Hz, 2H), 2.29 (s, 3H), 1.84 (d, *J =* 7.0 Hz, 3H). MS = 543.0 [M+H]⁺. The second eluted isomer **Example 70:** ¹H NMR (600 MHz, CD₃OD) δ 9.39 (s, 1H), 9.01 (d, *J =* 2.1 Hz, 1H), 8.15 (s, 1H), 8.14 (s, 1H), 7.86 - 7.81 (m, 1H), 7.76 (d, *J =* 0.5 Hz, 1H), 7.67 (d, *J =* 8.4 Hz, 1H), 7.00 (t, *J =* 54.7 Hz, 1H), 5.68 (q, *J =* 7.1 Hz, 1H), 4.21 - 4.03 (m, 4H), 2.37 (p, *J =* 7.5 Hz, 2H), 2.29 (s, 3H), 1.84 (d, *J =* 7.0 Hz, 3H). MS = 543.0 [M+H]⁺.

**Table AAE. The following compounds were prepared using procedures similar to those described above using the appropriate starting materials. In some examples, racemic products or diatereomeric mixtures were separated using chiral columns (SFC or HPLC) specified in the table.**

| **Example** | **Structure** | **Name** | **[M+H]⁺ or [M+Na]⁺** | **Chiral column** |
|---|---|---|---|---|
| **71** | 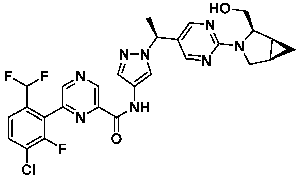 | 6-(3-Chloro-6-(difluoromethyl)-2-fluorophenyl)-*N*-(1-((*S* or *R)-1-(2-((1S,2R,5R* or 1*R*,2*S*,5*S*)-2-(hydroxymethyl)-3-azabicyclo[3.1.0]hexan-3-yl)pyrimidin-5-yl)ethyl)-1*H-*pyrazol-4-yl)pyrazine-2-carboxamide | Calcd.: 585.2 | DAICEL YMC CHIRAL Amylose-C |
| | | | Found: 585.2 | |
| **72** | 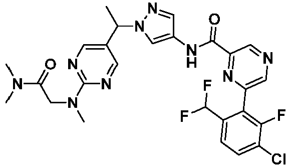 | 6-(3-Chloro-6-(difluoromethyl)-2-fluorophenyl)-*N*-(1-(1-(2-((2-(dimethylamino)-2-oxoethyl)(methyl)amino)pyrim idin-5-yl)ethyl)-1*H*-pyrazol-4-yl)pyrazine-2-carboxamide | Calcd.: 588.0 | |
| | | | Found: 588.2 | |
| **73** | 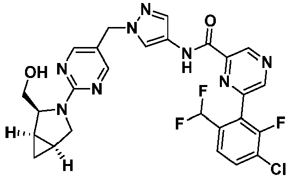 | 6-(3-Chloro-6-(difluoromethyl)-2-fluorophenyl)-*N*-(1-((2-((1*R,*2*S,*5*S)-2-*(hydroxymethyl)-3-azabicyclo[3.1.0]hexan-3-yl)pyrimidin-5-yl)methyl)-1*H-*pyrazol-4-yl)pyrazine-2-carboxamide | Calcd.: 571.2 | |
| | | | Found: 571.4 | |
| **74** | 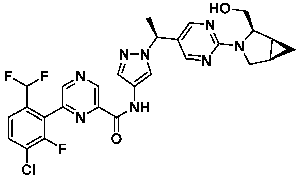 | 6-(3-Chloro-6-(difluoromethyl)-2-fluorophenyl)-N-(1-((S or *R)-1-(2-((1S,2R,5R* or 1R,2S,5S)-2-(hydroxymethyl)-3-azabicyclo[3.1.0]hexan-3-yl)pyrimidin-5-yl)ethyl)-1*H-*pyrazol-4-yl)pyrazine-2-carboxamide | Calcd.: 585.2 | DAICEL YMC CHIRAL Amylose-C |
| | | | Found: 585.2 | |
| **75** | 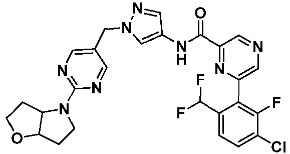 | 6-(3-Chloro-6-(difluoromethyl)-2-fluorophenyl)-*N*-(1-((2-(hexahydro-4*H*-furo[3,2-b]pyrrol-4-yl)pyrimidin-5-yl)methyl)-1*H*-pyrazol-4-yl)pyrazine-2-carboxamide | Calcd.: 571.0 | |
| | | | Found: 571.3 | |
| **76** | 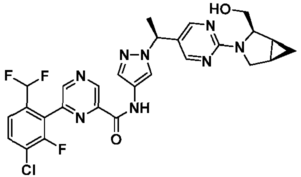 | 6-(3-Chloro-6-(difluoromethyl)-2-fluorophenyl)-*N*-(1-((S or *R)-1-(2-((1S,2R,5R* or 1*R*,2*S*,5*S*)-2-(hydroxymethyl)-3-azabicyclo[3.1.0]hexan-3-yl)pyrimidin-5-yl)ethyl)-1*H-*pyrazol-4-yl)pyrazine-2-carboxamide | Calcd.: 585.2 | DAICEL YMC CHIRAL Amylose-C |
| | | | Found: 585.2 | |
| **77** | 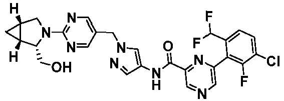 | 6-(3-Chloro-6-(difluoromethyl)-2-fluorophenyl)-*N*-(1-((2-*((1R,2S,5S* or 1*S,*2*R,*5*R*)-2-(hydroxymethyl)-3-azabicyclo[3.1.0]hexan-3-yl)pyrimidin-5-yl)methyl)-1*H-*pyrazol-4-yl)pyrazine-2-carboxamide | Calcd.: 571.2 | AS-H |
| | | | Found: 571.4 | |
| **78** | 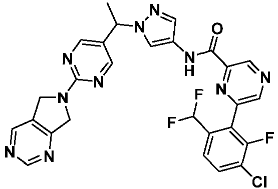 | 6-(3-Chloro-6-(difluoromethyl)-2-fluorophenyl)-*N*-(1-(1-(2-(5,7-dihydro-6*H*-pyrrolo[3,4-d]pyrimidin-6-yl)pyrimidin-5-yl)ethyl)-1*H*-pyrazol-4-yl)pyrazine-2-carboxamide | Calcd.: 593.0 | |
| | | | Found: 593.1 | |
| **79** | 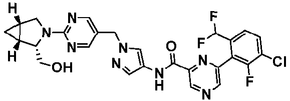 | 6-(3-Chloro-6-(difluoromethyl)-2-fluorophenyl)-*N*-(1-((2-*((1R,2S,5S* or 1*S*,2*R*,5*R*)-2-(hydroxymethyl)-3-azabicyclo[3.1.0]hexan-3-yl)pyrimidin-5-yl)methyl)-1*H-*pyrazol-4-yl)pyrazine-2-carboxamide | Calcd.: 571.2 | AS-H |
| | | | Found: 571.4 | |
| **80** | 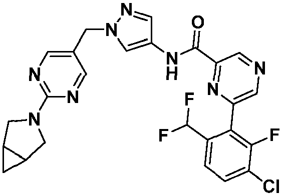 | *N-*(1-((2-(3-Azabicyclo[3.1.0]hexan-3-yl)pyrimidin-5-yl)methyl)-1*H-*pyrazol-4-yl)-6-(3-chloro-6-(difluoromethyl)-2-fluorophenyl)pyrazine-2-carboxamide | Calcd.: 541.1 | |
| | | | Found: 541.2 | |
| **81** | 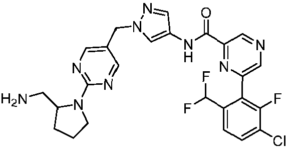 | *N*-(1-((2-(2-(Aminomethyl)pyrrolidin-1-yl)pyrimidin-5-yl)methyl)-1*H-*pyrazol-4-yl)-6-(3-chloro-6-(difluoromethyl)-2-fluorophenyl)pyrazine-2-carboxamide | Calcd.: 558.0 | |
| | | | Found: 557.9 | |
| **82** | 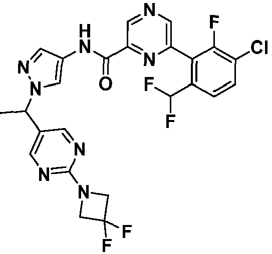 | 6-(3-Chloro-6-(difluoromethyl)-2-fluorophenyl)-*N*-(1-(1-(2-(3,3-difluoroazetidin-1-yl)pyrimidin-5-yl)ethyl)-1*H-*pyrazol-4-yl)pyrazine-2-carboxamide | Calcd.: 565.9 | |
| | | | Found: 566.0 | |
| **83** | 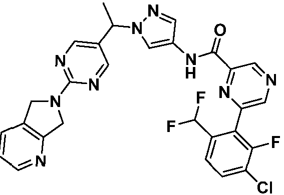 | 6-(3-Chloro-6-(difluoromethyl)-2-fluorophenyl)-*N*-(1-(1-(2-(5,7-dihydro-6*H*-pyrrolo[3,4-b]pyridin-6-yl)pyrimidin-5-yl)ethyl)-1*H*-pyrazol-4-yl)pyrazine-2-carboxamide | Calcd.: 592.2 | |
| | | | Found: 592.2 | |
| **84** | 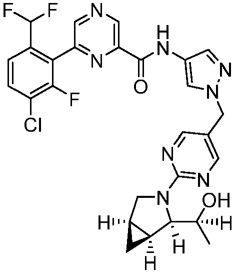 | 6-(3-Chloro-6-(difluoromethyl)-2-fluorophenyl)-*N*-(1-((2-((1*S,*2*R,*5*R)-*2-((*S*)-1-hydroxyethyl)-3-azabicyclo[3.1.0]hexan-3-yl)pyrimidin-5-yl)methyl)-1*H-*pyrazol-4-yl)pyrazine-2-carboxamide | Calcd.: 585.2 | |
| | | | Found: 585.3 | |
| **85** | 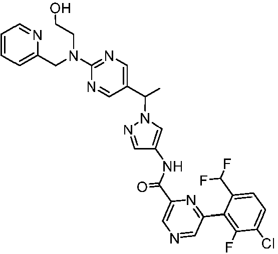 | 6-(3-Chloro-6-(difluoromethyl)-2-fluorophenyl)-*N*-(1-(1-(2-((2-hydroxyethyl)(pyridin-2-ylmethyl)amino)pyrimidin-5-yl)ethyl)-1*H*-pyrazol-4-yl)pyrazine-2-carboxamide | Calcd.: 623.2 | |
| | | | Found: 624.2 | |
| **86** | 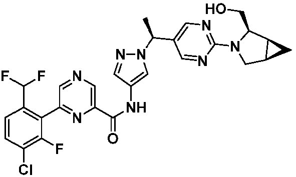 | 6-(3-Chloro-6-(difluoromethyl)-2-fluorophenyl)-*N*-(1-((S or *R)-1-*(2-((1*S,*2*R,*5*R* or 1*R*,2*S*,5*S*)-2-(hydroxymethyl)-3-azabicyclo[3.1.0]hexan-3-yl)pyrimidin-5-yl)ethyl)-1*H-*pyrazol-4-yl)pyrazine-2-carboxamide | Calcd.: 585.2 | |
| | | | Found: 585.2 | |
| **87** | 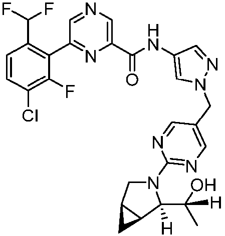 | 6-(3-Chloro-6-(difluoromethyl)-2-fluorophenyl)-*N*-(1-((2-((1*S,*2*R,*5*R)-*2-((*R*)-1-hydroxyethyl)-3-azabicyclo[3.1.0]hexan-3-yl)pyrimidin-5-yl)methyl)-1*H-*pyrazol-4-yl)pyrazine-2-carboxamide | Calcd.: 585.2 | |
| | | | Found: 585.3 | |
| **88** | 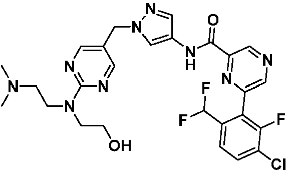 | 6-(3-Chloro-6-(difluoromethyl)-2-fluorophenyl)-*N*-(1-((2-((2-(dimethylamino)ethyl)(2-hydroxyethyl)amino)pyrimidin -5-yl)methyl)-1*H*-pyrazol-4-yl)pyrazine-2-carboxamide | Calcd.: 590.2 | |
| | | | Found: 590.3 | |
| **89** | 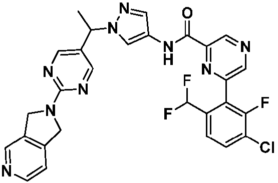 | 6-(3-Chloro-6-(difluoromethyl)-2-fluorophenyl)-*N*-(1-(1-(2-(1,3-dihydro-2*H*-pyrrolo[3,4-c]pyridin-2-yl)pyrimidin-5-yl)ethyl)-1*H*-pyrazol-4-yl)pyrazine-2-carboxamide | Calcd.: 592.2 | |
| | | | Found: 592.1 | |
| **90** | 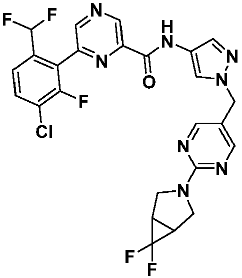 | 6-(3-Chloro-6-(difluoromethyl)-2-fluorophenyl)-*N*-(1-((2-(6,6-difluoro-3-azabicyclo[3.1.0]hexan-3-yl)pyrimidin-5-yl)methyl)-1*H-*pyrazol-4-yl)pyrazine-2-carboxamide | Calcd.: 577.1 | |
| | | | Found: 577.4 | |
| **91** | 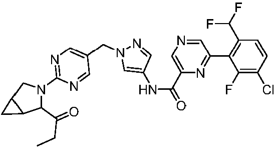 | 6-(3-Chloro-6-(difluoromethyl)-2-fluorophenyl)-*N*-(1-((2-(2-propionyl-3-azabicyclo[3.1.0]hexan-3-yl)pyrimidin-5-yl)methyl)-1*H-*pyrazol-4-yl)pyrazine-2-carboxamide | Calcd.: 597.2 | |
| | | | Found: 597.2 | |
| **92** | 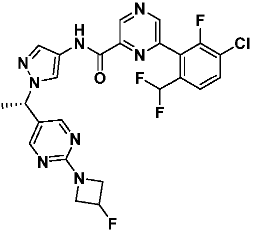 | (*S*)-6-(3-Chloro-6-(difluoromethyl)-2-fluorophenyl)-*N*-(1-(1-(2-(3-fluoroazetidin-1-yl)pyrimidin-5-yl)ethyl)-1*H* pyrazol-4-yl)pyrazine-2-carboxamide | Calcd.: 547.1 | |
| | | | Found: 547.3 | |
| **93** | 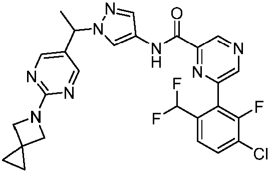 | (*R* or *S*)-N-(1-(1-(2-(5-Azaspiro [2.3]hexan-5-yl)pyrimidin-5-yl)ethyl)-1*H-*pyrazol-4-yl)-6-(3-chloro-6-(difluoromethyl)-2-fluorophenyl)pyrazine-2-carboxamide | Calcd.: 555.2 | AD-H |
| | | | Found: 555.3 | |
| **94** | 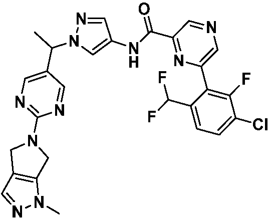 | 6-(3-Chloro-6-(difluoromethyl)-2-fluorophenyl)-*N*-(1-(1-(2-(1-methyl-4,6-dihydropyrrolo[3,4-c]pyrazol-5(1*H*)-yl)pyrimidin-5-yl)ethyl)-1*H*-pyrazol-4-yl)pyrazine-2-carboxamide | Calcd.: 595.2 | |
| | | | Found: 595.1 | |
| **95** | 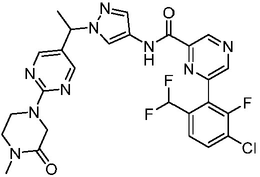 | 6-(3-Chloro-6-(difluoromethyl)-2-fluorophenyl)-*N*-(1-(1-(2-(4-methyl-3-oxopiperazin-1-yl)pyrimidin-5-yl)ethyl)-1*H-*pyrazol-4-yl)pyrazine-2-carboxamide | Calcd.: 586.2 | |
| | | | Found: 586.1 | |
| **96** | 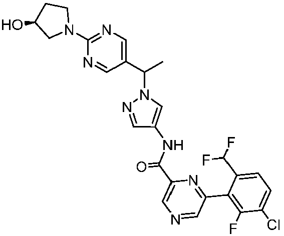 | 6-(3-Chloro-6-(difluoromethyl)-2-fluorophenyl)-*N*-(1-((*R* and *S*)-1-(2-((S)-3-hydroxypyrrolidin-1-yl)pyrimidin-5-yl)ethyl)-1*H-*pyrazol-4-yl)pyrazine-2-carboxamide | Calcd. 558.2 | |
| | | | Found: 559.1 | |
| **97** | 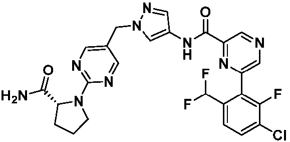 | *(R)-N-(1-((2-(2-*Carbamoylpyrrolidin-1-yl)pyrimidin-5-yl)methyl)-1*H-*pyrazol-4-yl)-6-(3-chloro-6-(difluoromethyl)-2-fluorophenyl)pyrazine-2-carboxamide | Calcd. 572.2 | |
| | | | Found: 572.3 | |
| **98** | 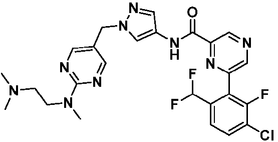 | 6-(3-Chloro-6-(difluoromethyl)-2-fluorophenyl)-*N*-(1-((2-((2-(dimethyl-amino)ethyl)(methyl)amino) pyrimidin-5-yl)methyl)-1*H-*pyrazol-4-yl)pyrazine-2-carboxamide | Calcd. 560.2 | |
| | | | Found: 560.4 | |
| **99** | 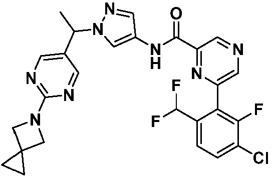 | *N-(1-(1-(2-(5-*Azaspiro[2.3]hexan-5-yl)pyrimidin-5-yl)ethyl)-1*H-*pyrazol-4-yl)-6-(3-chloro-6-(difluoromethyl)-2-fluorophenyl)pyrazine-2-carboxamide | Calcd.: 555.2 | |
| | | | Found: 555.3 | |
| **100** | 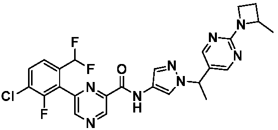 | 6-(3-Chloro-6-(difluoromethyl)-2-fluorophenyl)-*N*-(1-((R and *S*)-1-(2-((*R* and S)-2-methylazetidin-1-yl)pyrimidin-5-yl)ethyl)-1*H*-pyrazol-4-yl)pyrazine-2-carboxamide | Calcd.: 543.2 | |
| | | | Found: 543.3 | |
| **101** | 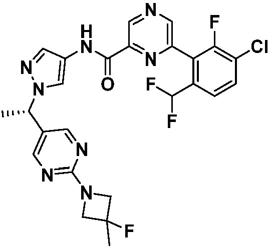 | (*S*)-6-(3-Chloro-6-(difluoromethyl)-2-fluorophenyl)-*N*-(1-(1-(2-(3-fluoro-3-methylazetidin-1-yl)pyrimidin-5-yl)ethyl)-1*H-*pyrazol-4-yl)pyrazine-2-carboxamide | Calcd.: 561.2 | |
| | | | Found: 561.0 | |
| **102** | 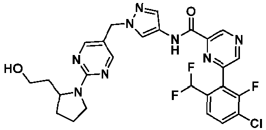 | (*S* or *R*)-6-(3-Chloro-6-(difluoromethyl)-2-fluorophenyl)-*N*-(1-((2-(2-(2-hydroxyethyl)pyrrolidin-1-yl)pyrimidin-5-yl)methyl)-1*H-*pyrazol-4-yl)pyrazine-2-carboxamide | Calcd.: 573.2 | DAICEL CHIRALPAK AD |
| | | | Found: 573.3 | |
| **103** | 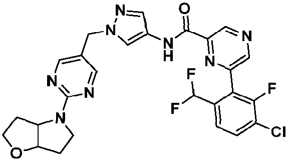 | 6-(3-Chloro-6-(difluoromethyl)-2-fluorophenyl)-*N*-(1-((2-((3a*S*,6a*S* or 3a*R*,6a*R*)-hexahydro-4*H*-furo[3,2-*b*]pyrrol-4-yl)pyrimidin-5-yl)methyl)-1*H*-pyrazol-4-yl)pyrazine-2-carboxamide | Calcd.: 571.2 | AD-H |
| | | | Found: 571.2 | |
| **104** | 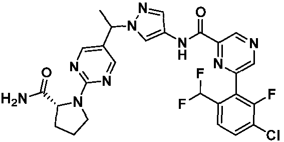 | *N-(1-((R* and *S*)-1-(2-((*R*)-2-Carbamoylpyrrolidin-1-yl)pyrimidin-5-yl)ethyl)-1*H-*pyrazol-4-yl)-6-(3-chloro-6-(difluoromethyl)-2-fluorophenyl)pyrazine-2-carboxamide | Calcd.: 586.2 | |
| | | | Found: 586.0 | |
| **105** | 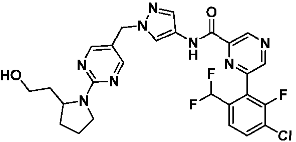 | (*S* or *R*)-6-(3-Chloro-6-(difluoromethyl)-2-fluorophenyl)-*N*-(1-((2-(2-(2-hydroxyethyl)pyrrolidin-1-yl)pyrimidin-5-yl)methyl)-1*H-*pyrazol-4-yl)pyrazine-2-carboxamide | Calcd.: 573.2 | DAICEL CHIRALPAK AD |
| | | | Found: 573.3 | |
| **106** | 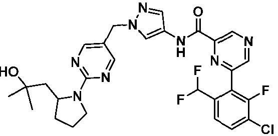 | 6-(3-Chloro-6-(difluoromethyl)-2-fluorophenyl)-*N*-(1-((2-(2-(2-hydroxy-2-methylpropyl)pyrrolidin-1-yl)pyrimidin-5-yl)methyl)-1*H-*pyrazol-4-yl)pyrazine-2-carboxamide | Calcd.: 601.2 | |
| | | | Found: 601.2 | |
| **107** | 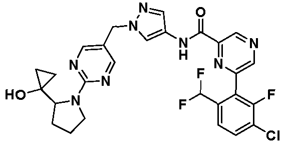 | 6-(3-Chloro-6-(difluoromethyl)-2-fluorophenyl)-*N*-(1-((2-(2-(1-hydroxycyclopropyl)pyrrolidin -1-yl)pyrimidin-5-yl)methyl)-1*H*-pyrazol-4-yl)pyrazine-2-carboxamide | Calcd.: 585.2 | |
| | | | Found: 585.4 | |
| **108** | 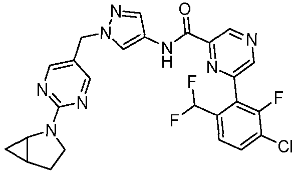 | *N-(1-((2-(2-*Azabicyclo[3.1.0]hexan-2-yl)pyrimidin-5-yl)methyl)-1*H-*pyrazol-4-yl)-6-(3-chloro-6-(difluoromethyl)-2-fluorophenyl)pyrazine-2-carboxamide | Calcd.: 585.2 | |
| | | | Found: 585.4 | |
| **109** | 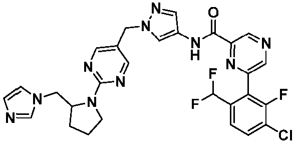 | *N*-(1-((2-(2-((1*H*-Imidazol-1-yl)methyl)pyrrolidin-1-yl)pyrimidin-5-yl)methyl)-1*H-*pyrazol-4-yl)-6-(3-chloro-6-(difluoromethyl)-2-fluorophenyl)pyrazine-2-carboxamide | Calcd.: 609.2 | |
| | | | Found: 609.4 | |
| **110** | 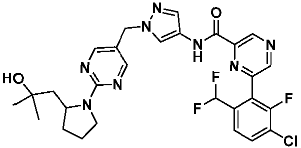 | (*S* or *R*)-6-(3-Chloro-6-(difluoromethyl)-2-fluorophenyl)-*N*-(1-((2-(2-(2-hydroxy-2-methylpropyl)pyrrolidin-1-yl)pyrimidin-5-yl)methyl)-1*H-*pyrazol-4-yl)pyrazine-2-carboxamide | Calcd.: 601.2 | |
| | | | Found: 601.2 | |
| **111** | 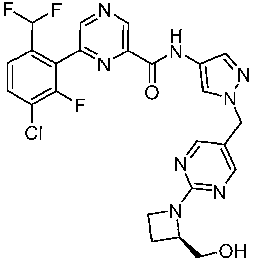 | (*R*)-6-(3-Chloro-6-(difluoromethyl)-2-fluorophenyl)-*N*-(1-((2-(2-(hydroxymethyl)azetidin-1-yl)pyrimidin-5-yl)methyl)-1*H-*pyrazol-4-yl)pyrazine-2-carboxamide | Calcd.: 545.1 | |
| | | | Found: 545.0 | |
| **112** | 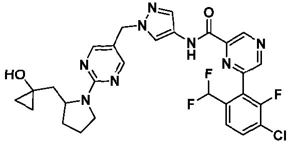 | (*S* or *R*)-6-(3-Chloro-6-(difluoromethyl)-2-fluorophenyl)-*N*-(1-((2-(2-((1-hydroxycyclopropyl)methyl)py rrolidin-1-yl)pyrimidin-5-yl)methyl)-1*H*-pyrazol-4-yl)pyrazine-2-carboxamide | Calcd.: 599.2 | |
| | | | Found: 599.2 | |
| **113** | 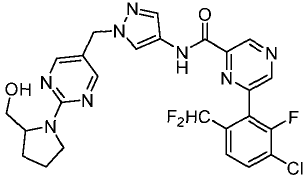 | 6-(3-Chloro-6-(difluoromethyl)-2-fluorophenyl)-*N*-(1-((2-(2-(hydroxymethyl)pyrrolidin-1-yl)pyrimidin-5-yl)methyl)-1*H-*pyrazol-4-yl)pyrazine-2-carboxamide | Calcd.: 559.2 | |
| | | | Found: 559.3 | |
| **114** | 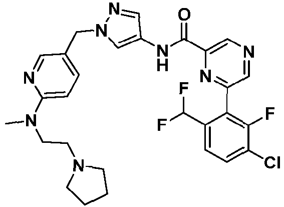 | 6-(3-Chloro-6-(difluoromethyl)-2-fluorophenyl)-*N*-(1-((6-(methyl(2-(pyrrolidin-1-yl)ethyl)amino)pyridin-3-yl)methyl)-1*H*-pyrazol-4-yl)pyrazine-2-carboxamide | Calcd.: 585.2 | |
| | | | Found: 585.2 | |
| **115** | 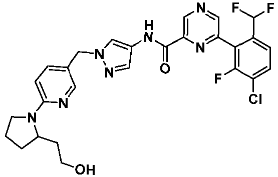 | 6-(3-Chloro-6-(difluoromethyl)-2-fluorophenyl)-*N*-(1-((6-(2-(2-hydroxyethyl)pyrrolidin-1-yl)pyridin-3-yl)methyl)-1*H-*pyrazol-4-yl)pyrazine-2-carboxamide | Calcd.: 572.2 | |
| | | | Found: 572.1 | |
| **116** | 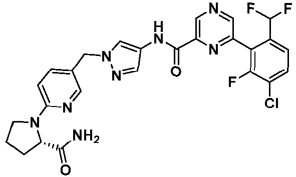 | (*S*)-*N*-(1-((6-(2-Carbamoylpyrrolidin-1-yl)pyridin-3-yl)methyl)-1*H-*pyrazol-4-yl)-6-(3-chloro-6-(difluoromethyl)-2-fluorophenyl)pyrazine-2-carboxamide | Calcd.: 571.2 | |
| | | | Found: 571.1 | |
| **117** | 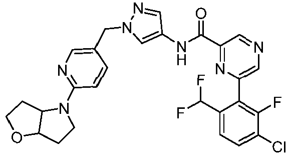 | 6-(3-Chloro-6-(difluoromethyl)-2-fluorophenyl)-*N*-(1-((6-(hexahydro-4*H*-furo[3,2-b]pyrrol-4-yl)pyridin-3-yl)methyl)-1*H*-pyrazol-4-yl)pyrazine-2-carboxamide | Calcd.: 570.2 | |
| | | | Found: 570.3 | |
| **118** | 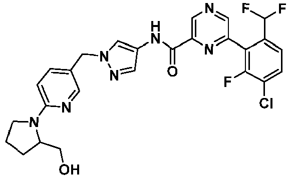 | 6-(3-Chloro-6-(difluoromethyl)-2-fluorophenyl)-*N*-(1-((6-(2-(hydroxymethyl)pyrrolidin-1-yl)pyridin-3-yl)methyl)-1*H-*pyrazol-4-yl)pyrazine-2-carboxamide | Calcd.: 558.2 | |
| | | | Found: 558.0 | |
| **119** | 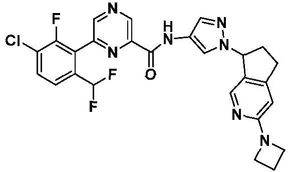 | (*R* or *S*)-*N*-(1-(3-(Azetidin-1-yl)-6,7-dihydro-5*H-*cyclopenta[c]pyridin-7-yl)-1*H-*pyrazol-4-yl)-6-(3-chloro-6-(difluoromethyl)-2-fluorophenyl)pyrazine-2-carboxamide | Calcd.: 540.2 | |
| | | | Found: 540.1 | |
| **120** | 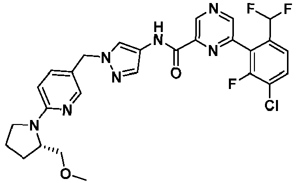 | (*S*)-6-(3-Chloro-6-(difluoromethyl)-2-fluorophenyl)-*N*-(1-((6-(2-(methoxymethyl)pyrrolidin-1-yl)pyridin-3-yl)methyl)-1*H-*pyrazol-4-yl)pyrazine-2-carboxamide | Calcd.: 572.2 | |
| | | | Found: 572.1 | |
| **121** | 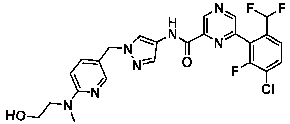 | 6-(3-Chloro-6-(difluoromethyl)-2-fluorophenyl)-*N*-(1-((6-((2-hydroxyethyl)(methyl)amino)p yridin-3-yl)methyl)-1*H-*pyrazol-4-yl)pyrazine-2-carboxamide | Calcd.: 532.1 | |
| | | | Found: 532.1 | |
| **122** | 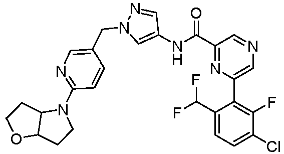 | 6-(3-Chloro-6-(difluoromethyl)-2-fluorophenyl)-*N*-(1-((6-((3aR,6aR or 3aS,6aS)-hexahydro-4*H*-furo[3,2-b]pyrrol-4-yl)pyridin-3-yl)methyl)-1*H*-pyrazol-4-yl)pyrazine-2-carboxamide | Calcd.: 570.2 | OJ-H |
| | | | Found: 570.3 | |
| **123** | 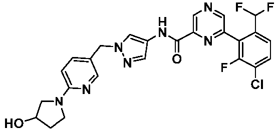 | 6-(3-Chloro-6-(difluoromethyl)-2-fluorophenyl)-*N*-(1-((6-(3-hydroxypyrrolidin-1-yl)pyridin-3-yl)methyl)-1*H-*pyrazol-4-yl)pyrazine-2-carboxamide | Calcd.: 544.1 | |
| | | | Found: 544.1 | |
| **125** | 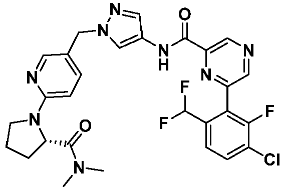 | (*S*)-6-(3-Chloro-6-(difluoromethyl)-2-fluorophenyl)-*N*-(1-((6-(2-(dimethylcarbamoyl)pyrrolidin -1-yl)pyridin-3-yl)methyl)-1H-pyrazol-4-yl)pyrazine-2-carboxamide | Calcd.: 599.1 | |
| | | | Found: 598.1 | |
| **126** | 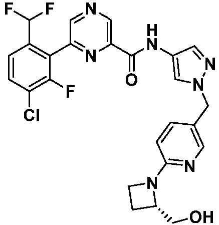 | (*S*)-6-(3-Chloro-6-(difluoromethyl)-2-fluorophenyl)-*N*-(1-((6-(2-(hydroxymethyl)azetidin-1-yl)pyridin-3-yl)methyl)-1*H-*pyrazol-4-yl)pyrazine-2-carboxamide | Calcd.: 544.1 | |
| | | | Found: 544.0 | |
| **127** | 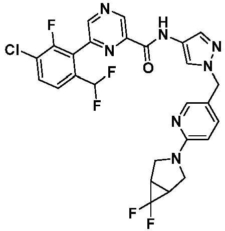 | 6-(3-Chloro-6-(difluoromethyl)-2-fluorophenyl)-*N*-(1-((6-(6,6-difluoro-3-azabicyclo[3.1.0]hexan-3-yl)pyridin-3-yl)methyl)-1*H-*pyrazol-4-yl)pyrazine-2-carboxamide | Calcd.: 576.1 | |
| | | | Found: 576.1 | |
| **128** | 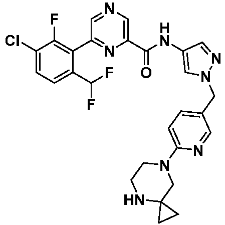 | *N*-(1-((6-(4,7-Diazaspiro[2.5]octan-7-yl)pyridin-3-yl)methyl)-1*H-*pyrazol-4-yl)-6-(3-chloro-6-(difluoromethyl)-2-fluorophenyl)pyrazine-2-carboxamide | Calcd.: 569.2 | |
| | | | Found: 569.4 | |
| **129** | 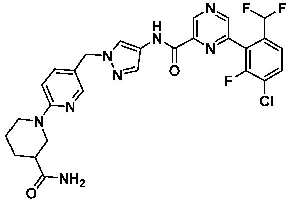 | *N*-(1-((6-(3-Carbamoylpiperidin-1-yl)pyridin-3-yl)methyl)-1*H-*pyrazol-4-yl)-6-(3-chloro-6-(difluoromethyl)-2-fluorophenyl)pyrazine-2-carboxamide | Calcd.: 585.2 | |
| | | | Found: 585.1 | |
| **130** | 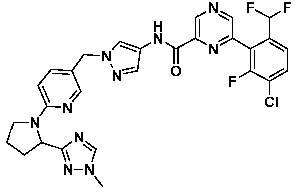 | 6-(3-Chloro-6-(difluoromethyl)-2-fluorophenyl)-*N*-(1-((6-(2-(1-methyl-1*H*-1,2,4-triazol-3-yl)pyrrolidin-1-yl)pyridin-3-yl)methyl)-1*H*-pyrazol-4-yl)pyrazine-2-carboxamide | Calcd.: 609.2 | |
| | | | Found: 609.1 | |
| **131** | 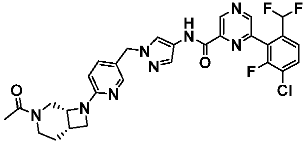 | *N*-(1-((6-((1*R*,6*S*)-3-Acetyl-3,8-diazabicyclo[4.2.0]octan-8-yl)pyridin-3-yl)methyl)-1*H-*pyrazol-4-yl)-6-(3-chloro-6-(difluoromethyl)-2-fluorophenyl)pyrazine-2-carboxamide | Calcd.: 611.2 | |
| | | | Found: 611.1 | |
| **132** | 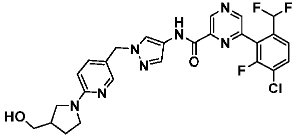 | 6-(3-Chloro-6-(difluoromethyl)-2-fluorophenyl)-*N*-(1-((6-(3-(hydroxymethyl)pyrrolidin-1-yl)pyridin-3-yl)methyl)-1*H-*pyrazol-4-yl)pyrazine-2-carboxamide | Calcd.: 558.2 | |
| | | | Found: 558.0 | |
| **133** | 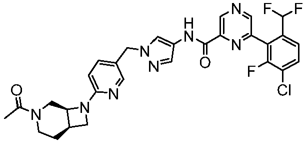 | *N*-(1-((6-((1*S*,6*R*)-3-Acetyl-3,8-diazabicyclo[4.2.0]octan-8-yl)pyridin-3-yl)methyl)-*1H-*pyrazol-4-yl)-6-(3-chloro-6-(difluoromethyl)-2-fluorophenyl)pyrazine-2-carboxamide | Calcd.: 611.2 | |
| | | | Found: 611.2 | |

### Examples 134 and 135

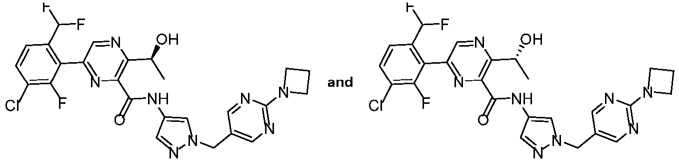

### (S)-N-(1-((2-(Azetidin-1-yl)pyrimidin-5-yl)methyl)-1H-pyrazol-4-yl)-6-(3-chloro-6-(difluoromethyl)-2-fluorophenyl)-3-(1-hydroxyethyl)pyrazine-2-carboxamide and (R)-N-(1-((2-(Azetidin-1-yl)pyrimidin-5-yl)methyl)-1H-pyrazol-4-yl)-6-(3-chloro-6-(difluoromethyl)-2-fluorophenyl)-3-(1-hydroxyethyl)pyrazine-2-carboxamide (Scheme AAF)

To a solution of *N*-(1-((2-(azetidin-1-yl)pyrimidin-5-yl)methyl)-1*H*-pyrazol-4-yl)-6-(3-chloro-6-(difluoromethyl)-2-fluorophenyl)-3-vinylpyrazine-2-carboxamide (41 mg, 0.076 mmol) in IPA (5 mL) was added phenylsilane (21 mg, 0.19 mmol) and tris(2,2,6,6-tetramethyl-3,5-heptanedionato)manganese(III) (9.2 mg, 0.015 mmol). The reaction atmosphere was replaced with O₂ via a balloon, and the resulting mixture was vigorously stirred at rt for 12 h. The reaction was concentrated and partitioned between water and EtOAc. The layers were separated, and the aq. layer was extracted with EtOAc. The combined organic layers were washed with brine, dried over anhydrous Na₂SO₄, filtered and concentrated to afford a crude residue that was purified by reversed-phase preparatory-HPLC (C18 stationary phase, water (0.1%TFA) -ACN Begin B 33%, End B 63%) to give a racemic mixture that was separated by SFC (Phenomenex-Cellulose, 0.1% NH₃H₂O-MeOH Begin B 55% End B 55%) to give the title compounds. The first eluted isomer **Example 134:** ¹H NMR (400 MHz, CD₃OD) δ 8.97 (d, *J =* 2.2 Hz, 1H), 8.29 (s, 2H), 8.21 (s, 1H), 7.80 (t, *J =* 7.8 Hz, 1H), 7.61-7.70 (m, 2H), 6.76-7.09 (m, 1H), 5.80 (br d, *J* = 6.6 Hz, 1H), 5.15 (s, 2H), 4.10 (t, *J =* 7.6 Hz, 4H), 2.35 (br t, *J =* 7.5 Hz, 2H), 1.60 (d, *J =* 6.4 Hz, 3H); MS = 559.2 [M+1]⁺. The second eluted isomer **Example 135:** ¹H NMR (400 MHz, CD₃OD) δ 8.97 (d, *J = 2.2* Hz, 1H), 8.29 (s, 2H), 8.21 (s, 1H), 7.80 (t, *J =* 7.8 Hz, 1H), 7.60-7.72 (m, 2H), 6.74-7.09 (m, 1H), 5.80 (q, *J =* 6.4 Hz, 1H), 5.15 (s, 2H), 4.10 (t, *J =* 7.5 Hz, 4H), 2.36 (t, *J =* 7.6 Hz, 2H), 1.60 (d, *J =* 6.6 Hz, 3H). MS = 559.2 [M+H]⁺.

### Example 136

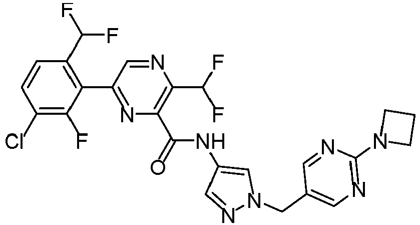

### N-(1-((2-(Azetidin-1-yl)pyrimidin-5-yl)methyl)-1H-pyrazol-4-yl)-6-(3-chloro-6-(difluoromethyl)-2-fluorophenyl)-3-(difluoromethyl)pyrazine-2-carboxamide (Scheme AAG)

Step 1. *N*-(1-((2-(Azetidin-1-yl)pyrimidin-5-yl)methyl)-1*H*-pyrazol-4-yl)-6-(3-chloro-6-(difluoromethyl)-2-fluorophenyl)-*N*-((2-(trimethylsilyl)ethoxy)methyl)-3-vinylpyrazine-2-carboxamide: To a solution of *N*-(1-((2-(azetidin-1-yl)pyrimidin-5-yl)methyl)-1*H*-pyrazol-4-yl)-6-(3-chloro-6-(difluoromethyl)-2-fluorophenyl)-3-vinylpyrazine-2-carboxamide (300 mg, 0.555 mmol) in DMF (4 mL) was added NaH (44.4 mg, 1.11 mmol, 60 wt% in mineral spirits), and the resulting mixture was stirred at rt for 5 min. SEM-Cl (0.20 mL, 1.1 mmol) was added, and the mixture continued stirring at rt for 2 h. The reaction was diluted with water and extracted with EtOAc. The combined organic layers were washed with brine, dried over anhydrous Na₂SO₄, filtered and concentrated to give a crude residue that was purified by silica gel chromatography (EtOAc/petroleum ether) to give the title compound. MS = 671.2 [M+H]⁺.

Step 2. *N*-(1-((2-(Azetidin-1-yl)pyrimidin-5-yl)methyl)-1*H*-pyrazol-4-yl)-6-(3-chloro-6-(difluoromethyl)-2-fluorophenyl)-3-formyl-*N*-((2-(trimethylsilyl)ethoxy)methyl)pyrazine-2-carboxamide: To a solution of *N*-(1-((2-(azetidin-1-yl)pyrimidin-5-yl)methyl)-1*H*-pyrazol-4-yl)-6-(3-chloro-6-(difluoromethyl)-2-fluorophenyl)-*N*-((2-(trimethylsilyl)ethoxy)methyl)-3-vinylpyrazine-2-carboxamide (100 mg, 0.149 mmol) in THF (5 mL) and water (2 mL) were added osmium tetroxide (0.76 mL, 0.030 mmol, 10 mg/mL aq. solution) and sodium periodate (159 mg, 0.745 mmol), and the resulting mixture was stirred at rt for 12 h. The reaction was quenched with satd. aq. Na₂S₂O₃ and allowed to stir at rt for 30 min, at which time, the mixture was extracted with EtOAc. The combined organic layers were washed with brine, dried over anhydrous Na₂SO₄, filtered and concentrated to give the title compound which was used in next step directly. MS = 673.2 [M+H]⁺.

Step 3. *N*-(1-((2-(Azetidin-1-yl)pyrimidin-5-yl)methyl)-1*H*-pyrazol-4-yl)-6-(3-chloro-6-(difluoromethyl)-2-fluorophenyl)-3-(difluoromethyl)-*N*-((2-(trimethylsilyl)ethoxy)methyl)pyrazine-2-carboxamide: To a solution of *N*-(1-((2-(azetidin-1-yl)pyrimidin-5-yl)methyl)-1*H*-pyrazol-4-yl)-6-(3-chloro-6-(difluoromethyl)-2-fluorophenyl)-3-formyl-N-((2-(trimethylsilyl)ethoxy)methyl)pyrazine-2-carboxamide (60 mg, 0.089 mmol) in THF (2 mL) was added DAST (0.12 mL, 0.89 mmol) at 0 °C, and the resulting mixture was warmed to 35 °C and allowed to stir at 35 °C for 12 h. The reaction was quenched with satd. aq. NaHCO₃ and water, and the resulting mixture was extracted with EtOAc. The combined organic layers were washed with brine, dried over anhydrous Na₂SO₄, filtered and concentrated to give a crude residue that was purified by preparatory-TLC ((1:1)EtOAc/petroleum ether) to give the title compound. MS = 695.1 [M+H]⁺.

Step 4. *N*-(1-((2-(Azetidin-1-yl)pyrimidin-5-yl)methyl)-1*H*-pyrazol-4-yl)-6-(3-chloro-6-(difluoromethyl)-2-fluorophenyl)-3-(difluoromethyl)pyrazine-2-carboxamide: A solution of *N-*(1-((2-(azetidin-1-yl)pyrimidin-5-yl)methyl)-1*H*-pyrazol-4-yl)-6-(3-chloro-6-(difluoromethyl)-2-fluorophenyl)-3-(difluoromethyl)-N-((2-(trimethylsilyl)ethoxy)methyl)pyrazine-2-carboxamide (40 mg, 0.058 mmol) in TFA (1 mL) was stirred at 50 °C for 12 h. The mixture was concentrated purified by reversed-phase preparatory-HPLC (C18 stationary phase, water (0.1%TFA) - ACN) to afford the title compound. ¹H NMR (400 MHz, CD₃OD) δ 9.13 (d, *J* = 2.4 Hz, 1H), 8.43 (s, 2H), 8.28 (s, 1H), 7.75-8.04 (m, 2H), 7.73 (d, *J=* 0.8 Hz, 1H), 7.69 (d, *J =* 8.6 Hz, 1H), 6.82-7.15 (m, 1H), 5.23 (s, 2H), 4.23 (t, *J =* 7.8 Hz, 4H), 2.39-2.52 (m, 2H). MS = 565.1 [M+H]⁺.

### Example 137

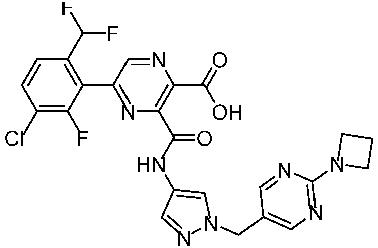

### 3-((1-((2-(Azetidin-1-yl)pyrimidin-5-yl)methyl)-1H-pyrazol-4-yl)carbamoyl)-5-(3-chloro-6-(difluoromethyl)-2-fluorophenyl)pyrazine-2-carboxylic acid (Scheme AAH)

Step 1. 3-((1-((2-(Azetidin-1-yl)pyrimidin-5-yl)methyl)-1*H*-pyrazol-4-yl)((2-(trimethylsilyl)ethoxy)-methyl)carbamoyl)-5-(3-chloro-6-(difluoromethyl)-2-fluorophenyl)pyrazine-2-carboxylic acid: To a solution of *N*-(1-((2-(azetidin-1-yl)pyrimidin-5-yl)methyl)-1*H*-pyrazol-4-yl)-6-(3-chloro-6-(difluoro-methyl)-2-fluorophenyl)-3-formyl-N-((2-(trimethylsilyl)ethoxy)methyl)pyrazine-2-carboxamide (50 mg, 0.074 mmol) in water (1 mL) and ACN (1 mL) were added sodium dihydrogenphosphate dihydrate (58 mg, 0.37 mmol), sulfamic acid (36 mg, 0.37 mmol) and sodium chlorite (20 mg, 0.22 mmol), and the resulting mixture was stirred at 15 °C for 30 min. The reaction was diluted with water and extracted with EtOAc, and the combined organic layers were washed with brine, dried over anhydrous Na₂SO₄, filtered and concentrated to afford a crude residue that was purified by reversed-phase preparatory-HPLC (C18 stationary phase, water (0.1%TFA) - ACN) to afford the title compound. MS = 689.1 [M+H]⁺.

Step 2. 3-((1-((2-(Azetidin-1-yl)pyrimidin-5-yl)methyl)-1*H*-pyrazol-4-yl)carbamoyl)-5-(3-chloro-6-(difluoromethyl)-2-fluorophenyl)pyrazine-2-carboxylic acid: The title compound was prepared following procedures similar to those described above for Intermediate BF, Step 3, in which the final compound was purified by reversed-phase preparatory-HPLC (C18 stationary phase, water (0.1%TFA) - ACN). ¹H NMR (400 MHz, CD₃OD) δ 9.00 (br s, 1H), 8.32 (s, 2H), 8.18 (s, 1H), 7.84 (br t, *J =* 7.6 Hz, 1H), 7.62-7.70 (m, 2H), 6.81-7.18 (m, 1H), 5.17 (s, 2H), 4.05-4.19 (m, 4H), 2.38 (t, *J =* 7.6 Hz, 2H). MS = 559.1 [M+H]⁺.

### Example 138

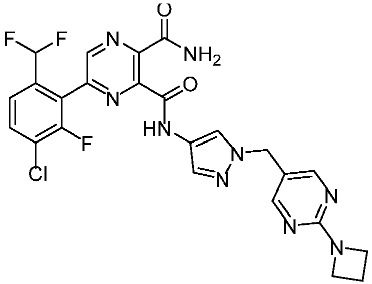

### N²-(1-((2-(Azetidin-1-yl)pyrimidin-5-yl)methyl)-1H-pyrazol-4-yl)-6-(3-chloro-6-(difluoromethyl)-2-fluorophenyl)pyrazine-2,3-dicarboxamide (Scheme AAI)

Step 1. *N*²-(1-((2-(Azetidin-1-yl)pyrimidin-5-yl)methyl)-1*H*-pyrazol-4-yl)-6-(3-chloro-6-(difluoromethyl)-2-fluorophenyl)-*N*²-((2-(trimethylsilyl)ethoxy)methyl)pyrazine-2,3-dicarboxamide:*H -* fluorophenyl)pyrazine-2-carboxylic acid (13 mg, 0.019 mmol), HATU (8.6 mg, 0.023 mmol) and DIEA (10 µL, 0.057 mmol) in DCM (2 mL) was added NH₄Cl (1.2 mg, 0.023 mmol), and the resulting mixture was allowed to stir at rt for 1 h. The reaction was diluted with water and extracted with DCM. The combined organic layers were washed with brine, dried over Na₂SO₄, filtered and concentrated to give the title compound which was used in next step directly. MS = 688.2 (M+H).

Step 2. *N*²-(1-((2-(Azetidin-1-yl)pyrimidin-5-yl)methyl)-1*H*-pyrazol-4-yl)-6-(3-chloro-6-(difluoromethyl)-2-fluorophenyl)pyrazine-2,3-dicarboxamide:¹H NMR (400 MHz, CD₃OD) δ 8.98 (d, *J* = 2.7 Hz, 1H), 8.37 (s, 2H), 8.18 (s, 1H), 7.78-7.86 (m, 1H), 7.61-7.68 (m, 2H), 6.82-7.11 (m, 1H), 5.19 (s, 2H), 4.12-4.22 (m, 4H), 2.41 (t, *J =* 7.7 Hz, 2H). MS = 558.1 [M+H]⁺.

### Example 139

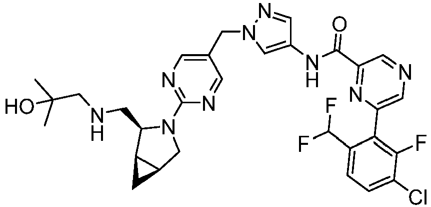

### 6-(3-Chloro-6-(difluoromethyl)-2-fluorophenyl)-N-(1-((2-((1R,2S,5S)-2-(((2-hydroxy-2-methylpropyl)amino)methyl)-3-azabicyclo[3.1.0]hexan-3-yl)pyrimidin-5-yl)methyl)-1H-pyrazol-4-yl)pyrazine-2-carboxamide (Scheme AAJ)

Step 1. 6-(3-Chloro-6-(difluoromethyl)-2-fluorophenyl)-*N*-(1-((2-((1*R,*2*S*,5*S*)-2-formyl-3-azabicyclo[3.1.0]hexan-3-yl)pyrimidin-5-yl)methyl)-1*H*-pyrazol-4-yl)pyrazine-2-carboxamide: Dess-Martin Periodinane (66 mg, 0.16 mmol) and sodium bicarbonate (16 mg, 0.19 mmol) were added to a stirred solution of 6-(3-chloro-6-(difluoromethyl)-2-fluorophenyl)-*N*-(1-((2-((1*R*,2*S*,5*S*)-2-(hydroxymethyl)-3-azabicyclo[3.1.0]hexan-3-yl)pyrimidin-5-yl)methyl)-1*H-*pyrazol-4-yl)pyrazine-2-carboxamide (74 mg, 0.13 mmol) in DCM (4 mL) at rt, and the resulting mixture was stirred at rt for 3 h. The reaction was quenched with 2M aq. NaS₂O₃ and extracted with DCM. The combined organic layers were dried (Na₂SO₄), filtered and concentrated to afford a crude residue that was purified by silica gel chromatography ((3:1) EtOAc:EtOH/hexanes) to give the title compound. MS = 569.2 [M + 1]⁺.

Step 2. 6-(3-Chloro-6-(difluoromethyl)-2-fluorophenyl)-*N*-(1-((2-((1*R,*2*S*,5*S*)-2-(((2-hydroxy-2-methylpropyl)amino)methyl)-3-azabicyclo[3.1.0]hexan-3-yl)pyrimidin-5-yl)methyl)-1*H*-pyrazol-4-yl)pyrazine-2-carboxamide: 1-Amino-2-methylpropan-2-ol (10 mg, 0.11 mmol) was added to a stirred solution of 6-(3-chloro-6-(difluoromethyl)-2-fluorophenyl)-*N*-(1-((2-((1*R*,2*R*,5*R*)-2-formylbicyclo[3.1.0]hexan-3-yl)pyrimidin-5-yl)methyl)-1*H*-pyrazol-4-yl)pyrazine-2-carboxamide (16 mg, 0.028 mmol) in DCE ( 1 mL, 5% AcOH), and the resulting mixture was stirred at rt for 0.5 h. Sodium triacetoxyboronhydride (12 mg, 0.056 mmol) was added to this mixture, which was stirred at rt for 3 h. The reaction was quenched with 1M Na₂CO₃ and extracted with DCM. The combined organic layers were dried (Na₂SO₄), filtered and concentrated to afford a crude residue that was purified by reversed-phase preparatory-HPLC (C18 stationary phase, water (0.1%TFA) - ACN) to afford the title compound. ¹H NMR (400 MHz, DMSO-*d*₆) δ 10.83 (s, 1H), 9.84 (br, 1H), 9.32 (s, 1H), 9.14 (s, 1H), 8.79 (br, 1H), 8.44 (s, 2H), 825 (s, 1H), 7.98 (t, *J =* 7.8 Hz, 1H), 7.70 (m, 2H), 7.17 (t, *J =* 54.3 Hz, 1H), 5.20 (s, 2H), 4.22 - 4.19 (m, 1H), 3.79-3.75 (m, 1H), 3.66 - 3.61 (m, 1H), 3.50-3.41 (m, 1H), 3.16 - 2.99 (m, 2H), 2.85-2.74 (m, 1H), 1.97 - 1.86 (m, 1H), 1.71 - 1.63 (m, 1H), 1.23 (s, 3H), 1.21 (s, 3H), 0.70 - 0.63 (m, 1H), 0.43 - 0.38 (m, 1H). MS = 642.2 [M+H]⁺.

**Table AAJ. The following compounds were prepared using procedures similar to those described above using the appropriate starting materials. In some examples, racemic products or diatereomeric mixtures were separated using chiral columns (SFC or HPLC) specified in the table.**

| **Example** | **Structure** | **IUPAC Name** | **[M+H]⁺ or [M+Na]⁺** | **Chiral Column** |
|---|---|---|---|---|
| **140** | 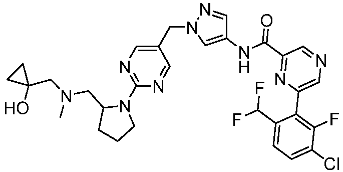 | (*S* or *R*)-6-(3-Chloro-6-(difluoromethyl)-2-fluorophenyl)-N-(1-((2-(2-((((1-hydroxycyclopropyl)methyl)(methyl) amino)methyl)pyrrolidin-1-yl)pyrimidin-5-yl)methyl)-1*H-*pyrazol-4-yl)pyrazine-2-carboxamide | Calcd.: 642.1 | Chiral IG column second peak |
| | | | Found: 642.2 | |
| **141** | 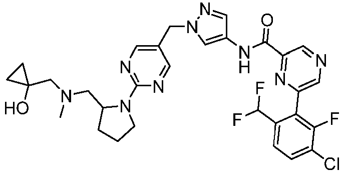 | (*R* or *S*)-6-(3-Chloro-6-(difluoromethyl)-2-fluorophenyl)-N-(1-((2-(2-((((1-hydroxycyclopropyl)methyl)(methyl) amino)methyl)pyrrolidin-1-yl)pyrimidin-5-yl)methyl)-1*H-*pyrazol-4-yl)pyrazine-2-carboxamide | Calcd.: 642.1 | Chiral IG column First peak |
| | | | Found: 642.2 | |
| **142** | 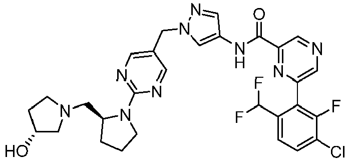 | 6-(3-Chloro-6-(difluoromethyl)-2-fluorophenyl)-*N*-(1-((2-((*S*)-2-(((*R*)-3-hydroxypyrrolidin-1-yl)methyl)pyrrolidin-1-yl)pyrimidin-5-yl)methyl)-1*H*-pyrazol-4-yl)pyrazine-2-carboxamide | Calcd.: 628.1 | |
| | | | Found: 628.3 | |
| **143** | 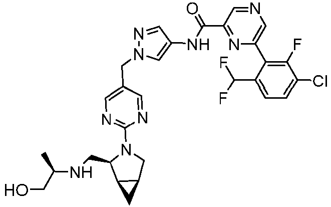 | 6-(3-Chloro-6-(difluoromethyl)-2-fluorophenyl)-*N*-(1-((2-((1*R*,2*S*,5*S*)-2-((((*R*)-1-hydroxypropan-2-yl)amino)methyl)-3-azabicyclo[3.1.0]hexan-3-yl)pyrimidin-5-yl)methyl)-1*H-*pyrazol-4-yl)pyrazine-2-carboxamide | Calcd.: 628.1 | |
| | | | Found: 628.2 | |
| **144** | 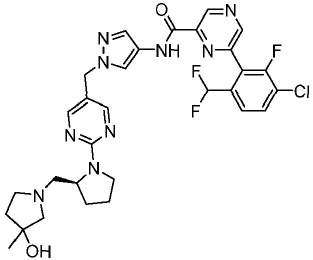 | 6-(3-Chloro-6-(difluoromethyl)-2-fluorophenyl)-*N*-(1-((2-((2*S*)-2-((3-hydroxy-3-methylpyrrolidin-1-yl)methyl)pyrrolidin-1-yl)pyrimidin-5-yl)methyl)-1*H*-pyrazol-4-yl)pyrazine-2-carboxamide | Calcd.: 642.1 | |
| | | | Found: 642.3 | |
| **145** | 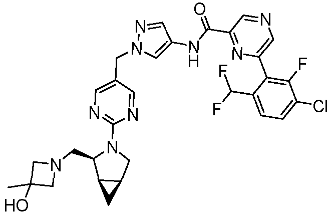 | 6-(3-Chloro-6-(difluoromethyl)-2-fluorophenyl)-*N*-(1-((2-((1*R*,2*S*,5*S*)-2-((3-hydroxy-3-methylazetidin-1-yl)methyl)-3-azabicyclo[3.1.0]hexan-3-yl)pyrimidin-5-yl)methyl)-1*H-*pyrazol-4-yl)pyrazine-2-carboxamide | Calcd.: 640.0 | |
| | | | Found: 640.2 | |
| **146** | 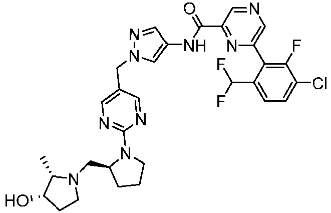 | 6-(3-Chloro-6-(difluoromethyl)-2-fluorophenyl)-*N*-(1-((2-((*S*)-2-(((2*S*,3*S*)-3-hydroxy-2-methylpyrrolidin-1-yl)methyl)pyrrolidin-1-yl)pyrimidin-5-yl)methyl)-1*H*-pyrazol-4-yl)pyrazine-2-carboxamide | Calcd.: 642.1 | |
| | | | Found: 642.2 | |
| **147** | 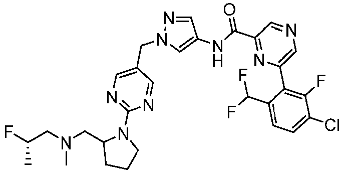 | 6-(3-Chloro-6-(difluoromethyl)-2-fluorophenyl)-*N*-(1-((2-(2-((((S)-2-fluoropropyl)(methyl)amino)methyl)p yrrolidin-1-yl)pyrimidin-5-yl)methyl)-1*H*-pyrazol-4-yl)pyrazine-2-carboxamide | Calcd.: 632.1 | |
| | | | Found: 632.4 | |
| **148** | 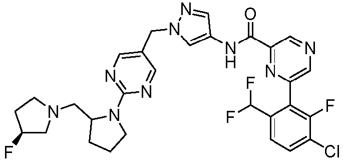 | 6-(3-Chloro-6-(difluoromethyl)-2-fluorophenyl)-*N*-(1-((2-(2-(((S)-3-fluoropyrrolidin-1-yl)methyl)pyrrolidin-1-yl)pyrimidin-5-yl)methyl)-1*H*-pyrazol-4-yl)pyrazine-2-carboxamide | Calcd.: 630.0 | |
| | | | Found: 630.4 | |
| **149** | 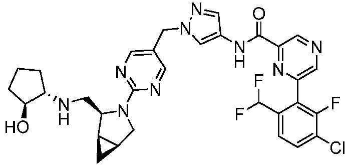 | 6-(3-Chloro-6-(difluoromethyl)-2-fluorophenyl)-*N*-(1-((2-((1R,2S,5S)-2-((((1*S*,2*S*)-2-hydroxycyclopentyl)amino)methyl)-3-azabicyclo[3.1.0]hexan-3-yl)pyrimidin-5-yl)methyl)-1*H-*pyrazol-4-yl)pyrazine-2-carboxamide | Calcd.: 654.1 | |
| | | | Found: 654.2 | |
| **150** | 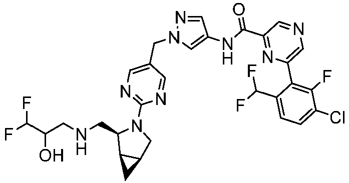 | 6-(3-Chloro-6-(difluoromethyl)-2-fluorophenyl)-*N*-(1-((2-((1R,2S,5S)-2-(((3,3-difluoro-2-hydroxypropyl)amino)methyl)-3-azabicyclo[3.1.0]hexan-3-yl)pyrimidin-5-yl)methyl)-1*H-*pyrazol-4-yl)pyrazine-2-carboxamide | Calcd.: 664.0 | |
| | | | Found: 664.1 | |
| **151** | 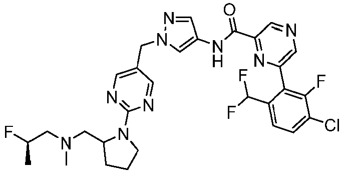 | 6-(3-Chloro-6-(difluoromethyl)-2-fluorophenyl)-*N*-(1-((2-(2-((((R)-2-fluoropropyl)(methyl)amino)methyl)p yrrolidin-1-yl)pyrimidin-5-yl)methyl)-1*H*-pyrazol-4-yl)pyrazine-2-carboxamide | Calcd.: 632.1 | |
| | | | Found: 632.4 | |
| **152** | 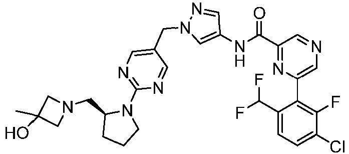 | (*S*)-6-(3-Chloro-6-(difluoromethyl)-2-fluorophenyl)-*N*-(1-((2-(2-((3-hydroxy-3-methylazetidin-1-yl)methyl)pyrrolidin-1-yl)pyrimidin-5-yl)methyl)-1*H*-pyrazol-4-yl)pyrazine-2-carboxamide | Calcd.: 628.1 | |
| | | | Found: 628.2 | |
| **153** | 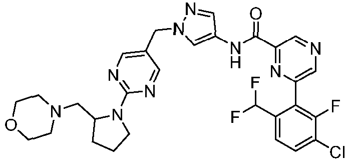 | 6-(3-Chloro-6-(difluoromethyl)-2-fluorophenyl)-*N*-(1-((2-(2-(morpholinomethyl)pyrrolidin-1-yl)pyrimidin-5-yl)methyl)-1*H-*pyrazol-4-yl)pyrazine-2-carboxamide | Calcd.: 628.1 | |
| | | | Found: 628.4 | |
| **154** | 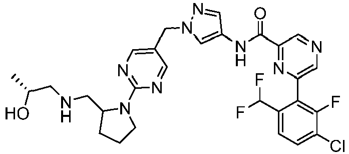 | 6-(3-Chloro-6-(difluoromethyl)-2-*fluorophenyl)-N-(1-((2-(2-((((R)-2-*hydroxypropyl)amino)methyl)pyrroli din-1-yl)pyrimidin-5-yl)methyl)-1*H-*pyrazol-4-yl)pyrazine-2-carboxamide | Calcd.: 616.0 | |
| | | | Found: 616.4 | |
| **155** | 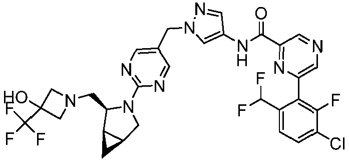 | 6-(3-Chloro-6-(difluoromethyl)-2-fluorophenyl)-*N*-(1-((2-((1R,2S,5S)-2-((3-hydroxy-3-(trifluoromethyl)azetidin-1-yl)methyl)-3-azabicyclo[3.1.0]hexan-3-yl)pyrimidin-5-yl)methyl)-1*H-*pyrazol-4-yl)pyrazine-2-carboxamide | Calcd.: 694.0 | |
| | | | Found: 694.1 | |
| **156** | 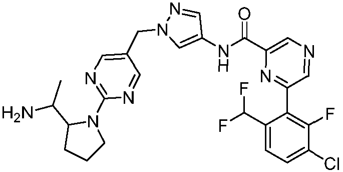 | *N*-(1-((2-(2-(1-Aminoethyl)pyrrolidin-1-yl)pyrimidin-5-yl)methyl)-1*H-*pyrazol-4-yl)-6-(3-chloro-6-(difluoromethyl)-2-fluorophenyl)pyrazine-2-carboxamide | Calcd.: 572.0 | CHIRAL PAK IG Mixture of peak 1 and 2 |
| | | | Found: 572.2 | |
| **157** | 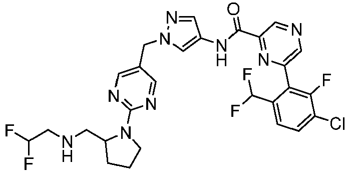 | 6-(3-Chloro-6-(difluoromethyl)-2-fluorophenyl)-*N*-(1-((2-(2-(((2,2-difluoroethyl)amino)methyl)pyrrolidi n-1-yl)pyrimidin-5-yl)methyl)-1*H-*pyrazol-4-yl)pyrazine-2-carboxamide | Calcd.: 622.0 | |
| | | | Found: 622.3 | |
| **158** | 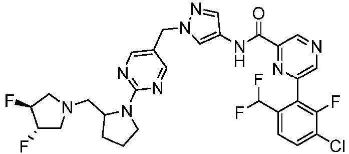 | 6-(3-Chloro-6-(difluoromethyl)-2-fluorophenyl)-*N*-(1-((2-(2-(((3S,4S)-3,4-difluoropyrrolidin-1-yl)methyl)pyrrolidin-1-yl)pyrimidin-5-yl)methyl)-1*H*-pyrazol-4-yl)pyrazine-2-carboxamide | Calcd.: 648.0 | |
| | | | Found: 648.4 | |
| **159** | 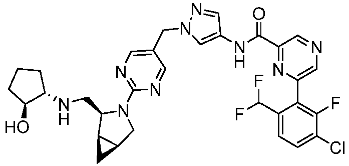 | 6-(3-Chloro-6-(difluoromethyl)-2-fluorophenyl)-*N*-(1-((2-((1*R*,2*S*,5*S*)-2-((((1*S*,2*S*)-2-hydroxycyclopentyl)amino)methyl)-3-azabicyclo[3.1.0]hexan-3-yl)pyrimidin-5-yl)methyl)-1*H-*pyrazol-4-yl)pyrazine-2-carboxamide | Calcd.: 654.1 | |
| | | | Found: 654.2 | |
| **160** | 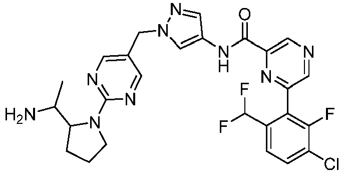 | *N*-(1-((2-(2-(1-Aminoethyl)pyrrolidin-1-yl)pyrimidin-5-yl)methyl)-1*H-*pyrazol-4-yl)-6-(3-chloro-6-(difluoromethyl)-2-fluorophenyl)pyrazine-2-carboxamide | Calcd.: 572.0 | CHIRAL PAK IG Peak 3 |
| | | | Found: 572.1 | |
| **161** | 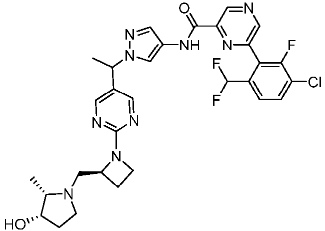 | 6-(3-Chloro-6-(difluoromethyl)-2-fluorophenyl)-*N*-(1-(1-(2-((S)-2-(((2*S*,3*S*)-3-hydroxy-2-methylpyrrolidin-1-yl)methyl)azetidin-1-yl)pyrimidin-5-yl)ethyl)-1*H*-pyrazol-4-yl)pyrazine-2-carboxamide | Calcd. 642.2 | |
| | | | found 642.2 | |
| **162** | 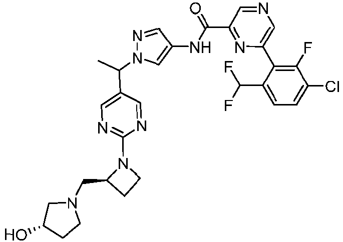 | 6-(3-Chloro-6-(difluoromethyl)-2-fluorophenyl)-*N*-(1-(1-(2-((*S*)-2-(((*S*)-3-hydroxypyrrolidin-1-yl)methyl)azetidin-1-yl)pyrimidin-5-yl)ethyl)-1*H*-pyrazol-4-yl)pyrazine-2-carboxamide | Calcd. 628.2 | |
| | | | found 628.2 | |
| **163** | 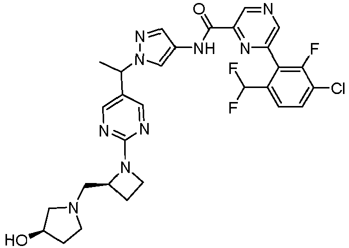 | 6-(3-Chloro-6-(difluoromethyl)-2-fluorophenyl)-*N*-(1-(1-(2-((S)-2-(((R)-3-hydroxypyrrolidin-1-yl)methyl)azetidin-1-yl)pyrimidin-5-yl)ethyl)-1*H*-pyrazol-4-yl)pyrazine-2-carboxamide | Calcd. 628.2 | |
| | | | found 628.3 | |
| **164** | 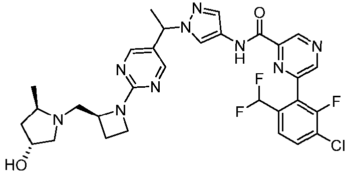 | 6-(3-Chloro-6-(difluoromethyl)-2-fluorophenyl)-*N*-(1-(1-(2-((S)-2-(((2*R*,4*R*)-4-hydroxy-2-methylpyrrolidin-1-yl)methyl)azetidin-1-yl)pyrimidin-5-yl)ethyl)-1*H*-pyrazol-4-yl)pyrazine-2-carboxamide | Calcd. 641.2 | |
| | | | found 642.2 | |
| **165** | 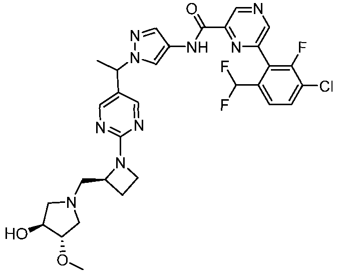 | 6-(3-Chloro-6-(difluoromethyl)-2-fluorophenyl)-*N*-(1-(1-(2-((S)-2-(((3*S*,4*S*)-3-hydroxy-4-methoxypyrrolidin-1-yl)methyl)azetidin-1-yl)pyrimidin-5-yl)ethyl)-1*H*-pyrazol-4-yl)pyrazine-2-carboxamide | Calcd. 658.2 | |
| | | | found 658.3 | |
| **166** | 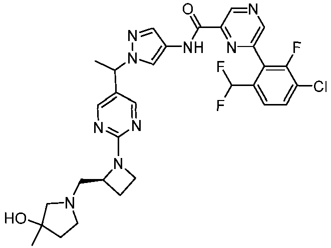 | 6-(3-Chloro-6-(difluoromethyl)-2-fluorophenyl)-*N*-(1-(1-(2-((2*S*)-2-((3-hydroxy-3-methylpyrrolidin-1-yl)methyl)azetidin-1-yl)pyrimidin-5-yl)ethyl)-1*H*-pyrazol-4-yl)pyrazine-2-carboxamide | Calcd. 642.2 | |
| | | | found 642.3 | |
| **167** | 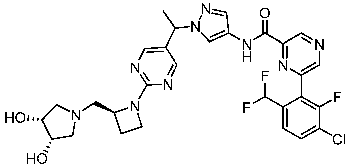 | 6-(3-Chloro-6-(difluoromethyl)-2-fluorophenyl)-*N*-(1-(1-(2-((S)-2-(((3*R*,4*S*)-3,4-dihydroxypyrrolidin-1-yl)methyl)azetidin-1-yl)pyrimidin-5-yl)ethyl)-1*H*-pyrazol-4-yl)pyrazine-2-carboxamide | Calcd. 643.2 | |
| | | | found 644.1 | |
| **168** | 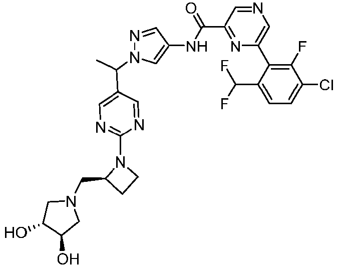 | 6-(3-Chloro-6-(difluoromethyl)-2-fluorophenyl)-*N*-(1-(1-(2-((S)-2-(((3*R*,4*R*)-3,4-dihydroxypyrrolidin-1-yl)methyl)azetidin-1-yl)pyrimidin-5-yl)ethyl)-1*H*-pyrazol-4-yl)pyrazine-2-carboxamide | Calcd. 644.2 | |
| | | | found 644.1 | |
| **169** | 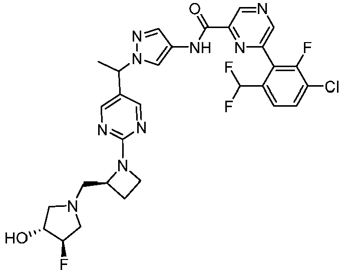 | 6-(3-Chloro-6-(difluoromethyl)-2-fluorophenyl)-*N*-(1-(1-(2-((S)-2-(((3*R*,4*R*)-3-fluoro-4-hydroxypyrrolidin-1-yl)methyl)azetidin-1-yl)pyrimidin-5-yl)ethyl)-1*H*-pyrazol-4-yl)pyrazine-2-carboxamide | Calcd. 646.2 | |
| | | | found 646.2 | |
| **170** | 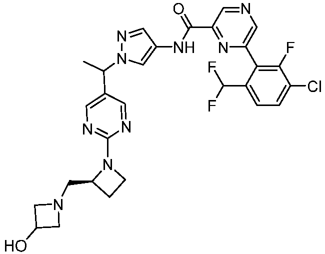 | 6-(3-Chloro-6-(difluoromethyl)-2-fluorophenyl)-*N*-(1-(1-(2-((S)-2-((3-hydroxyazetidin-1-yl)methyl)azetidin-1-yl)pyrimidin-5-yl)ethyl)-1*H*-pyrazol-4-yl)pyrazine-2-carboxamide | Calcd. 614.2 | |
| | | | found 614.3 | |
| **171** | 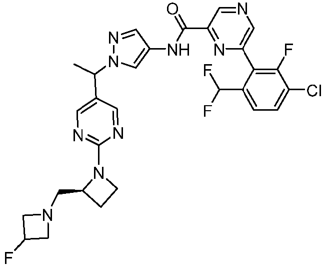 | 6-(3-Chloro-6-(difluoromethyl)-2-fluorophenyl)-*N*-(1-(1-(2-((S)-2-((3-fluoroazetidin-1-yl)methyl)azetidin-1-yl)pyrimidin-5-yl)ethyl)-1*H*-pyrazol-4-yl)pyrazine-2-carboxamide | Calcd. 616.2 | |
| | | | found 616.2 | |
| **172** | 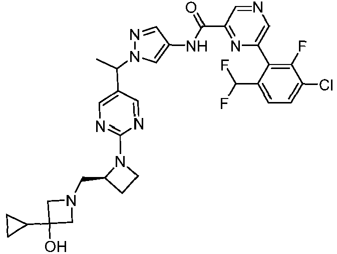 | 6-(3-Chloro-6-(difluoromethyl)-2-fluorophenyl)-*N*-(1-(1-(2-((*S*)-2-((3-cyclopropyl-3-hydroxyazetidin-1-yl)methyl)azetidin-1-yl)pyrimidin-5-yl)ethyl)-1*H*-pyrazol-4-yl)pyrazine-2-carboxamide | Calcd. 654.2 | |
| | | | found 654.2 | |
| **173** | 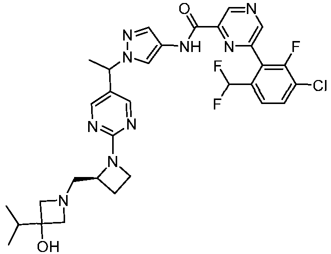 | 6-(3-Chloro-6-(difluoromethyl)-2-fluorophenyl)-*N*-(1-(1-(2-((S)-2-((3-hydroxy-3-isopropylazetidin-1-yl)methyl)azetidin-1-yl)pyrimidin-5-yl)ethyl)-1*H*-pyrazol-4-yl)pyrazine-2-carboxamide | Calcd. 656.2 | |
| | | | found 656.3 | |
| **174** | 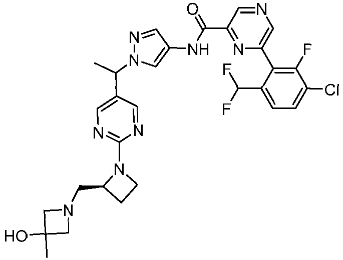 | 6-(3-Chloro-6-(difluoromethyl)-2-fluorophenyl)-*N*-(1-(1-(2-((S)-2-((3-hydroxy-3-methylazetidin-1-yl)methyl)azetidin-1-yl)pyrimidin-5-yl)ethyl)-1*H*-pyrazol-4-yl)pyrazine-2-carboxamide | Calcd. 628.2 | |
| | | | found 628.2 | |
| **175** | 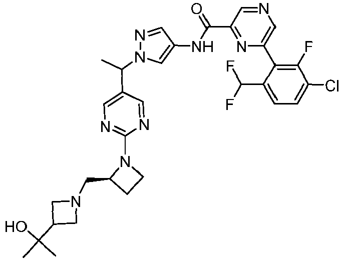 | 6-(3-Chloro-6-(difluoromethyl)-2-fluorophenyl)-*N*-(1-(1-(2-((S)-2-((3-(2-hydroxypropan-2-yl)azetidin-1-yl)methyl)azetidin-1-yl)pyrimidin-5-yl)ethyl)-1*H*-pyrazol-4-yl)pyrazine-2-carboxamide | Calcd. 656.2 | |
| | | | found 656.2 | |
| **176** | 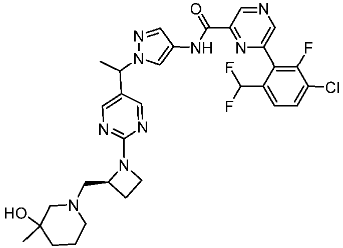 | 6-(3-Chloro-6-(difluoromethyl)-2-fluorophenyl)-*N*-(1-(1-(2-((2*S*)-2-((3-hydroxy-3-methylpiperidin-1-yl)methyl)azetidin-1-yl)pyrimidin-5-yl)ethyl)-1*H*-pyrazol-4-yl)pyrazine-2-carboxamide | Calcd. 656.2 | |
| | | | found 656.3 | |
| **177** | 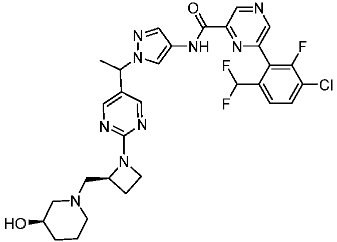 | 6-(3-Chloro-6-(difluoromethyl)-2-fluorophenyl)-*N*-(1-(1-(2-((S)-2-(((R)-3-hydroxypiperidin-1-yl)methyl)azetidin-1-yl)pyrimidin-5-yl)ethyl)-1*H*-pyrazol-4-yl)pyrazine-2-carboxamide | Calcd. 642.2 | |
| | | | found 642.3 | |
| **178** | 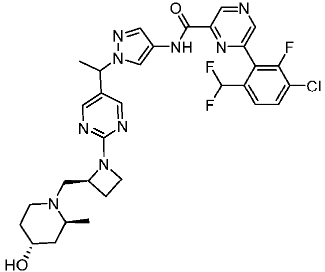 | 6-(3-Chloro-6-(difluoromethyl)-2-fluorophenyl)-*N*-(1-(1-(2-((S)-2-(((2*S*,4*R*)-4-hydroxy-2-methylpiperidin-1-yl)methyl)azetidin-1-yl)pyrimidin-5-yl)ethyl)-1*H-*pyrazol-4-yl)pyrazine-2-carboxamide | Calcd. 656.2 | |
| | | | found 656.3 | |
| **179** | 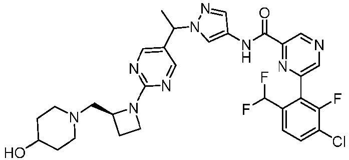 | 6-(3-Chloro-6-(difluoromethyl)-2-fluorophenyl)-*N*-(1-(1-(2-((S)-2-((4-hydroxypiperidin-1-yl)methyl)azetidin-1-yl)pyrimidin-5-yl)ethyl)-1*H*-pyrazol-4-yl)pyrazine-2-carboxamide | Calcd. 641.2 | |
| | | | found 642.3 | |
| **180** | 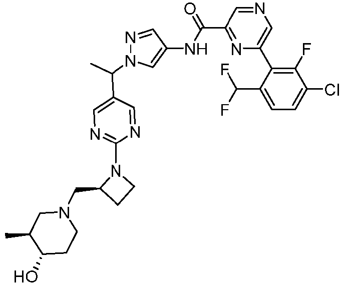 | 6-(3-Chloro-6-(difluoromethyl)-2-fluorophenyl)-*N*-(1-(1-(2-((S)-2-(((3*S*,4*S*)-4-hydroxy-3-methylpiperidin-1-yl)methyl)azetidin-1-yl)pyrimidin-5-yl)ethyl)-1*H-*pyrazol-4-yl)pyrazine-2-carboxamide | Calcd. 656.2 | |
| | | | found 656.3 | |
| **181** | 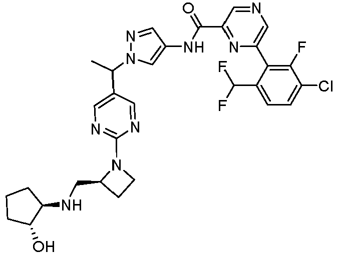 | 6-(3-Chloro-6-(difluoromethyl)-2-fluorophenyl)-*N*-(1-(1-(2-((S)-2-((((1*R*,2*R*)-2-hydroxycyclopentyl)amino)methyl)az etidin-1-yl)pyrimidin-5-yl)ethyl)-1*H-*pyrazol-4-yl)pyrazine-2-carboxamide | Calcd. 642.2 | |
| | | | found 642.3 | |
| **182** | 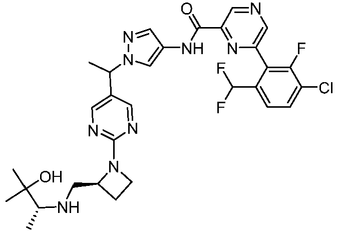 | 6-(3-Chloro-6-(difluoromethyl)-2-fluorophenyl)-*N*-(1-(1-(2-((S)-2-((((*R*)-3-hydroxy-3-methylbutan-2-yl)amino)methyl)azetidin-1-yl)pyrimidin-5-yl)ethyl)-1*H*-pyrazol-4-yl)pyrazine-2-carboxamide | Calcd. 644.2 | |
| | | | found 644.3 | |
| **183** | 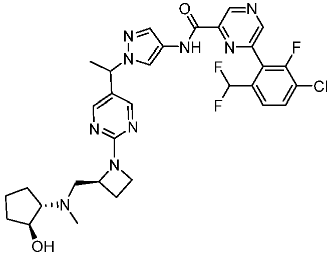 | 6-(3-Chloro-6-(difluoromethyl)-2-fluorophenyl)-*N*-(1-(1-(2-((S)-2-((((1*S*,2*S*)-2-hydroxycyclopentyl)(methyl)amino) methyl)azetidin-1-yl)pyrimidin-5-yl)ethyl)-1*H*-pyrazol-4-yl)pyrazine-2-carboxamide | Calcd. 656.2 | |
| | | | found 656.2 | |
| **184** | 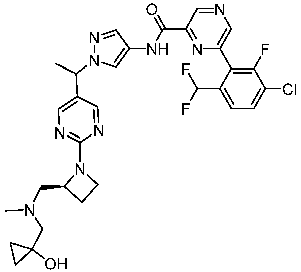 | 6-(3-Chloro-6-(difluoromethyl)-2-fluorophenyl)-*N*-(1-(1-(2-((S)-2-((((1*S*,2*S*)-2-hydroxycyclopentyl)(methyl)amino) methyl)azetidin-1-yl)pyrimidin-5-yl)ethyl)-1*H*-pyrazol-4-yl)pyrazine-2-carboxamide | Calcd. 642.2 | |
| | | | found 642.3 | |
| **185** | 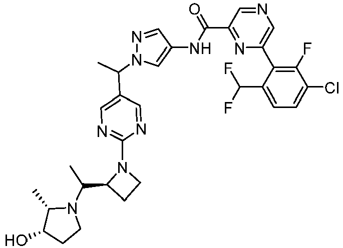 | 6-(3-Chloro-6-(difluoromethyl)-2-fluorophenyl)-*N*-(1-(1-(2-((S)-2-((R)-1-((2*S*,3*S*)-3-hydroxy-2-methylpyrrolidin-1-yl)ethyl)azetidin-1-yl)pyrimidin-5-yl)ethyl)-1*H-*pyrazol-4-yl)pyrazine-2-carboxamide | Calcd. 656.2 | |
| | | | found 656.2 | |
| **186** | 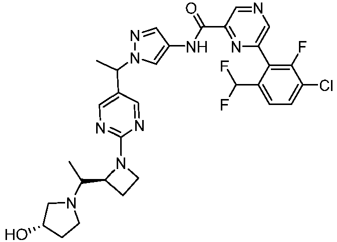 | 6-(3-Chloro-6-(difluoromethyl)-2-fluorophenyl)-*N*-(1-(1-(2-((*S*)-2-((*R*)-1-((*S*)-3-hydroxypyrrolidin-1-yl)ethyl)azetidin-1-yl)pyrimidin-5-yl)ethyl)-1*H*-pyrazol-4-yl)pyrazine-2-carboxamide | Calcd. 642.2 | |
| | | | found 642.3 | |
| **187** | 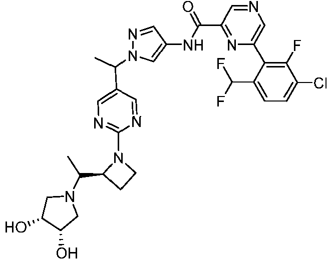 | 6-(3-Chloro-6-(difluoromethyl)-2-fluorophenyl)-*N*-(1-(1-(2-((S)-2-((R)-1-((3*R*,4*S*)-3,4-dihydroxypyrrolidin-1-yl)ethyl)azetidin-1-yl)pyrimidin-5-yl)ethyl)-1*H*-pyrazol-4-yl)pyrazine-2-carboxamide | Calcd. 658.2 | |
| | | | found 658.2 | |
| **188** | 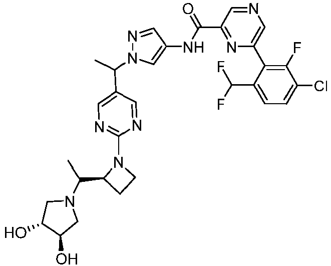 | 6-(3-Chloro-6-(difluoromethyl)-2-fluorophenyl)-*N*-(1-(1-(2-((*S*)-2-((*R*)-1-((3*R*,4*R*)-3,4-dihydroxypyrrolidin-1-yl)ethyl)azetidin-1-yl)pyrimidin-5-yl)ethyl)-1*H*-pyrazol-4-yl)pyrazine-2-carboxamide | Calcd. 658.2 | |
| | | | found 658.3 | |
| **189** | 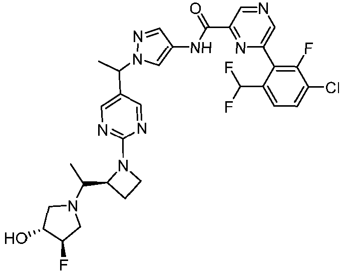 | 6-(3-Chloro-6-(difluoromethyl)-2-fluorophenyl)-*N*-(1-(1-(2-((S)-2-((R)-1-((3*R*,4*R*)-3-fluoro-4-hydroxypyrrolidin-1-yl)ethyl)azetidin-1-yl)pyrimidin-5-yl)ethyl)-1*H*-pyrazol-4-yl)pyrazine-2-carboxamide | Calcd. 660.2 | |
| | | | found 660.2 | |
| **190** | 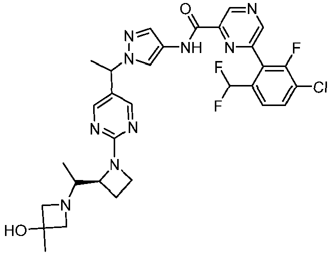 | 6-(3-Chloro-6-(difluoromethyl)-2-fluorophenyl)-*N*-(1-(1-(2-((S)-2-((*R*)-1-(3-hydroxy-3-methylazetidin-1-yl)ethyl)azetidin-1-yl)pyrimidin-5-yl)ethyl)-1*H*-pyrazol-4-yl)pyrazine-2-carboxamide | Calcd. 642.2 | |
| | | | found 642.3 | |
| **191** | 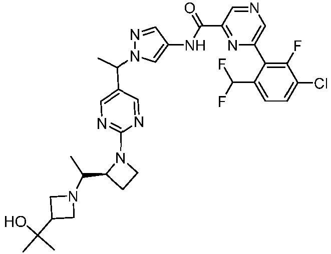 | 6-(3-Chloro-6-(difluoromethyl)-2-fluorophenyl)-*N*-(1-(1-(2-((*S*)-2-((*R*)-1-(3-(2-hydroxypropan-2-yl)azetidin-1-yl)ethyl)azetidin-1-yl)pyrimidin-5-yl)ethyl)-1*H*-pyrazol-4-yl)pyrazine-2-carboxamide | Calcd. 670.3 | |
| | | | found 670.3 | |
| **192** | 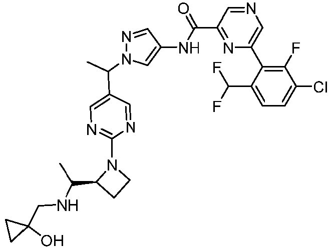 | 6-(3-Chloro-6-(difluoromethyl)-2-fluorophenyl)-*N*-(1-(1-(2-((*S*)-2-((*R*)-1-(((1-hydroxycyclopropyl)methyl)amino)et hyl)azetidin-1-yl)pyrimidin-5-yl)ethyl)-1*H*-pyrazol-4-yl)pyrazine-2-carboxamide | Calcd. 642.2 | |
| | | | found 642.3 | |
| **193** | 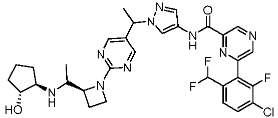 | 6-(3-Chloro-6-(difluoromethyl)-2-fluorophenyl)-*N*-(1-(1-(2-((S)-2-((R)-1-(((1*R*,2*R*)-2-hydroxycyclopentyl)amino)ethyl)azet idin-1-yl)pyrimidin-5-yl)ethyl)-1*H-*pyrazol-4-yl)pyrazine-2-carboxamide | Calcd. 655.2 | |
| | | | found 656.3 | |
| **194** | 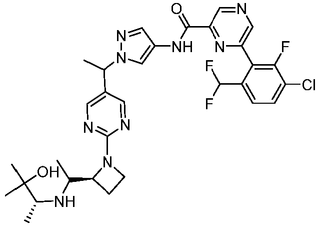 | 6-(3-Chloro-6-(difluoromethyl)-2-fluorophenyl)-*N*-(1-(1-(2-((S)-2-((R)-1-(((*R*)-3-hydroxy-3-methylbutan-2-yl)amino)ethyl)azetidin-1-yl)pyrimidin-5-yl)ethyl)-1*H*-pyrazol-4-yl)pyrazine-2-carboxamide | Calcd. 658.3 | |
| | | | found 658.3 | |

### Example 195

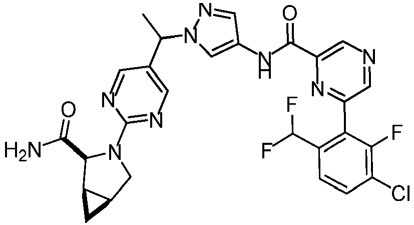

### (cis)-3 -(5-(1-(4-(6-(3-Chloro-6-(difluoromethyl)-2-fluorol)henyl)pyrazine-2-carboxamido)-1H-pyrazol-1-yl)ethyl)pyrimidin-2-yl)-3-azabicyclo[3.1.0]hexane-2-carboxamide (Scheme AAK)

To a mixture of (*cis*)-3-(5-(1-(4-(6-(3-chloro-6-(difluoromethyl)-2-fluorophenyl)pyrazine-2-carboxamido)-1*H*-pyrazol-1-yl)ethyl)pyrimidin-2-yl)-3-azabicyclo[3.1.0]hexane-2-carboxylic acid (320 mg, 0.534 mmol) in DMF (3 mL) was added 1-hydroxy-1*H*-benzotriazole, ammonium salt (163 mg, 1.07 mmol) and EDC (205 mg, 1.07 mmol), and the resulting mixture was stirred at rt for 16 h. The reaction was filtered, and the filtrate was purified by reversed-phase preparatory-HPLC (C18 stationary phase, water (0.1%TFA) - ACN) to afford the title compound. ¹H NMR (400 MHz, CDCl₃) δ 9.47-9.50 (m, 2H), 9.05 (d, *J* = 2.8 Hz, 1H), 8.31 (d, *J =* 2.8 Hz, 2H), 8.17 (s, 1H), 7.72-7.74 (m, 1H), 7.63-7.68 (m, 1H), 7.59 (s, 1H), 6.59 (t, *J =* 56.0 Hz, 1H), 5.90-6.01 (m, 2H), 5.38-5.42 (m, 1H), 4.48 (d, *J =* 5.6 Hz, 1H), 3.88 (d, *J =* 11.2 Hz, 1H), 3.71-3.76 (m, 1H), 2.09-2.12 (m, 1H), 1.90 (d, *J =* 7.2 Hz, 3H), 1.78-1.81 (m, 1H), 0.81-0.86 (m, 1H), 0.70-0.72 (m, 1H). MS = 598.1 [M+H]⁺.

### Examples 196 - 199

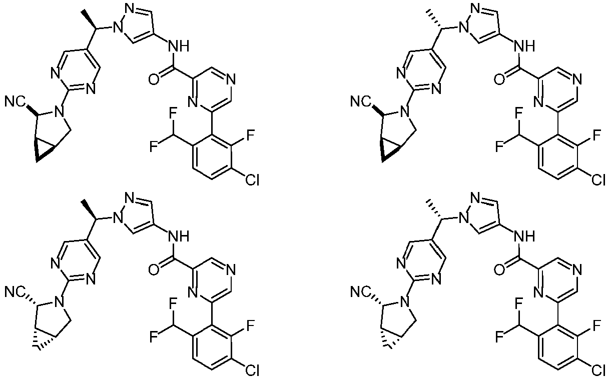

### 6-(3-Chloro-6-(difluoromethyl)-2-fluorophenyl)-N-(1-((R)-1-(2-((1R,2S,5S)-2-cyano-3-azabicyclo[3.1.0]hexan-3-yl)pyrimidin-5-yl)ethyl)-1H-pyrazol-4-yl)pyrazine-2-carboxamide: 6-(3-chloro-6-(difluoromethyl)-2-fluorophenyl)-N-(1-((R)-1-(2-((1S,2R,5R)-2-cyano-3-azabicyclo[3.1.0]hexan-3-yl)pyrimidin-5-yl)ethyl)-1H-pyrazol-4-yl)pyrazine-2-carboxamide, 6-(3-chloro-6-(difluoromethyl)-2-fluorophenyl)-N-(1-((S)-1-(2-((1R,2S,5S)-2-cyano-3-azabicyclo[3.1.0]hexan-3-yl)pyrimidin-5-yl)ethyl)-1H-pyrazol-4-yl)pyrazine-2-carboxamide: 6-(3-chloro-6-(difluoromethyl)-2-fluorophenyl)-N-(1-((S)-1-(2-((1S,2R,5R)-2-cyano-3-azabicyclo[3.1.0]hexan-3-yl)pyrimidin-5-yl)ethyl)-1H-pyrazol-4-yl)pyrazine-2-carboxamide (Scheme AAK)

To a mixture of (*cis*)-3-(5-(1-(4-(6-(3-chloro-6-(difluoromethyl)-2-fluorophenyl)pyrazine-2-carboxamido)-1*H*-pyrazol-1-yl)ethyl)pyrimidin-2-yl)-3-azabicyclo[3.1.0]hexane-2-carboxamide (220 mg, 0.368 mmol) in DCM (5 mL) was added TEA (77 µL, 0.55 mmol). TFAA (78 µL, 0.55 mmol) was added to the mixture at 0 °C, and the resulting mixture was warmed to rt and allowed to stir for 16 h. The reaction was concentrated, and the crude residue was purified by preparatory-TLC ((1:1) EtOAc/petroleum ether) to give the mixture of four stereoisomers, which were separated by Chiral SFC (DAICEL CHIRALPAK AD 0.1%NH₃H₂O EtOH, Begin B 55%, End B 55%) to the first eluting isomer **Example 196:** ¹H NMR (400 MHz, CDCl₃) δ 9.51 (s, 1H), 9.46 (s, 1H), 9.04 (d, *J =* 2.4 Hz, 1H), 8.35 (s, 2H), 8.16 (s, 1H), 7.74 (m, 1H), 7.66 (d, *J =* 8.4 Hz, 1H), 7.59 (s, 1H), 6.59 (t, *J =* 54.0 Hz, 1H), 5.41 (q, *J* = 6.8 Hz, 1H), 4.72 (d, *J* = 5.2 Hz, 1H), 3.86 (d, *J =* 7.6 Hz, 1H), 3.63 (dd, *J* = 10.8 Hz, 4.0 Hz, 1H), 2.10-2.12 (m, 1H), 1.91 (d, *J =* 8.0 Hz, 4H), 1.06-1.08 (m, 1H), 0.79-0.80 (m, 1H). MS = 580.1 [M+H]⁺.
The mixture of other three isomers required an additional separation by chiral SFC (REGIS (s,s) WHELK-O1, 0.1%NH₃ H₂O-EtOH Begin B 45%, End B 45%). The second overall eluting isomer **Example 197:** ¹H NMR (400 MHz, CDCl₃) δ 9.51 (s, 1H), 9.46 (s, 1H), 9.04 (d, *J =* 2.4 Hz, 1H), 8.33 (s, 2H), 8.17 (s, 1H), 7.74 (m, 1H), 7.66 (d, *J =* 8.4 Hz, 1H), 7.60 (s, 1H), 6.59 (t, *J* = 54.0 Hz, 1H), 5.42 (q, *J =* 6.8 Hz, 1H), 4.71 (d, *J =* 5.2 Hz, 1H), 3.86 (d, *J =* 7.6 Hz, 1H), 3.63 (dd, *J =* 10.8 Hz, 4.0 Hz, 1H), 2.09-2.12 (m, 1H), 1.91 (d, *J =* 8.0 Hz, 4H), 1.04-1.07 (m, 1H), 0.77-0.81 (m, 1H). MS = 580.1 [M+H]⁺. The third overall eluting isomer **Example 198:** ¹H NMR (400 MHz, CDCl₃) δ 9.50 (s, 1H), 9.46 (s, 1H), 9.04 (d, *J* = 2.4 Hz, 1H), 8.35 (s, 2H), 8.17 (s, 1H), 7.74 (m, 1H), 7.66 (d, *J =* 8.4 Hz, 1H), 7.60 (s, 1H), 6.59 (t, *J =* 54.0 Hz, 1H), 5.42 (q, *J* = 6.8 Hz, 1H), 4.71 (d, *J =* 5.2 Hz, 1H), 3.86 (d, *J =* 7.6 Hz, 1H), 3.63 (dd, *J =* 10.8 Hz, 4.0 Hz, 1H), 2.09-2.12 (m, 1H), 1.91 (d, *J =* 8.0 Hz, 4H), 1.04-1.10 (m, 1H), 0.77-0.81 (m, 1H). MS = 580.1 [M+H]⁺. The fourth overall eluting isomer **Example 199:** ¹H NMR (400 MHz, CDCl₃) δ 9.51 (s, 1H), 9.46 (s, 1H), 9.04 (d, *J =* 2.4 Hz, 1H), 8.33 (s, 2H), 8.17 (s, 1H), 7.74 (m, 1H), 7.66 (d, *J* = 8.4 Hz, 1H), 7.60 (s, 1H), 6.59 (t, *J =* 54.0 Hz, 1H), 5.42 (q, *J =* 6.8 Hz, 1H), 4.71 (d, *J =* 5.2 Hz, 1H), 3.86 (d, *J =* 7.6 Hz, 1H), 3.63 (dd, *J =* 10.8 Hz, 4.0 Hz, 1H), 2.09-2.12 (m, 1H), 1.91 (d, *J =* 8.0 Hz, 4H), 1.04-1.09 (m, 1H), 0.77-0.81 (m, 1H). MS = 580.1 [M+H]⁺.

**Table AAK. The following compounds were prepared using procedures similar to those described above using the appropriate starting materials. In some examples, racemic products or diatereomeric mixtures were separated using chiral columns (SFC or HPLC) specified in the table.**

| **Example** | **Structure** | **IUPAC Name** | **[M+H]⁺ or [M+Na]⁺** | **Chiral Column** |
|---|---|---|---|---|
| **200** | 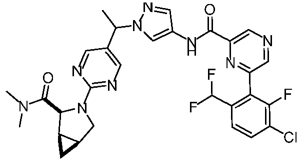 | (1*R*,2*S*,5*S* or 1*S*,2*R*,5*R*)-3-(5-((*R* or *S*)-1-(4-(6-(3-Chloro-6-(difluoromethyl)-2-fluorophenyl)pyrazine-2-carboxamido)-1*H*-pyrazol-1-yl)ethyl)pyrimidin-2-yl)-*N*,*N-*dimethyl-3-azabicyclo[3.1.0]hexane-2-carboxamide | [M/2+H]⁺ | DAICEL CHIRALPAK AD and IG |
| | | | Calcd.: 313.6 | |
| | | | Found: 313.7 | |
| **201** | 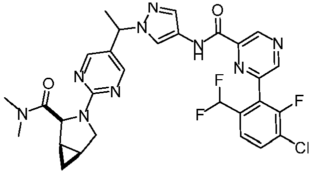 | (1*R*,2*S*,5*S* or 1*S*,2*R*,5*R*)-3-(5-((*S* or *R*)-1-(4-(6-(3-Chloro-6-(difluoromethyl)-2-fluorophenyl)pyrazine-2-carboxamido)-1*H*-pyrazol-1-yl)ethyl)pyrimidin-2-yl)-*N*,*N-*dimethyl-3-azabicyclo[3.1.0]hexane-2-carboxamide | [M/2+H]⁺ | DAICEL CHIRALPAK AD and IG |
| | | | Calcd.: 313.6 | |
| | | | Found: 313.6 | |
| **202** | 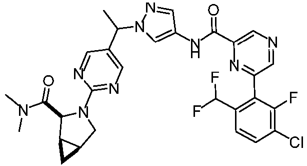 | (1*R*,2*S*,5*S* or 1*S,*2*R,*5*R*)-3-(5-((*R* or *S*)-1-(4-(6-(3-Chloro-6-(difluoromethyl)-2-fluorophenyl)pyrazine-2-carboxamido)-1*H*-pyrazol-1-yl)ethyl)pyrimidin-2-yl)-*N*,*N-*dimethyl-3-azabicyclo[3.1.0]hexane-2-carboxamide | Calcd.: 626.2 | DAICEL CHIRALPAK AD |
| | | | Found: 626.1 | |
| **203** | 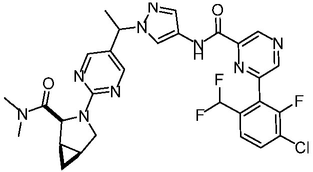 | (1*R*,2*S*,5*S* or 1*S*,2*R*,5*R*)-3-(5-((*S* or *R*)-1-(4-(6-(3-Chloro-6-(difluoromethyl)-2-fluorophenyl)pyrazine-2-carboxamido)-1*H*-pyrazol-1-yl)ethyl)pyrimidin-2-yl)-*N*,*N-*dimethyl-3-azabicyclo[3.1.0]hexane-2-carboxamide | [M/2+H]⁺ | DAICEL CHIRALPAK AD |
| | | | Calcd.: 313.6 | |
| | | | Found: 313.6 | |

### Example 204

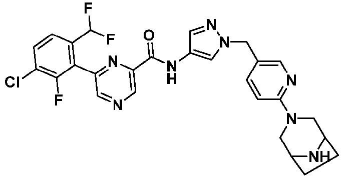

### N-(1-((6-(3,8-Diazabicyclo[3.2.1]octan-3-yl)pyridin-3-yl)methyl)-1H-pyrazol-4-yl)-6-(3-chloro-6-(difluoromethyl)-2-fluorophenyl)pyrazine-2-carboxamide (Scheme AAL)

TFA (274 µl) was added to a stirred mixture of tert-butyl 3-(5-((4-(6-(3-chloro-6-(difluoromethyl)-2-fluorophenyl)pyrazine-2-carboxamido)-1*H*-pyrazol-1-yl)methyl)pyridin-2-yl)-3,8-diazabicyclo[3.2.1]-octane-8-carboxylate (55 mg, 0.082 mmol) in DCM (548 µl) and the mixture was stirred at room temperature for 2 h.. After evaporation of the volatile components, the residue was purified by reversed-phase preparatory-HPLC (C18 stationary phase, water(0.1%TFA)-ACN to give the title compound. ¹H NMR (600 MHz, CD₃OD) δ 9.39 (s, 1H), 9.02 (d, *J =* 1.9 Hz, 1H), 8.23 (s, 1H), 8.14 (d, *J* = 2.0 Hz, 1H), 7.84 (dd, *J =* 7.8 Hz, 1H), 7.74 (s, 1H), 7.69 - 7.62 (m, 2H), 7.11 - 6.89 (m, 2H), 5.26 (s, 2H), 4.22 - 4.17 (m, 4H), 3.26 (d, *J =* 12.3 Hz, 2H), 2.18 - 1.94 (m, 4H). MS = 569.4 [M+H]⁺.

### Example 205

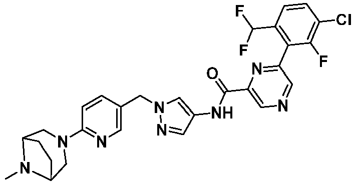

### 6-(3-Chloro-6-(difluoromethyl)-2-fluorophenyl)-N-(1-((6-(8-methyl-3,8-diazabicyclo[3.2.1]octan-3-yl)pyridin-3-yl)methyl)-1H-pyrazol-4-yl)pyrazine-2-carboxamide (Scheme AAL)

Sodium triacetoxyborohydride (47 mg, 0.22 mmol) was added to a stirred mixture of *N*-(1-((6-(3,8-diazabicyclo[3.2.1]octan-3-yl)pyridin-3-yl)methyl)-1*H*-pyrazol-4-yl)-6-(3-chloro-6-(difluoromethyl)-2-fluorophenyl)pyrazine-2-carboxamide (25 mg, 0.044 mmol), formaldehyde (16 µl, 0.22 mmol, 37 % w/v aq. solution), acetic acid (2.5 µl, 0.044 mmol) in MeOH (440 µl) and the mixture was stirred at rt for 4 h. After evaporation of the solvent, the residue was purified by reversed-phase preparatory-HPLC (C18 stationary phase, water(0.1%TFA)-ACN to give the title compound. ¹H NMR (600 MHz, CD₃OD) δ 9.39 (s, 1H), 9.02 (d, *J* = 1.9 Hz, 1H), 8.22 (s, 1H), 8.16 (d, *J* = 1.9 Hz, 1H), 7.84 (d, *J* = 7.8 Hz, 1H), 7.74 (s, 1H), 7.68 - 7.60 (m, 2H), 7.11 - 6.86 (m, 2H), 5.25 (s, 2H), 4.27 (d, *J =* 13.4 Hz, 2H), 4.08 (s, 2H), 2.89 (s, 3H), 2.30 (s, 2H), 2.07 (d, *J =* 8.5 Hz, 2H). MS = 583.4 [M+H]⁺.

### Example 206

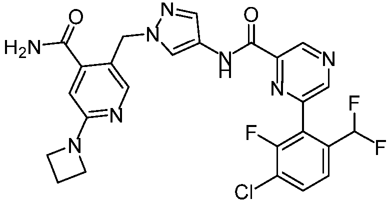

### N-(1-((6-(Azetidin-1-yl)-4-carbamoylpyridin-3-yl)methyl)-1H-pyrazol-4-yl)-6-(3-chloro-6-(difluoromethyl)-2-fluorophenyl)pyrazine-2-carboxamide

To a solution of *N*-(1-((6-(azetidin-1-yl)-4-cyanopyridin-3-yl)methyl)-1*H*-pyrazol-4-yl)-6-(3-chloro-6-(difluoromethyl)-2-fluorophenyl)pyrazine-2-carboxamide (60 mg, 0.11 mmol) in DMSO (3 mL) and EtOH (2 mL) at 0 °C were added K₂CO₃ (31 mg, 0.22 mmol) and H₂O₂ (0.029 mL, 0.33 mmol). After stirring at 0 °C for 1 h, the reaction was diluted with satd. aq. Na₂S₂O₃ and purified by reversed-phase preparatory-HPLC (C18 stationary phase, water(0.1%TFA)-ACN to give the title compoound: ¹H NMR (400 MHz, CD₃OD) δ 9.38 (s, 1H), 9.02 (d, *J* = 2.2 Hz, 1H), 8.19 (s, 1H), 7.72-7.86 (m, 3H), 7.66 (d, *J =* 8.6 Hz, 1H), 6.78-7.16 (m, 2H), 5.39 (s, 2H), 4.33 (t, *J =* 7.7 Hz, 4H), 2.52-2.65 (m, 2H). MS = 557.2 [M+H]⁺.

### Example 207

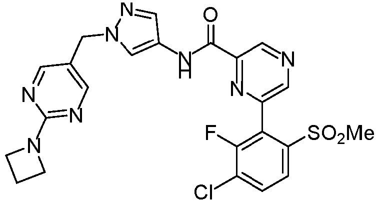

### N-(1-((2-(Azetidin-1-yl)pyrimidin-5-yl)methyl)-1H-pyrazol-4-yl)-6-(3-chloro-2-fluoro-6-(methylsulfonyl)phenyl)pyrazine-2-carboxamide

A mixture of N-(1-((2-(azetidin-1-yl)pyrimidin-5-yl)methyl)-1*H*-pyrazol-4-yl)-6-(3-chloro-2-fluoro-6-(methylsulfinyl)phenyl)pyrazine-2-carboxamide (30 mg, 0.057 mmol) and oxone (70 mg, 0.11 mmol) in THF (1 mL) and water (0.2 mL) was stirred at rt for 3 h. The reaction was quenched with satd. aq. Na₂SO₃ and extracted with DCM. The combined organic layers were dried over Na₂SO₄, filtered and concentrated to give a crude residue that was purified by reversed-phase preparatory-HPLC (C18 stationary phase, water (10 mM, NH₄HCO₃)-ACN to give the title compound. ¹H NMR (400 MHz, CDCl₃) δ 9.51 (d, *J* = 7.3 Hz, 1H), 9.27 (br s, 1H), 8.93 (br d, *J* - 9.3 Hz, 1H), 8.25 - 8.34 (m, 2H), 8.08 - 8.14 (m, 1H), 7.99 (br d, *J =* 8.6 Hz, 1H), 7.77 - 7.88 (m, 1H), 7.50 - 7.57 (m, 1H), 5.10 (br d, *J =* 9.5 Hz, 2H), 4.11 - 4.19 (m, 4H), 2.95 - 3.02 (m, 3H), 2.29 - 2.46 (m, 2H). MS = 543.1 [M+H]⁺.

### Example 208

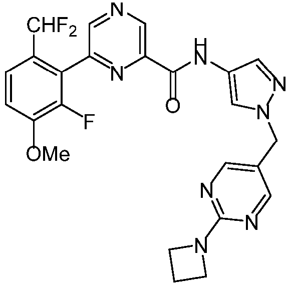

### N-(1-((2-(Azetidin-1-yl)pyrimidin-5-yl)methyl)-1H-pyrazol-4-yl)-6-(6-(difluoromethyl)-2-fluoro-3-methoxyphenyl)pyrazine-2-carboxamide

Me₄-^{t}BuXPhos Pd G3 (1.0 mg, 1.2 µmol) was added to a stirred mixture of Cs₂CO₃ (38 mg, 0.12 mmol) and *N*-(1-((2-(azetidin-1-yl)pyrimidin-5-yl)methyl)-1*H*-pyrazol-4-yl)-6-(3-chloro-6-(difluoromethyl)-2-fluorophenyl)pyrazine-2-carboxamide (30 mg, 0.058 mmol) in dioxane (0.5 mL) and MeOH (0.5 mL) in a glove box. The resulting mixture was heated to 100 °C for 8 h, at which time, the reaction was directly purified by reversed-phase preparatory-HPLC (C18 stationary phase, water(0.1%TFA)-ACN to give the title compound. ¹H NMR (400 MHz, CD₃OD) δ 9.37 (s, 1H), 8.98 (d, *J* = 2.2 Hz, 1H), 8.43 (s, 2H), 8.28 (s, 1H), 7.77 (s, 1H), 7.61 (d, *J =* 8.7 Hz, 1H), 7.41 (t, *J =* 8.4 Hz, 1H), 6.74 - 7.09 (t, *J =* 55.2 Hz, 1H), 5.24 (s, 2H), 4.24 (t, *J =* 7.6 Hz, 4H), 4.01 (s, 3H), 2.46 (quint, *J* = 7.7 Hz, 2H). MS = 511.1 [M+H]⁺.

### Factor Xla assay

The effectiveness of a compound of the present invention as an inhibitor of Coagulation factor XIa can be determined using a relevant purified serine protease, and an appropriate synthetic substrate. The rate of hydrolysis of the chromogenic or fluorogenic substrate by the relevant serine protease was measured both in the absence and presence of compounds of the present invention. Assays were conducted at rt or at 37 °C. Hydrolysis of the substrate resulted in release of amino trifluoromethylcoumarin (AFC), which was monitored spectrofluorometrically by measuring the increase in emission at 510 nm with excitation at 405 nm. A decrease in the rate of fluorescence change in the presence of inhibitor is indicative of enzyme inhibition. Such methods are known to one skilled in the art. The results of this assay are expressed as the half-maximal inhibitory concentrations (IC₅₀), or the inhibitory constant, Kᵢ.

Compounds were pre-incubated for 30 min at 25 °C with human (0.04 nM) factor XIa in 50 mM HEPES buffer with 150 mM sodium chloride, 5 mM calcium chloride, 0.1% PEG 8000, pH 7.4. factor XIa enzymatic activity was determined by addition of the substrate glycine-proline-arginine-7-amido-4-trifluoromethylcoumarin (GPR-AFC) and measurement of the fluorescence at 400/505 nm after a 60 min incubation at 25 °C. The % inhibition for each data point was calculated from the data and analyzed using the log (inhibitor) vs. response four parameters equation to determine the half-maximal inhibitory concentrations (IC₅₀). The IC₅₀ were converted to equilibrium inhibitory constants (Ki) using the Cheng-Prusoff equation.

The activities shown by this assay indicate that the compounds of the invention may be therapeutically useful for treating or preventing various cardiovascular and/or cerebrovascular thromboembolic conditions in patients suffering from unstable angina, acute coronary syndrome, refractory angina, myocardial infarction, transient ischemic attacks, atrial fibrillation, stroke such as thrombotic stroke or embolic stroke, venous thrombosis, coronary and cerebral arterial thrombosis, cerebral and pulmonary embolism, atherosclerosis, deep vein thrombosis, disseminated intravascular coagulation, and reocclusion or restenosis of recanalized vessels.

### Plasma Kallikrein assay

The effectiveness of a compound of the present invention as an inhibitor of plasma kallikrein can be determined using a relevant purified serine protease, and an appropriate synthetic substrate. The rate of hydrolysis of the chromogenic or fluorogenic substrate by the relevant serine protease was measured both in the absence and presence of compounds of the present invention. Assays were conducted at rt or at 37 °C. Hydrolysis of the substrate resulted in release of amino trifluoromethylcoumarin (AFC), which was monitored spectrofluorometrically by measuring the increase in emission at 510 nm with excitation at 405 nm. A decrease in the rate of fluorescence change in the presence of inhibitor is indicative of enzyme inhibition. Such methods are known to one skilled in the art. The results of this assay are expressed as the half-maximal inhibitory concentrations (IC₅₀), or the inhibitory constant, Kᵢ.

Plasma kallikrein determinations were made in 50 mM HEPES buffer at pH 7.4 containing 150 mM NaCl, 5 mM CaCl₂, and 0.1% PEG 8000 (polyethylene glycol; Fisher Scientific). Determinations were made using purified Human plasma kallikrein at a final concentration of 0.5 nM (Enzyme Research Laboratories) and the synthetic substrate, Acetyl-K-P-R-AFC (Sigma # C6608) at a concentration of 100 mM.

Activity assays were performed by diluting a stock solution of substrate at least tenfold to a final concentration ≤ 0.2 Km into a solution containing enzyme or enzyme equilibrated with inhibitor. Times required to achieve equilibration between enzyme and inhibitor were determined in control experiments. The reactions were performed under linear progress curve conditions and fluorescence increase measured at 405 Ex/510 Em nm. Values were converted to percent inhibition of the control reaction (after subtracting 100% Inhibition value). IC₅₀ was determined by inflection point from a four parameter logistic curve fit. Ki was calculated using the Cheng Prusoff equation, Ki = IC₅₀/(1+([S]/Km)).

The activities shown by this assay indicate that the compounds of the invention may be therapeutically useful for treating or preventing various ophthalmic, cardiovascular and/or cerebrovascular thromboembolic conditions in patients suffering from unstable angina, acute coronary syndrome, refractory angina, myocardial infarction, transient ischemic attacks, atrial fibrillation, stroke such as thrombotic stroke or embolic stroke, venous thrombosis, coronary and cerebral arterial thrombosis, cerebral and pulmonary embolism, atherosclerosis, deep vein thrombosis, disseminated intravascular coagulation, reocclusion or restenosis of recanalized vessels, hereditary angioedema, uveitis, posterior uveitis, wet age related macular edema, diabetic macular edema, diabetic retinopathy and retinal vein occlusion.
Plasma Kallikrein IC₅₀ (nM) and FXIa IC₅₀ (nM) for selected compounds are as follows:

| **Example** | **Plasma Kallikrein IC₅₀ (nM)** | **FXla IC₅₀ (nM)** |
|---|---|---|
| 1 | 10.2 | 921 |
| 2 | 15.8 | 1112 |
| 3 | 40.5 | 10000 |
| 4 | 23.1 | 1316 |
| 5 | 6.6 | 1348 |
| 6 | 22.9 | 1693 |
| 7 | 52.1 | 1424 |
| 8 | 4.2 | 458 |
| 9 | 5.2 | 5104 |
| 10 | 5.2 | 647 |
| 11 | 6.2 | 1706 |
| 12 | 6.2 | 3513 |
| 13 | 7.2 | 1845 |
| 14 | 8.0 | 312 |
| 15 | 8.1 | 3689 |
| 16 | 8.4 | 1053 |
| 17 | 10.2 | 1368 |
| 18 | 13.1 | 1999 |
| 19 | 14.6 | 4631 |
| 20 | 15.4 | 10000 |
| 21 | 17.0 | 973 |
| 22 | 22.3 | 4368 |
| 23 | 26.0 | 1757 |
| 24 | 26.8 | 10000 |
| 25 | 41.3 | 6090 |
| 26 | 45.9 | 5354 |
| 27 | 3.8 | 869 |
| 28 | 11.9 | 927 |
| 29 | 20.7 | 1774 |
| 30 | 7.8 | 2167 |
| 31 | 27.0 | 2718 |
| 32 | 8.1 | 1524 |
| 33 | 26.5 | 10000 |
| 34 | 28.1 | 3333 |
| 35 | 33.2 | 10000 |
| 36 | 44.3 | 4425 |
| 37 | 25.6 | 2696 |
| 38 | 5.0 | 2575 |
| 39 | 1.3 | 478 |
| 40 | 3.7 | 794 |
| 41 | 2.1 | 4212 |
| 42 | 4.7 | 1111 |
| 43 | 19.5 | 1174 |
| 44 | 10.3 | 1460 |
| 45 | 23.2 | 992 |
| 46 | 3.7 | 595 |
| 47 | 10.6 | 326 |
| 48 | 730.1 | 10000 |
| 49 | 17.6 | 10000 |
| 50 | 1122.0 | 10000 |
| 51 | 3772.0 | 10000 |
| 52 | 17.1 | 10000 |
| 53 | 30.7 | 10000 |
| 54 | 25.7 | 3032 |
| 55 | 24.4 | 3936 |
| 56 | 27.7 | 1488 |
| 57 | 6.8 | 1170 |
| 58 | 8.1 | 413 |
| 59 | 1.8 | 372 |
| 60 | 47.0 | 730 |
| 61 | 14.0 | 662 |
| 62 | 17.8 | 2149 |
| 63 | 17.3 | 5038 |
| 64 | 9.6 | 292 |
| 65 | 22.2 | 538 |
| 66 | 3.9 | 639 |
| 67 | 153.3 | 10000 |
| 68 | 26.3 | 10000 |
| 69 | 64.3 | 8066 |
| 70 | 1.4 | 485 |
| 71 | 2.5 | 2262 |
| 72 | 2.5 | 10000 |
| 73 | 3.7 | 1495 |
| 74 | 4.3 | 1015 |
| 75 | 5.0 | 10000 |
| 76 | 5.4 | 4635 |
| 77 | 5.6 | 2951 |
| 78 | 5.6 | 10000 |
| 79 | 5.9 | 2858 |
| 80 | 6.7 | 1993 |
| 81 | 7.4 | 3945 |
| 82 | 9.3 | 6013 |
| 83 | 9.4 | 8083 |
| 84 | 11.3 | 7241 |
| 85 | 11.8 | 10000 |
| 86 | 13.3 | 10000 |
| 87 | 13.6 | 8427 |
| 88 | 14.9 | 10000 |
| 89 | 17.3 | 10000 |
| 90 | 17.8 | 10000 |
| 91 | 17.9 | 10000 |
| 92 | 18.5 | 5017 |
| 93 | 19.9 | 4306 |
| 94 | 20.0 | 8234 |
| 95 | 21.7 | 10000 |
| 96 | 25.9 | 3714 |
| 97 | 26.9 | 8933 |
| 98 | 28.2 | 10000 |
| 99 | 29.8 | 3659 |
| 100 | 20.6 | 10000 |
| 101 | 31.2 | 7640 |
| 102 | 31.2 | 2252 |
| 103 | 31.3 | 10000 |
| 104 | 32.9 | 10000 |
| 105 | 36.8 | 3385 |
| 106 | 41.3 | 10000 |
| 107 | 43.5 | 10000 |
| 108 | 45.0 | 10000 |
| 109 | 45.9 | 10000 |
| 110 | 46.2 | 10000 |
| 111 | 46.5 | 2997 |
| 112 | 49.2 | 10000 |
| 113 | 65.1 | 10000 |
| 114 | 2.7 | 10000 |
| 115 | 3.5 | 10000 |
| 116 | 3.6 | 10000 |
| 117 | 5.4 | 10000 |
| 118 | 12.5 | 10000 |
| 119 | 14.5 | 3585 |
| 120 | 17.8 | 10000 |
| 121 | 18.4 | 10000 |
| 122 | 19.5 | 10000 |
| 123 | 27.3 | 3333 |
| 125 | 28.3 | 10000 |
| 126 | 30.5 | 10000 |
| 127 | 31.3 | 10000 |
| 128 | 32.2 | 10000 |
| 129 | 32.8 | 10000 |
| 130 | 35.0 | 10000 |
| 131 | 37.6 | 10000 |
| 132 | 49.0 | 10000 |
| 133 | 49.5 | 10000 |
| 134 | 9.4 | 852 |
| 135 | 2.8 | 120 |
| 136 | 8.6 | 838 |
| 137 | 21.2 | 4103 |
| 138 | 14.6 | 2322 |
| 139 | 0.3 | 502 |
| 140 | 0.2 | 1286 |
| 141 | 0.3 | 4734 |
| 142 | 0.4 | 2172 |
| 143 | 0.4 | 528 |
| 144 | 0.4 | 2343 |
| 145 | 0.5 | 366 |
| 146 | 0.6 | 7222 |
| 147 | 0.6 | 5497 |
| 148 | 0.6 | 2932 |
| 149 | 0.6 | 1477 |
| 150 | 0.6 | 650 |
| 151 | 1.0 | 7722 |
| 152 | 1.0 | 6017 |
| 153 | 1.1 | 10000 |
| 154 | 1.2 | 4133 |
| 155 | 1.8 | 3677 |
| 156 | 7.3 | 10000 |
| 157 | 9.3 | 10000 |
| 158 | 9.5 | 10000 |
| 159 | 12.0 | 10000 |
| 160 | 42.9 | 10000 |
| 161 | 2.0 | 9632 |
| 162 | 1.0 | 5189 |
| 163 | 1.2 | 4845 |
| 164 | 3.4 | 10000 |
| 165 | 1.4 | 4272 |
| 166 | 0.7 | 5769 |
| 167 | 1.0 | 5825 |
| 168 | 2.0 | 6351 |
| 169 | 1.5 | 9177 |
| 170 | 1.6 | 10000 |
| 171 | 4.9 | 10000 |
| 172 | 1.8 | 10000 |
| 173 | 1.0 | 8919 |
| 174 | 2.3 | 10000 |
| 175 | 0.6 | 5077 |
| 176 | 1.4 | 10000 |
| 177 | 1.2 | 10000 |
| 178 | 3.3 | 10000 |
| 179 | 0.7 | 9430 |
| 180 | 1.1 | 10000 |
| 181 | 4.2 | 10000 |
| 182 | 6.1 | 10000 |
| 183 | 4.2 | 10000 |
| 184 | 2.2 | 10000 |
| 185 | 1.5 | 10000 |
| 186 | 1.4 | 10000 |
| 187 | 1.0 | 3333 |
| 188 | 3.2 | 3333 |
| 189 | 1.2 | 10000 |
| 190 | 3.5 | 10000 |
| 191 | 1.1 | 10000 |
| 192 | 4.1 | 10000 |
| 193 | 8.1 | 10000 |
| 194 | 21.6 | 10000 |
| 195 | 11.0 | 8800 |
| 196 | 4.7 | 10000 |
| 197 | 411.6 | 10000 |
| 198 | 188.5 | 10000 |
| 199 | 1.5 | 4790 |
| 200 | 2.4 | 1123 |
| 201 | 6.2 | 10000 |
| 202 | 1.0 | 2257 |
| 203 | 77.8 | 10000 |
| 204 | 17.5 | 10000 |
| 205 | 14.1 | 10000 |
| 206 | 24.4 | 820 |
| 207 | 17.2 | 675 |
| 208 | 33.9 | 827 |

## Claims

1. A compound of the formula: 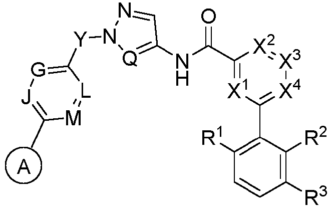 wherein 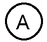 is selected from 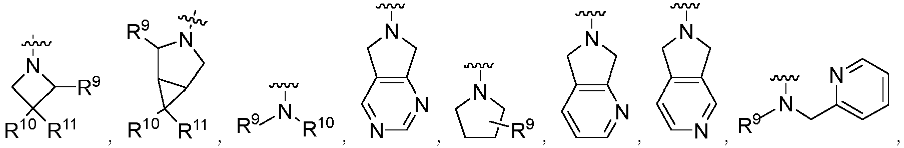 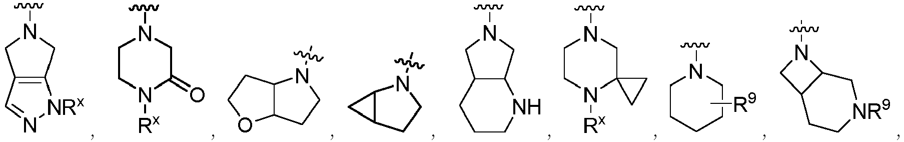 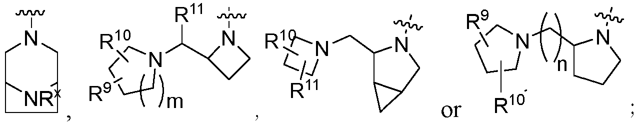
X¹ is N;
X² is CR⁴;
X³ is N;
X⁴ is CH;
Y is CR⁵R⁶ or SO₂;
Q is CH;
G is N or CR⁷;
J is N or CR⁷;
L is N or CR⁷;
M is N or CR⁷;
R¹ is selected from the group consisting of bromo, cyano, tetrazolyl, CHF₂, CF₃, OCHF₂ and SO₂CH₃;
R² is hydrogen or halo;
R³ is hydrogen, halo or OR^{x};
R⁴ is hydrogen, R^{x}, OR^{x}, (C=O)OR^{x}, (C=O)NR^{x}R^{y}, C₁₋₃ alkyl-OR^{x} or NR^{x}R^{y};
R⁵ is hydrogen or C₁₋₃ alkyl, which is optionally substituted with one to three substituents selected from deuterium, halo and hydroxy;
R⁶ is hydrogen, deuterium or C₁₋₃ alkyl;
or R⁵ and R⁶ can be taken together with the carbon atom between them to form a C₃₋₆ cycloalkyl group;
each R⁷ is independently selected from the group consisting of halo, cyano, R^{x}, OR^{x}, cyclopropyl, (C=O)NR^{x}R^{y} and NR^{x}R^{y};
or R⁵ and R⁷ can be taken together with the atoms between them to form a C₃₋₆ cycloalkyl group;
each R⁹ is independently selected from the group consisting of hydrogen, halo, hydroxy, cyano, triazolyl, cyclopropyl, (C=O)NR^{x}R^{y}, (C=O)R^{x} and C₁₋₃ alkyl, wherein said triazolyl group is optionally substituted with R^{x}, said cyclopropyl group is optionally substituted with R^{x} or OR^{x}, and said alkyl group is optionally substituted with one to four substituents selected from halo, hydroxy, heteroaryl, heterocyclyl, C₃₋₆ cycloalkyl, C₃₋₆ cycloalkyl(R¹⁰), OR^{x}, (C=O)NR^{×}R^{y} and NR^{x}R^{y};
R¹⁰ is hydrogen, halo, hydroxy or C₁₋₃ alkyl, wherein said alkyl is optionally substituted with one to four substituents selected from halo and hydroxy;
R¹¹ is hydrogen, halo, hydroxy or R^{x};
or R¹⁰ and R¹¹ can be taken together with the atoms between them to form a C₃₋₆ cycloalkyl group;
each R^{x} is independently hydrogen or C₁₋₆ alkyl, which is optionally substituted with one to four substituents selected from halo and hydroxy;
each R^{y} is independently selected from the group consisting of hydrogen, C₁₋₆ alkyl, C₃₋₆ cycloalkyl, CH₂-C₃₋₆ cycloalkyl and heterocyclyl, wherein said alkyl is optionally substituted with one to four substituents selected from halo and hydroxy, said cycloalkyl is optionally substituted with one or two R^{x} or OR^{x} and said heterocyclyl is optionally substituted with one or two R¹⁰;
m is an integer from zero to two;
n is an integer from zero to two;
or a pharmaceutically acceptable salt thereof.

2. The compound of Claim 1 wherein
wherein 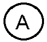 is 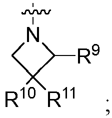 or a pharmaceutically acceptable salt thereof.

3. The compound of Claim 1 or Claim 2 wherein R4 is selected from the group consisting of hydrogen, CH3, CHF2, OCH3, NH2, N(CH3)2, (C=O)OH and (C=O)NH2, or a pharmaceutically acceptable salt thereof.

4. The compound of any of Claims 1-3 wherein R⁵ is hydrogen, CH₃, CHF₂ or CH₂OH; or a pharmaceutically acceptable salt thereof.

5. The compound of any of Claims 1-4 wherein J is N; M is N; G is CR⁷; L is CR⁷; or a pharmaceutically acceptable salt thereof.

6. The compound of Claim 1 selected from: , 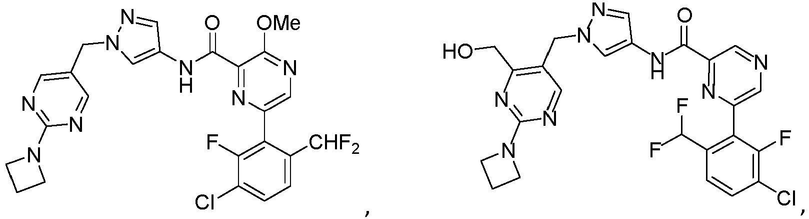 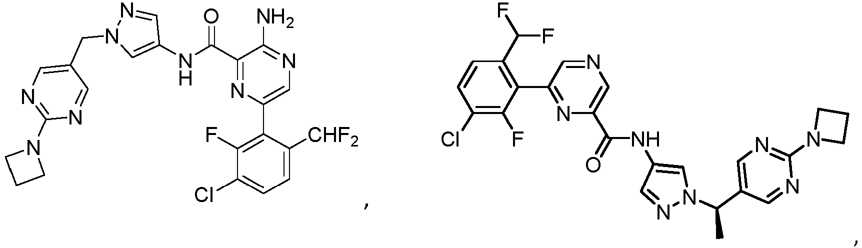 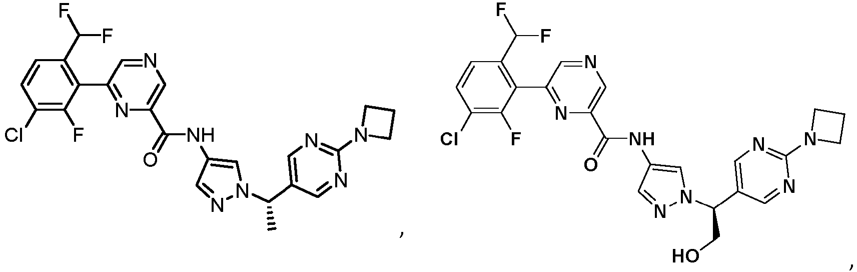 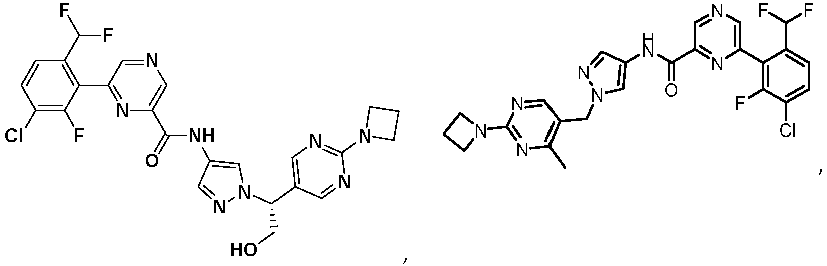 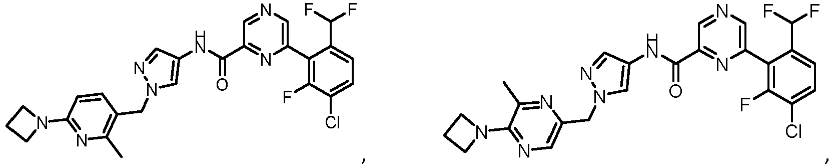 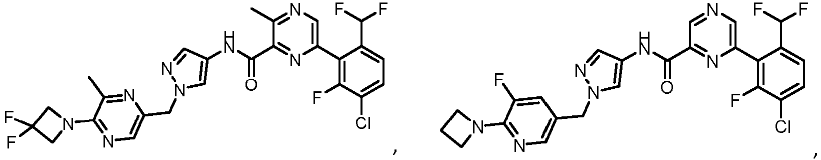 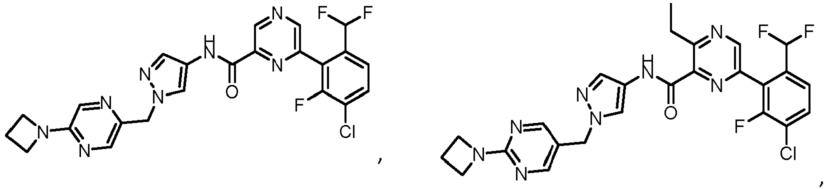 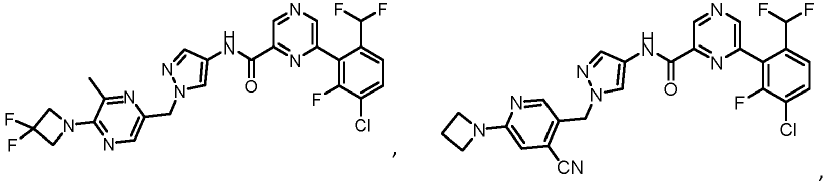 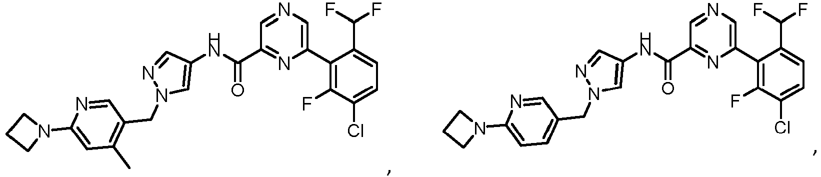 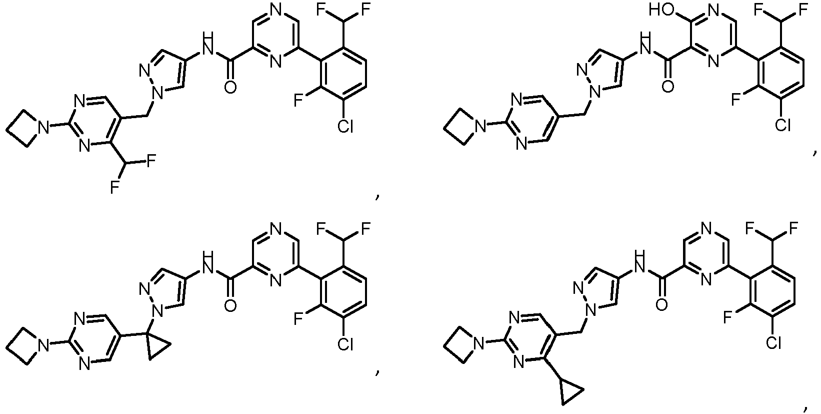 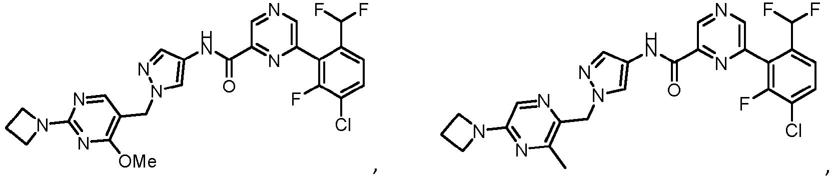 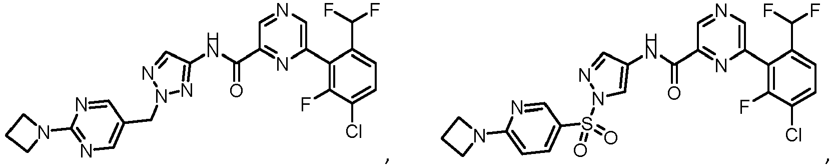 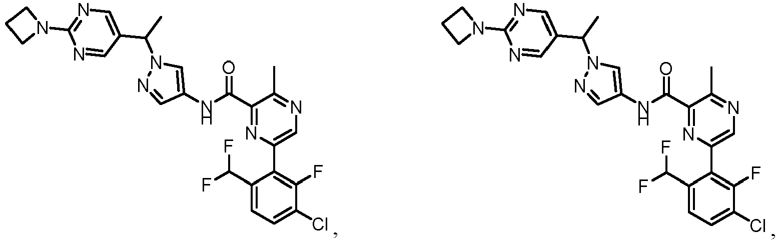 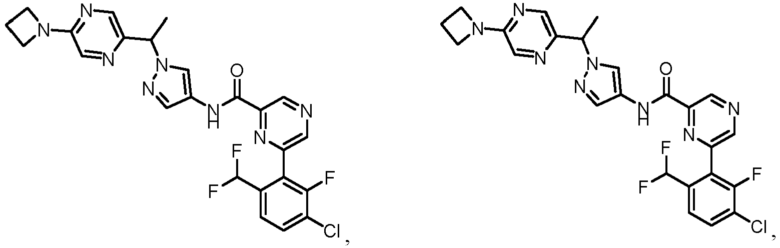 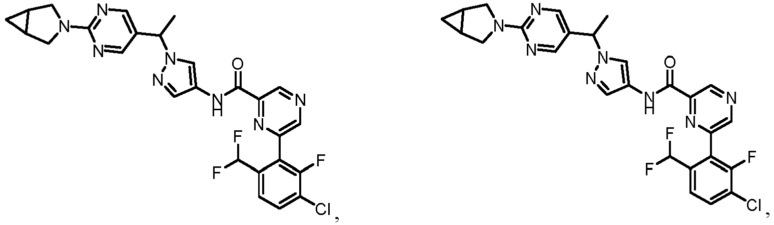 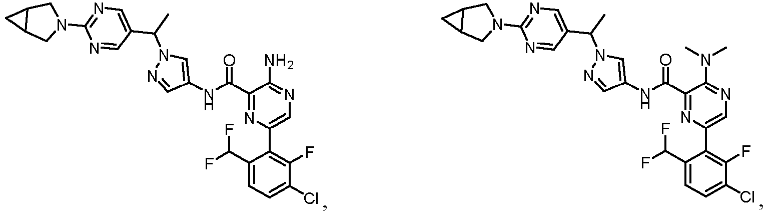 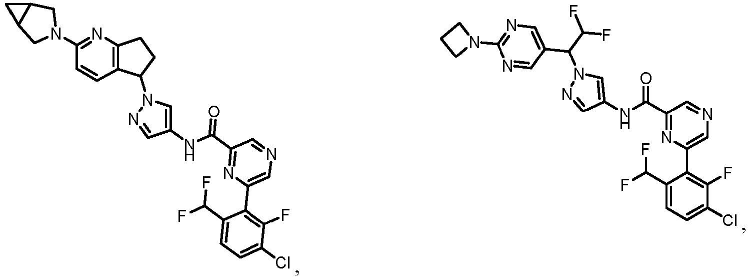 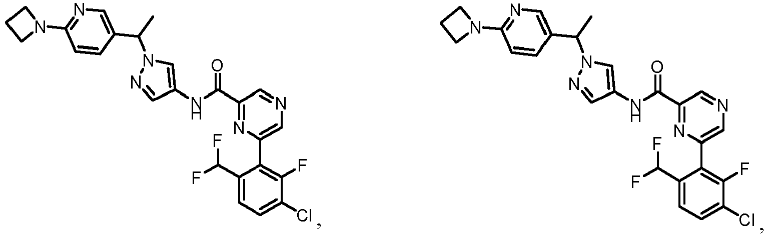 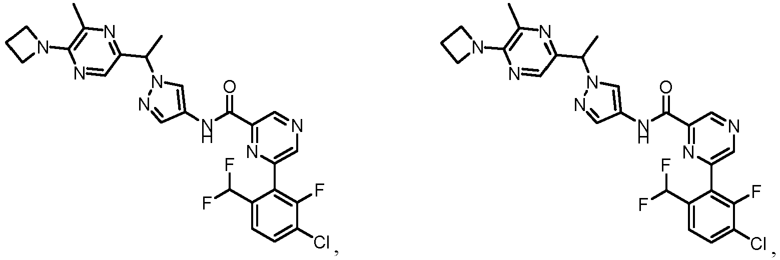 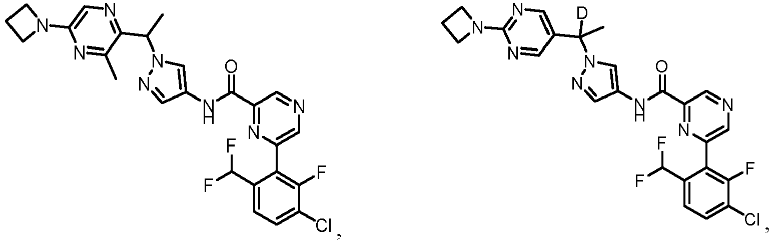 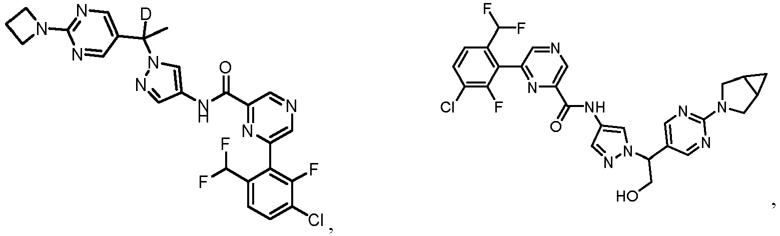 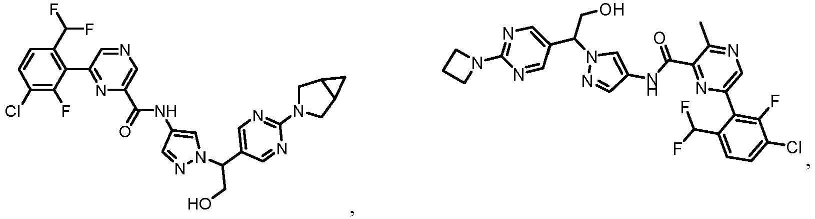 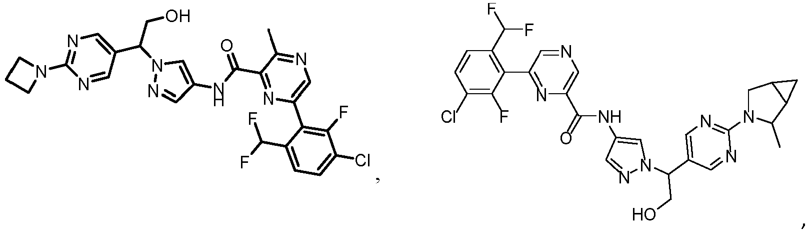 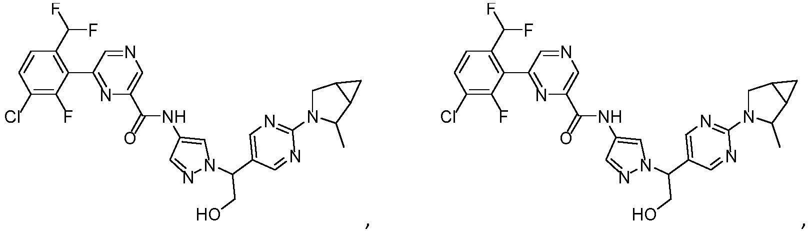 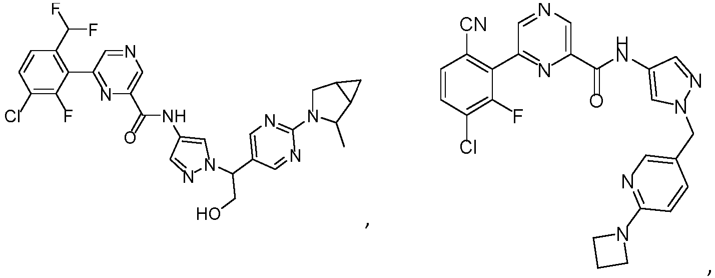 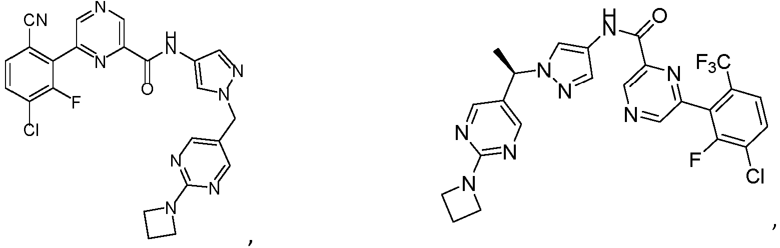 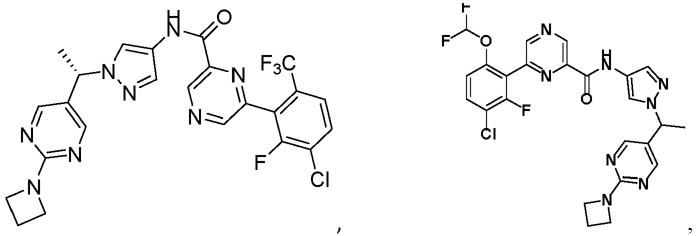 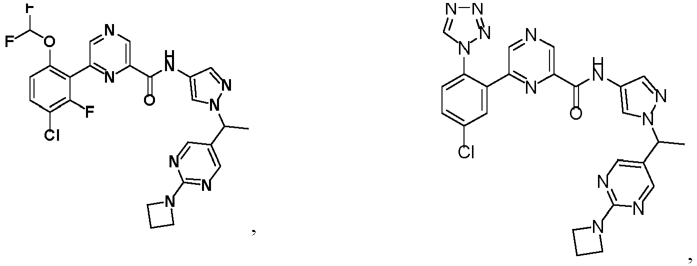 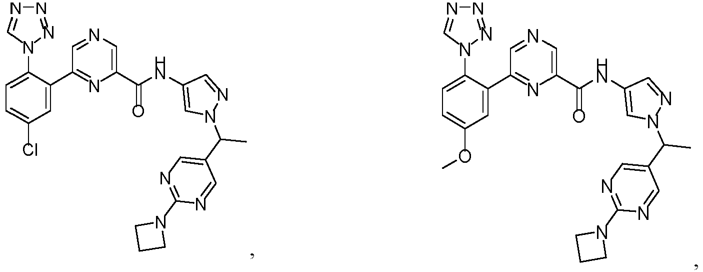 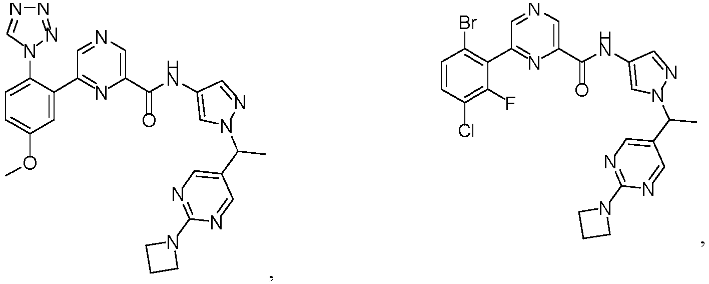 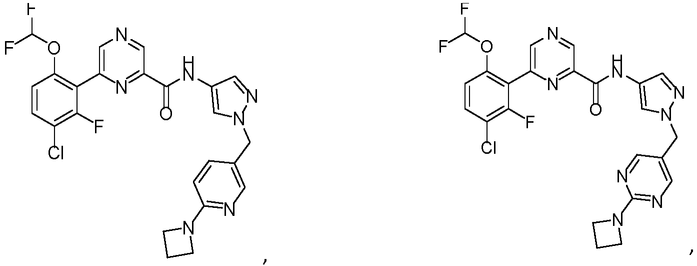 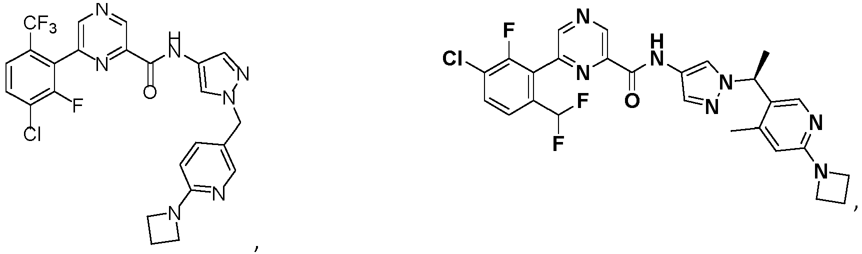 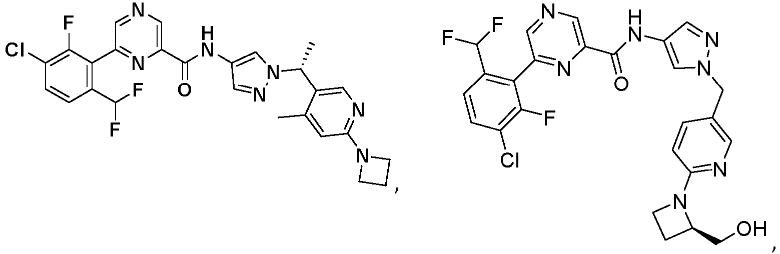 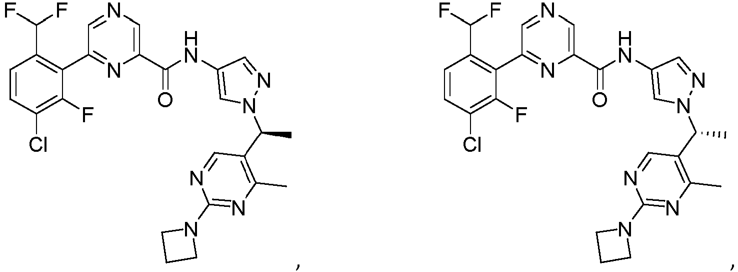 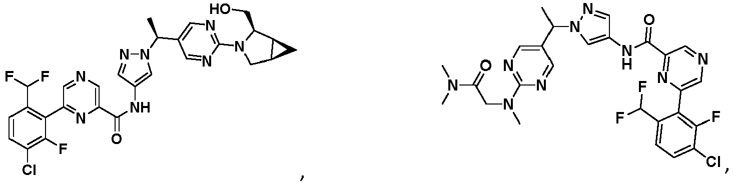 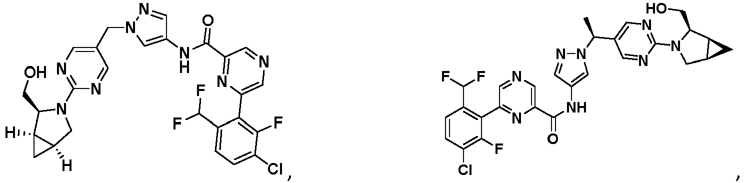 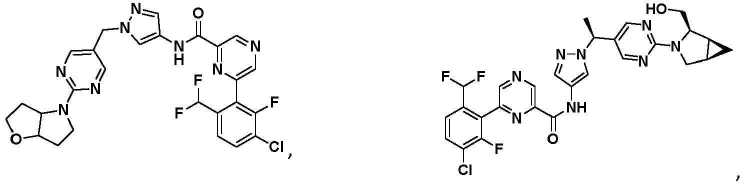 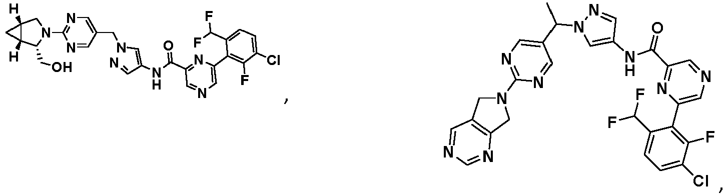 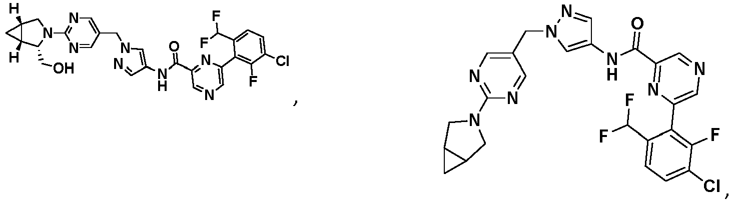 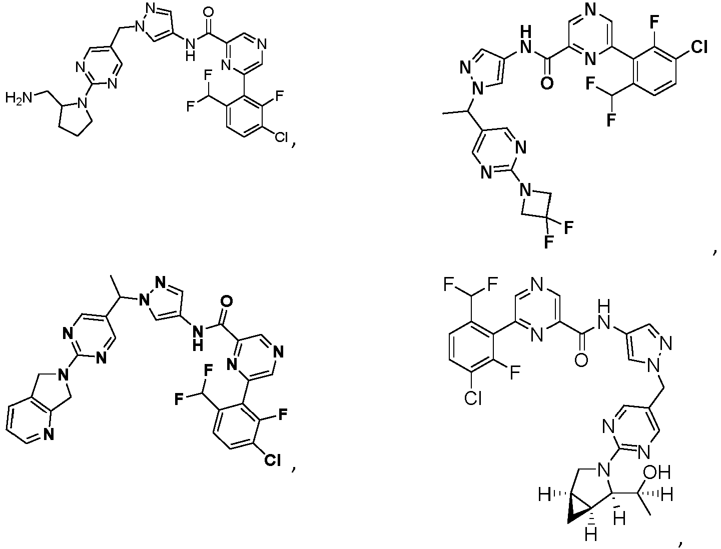 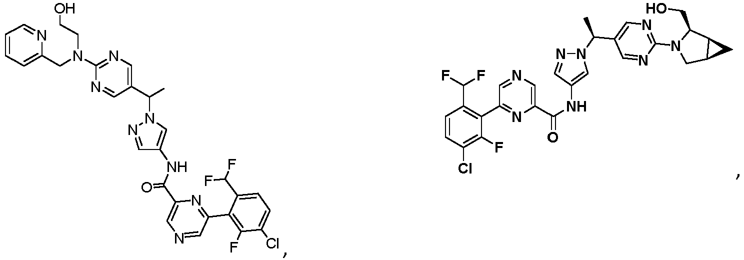 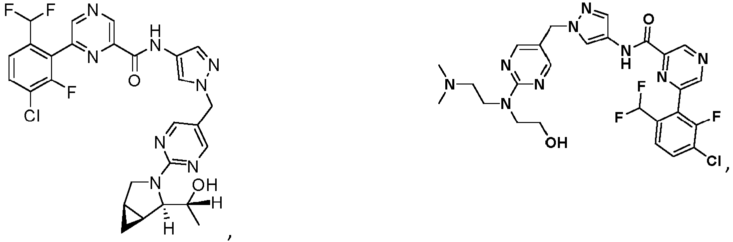 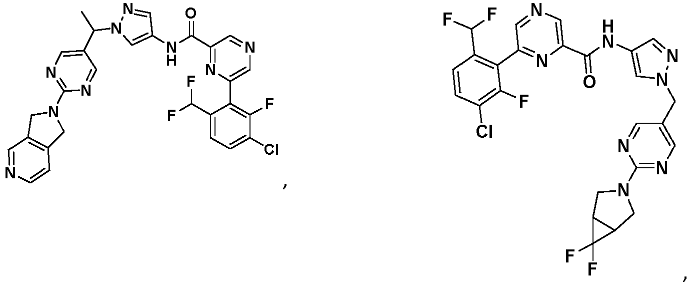 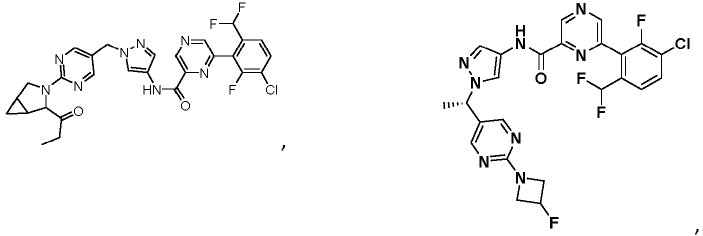 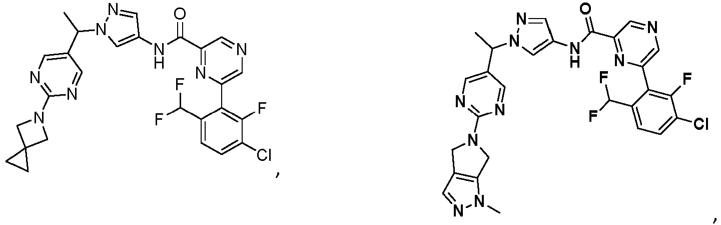 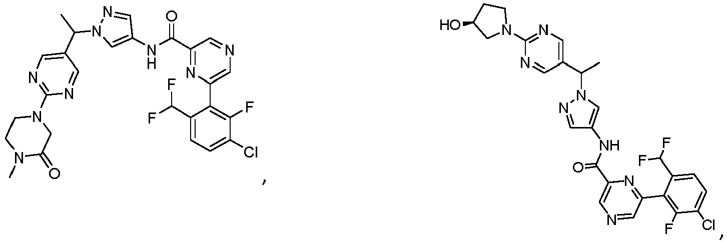 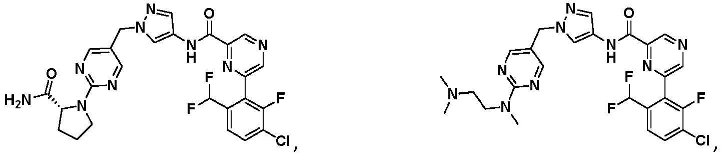 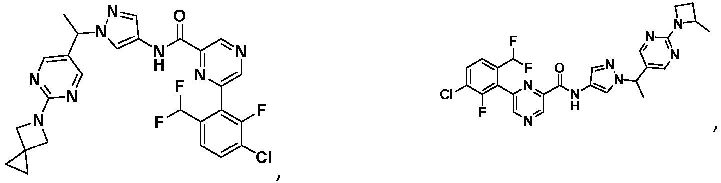 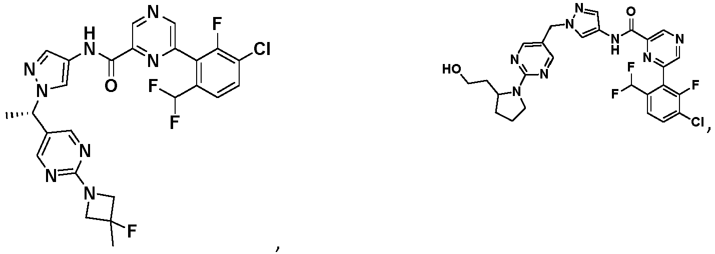 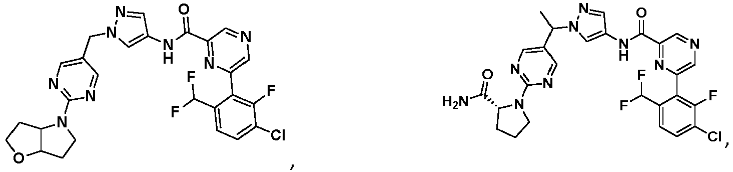 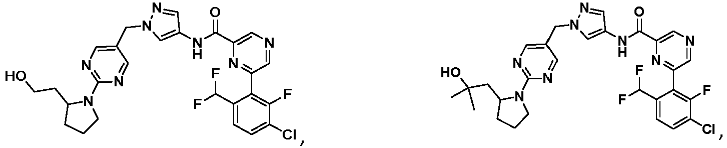 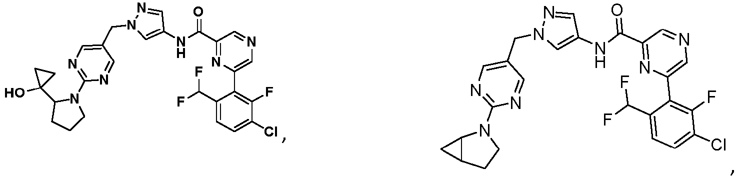 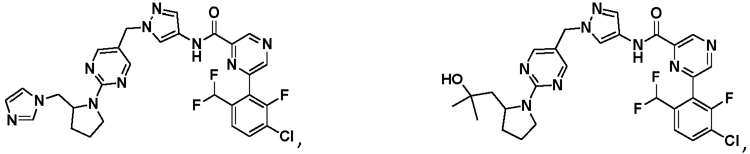 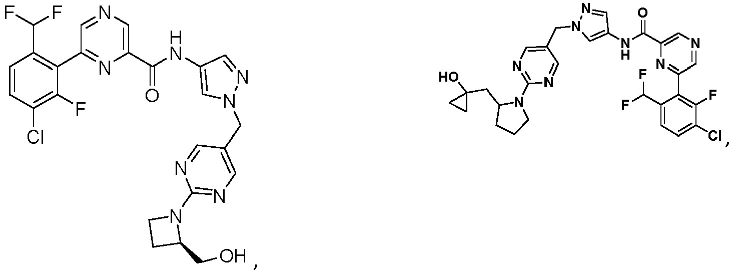 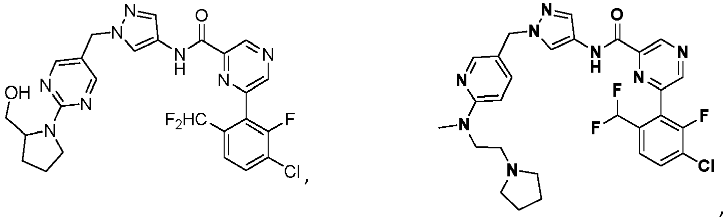 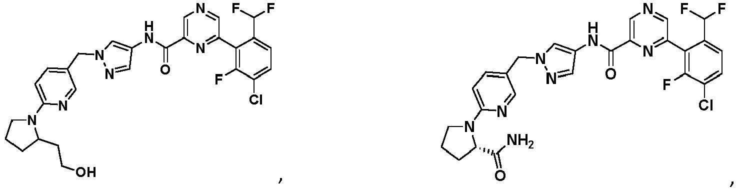 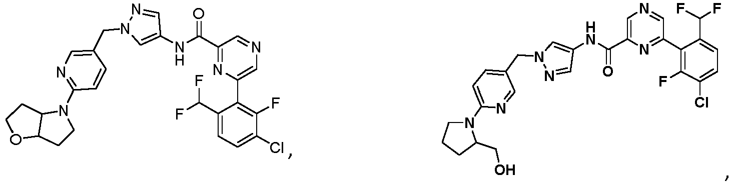 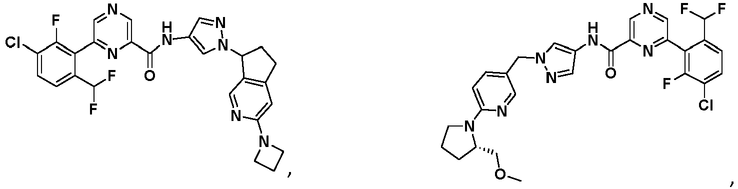 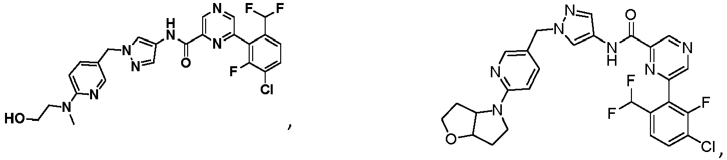 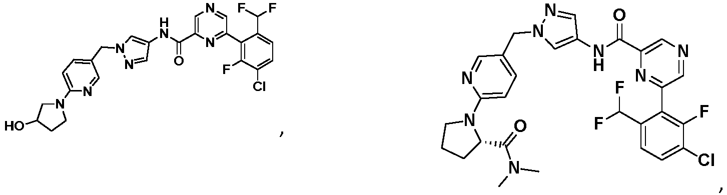 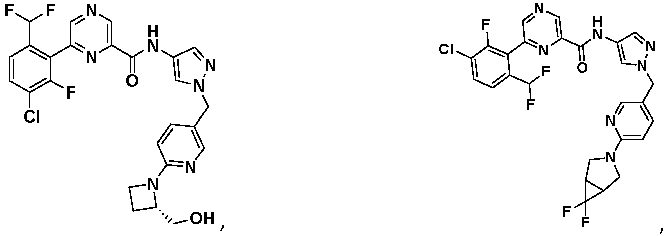 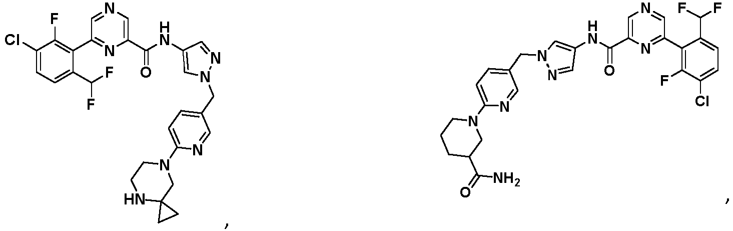 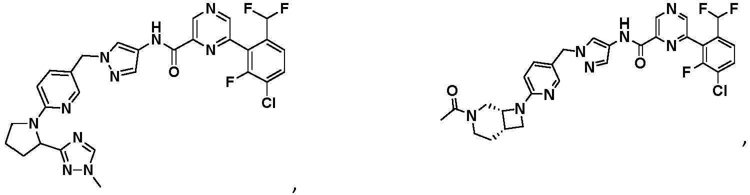 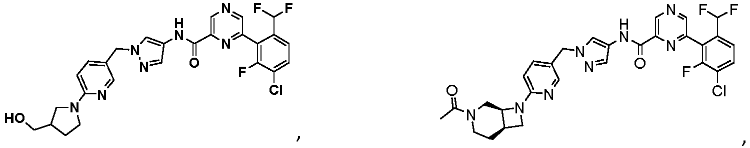 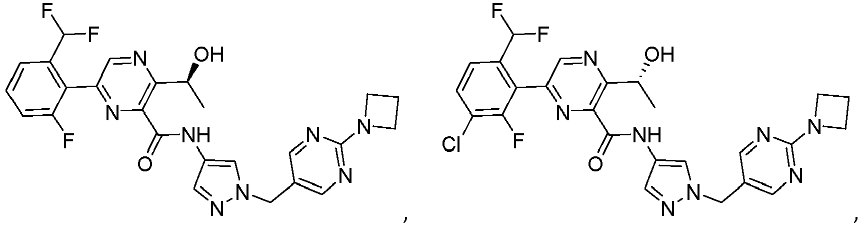 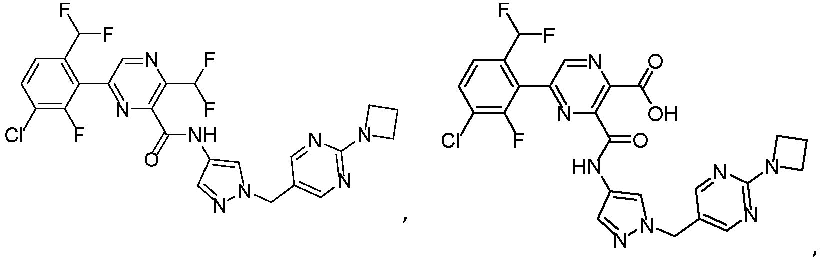 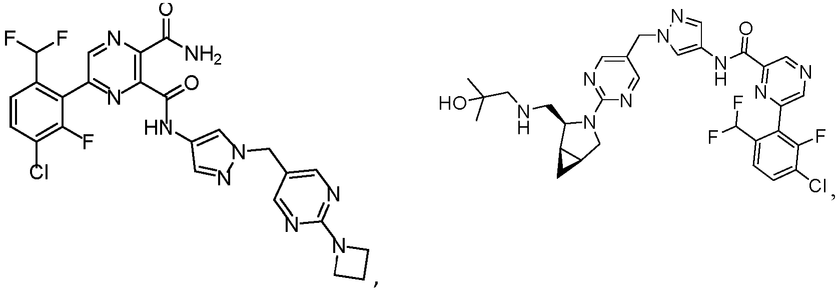 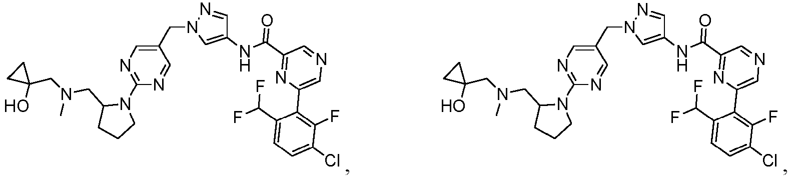 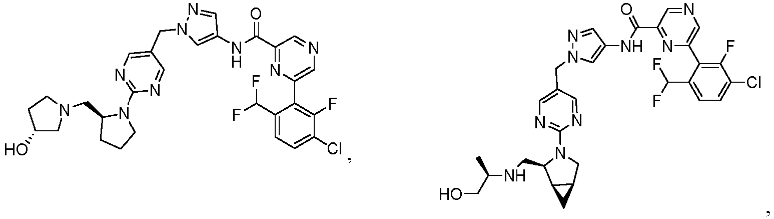 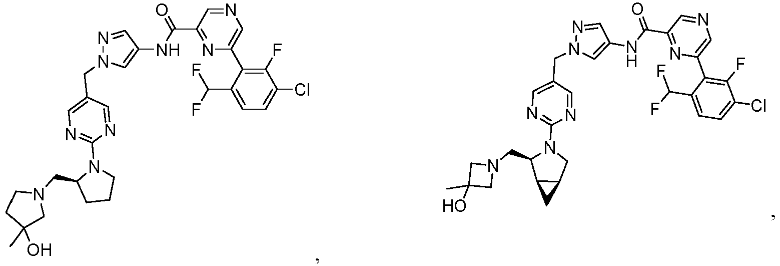 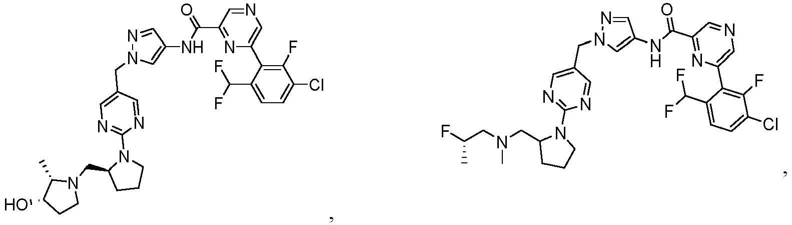 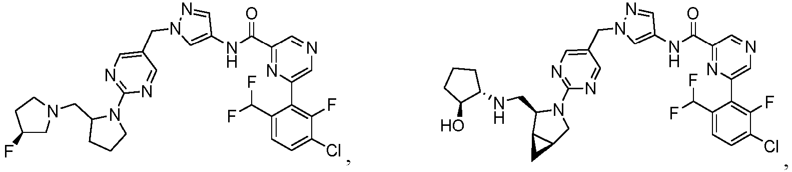 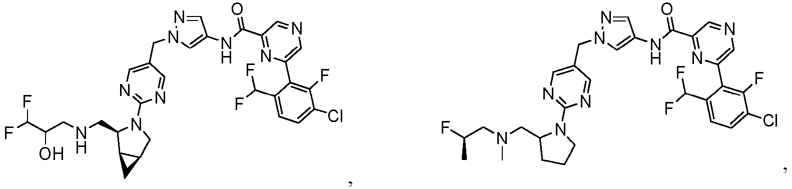 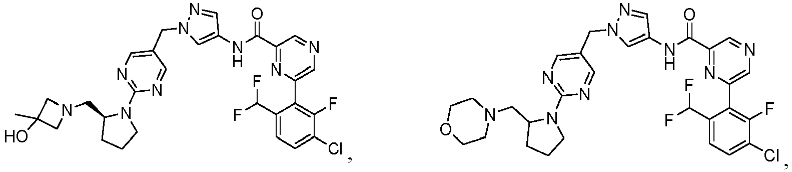 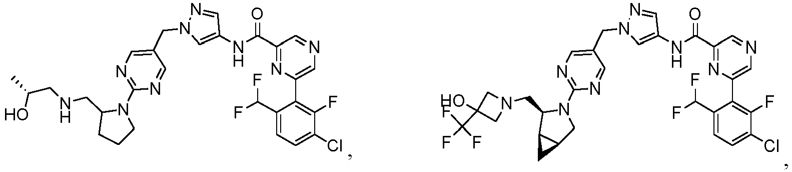 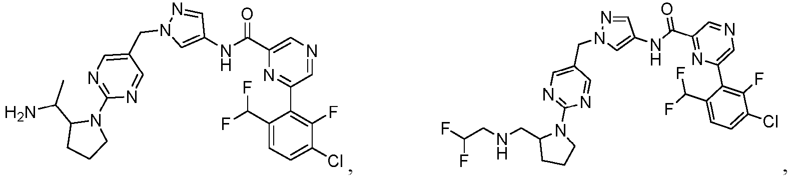 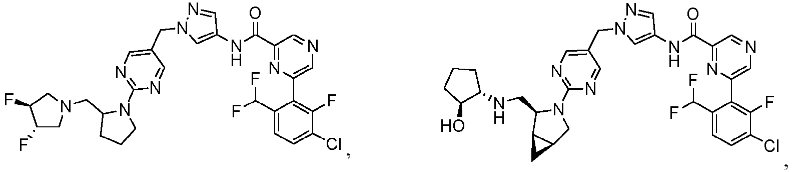 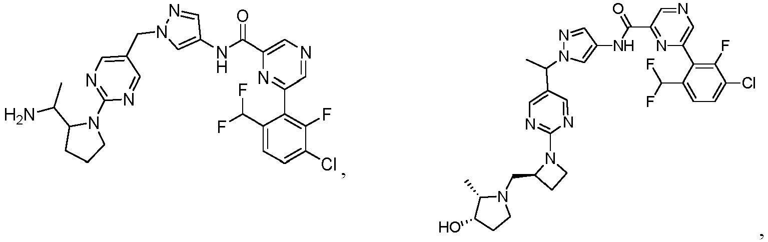 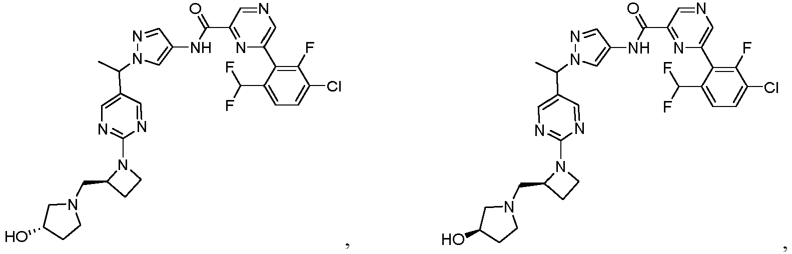 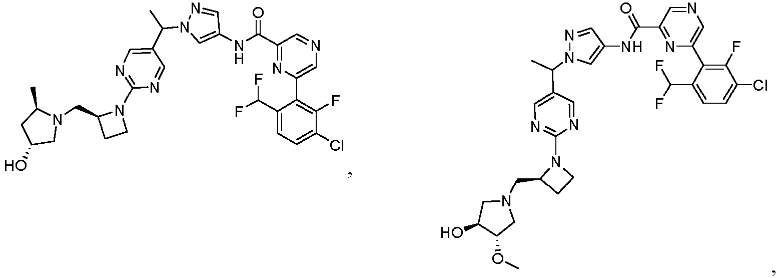 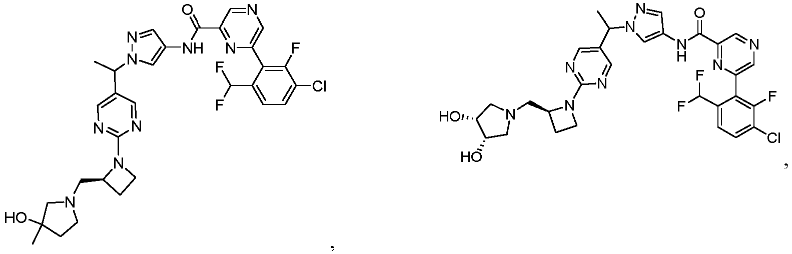 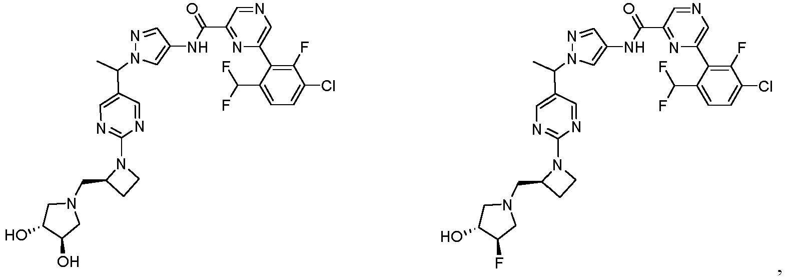 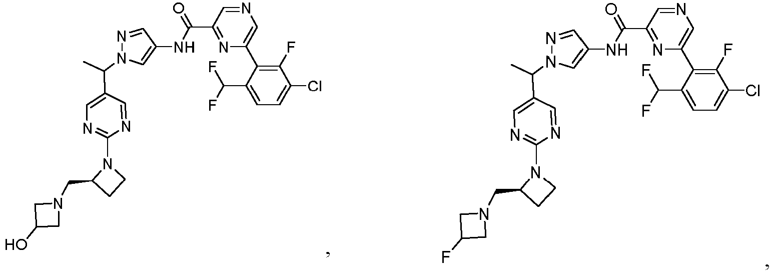 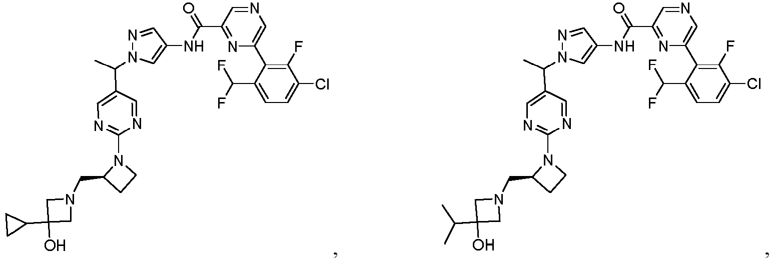 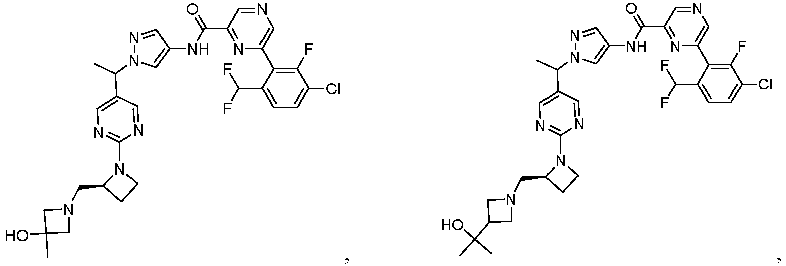 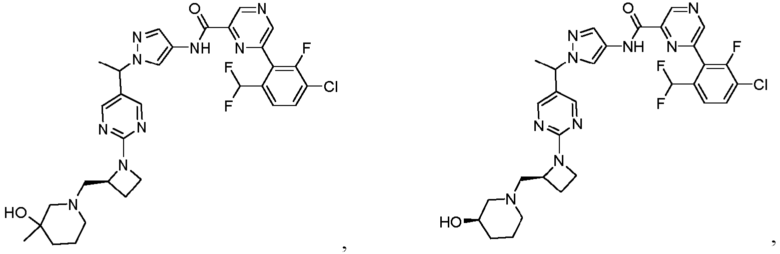 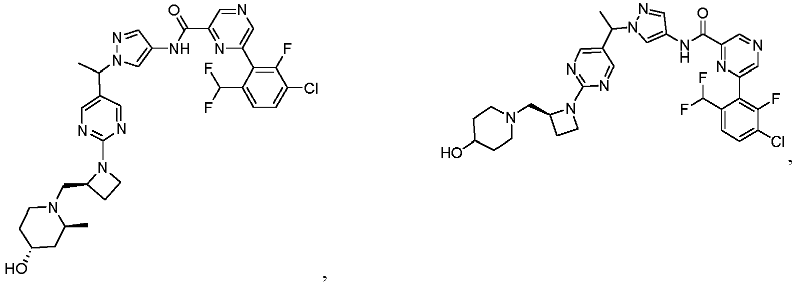 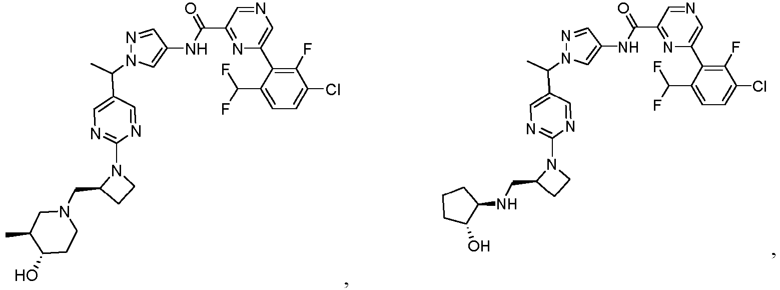 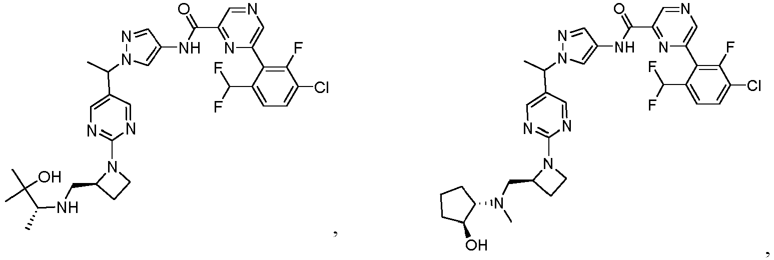 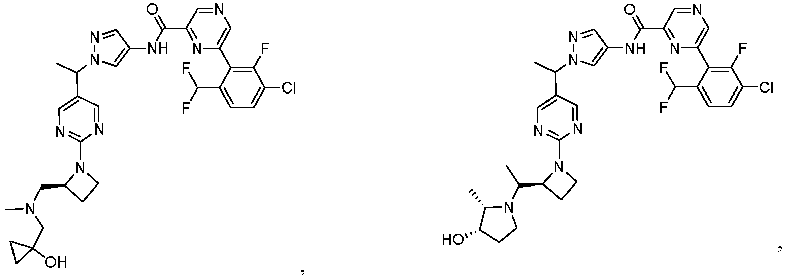 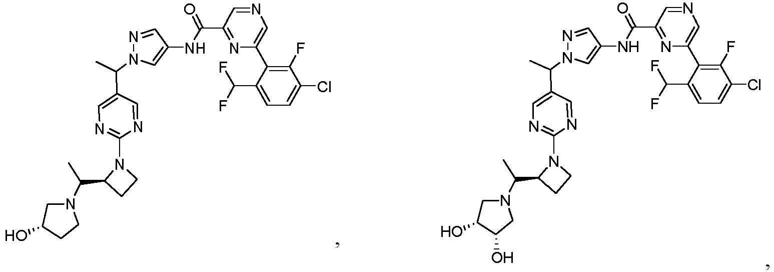 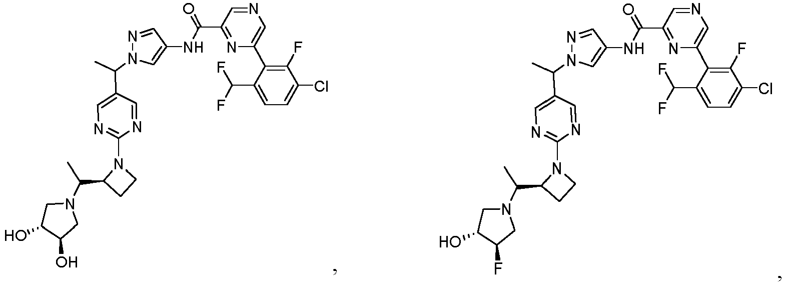 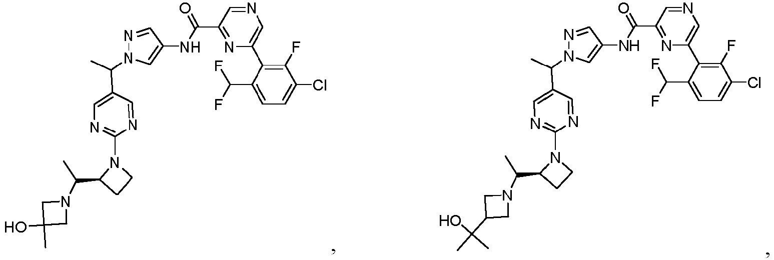 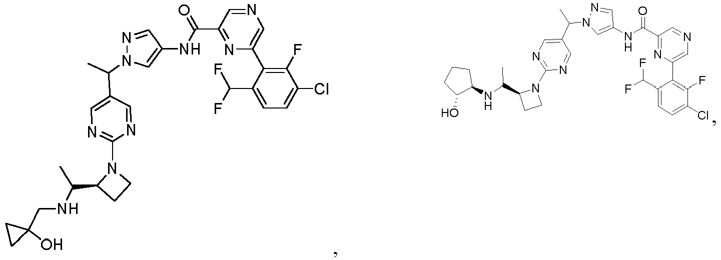 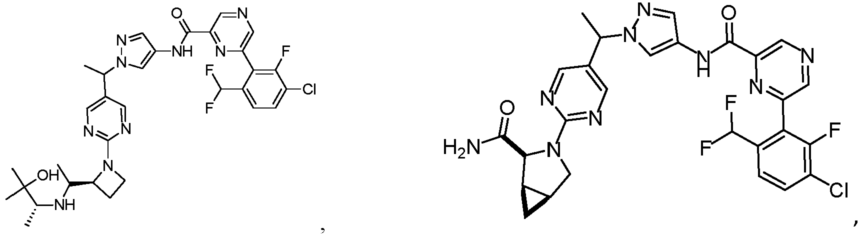 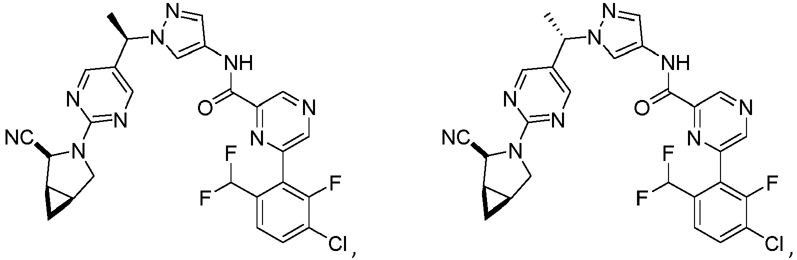 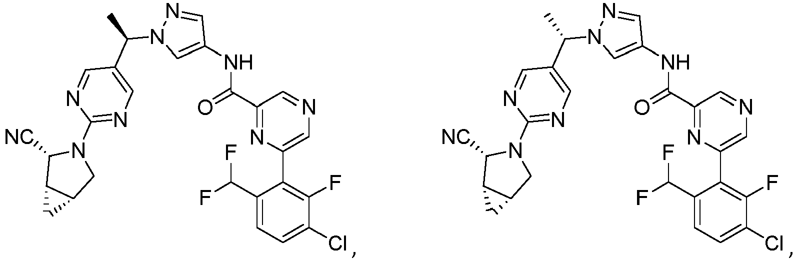 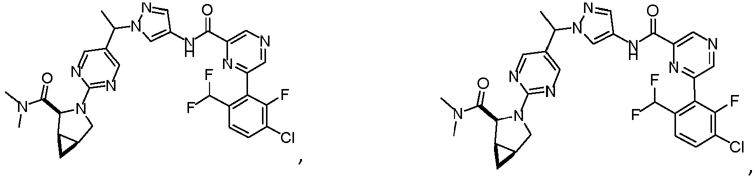 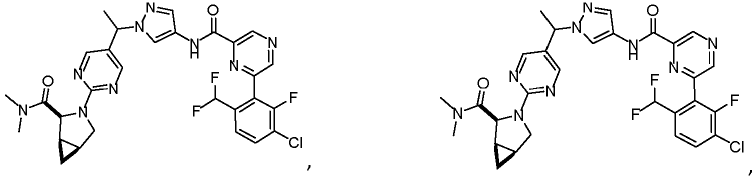 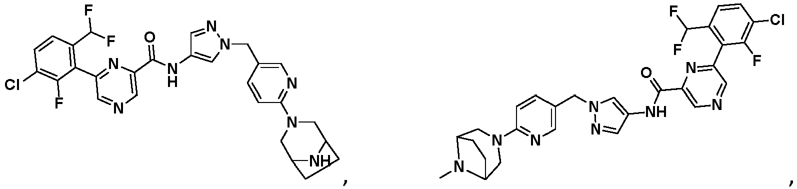 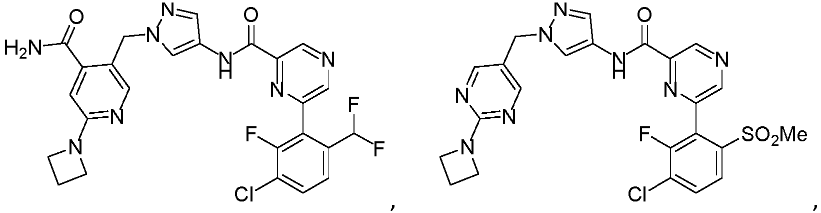 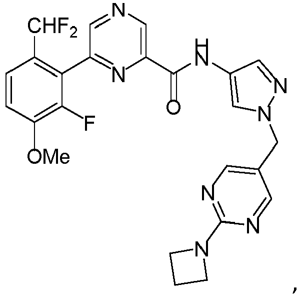 or a pharmaceutically acceptable salt thereof.

7. A pharmaceutical composition comprising a compound of any one of Claims 1-6 or a pharmaceutically acceptable salt thereof and a pharmaceutically acceptable carrier.

8. A compound of any of Claims 1-6 or pharmaceutically acceptable salt thereof, or a pharmaceutical composition of Claim 7, for use in a method for treating impaired visual activity, diabetic retinopathy, diabetic macular edema, retinal vein occlusion, hereditary angioedema, diabetes, pancreatitis, cerebral hemorrhage, nephropathy, cardiomyopathy, neuropathy, inflammatory bowel disease, arthritis, inflammation, septic shock, hypotension, cancer, adult respiratory distress syndrome, disseminated intravascular coagulation, blood coagulation during cardiopulmonary bypass surgery, bleeding from postoperative surgery, uveitis, posterior uveitis, wet age related macular edema.

9. A compound according to any one of Claims 1-6, or a pharmaceutically acceptable salt thereof, for use in therapy.

10. A combination comprising:
a compound of to any of Claims 1-6 or pharmaceutically acceptable salt thereof, or a pharmaceutical composition of Claim 7; and
another agent selected from the group consisting of anti-inflammatory agents, anti-VEGF agents, immunosuppressive agents, anticoagulants, antiplatelet agents, and thrombolytic agents.

11. The combination of claim 10, for use in therapy.

## Patentansprüche

1. Eine Verbindung der Formel: 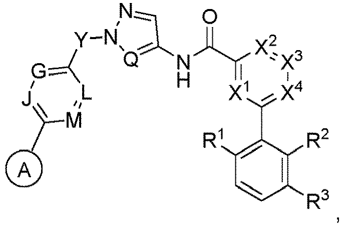 wobei 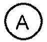 ausgewählt ist aus 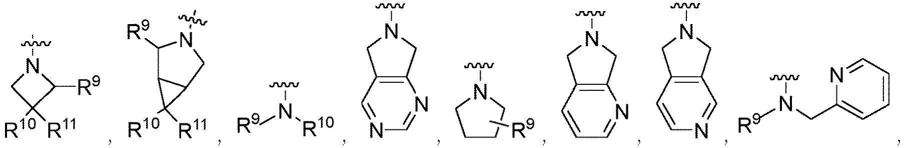 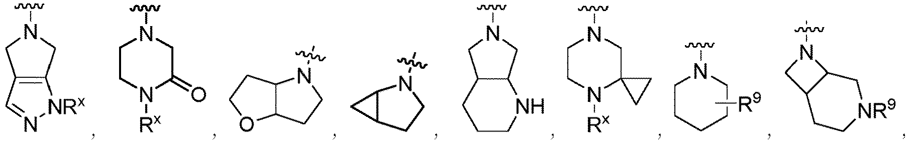 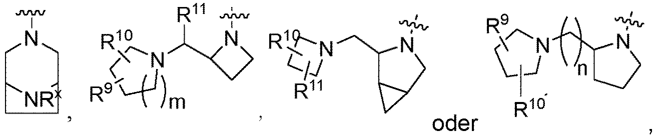
X¹ N ist,
X² CR⁴ ist,
X³ N ist,
X⁴ CH ist,
Y CR⁵R⁶ oder SO₂ ist,
Q CH ist,
G N oder CR⁷ ist,
J N oder CR⁷ ist,
L N oder CR⁷ ist,
M N oder CR⁷ ist,
R¹ ausgewählt ist aus der Gruppe bestehend aus Brom, Cyano, Tetrazolyl, CHF₂, CF₃, OCHF₂ und SO₂CH₃,
R² Wasserstoff oder Halogen ist,
R³ Wasserstoff, Halogen oder OR^{x} ist,
R⁴ Wasserstoff, R^{x}, OR^{x}, (C=O)OR^{x}, (C=O)NR^{x}R^{y}, C₁₋₃-Alkyl-OR^{x} oder NR^{x}R^{y} ist,
R⁵ Wasserstoff oder C₁₋₃-Alkyl ist, das gegebenenfalls substituiert ist mit einem bis drei Substituenten, ausgewählt aus Deuterium, Halogen und Hydroxy,
R⁶ Wasserstoff, Deuterium oder C₁₋₃-Alkyl ist,
oder R⁵ und R⁶ mit dem dazwischenliegenden Kohlenstoffatom zusammengenommen sein können unter Bildung einer C₃₋₆-Cycloalkylgruppe,
jedes R⁷ unabhängig ausgewählt ist aus der Gruppe bestehend aus Halogen, Cyano, R^{x}, OR^{x}, Cyclopropyl, (C=O)NR^{x}R^{y} und NR^{x}R^{y},
oder R⁵ und R⁷ mit den dazwischenliegenden Atomen zusammengenommen sein können unter Bildung einer C₃₋₆-Cycloalkylgruppe,
jedes R⁹ unabhängig ausgewählt ist aus der Gruppe bestehend aus Wasserstoff, Halogen, Hydroxy, Cyano, Triazolyl, Cyclopropyl, (C=O)NR^{x}R^{y}, (C=O)R^{x} und C₁₋₃-Alkyl, wobei die Triazolylgruppe gegebenenfalls substituiert ist mit R^{x}, die Cyclopropylgruppe gegebenenfalls substituiert ist mit R^{x} oder OR^{x}, und die Alkylgruppe gegebenenfalls substituiert ist mit einem bis vier Substituenten, ausgewählt aus Halogen, Hydroxy, Heteroaryl, Heterocyclyl, C₃₋₆-Cycloalkyl, C₃₋₆-Cycloalkyl(R¹⁰), OR^{x}, (C=O)NR^{x}R^{y} und NR^{x}R^{y},
R¹⁰ Wasserstoff, Halogen, Hydroxy oder C₁₋₃-Alky ist, wobei das Alkyl gegebenenfalls substituiert ist mit einem bis vier Substituenten, ausgewählt aus Halogen und Hydroxy,
R¹¹ Wasserstoff, Halogen, Hydroxy oder R^{x} ist,
oder R¹⁰ und R¹¹ mit den dazwischenliegenden Atomen zusammengenommen sein können unter Bildung einer C₃₋₆-Cycloalkylgruppe,
jedes R^{x} unabhängig Wasserstoff oder C₁₋₆-Alkyl ist, das gegebenenfalls substituiert ist mit einem bis vier Substituenten, ausgewählt aus Halogen und Hydroxy,
jedes R^{y} unabhängig ausgewählt ist aus der Gruppe bestehend aus Wasserstoff, C₁₋₆-Alkyl, C₃₋₆-Cycloalkyl, CH₂-C₃₋₆-Cycloalkyl und Heterocyclyl, wobei das Alkyl gegebenenfalls substituiert ist mit einem bis vier Substituenten, ausgewählt aus Halogen und Hydroxy, das Cycloalkyl gegebenenfalls substituiert ist mit einem oder zwei R^{x} oder OR^{x}, und das Heterocyclyl gegebenenfalls substituiert ist mit einem oder zwei R¹⁰,
m eine ganze Zahl von null bis zwei ist,
n eine ganze Zahl von null bis zwei ist,
oder ein pharmazeutisch annehmbares Salz davon.

2. Die Verbindung nach Anspruch 1, wobei 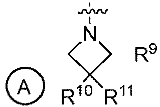 ist, oder ein pharmazeutisch annehmbares Salz davon.

3. Die Verbindung nach Anspruch 1 oder Anspruch 2, wobei R⁴ ausgewählt ist aus der Gruppe bestehend aus Wasserstoff, CH₃, CHF₂, OCH₄, NH₂, N(CH₃)₂, (C=O)OH und (C=O)NH₂, oder ein pharmazeutisch annehmbares Salz davon.

4. Die Verbindung nach einem der Ansprüche 1-3, wobei R⁵ Wasserstoff, CH₃, CHF₂ oder CH₂OH ist, oder ein pharmazeutisch annehmbares Salz davon.

5. Die Verbindung nach einem der Ansprüche 1-4, wobei J N ist, M N ist, G CR⁷ ist, L CR⁷ ist, oder ein pharmazeutisch annehmbares Salz davon.

6. Die Verbindung nach Anspruch 1, ausgewählt aus: 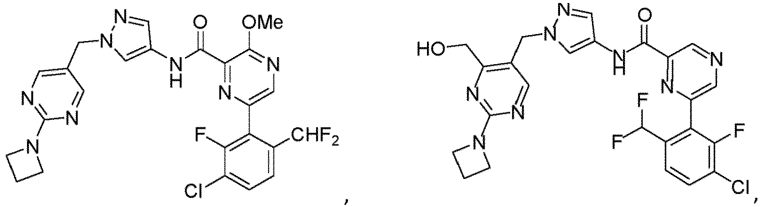 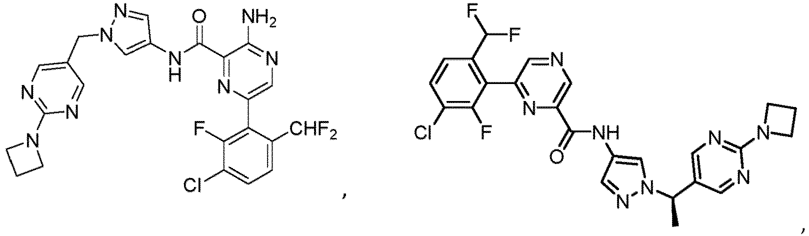 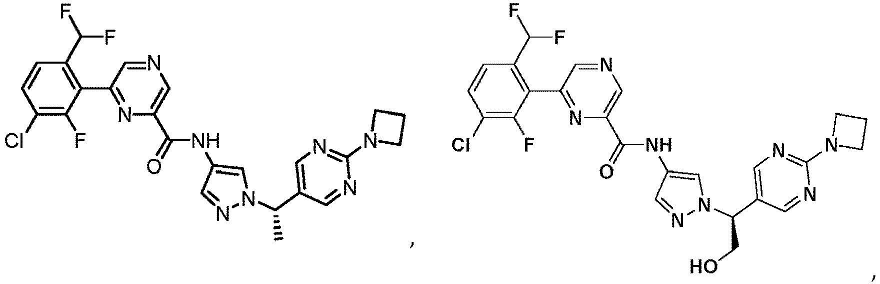 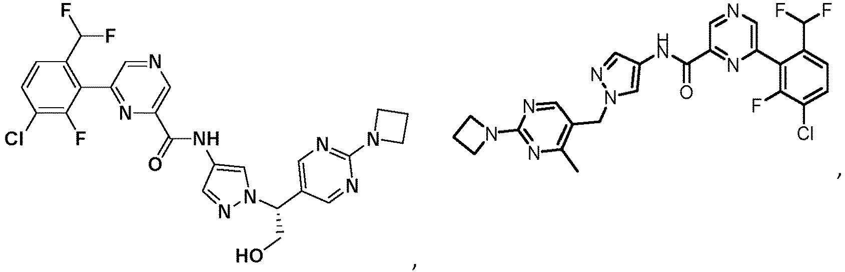 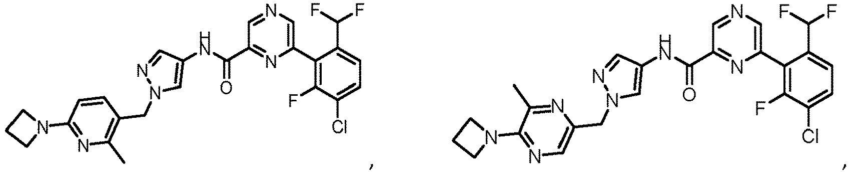 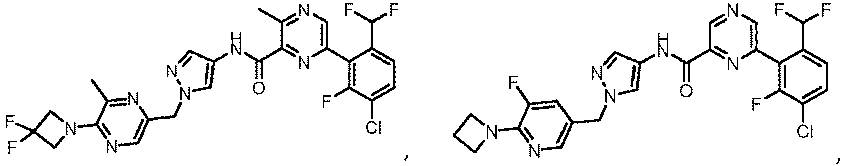 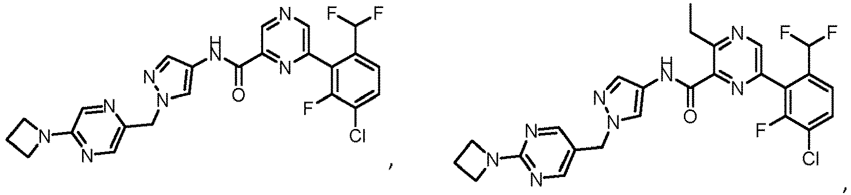 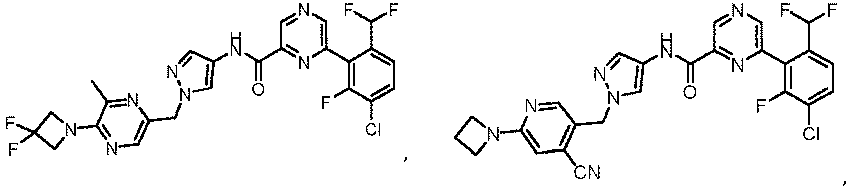 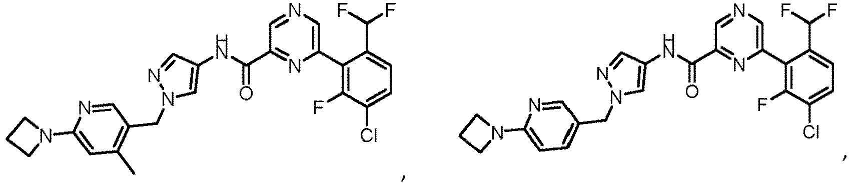 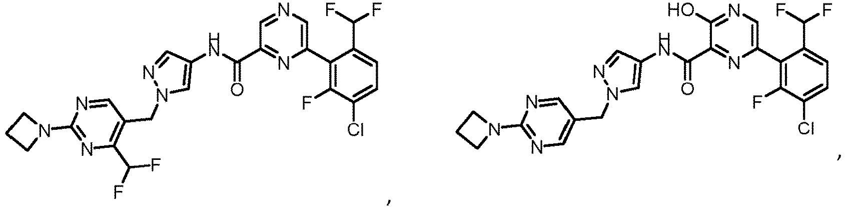 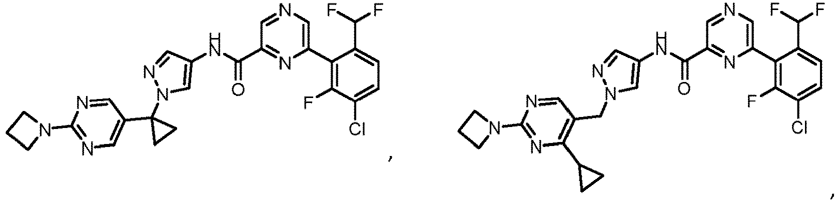 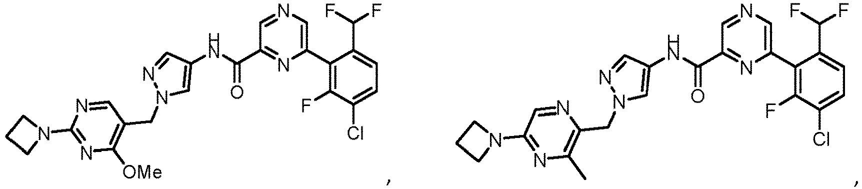 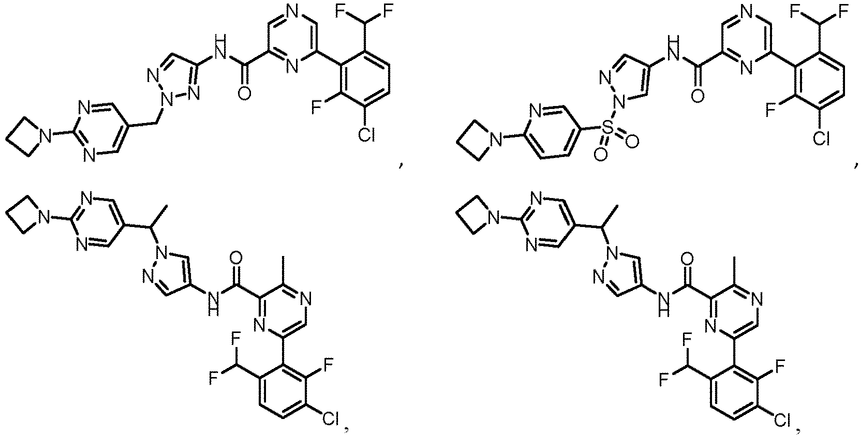 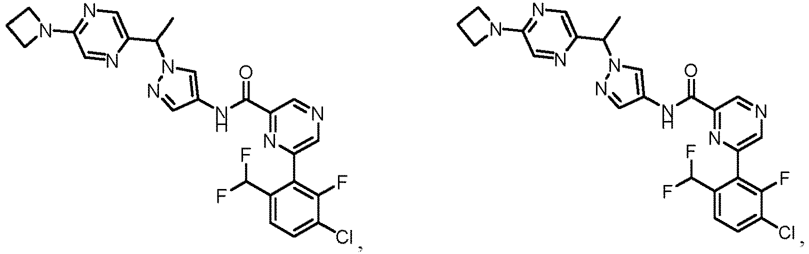 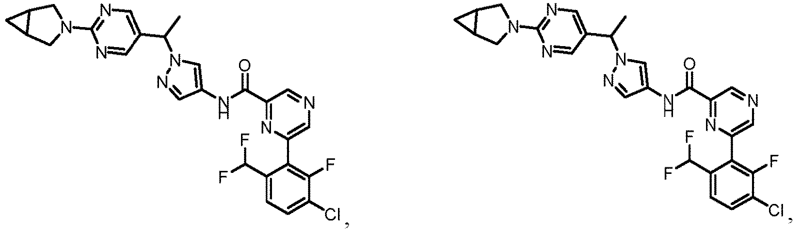 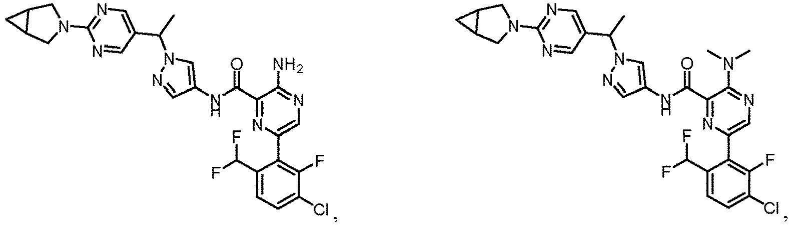 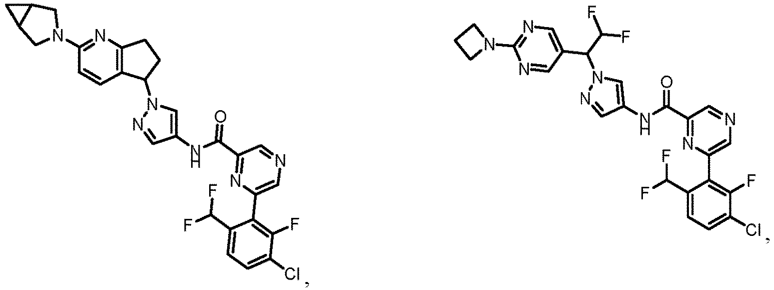 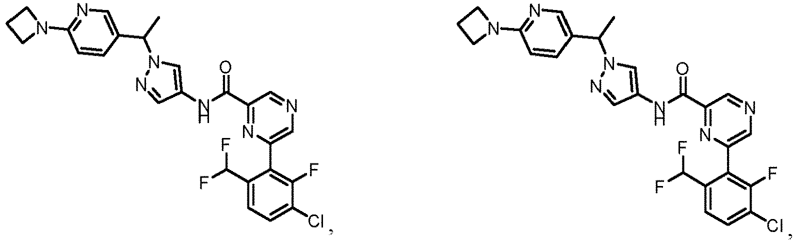 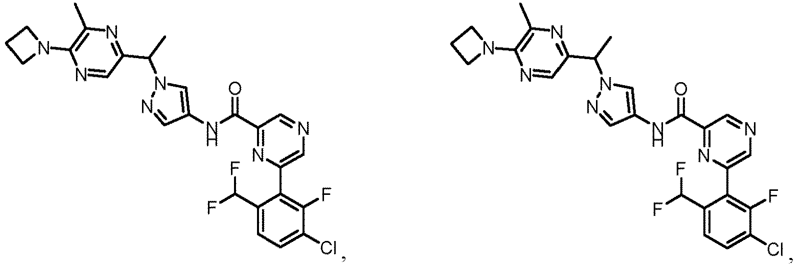 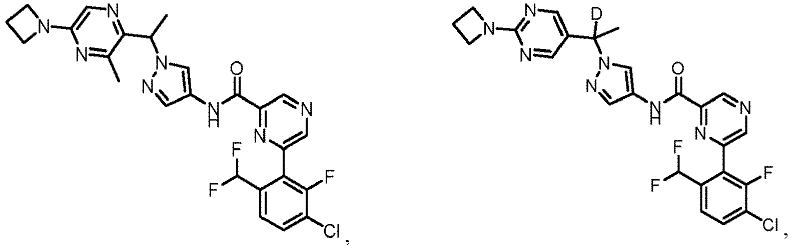 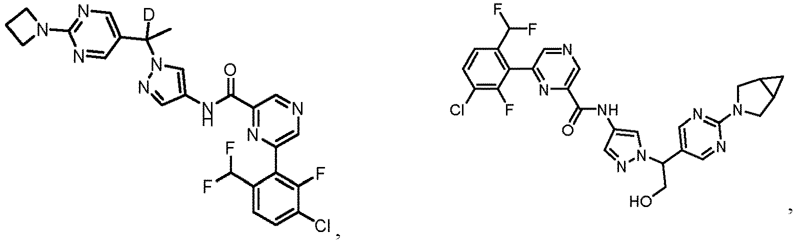 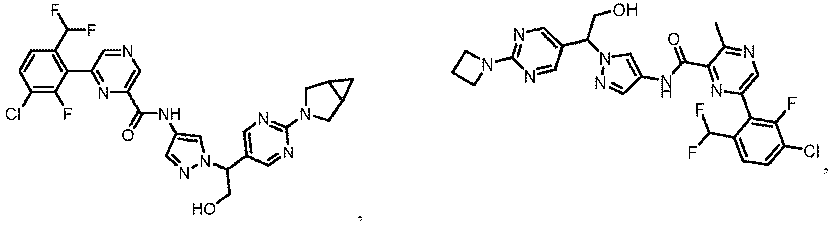 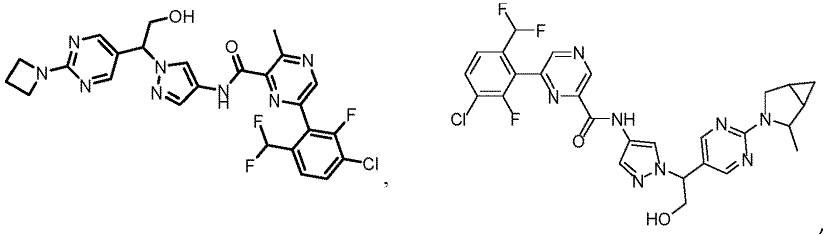 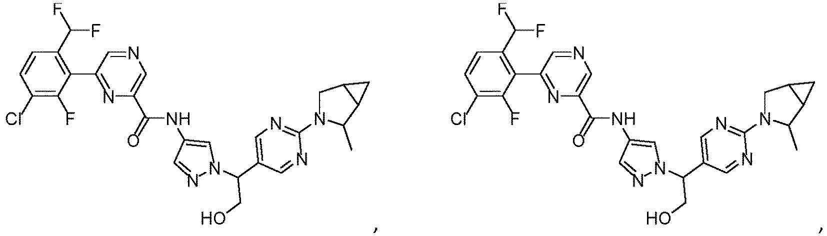 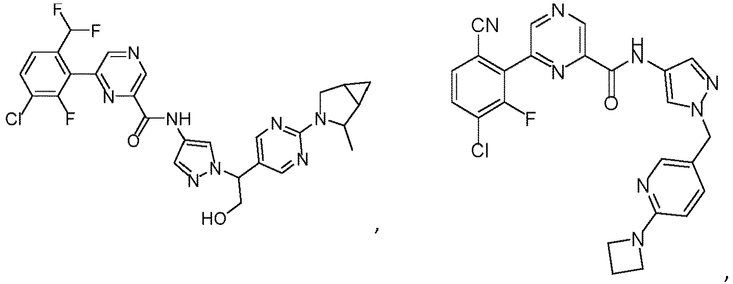 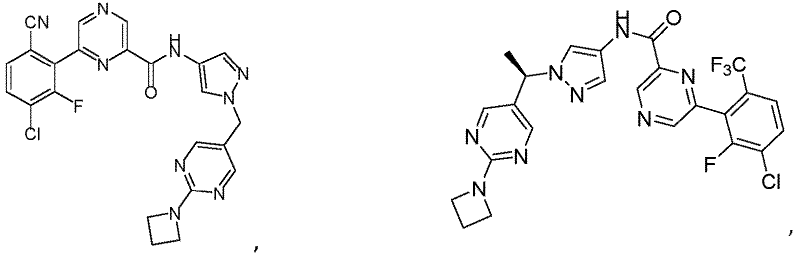 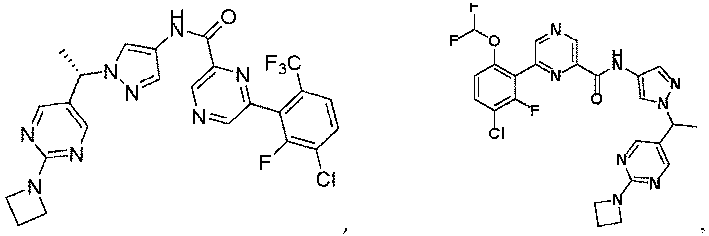 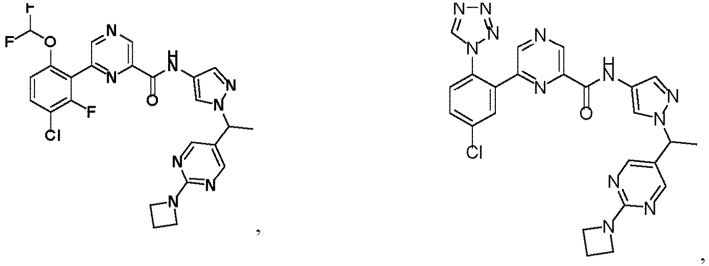 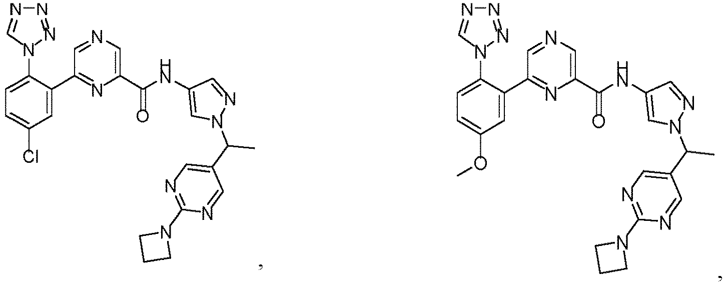 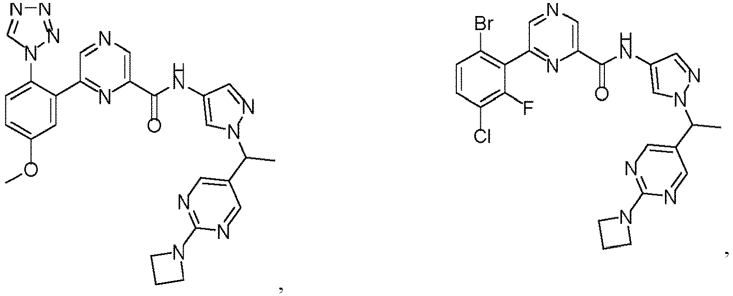 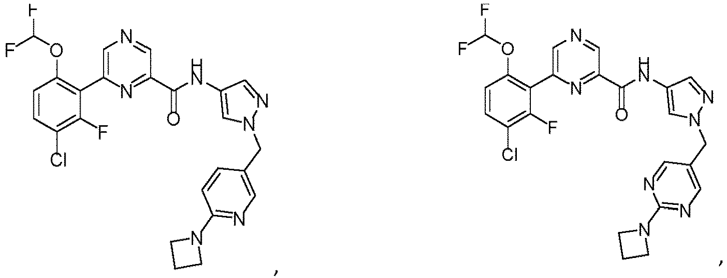 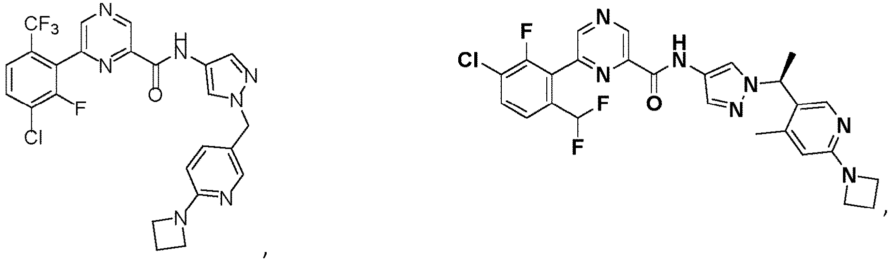 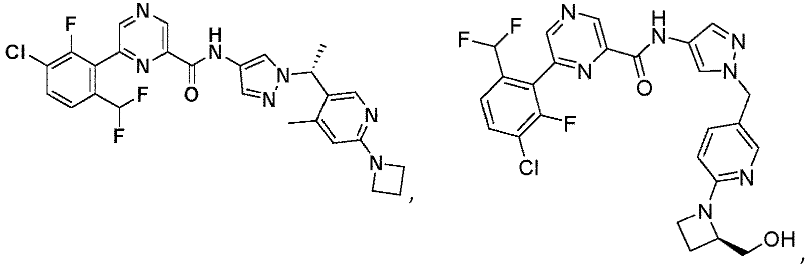 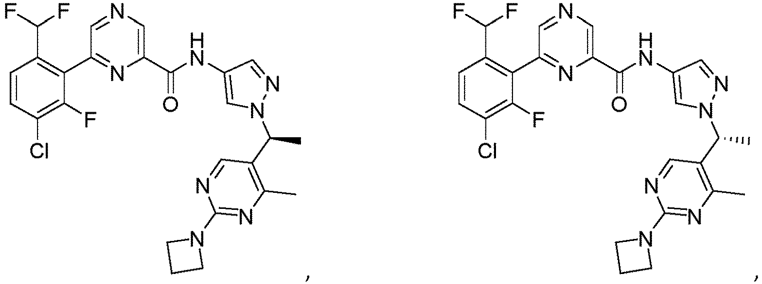 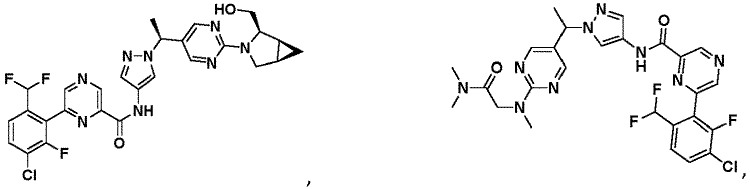 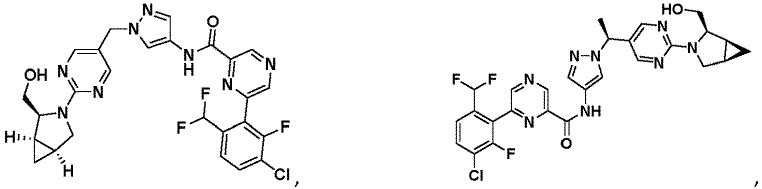 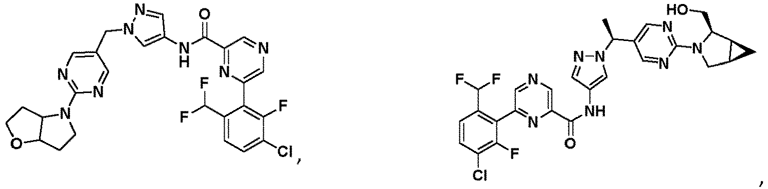 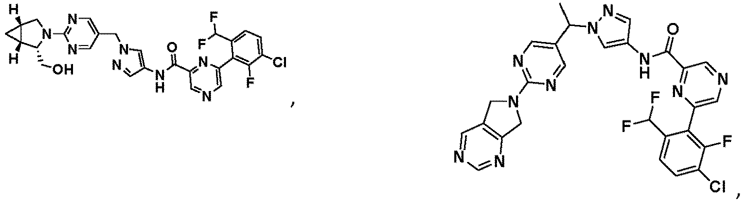 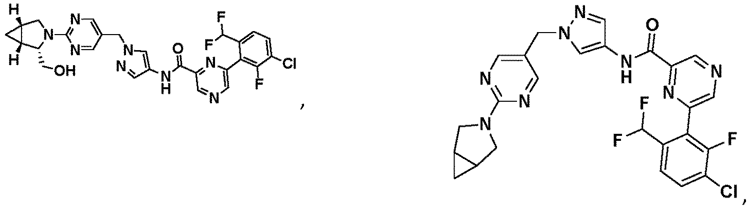 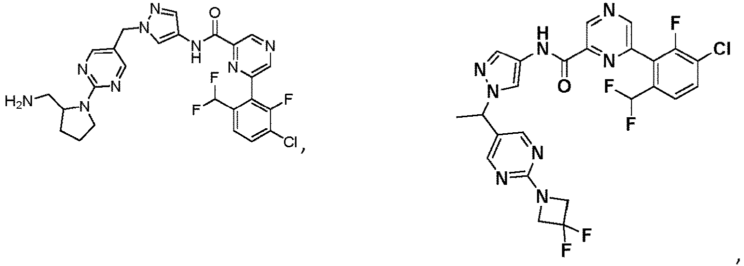 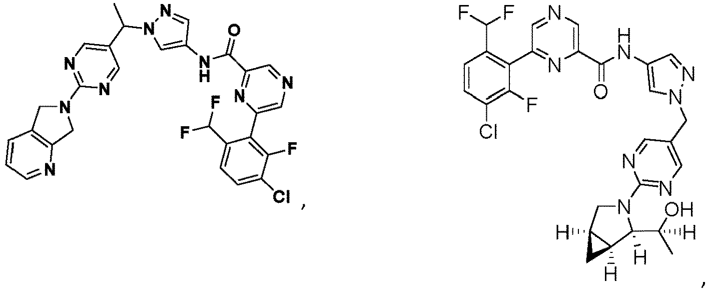 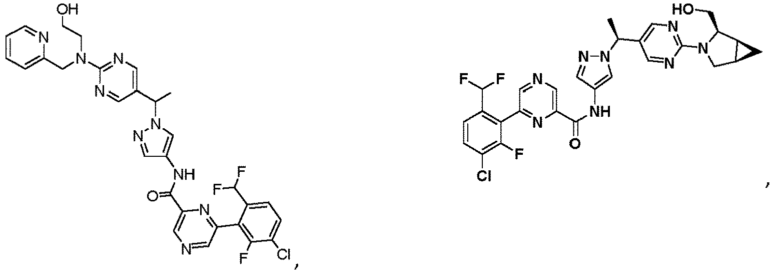 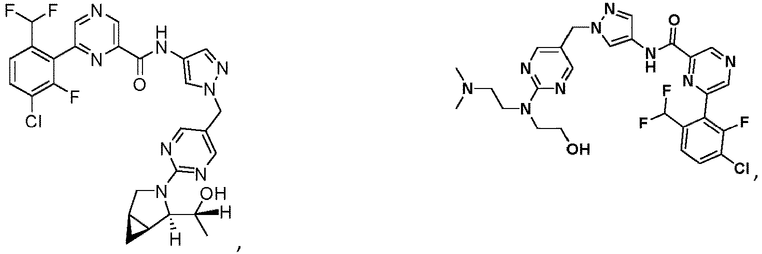 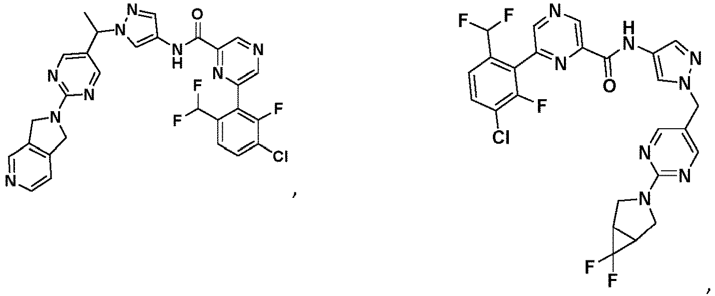 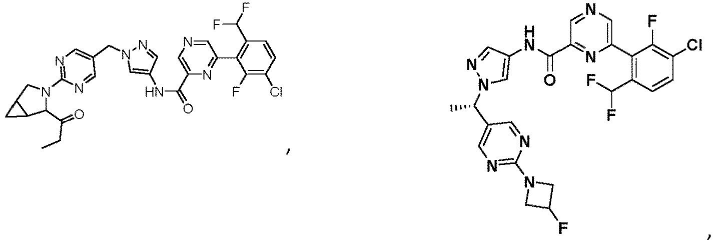 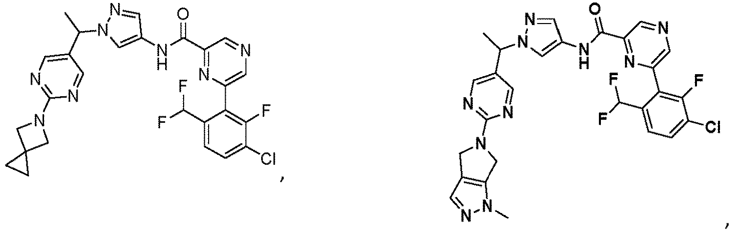 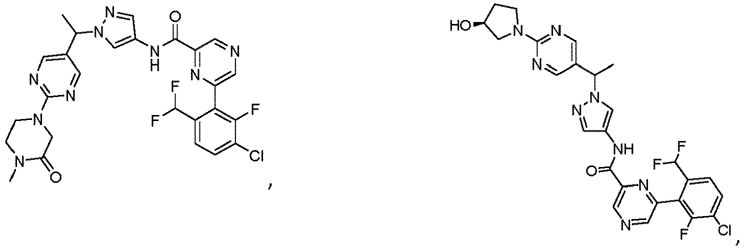 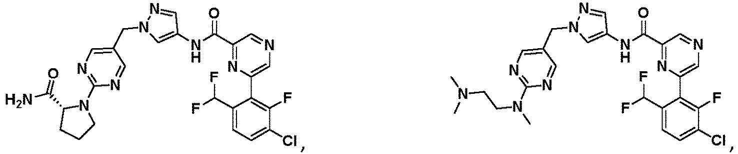 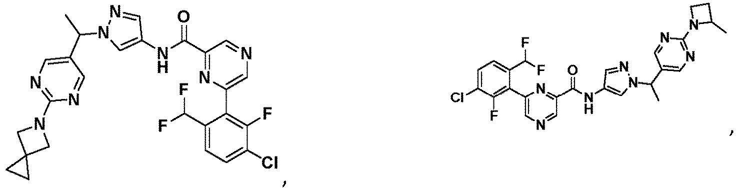 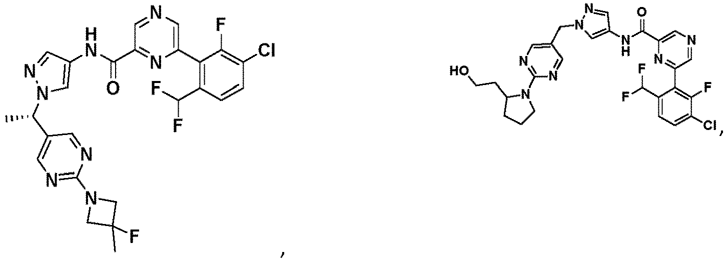 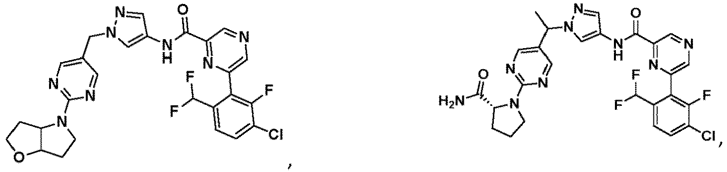 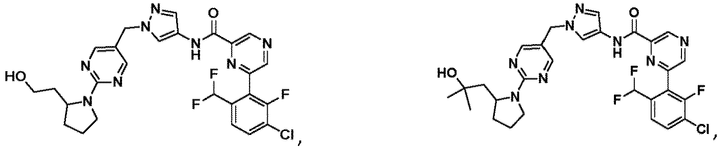 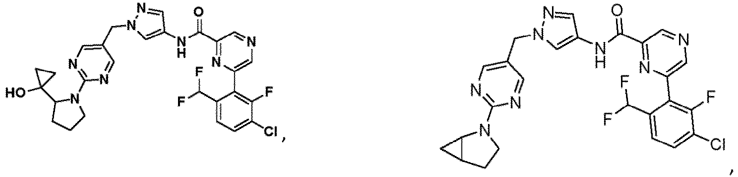 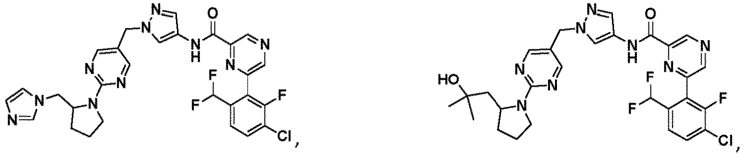 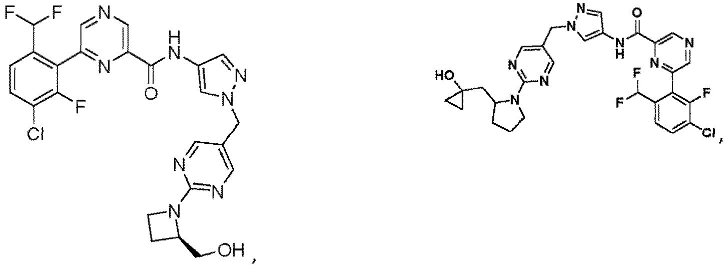 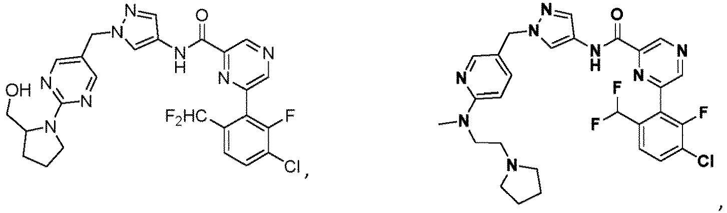 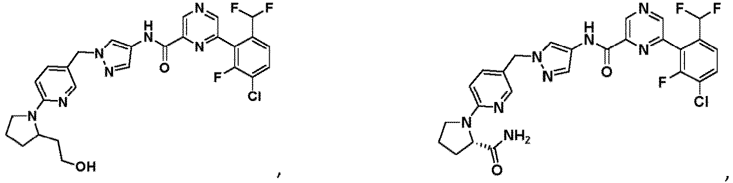 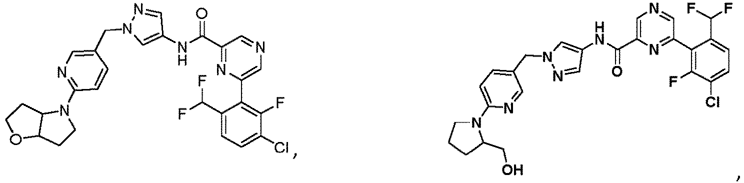 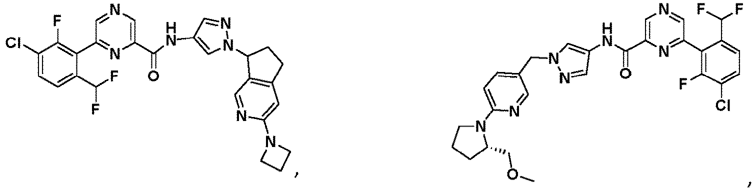 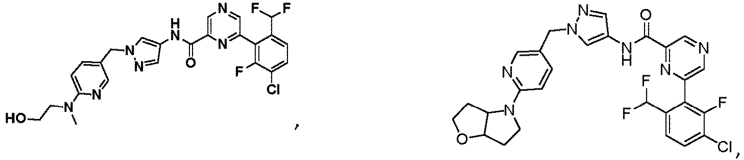 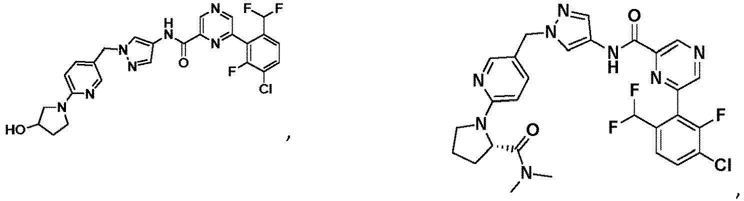 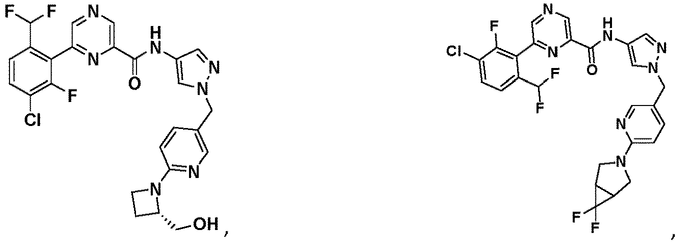 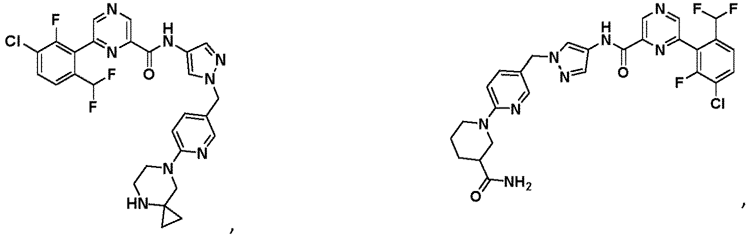 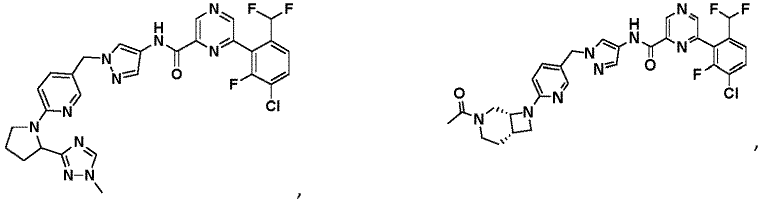 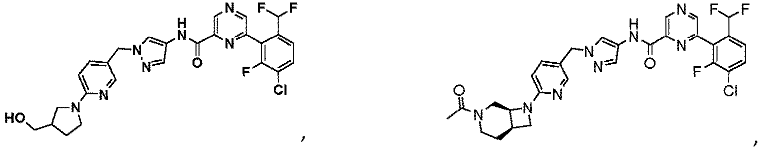 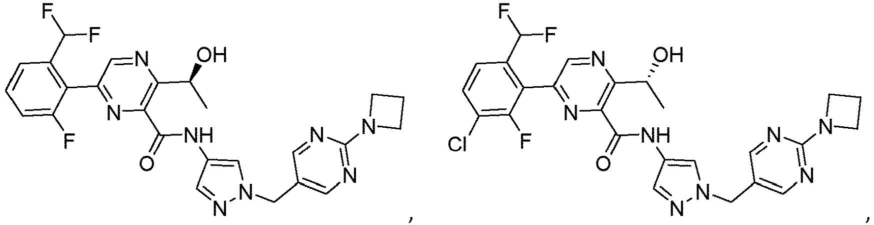 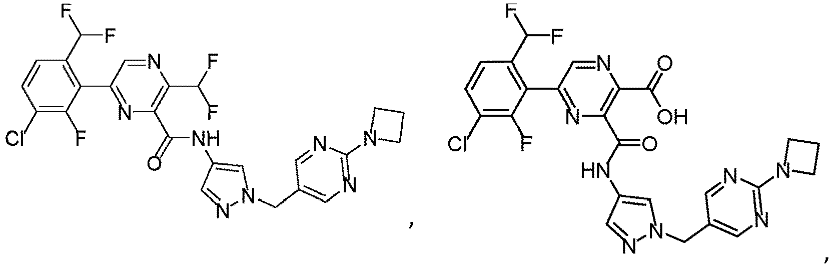 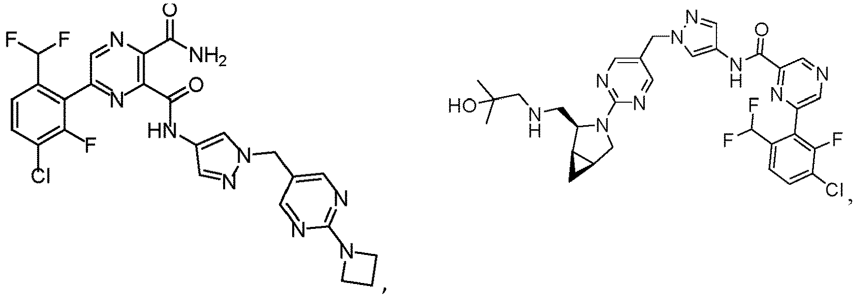 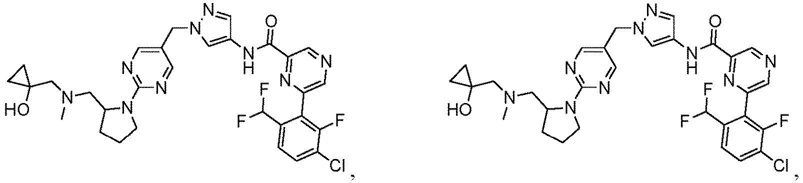 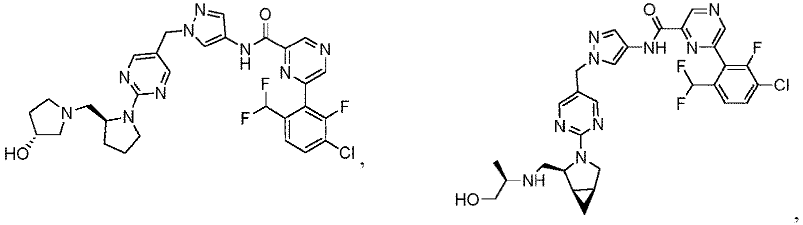 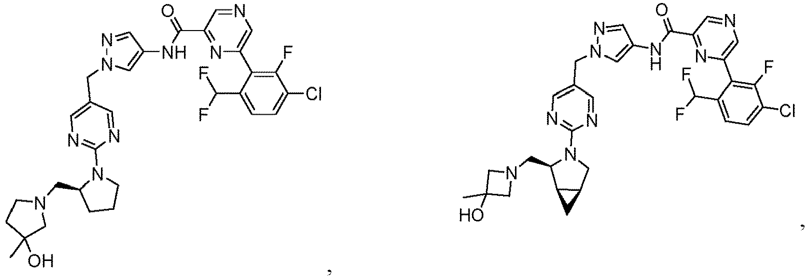 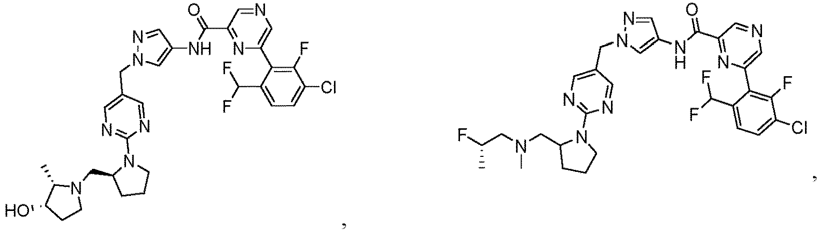 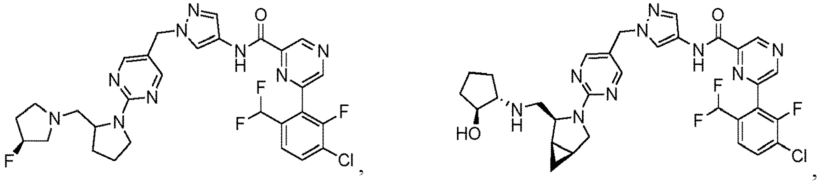 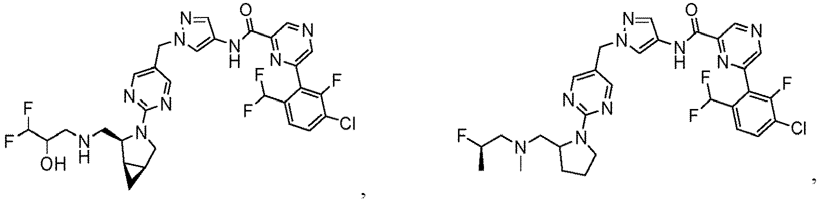 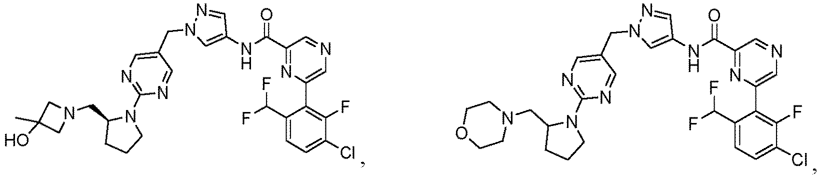 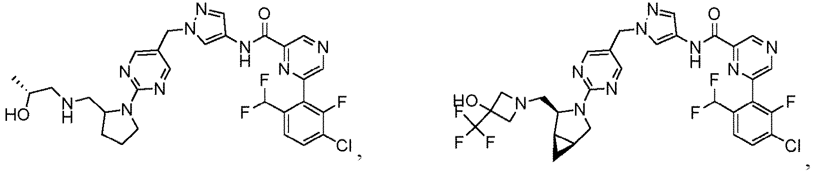 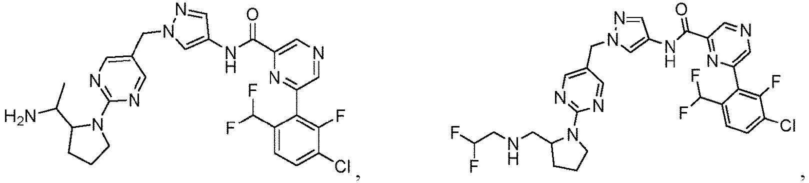 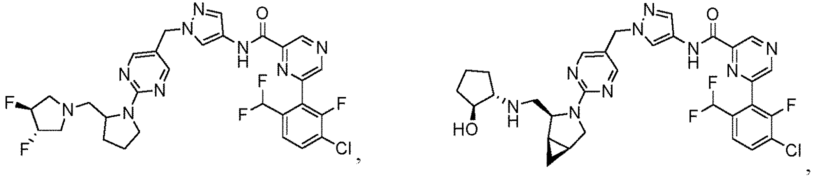 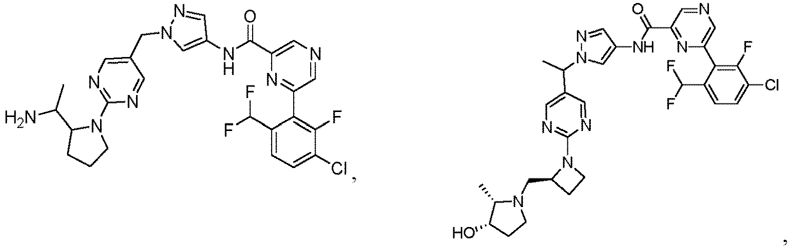 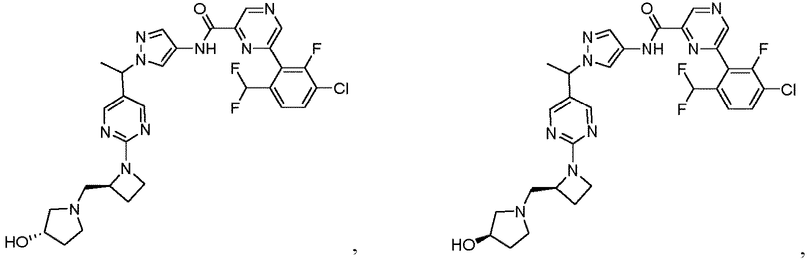 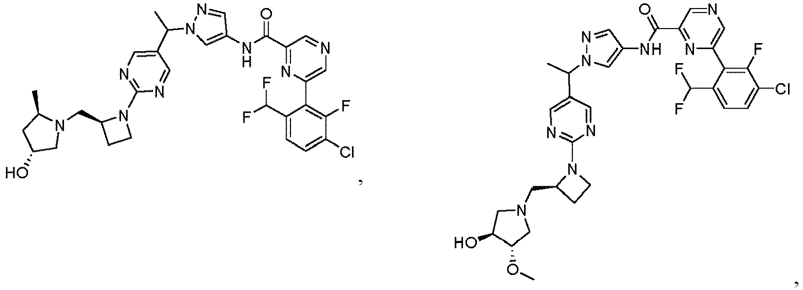 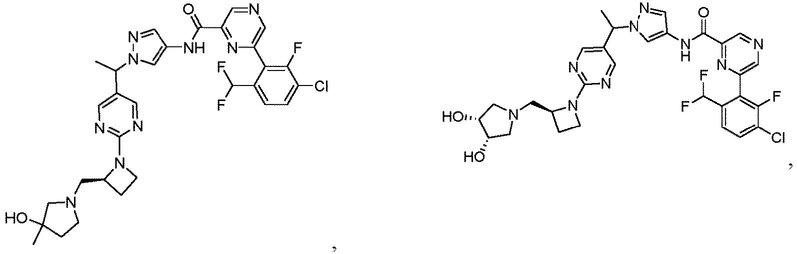 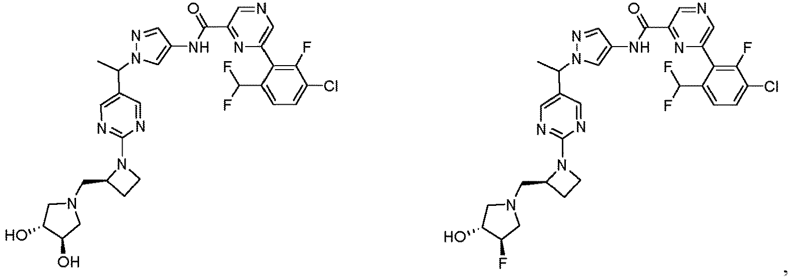 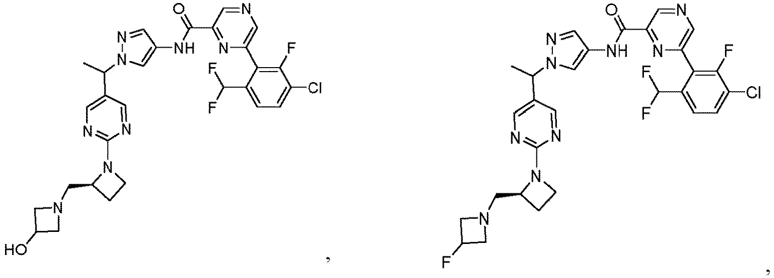 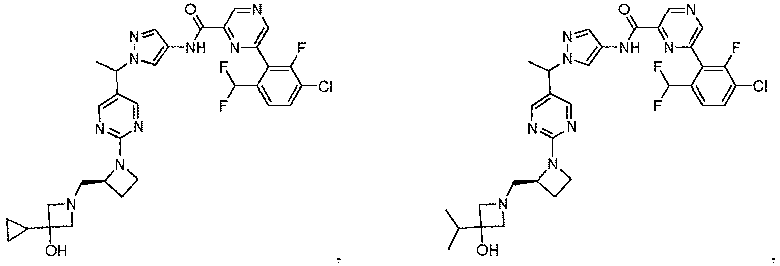 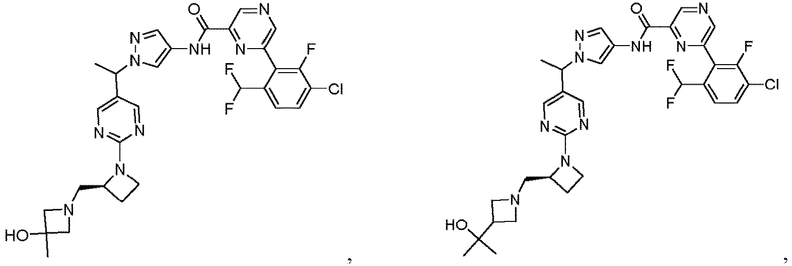 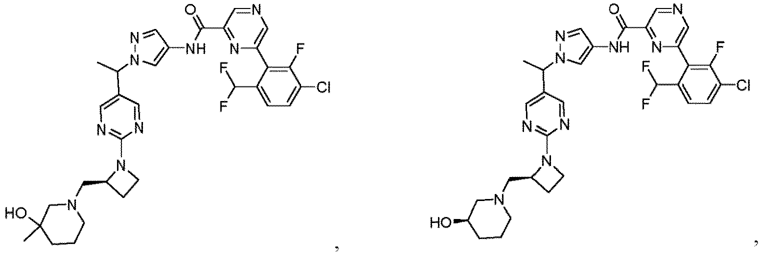 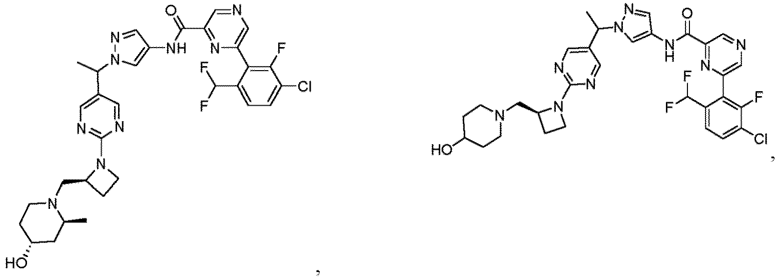 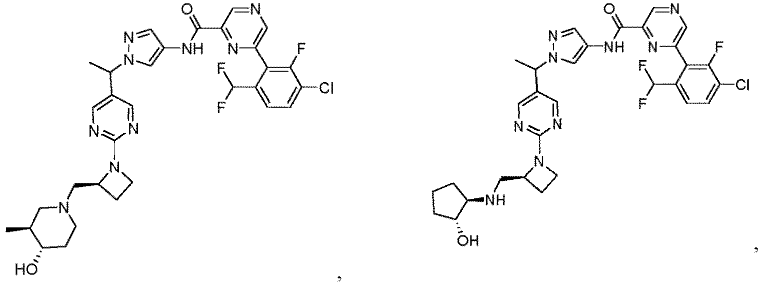 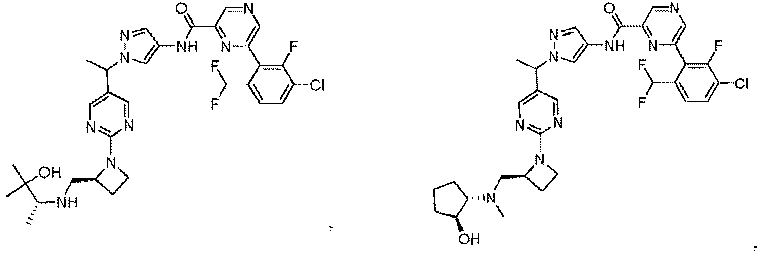 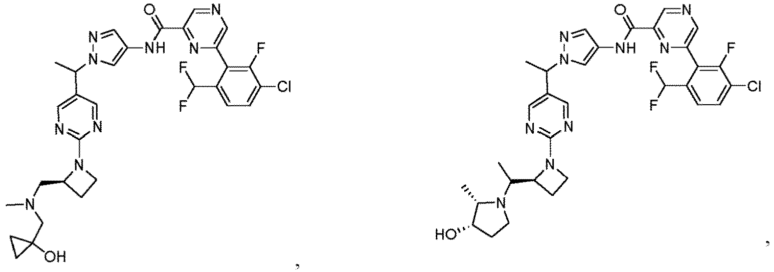 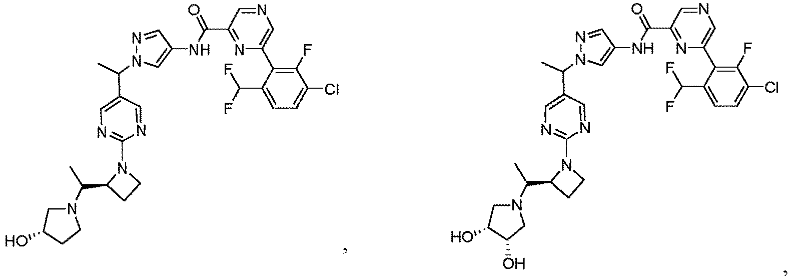 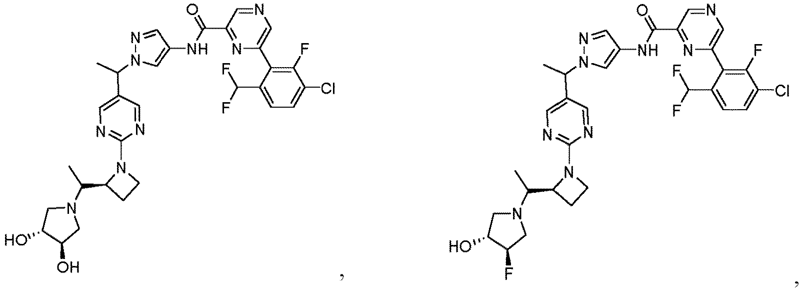 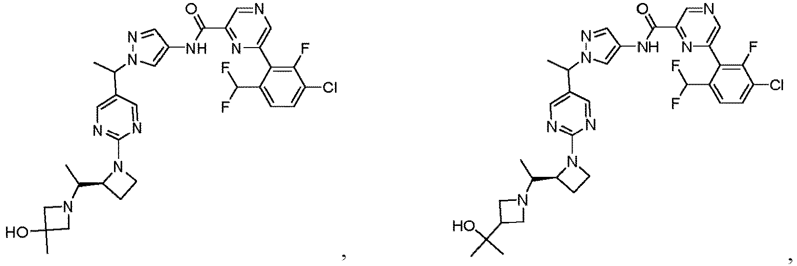 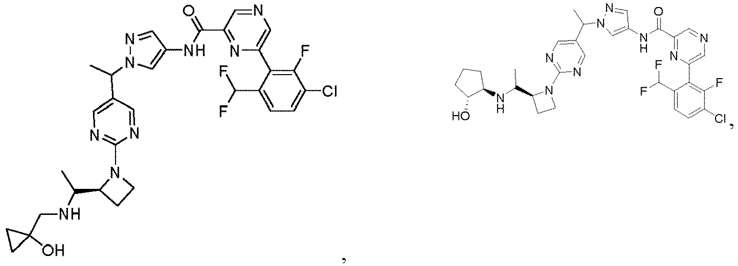 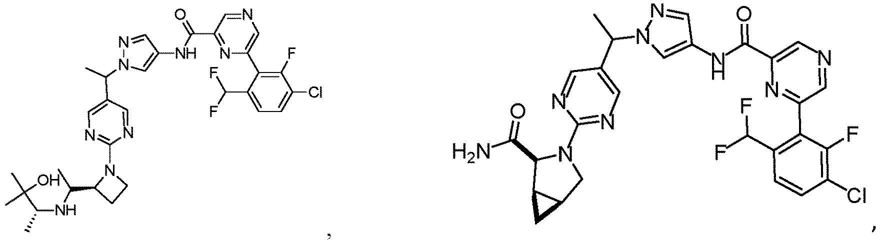 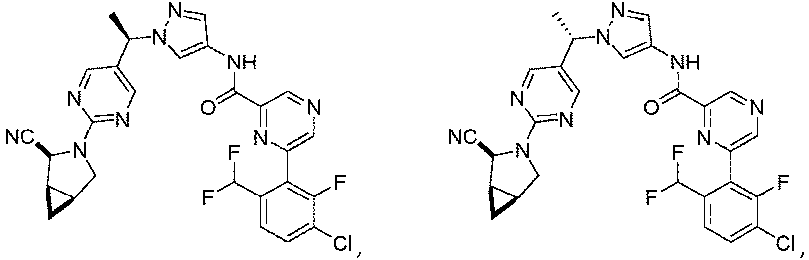 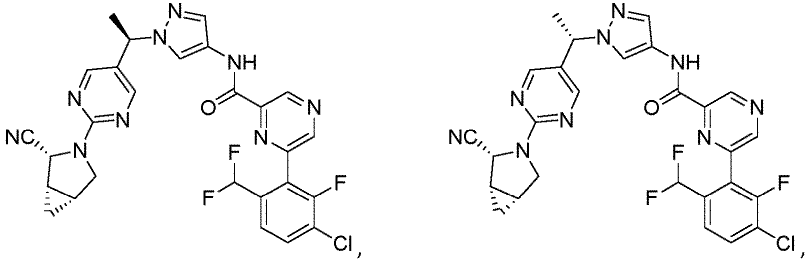 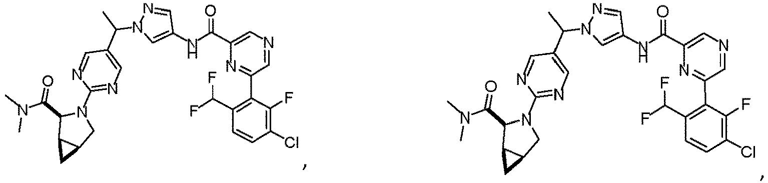 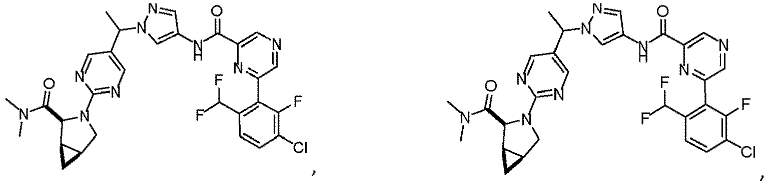 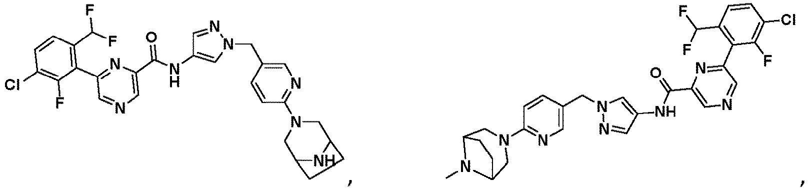 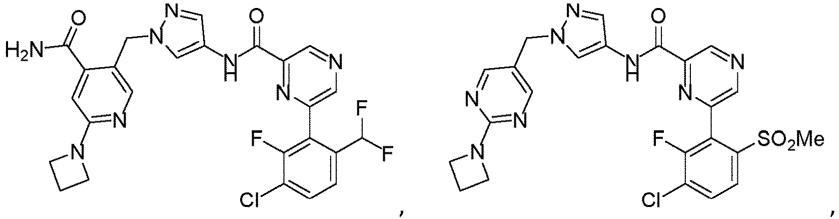 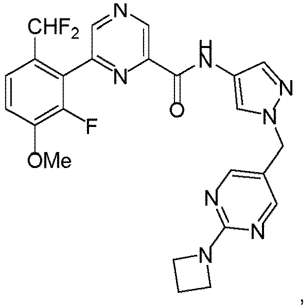 oder ein pharmazeutisch annehmbares Salz davon.

7. Eine pharmazeutische Zusammensetzung, die eine Verbindung nach einem der Ansprüche 1-6 oder ein pharmazeutisch annehmbares Salz davon und einen pharmazeutisch annehmbaren Träger umfasst.

8. Eine Verbindung nach einem der Ansprüche 1-6 oder ein pharmazeutisch annehmbares Salz davon oder eine pharmazeutische Zusammensetzung nach Anspruch 7 zur Verwendung bei einem Verfahren zur Behandlung von beeinträchtigter visueller Aktivität, diabetischer Retinopathie, diabetischem Makulaödem, retinalem Venenverschluss, hereditärem Angioödem, Diabetes, Pankreatitis, Hirnblutung, Nephropathie, Kardiomyopathie, Neuropathie, entzündlicher Darmerkrankung, Arthritis, Inflammation, septischem Schock, Hypotonie, Krebs, Atemnotsyndrom des Erwachsenen, disseminierter intravaskulärer Koagulation, Blutgerinnung während einer kardiopulmonalen Bypass-Operation, Blutungen aus postoperativen Eingriffen, Uveitis, posteriorer Uveitis, feuchtem altersbedingtem Makulaödem.

9. Eine Verbindung gemäß einem der Ansprüche 1-6 oder ein pharmazeutisch annehmbares Salz davon zur Verwendung in der Therapie.

10. Eine Kombination, die umfasst:
eine Verbindung nach einem der Ansprüche 1-6 oder ein pharmazeutisch annehmbares Salz davon oder eine pharmazeutische Zusammensetzung nach Anspruch 7, und
ein weiteres Mittel, ausgewählt aus der Gruppe bestehend aus Antiphlogistika, Anti-VEGF-Mitteln, Immunsuppressiva, Gerinnungshemmern, Thrombozytenaggregationshemmern und Thrombolytika.

11. Die Kombination nach Anspruch 10 zur Verwendung in der Therapie.

## Revendications

1. Composé de formule : 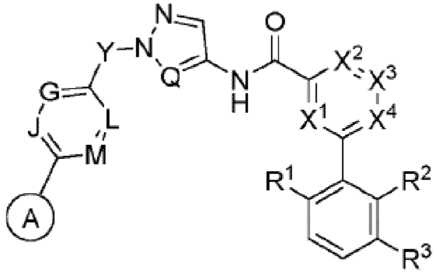 dans lequel 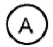 est choisi parmi : 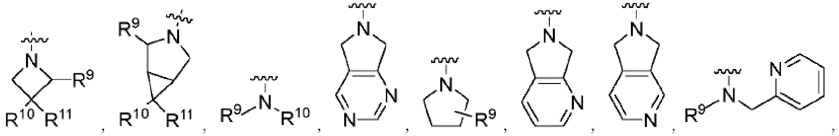 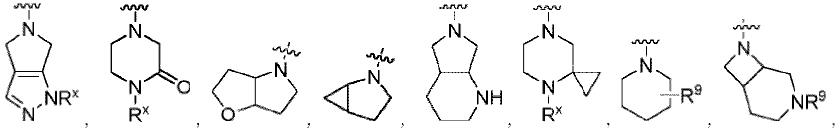 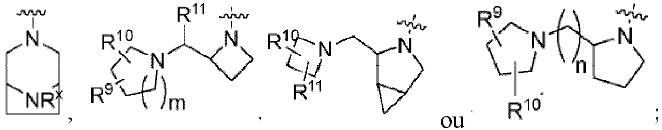
X¹ est N ;
X² est CR⁴ ;
X³ est N ;
X⁴ est CH ;
Y est CR⁵R⁶ ou SO₂ ;
Q est CH ;
G est N ou CR⁷ ;
J est N ou CR⁷ ;
L est N ou CR⁷ ;
M est N ou CR⁷ ;
R¹ est choisi dans le groupe constitué de : bromo, cyano, tétrazolyle, CHF₂, CF₃, OCHF₂ et SO₂CH₃ ;
R² est un hydrogène ou un halo ;
R³ est un hydrogène, un halo ou OR^{x} ;
R⁴ est un hydrogène, R^{x}, OR^{x}, (C=O)OR^{x}, (C=O)NR^{x}R^{y}, C₁₋₃ alkyl-OR^{x} ou NR^{x}R^{y} ;
R⁵ est un hydrogène ou un C₁₋₃ alkyle, qui est optionnellement substitué par un à trois substituants choisis parmi : deutérium, halo et hydroxy ;
R⁶ est un hydrogène, un deutérium ou un C₁₋₃ alkyle ;
ou R⁵ et R⁶ peuvent être pris ensemble avec l'atome de carbone entre eux pour former un groupe C₃₋₆ cycloalkyle ;
chaque R⁷ est indépendamment choisi dans le groupe constitué de : halo, cyano, R^{x}, OR^{x}, cyclopropyle, (C=O)NR^{x}R^{y} et NR^{x}R^{y} ;
ou R⁵ et R⁷ peuvent être pris ensemble avec les atomes entre eux pour former un groupe C₃₋₆ cycloalkyle ;
chaque R⁹ est indépendamment choisi dans le groupe constitué de : hydrogène, halo, hydroxy, cyano, triazolyle, cyclopropyle, (C=O)NR^{x}R^{y}, (C=O)R^{x} et C₁₋₃ alkyle, dans lequel ledit groupe triazolyle est optionnellement substitué par R^{x}, ledit groupe cyclopropyle est optionnellement substitué par R^{x} ou OR^{x} et ledit groupe alkyle est optionnellement substitué par un à quatre substituants choisis parmi : halo, hydroxy, hétéroaryle, hétérocyclyle, C₃₋₆ cycloalkyle, C₃₋₆ cycloalkyl(R¹⁰), OR^{x}, (C=O)NR^{x}R^{y} et NR^{x}R^{y} ;
R¹⁰ est un hydrogène, un halo, un hydroxy ou un C₁₋₃ alkyle, dans lequel ledit alkyle est optionnellement substitué par un à quatre substituants choisis parmi : halo et hydroxy ;
R¹¹ est un hydrogène, un halo, un hydroxy ou R^{x} ;
ou R¹⁰ et R¹¹ peuvent être pris ensemble avec les atomes entre eux pour former un groupe C₃₋₆ cycloalkyle ;
chaque R^{x} est indépendamment un hydrogène ou un C₁₋₆ alkyle, qui est optionnellement substitué par un à quatre substituants choisis parmi : halo et hydroxy ;
chaque R^{y} est indépendamment choisi dans le groupe constitué de : hydrogène, C₁₋₆ alkyle, C₃₋₆ cycloalkyle, CH₂-C₃₋₆ cycloalkyle et hétérocyclyle, dans lequel ledit alkyle est optionnellement substitué par un à quatre substituants choisis parmi : halo et hydroxy, ledit cycloalkyle est optionnellement substitué par un ou deux R^{x} ou OR^{x} et ledit hétérocyclyle est optionnellement substitué par un ou deux R¹⁰ ;
m est un nombre entier de zéro à deux ;
n est un nombre entier de zéro à deux ;
ou un sel pharmaceutiquement acceptable de celui-ci.

2. Composé selon la revendication 1, dans lequel :
dans lequel 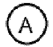 est 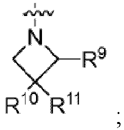 ou un sel pharmaceutiquement acceptable de celui-ci.

3. Composé selon la revendication 1 ou la revendication 2, dans lequel R4 est choisi dans le groupe constitué de : hydrogène, CH3, CHF2, OCH3, NH2, N(CH3)2, (C=O)OH et (C=O)NH2 ; ou un sel pharmaceutiquement acceptable de celui-ci.

4. Composé selon l'une quelconque des revendications 1-3, dans lequel R⁵ est un hydrogène, CH₃, CHF₂ ou CH₂OH ; ou un sel pharmaceutiquement acceptable de celui-ci.

5. Composé selon l'une quelconque des revendications 1-4, dans lequel J est N ; M est N ; G est CR⁷ ; L est CR⁷ ; ou un sel pharmaceutiquement acceptable de celui-ci.

6. Composé selon la revendication 1 choisi parmi : 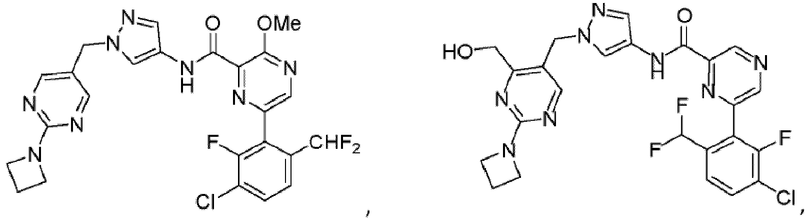 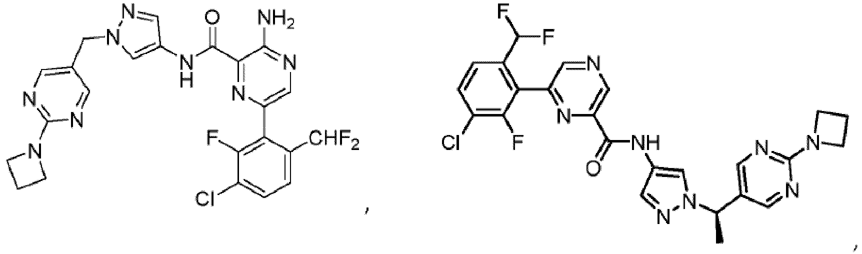 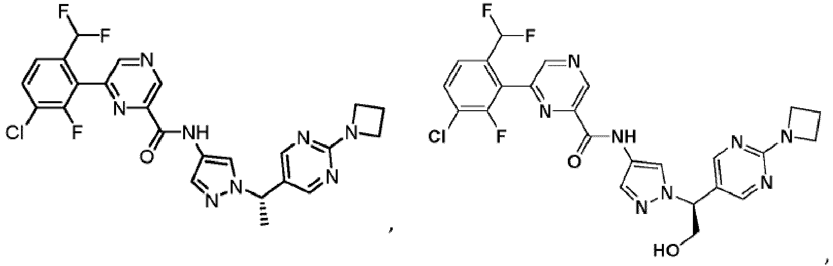 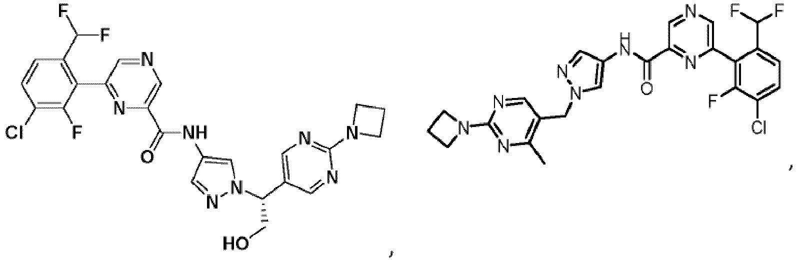 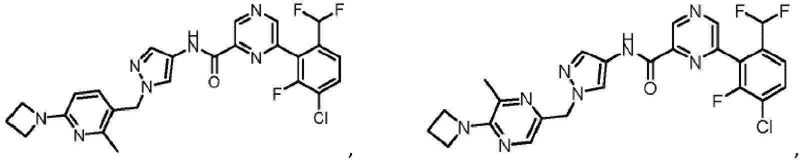 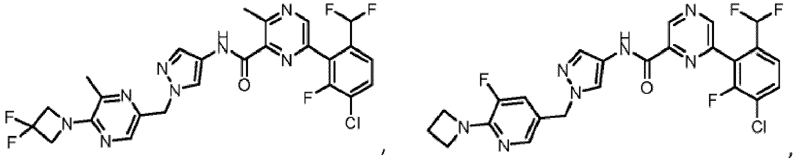 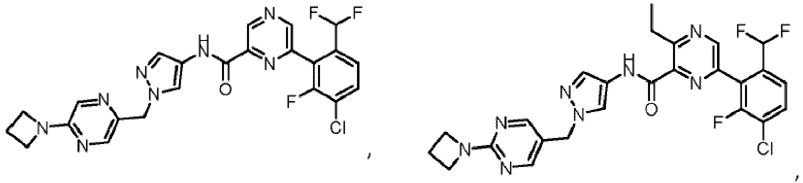 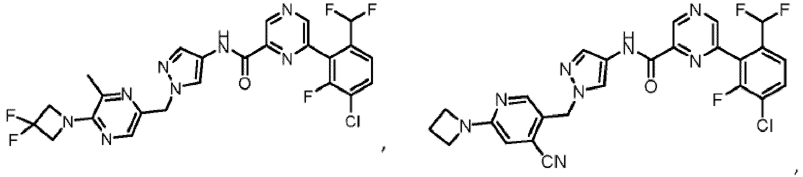 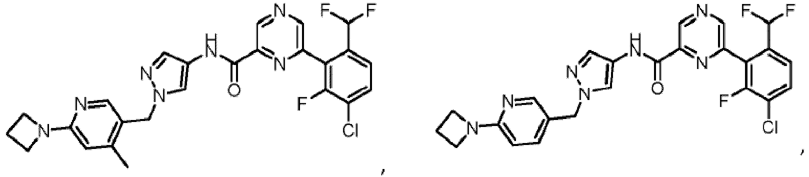 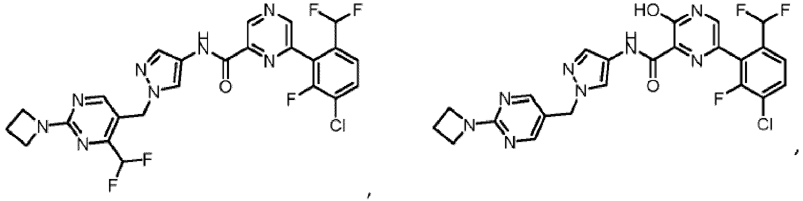 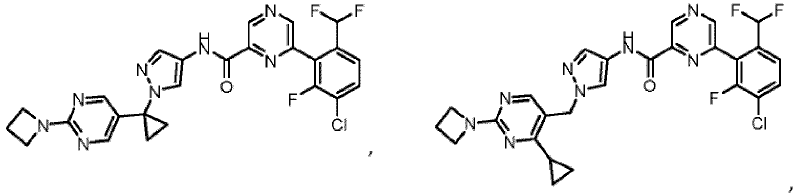 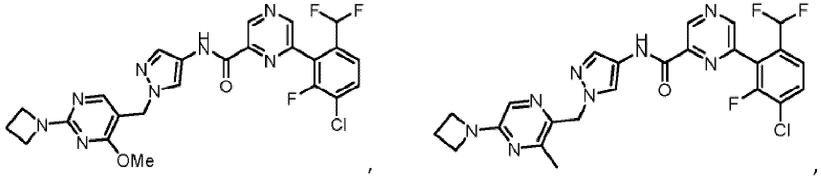 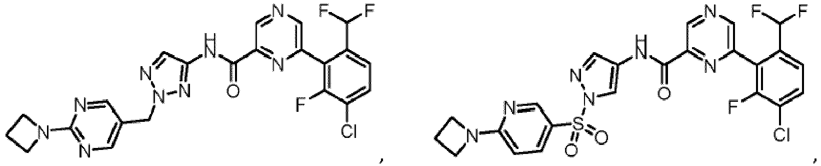 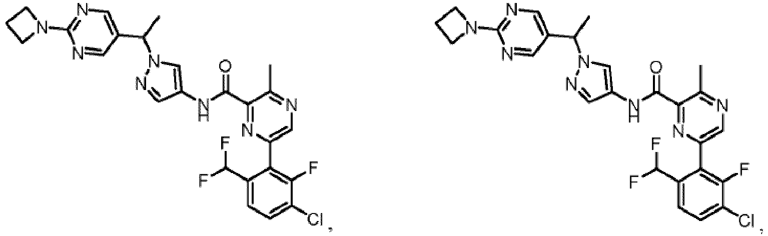 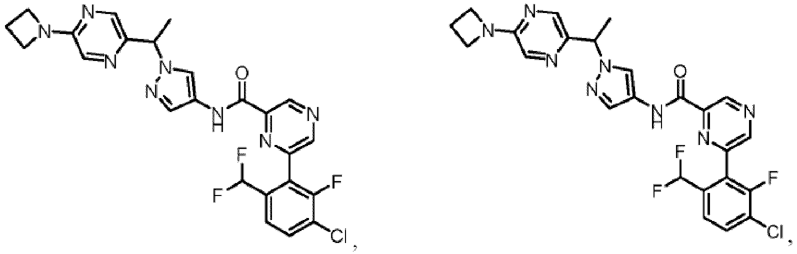 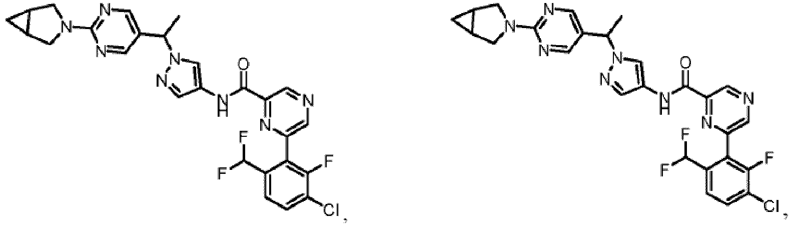 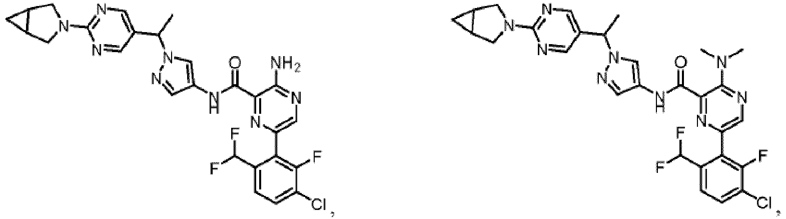 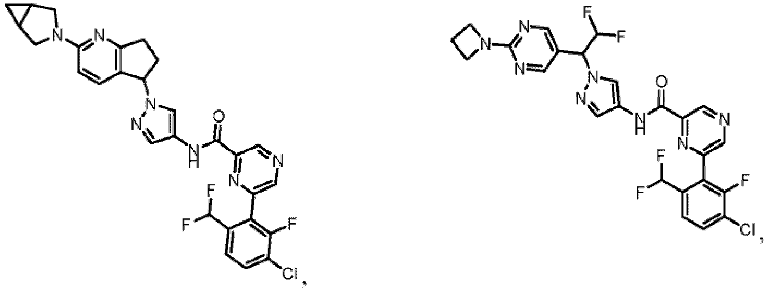 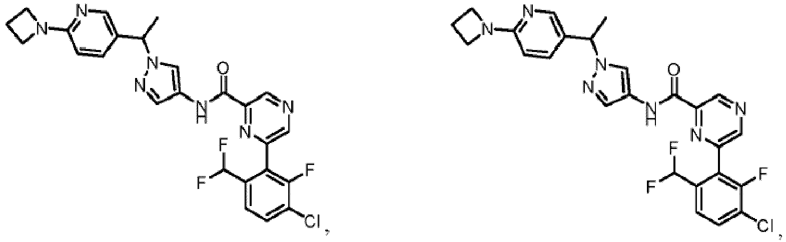 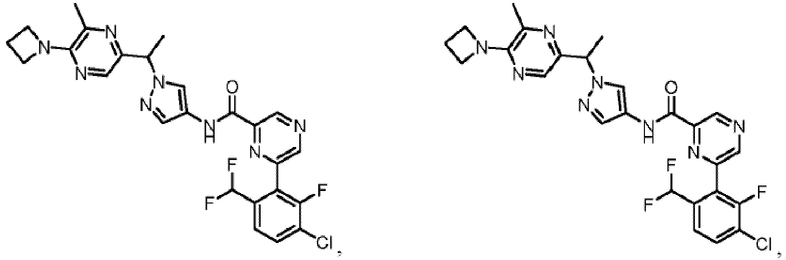 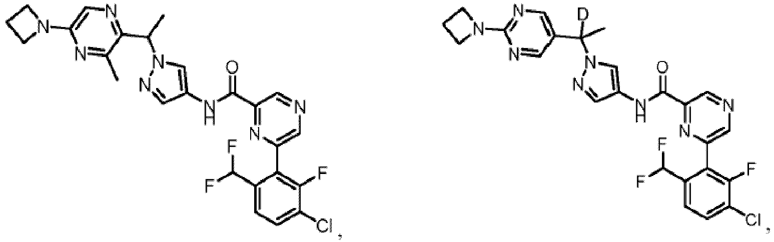 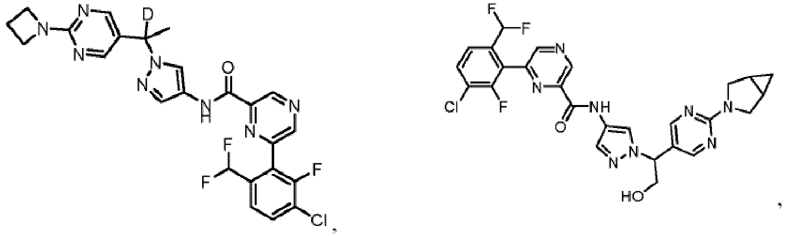 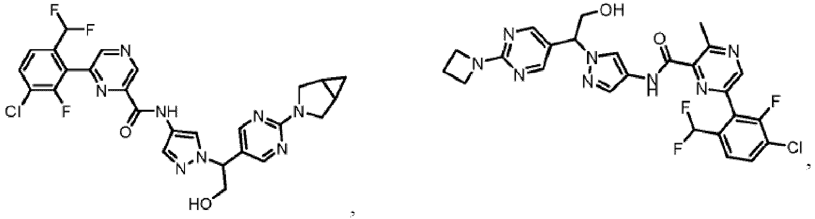 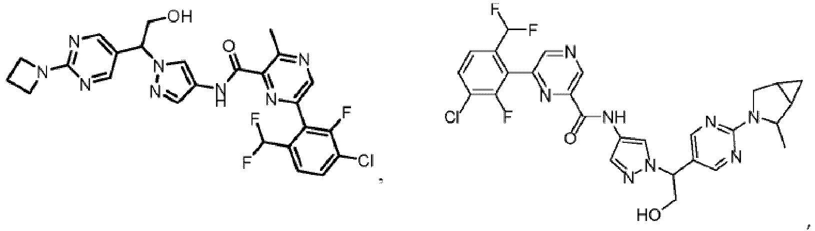 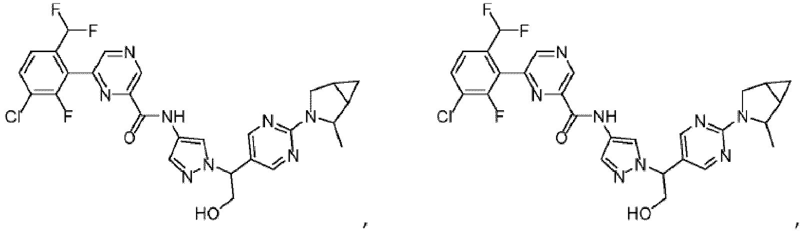 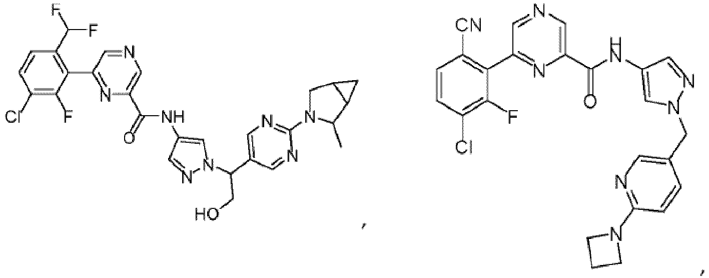 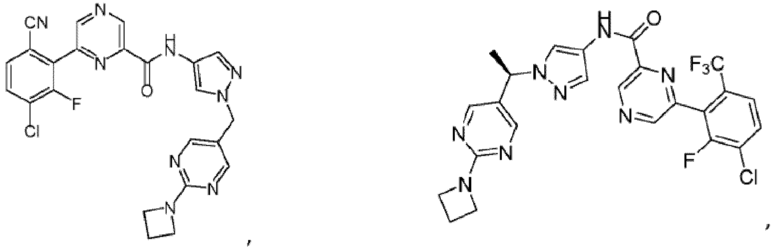 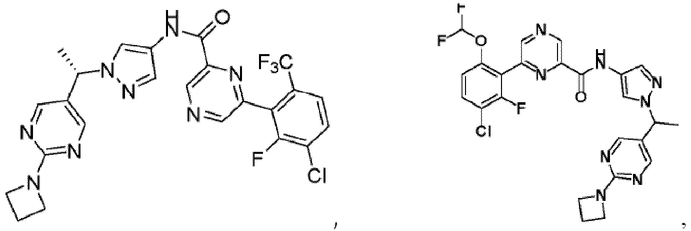 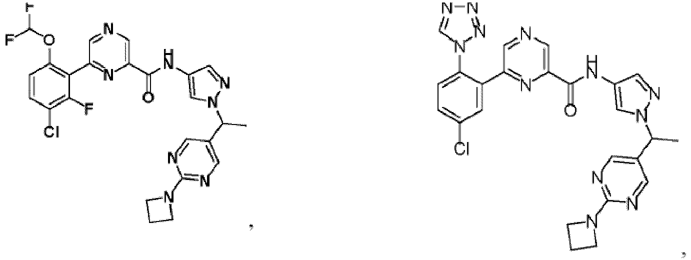 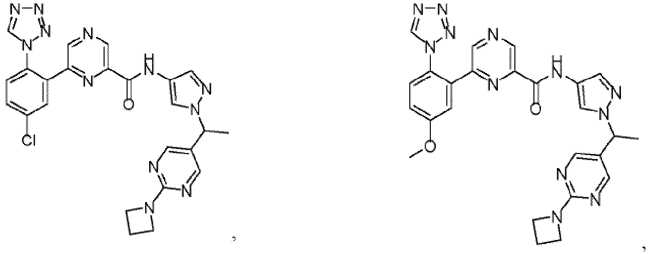 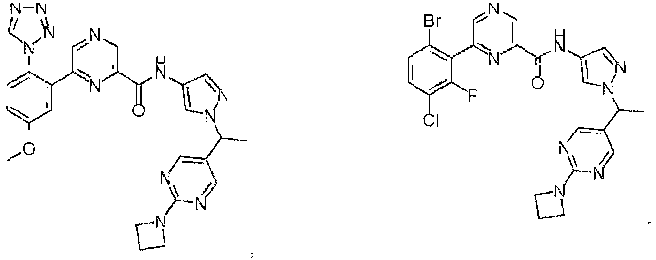 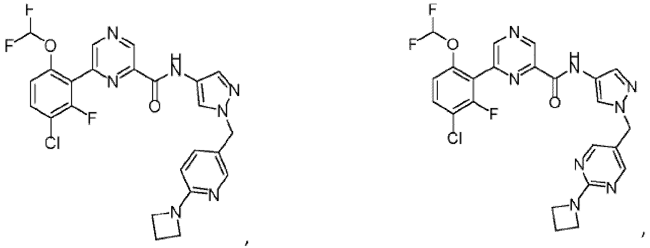 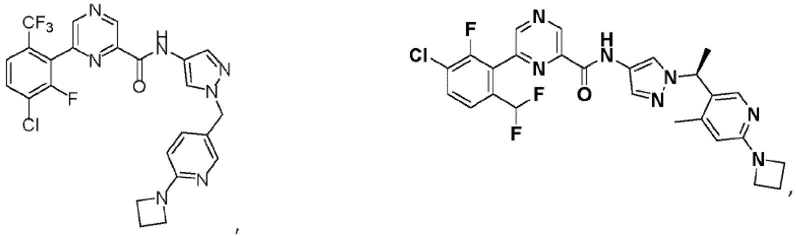 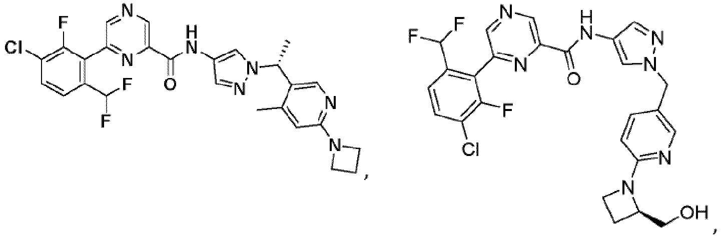 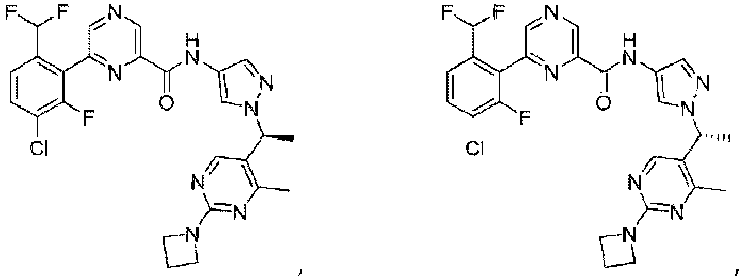 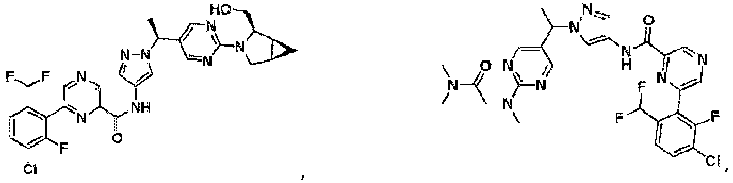 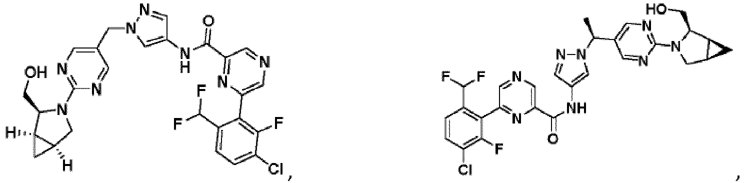 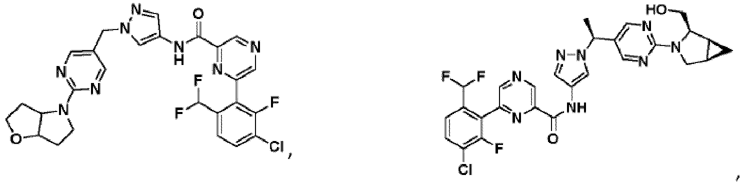 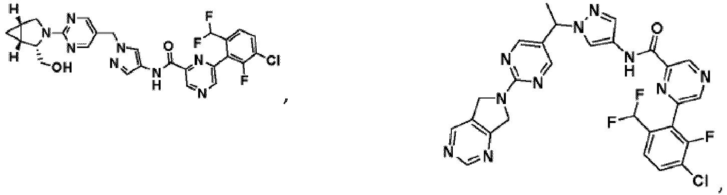 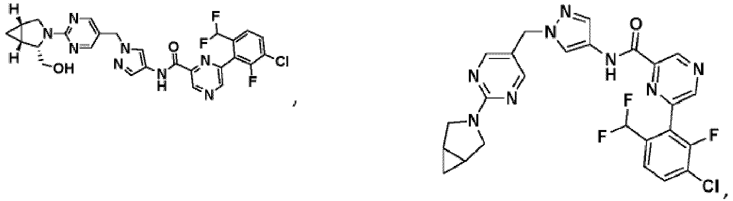 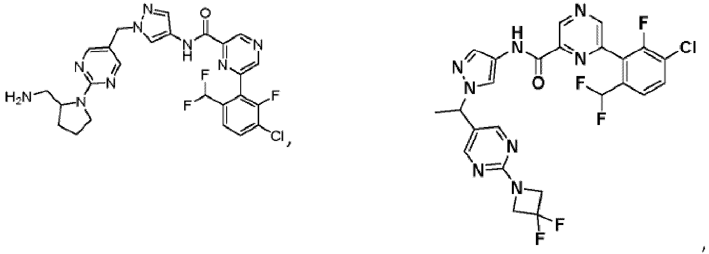 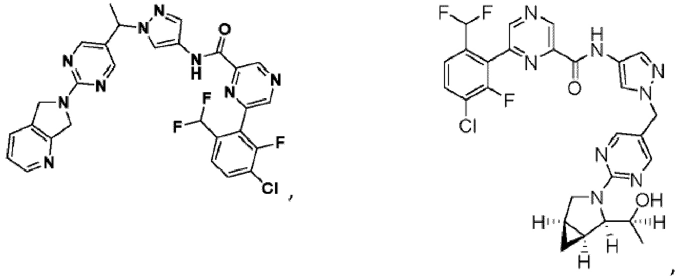 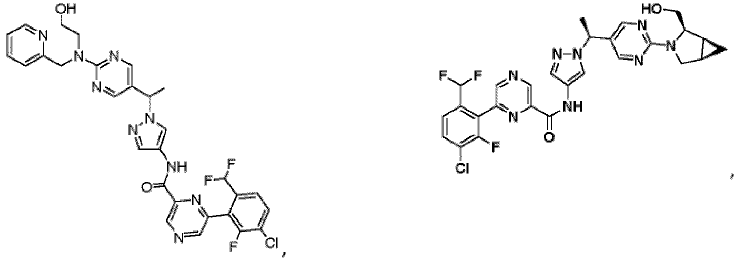 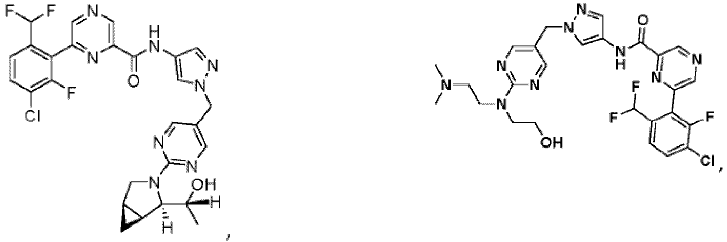 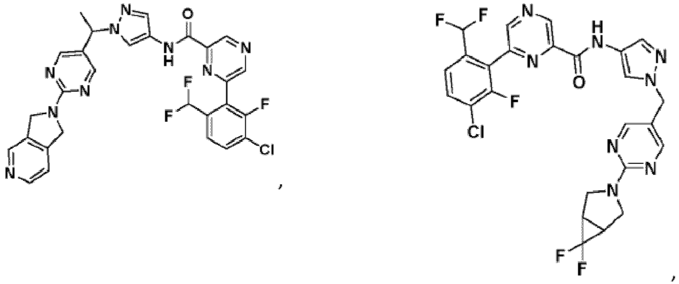 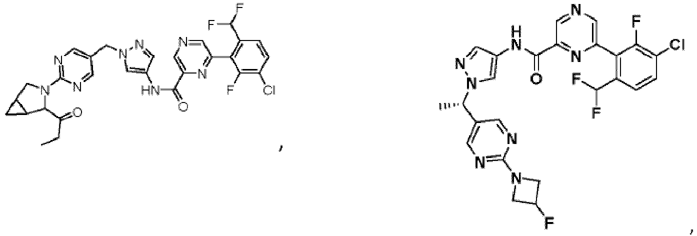 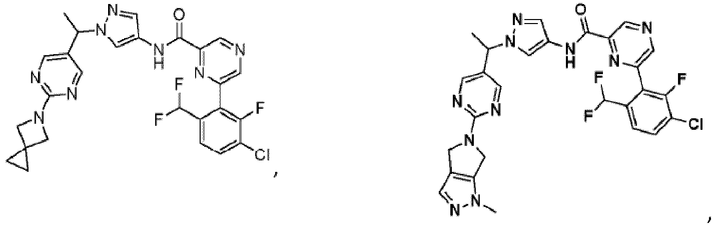 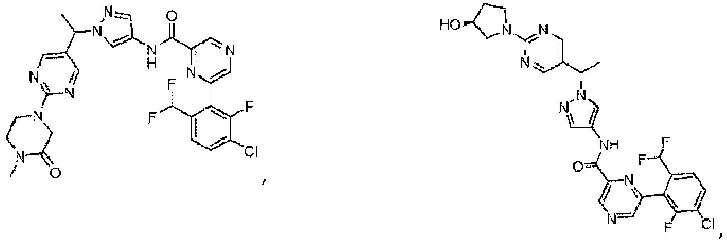 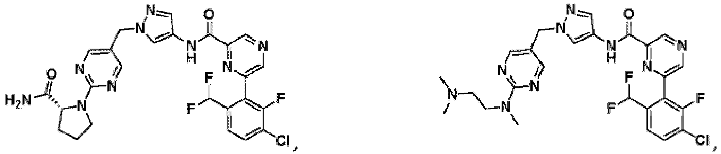 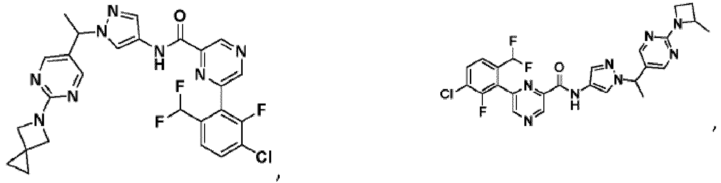 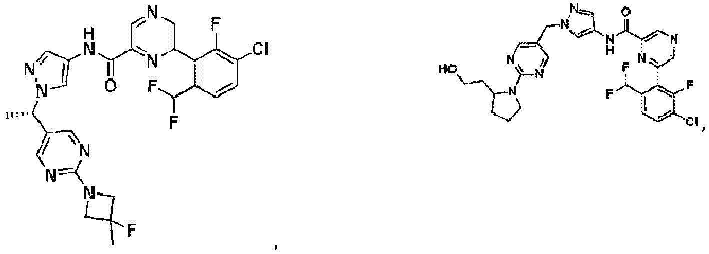 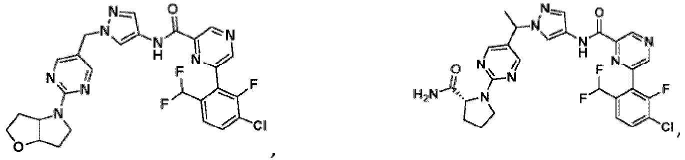 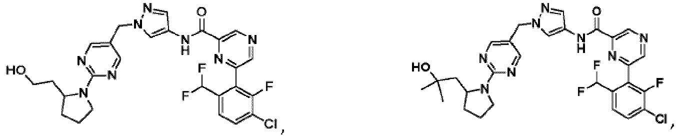 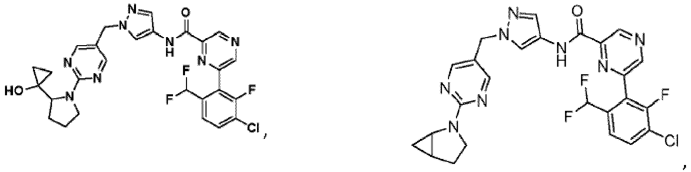 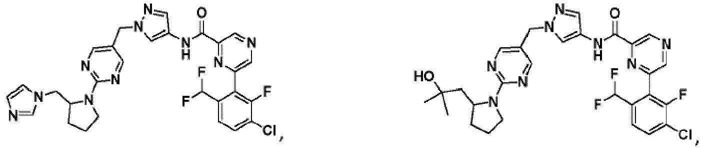 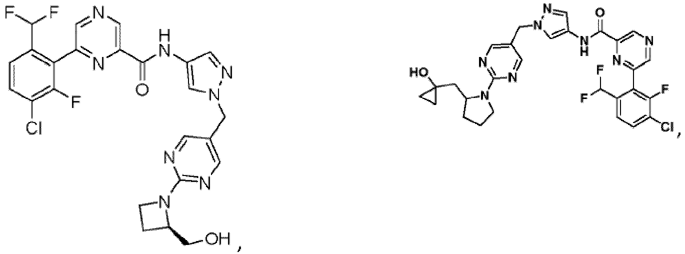 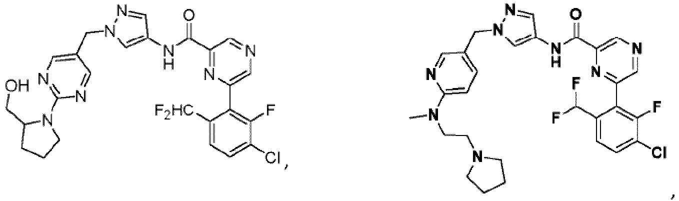 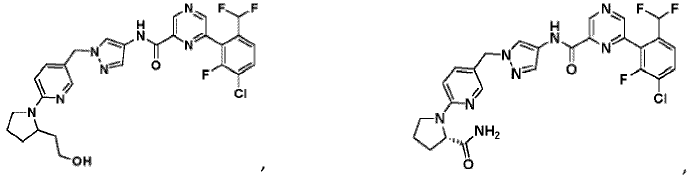 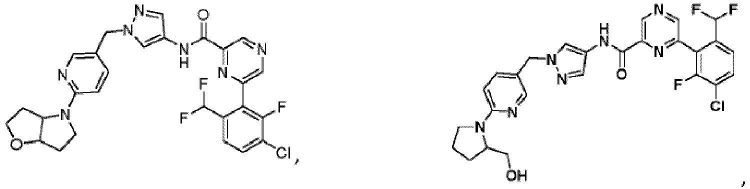 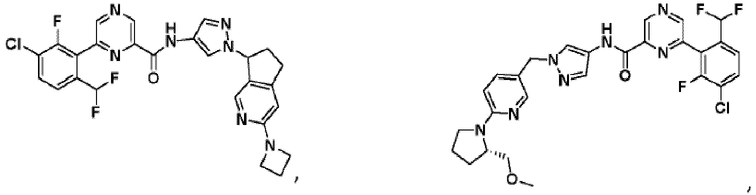 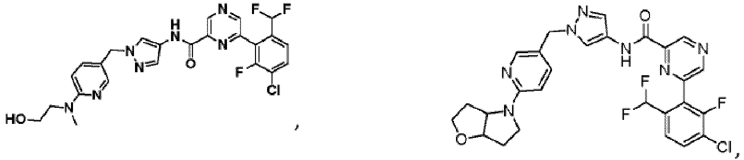 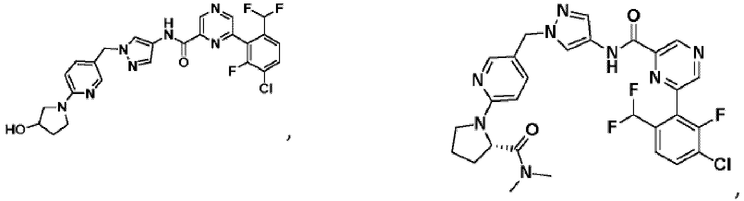 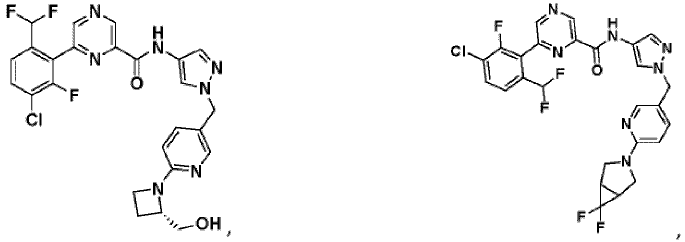 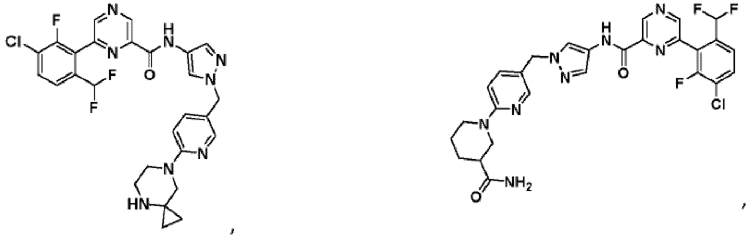 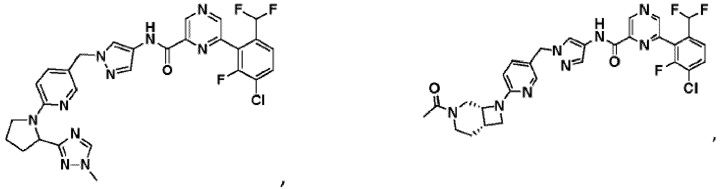 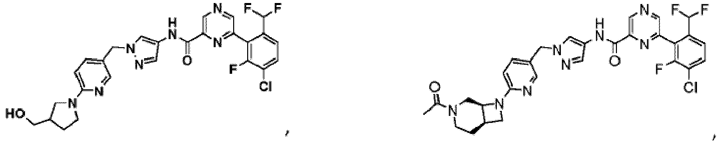 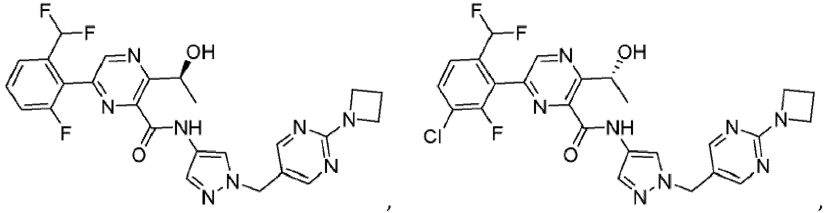 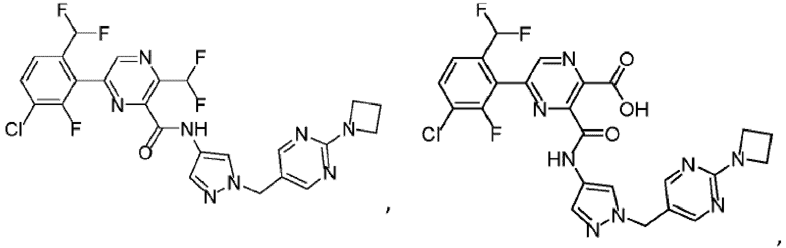 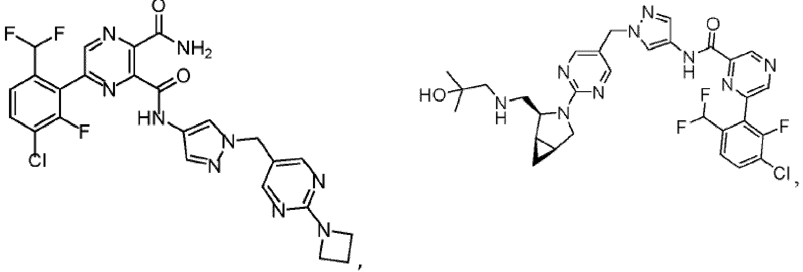 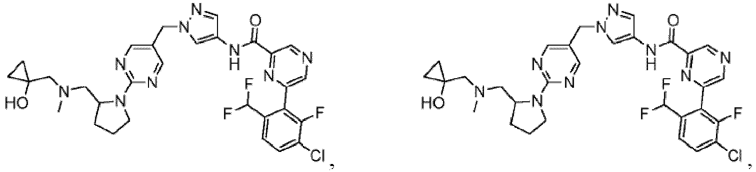 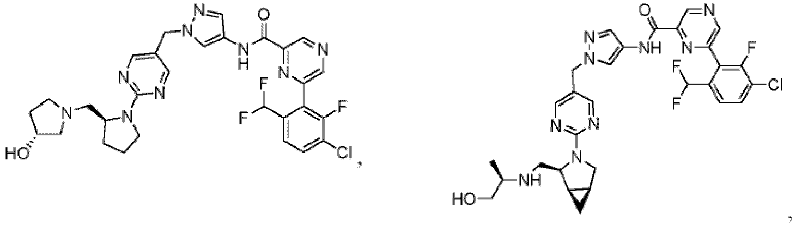 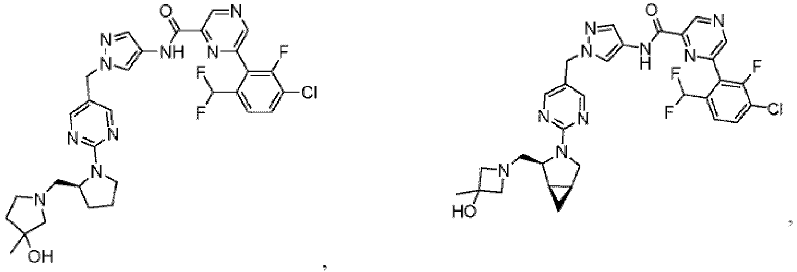 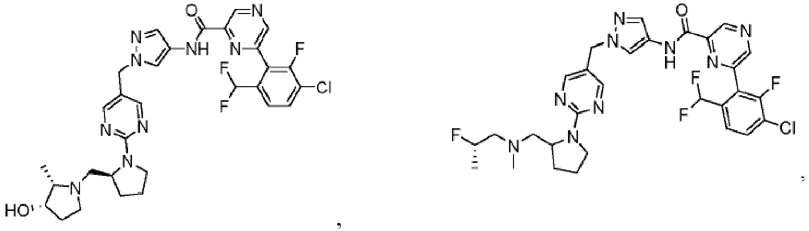 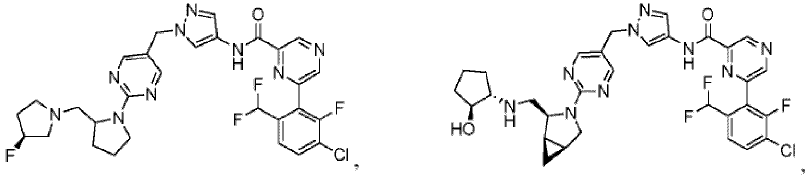 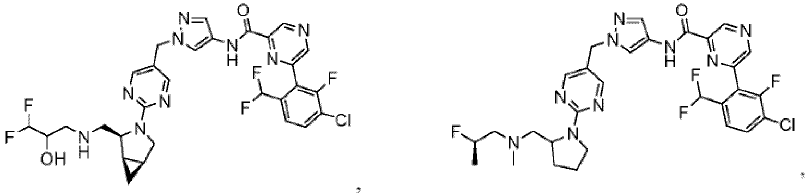 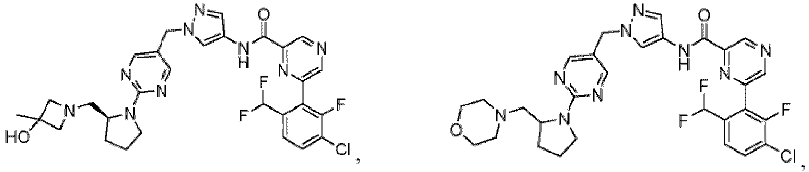 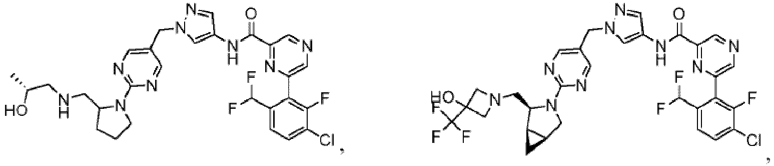 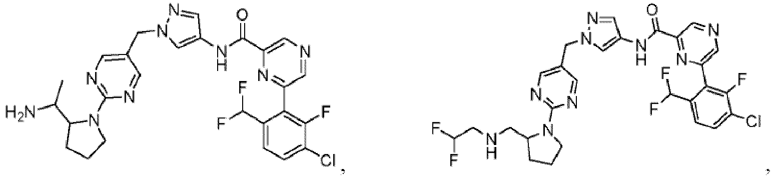 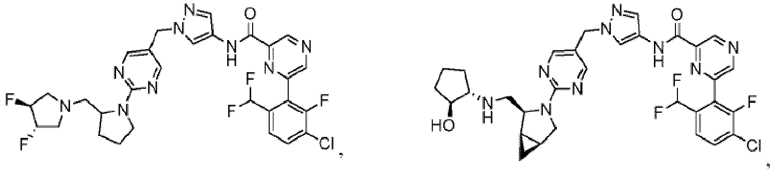 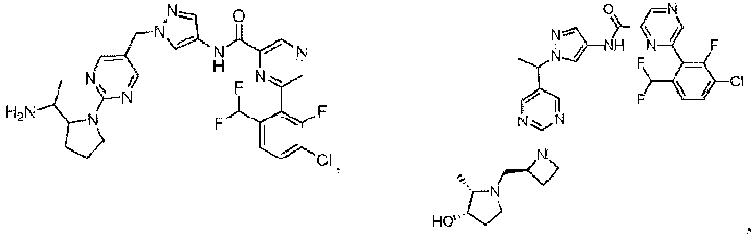 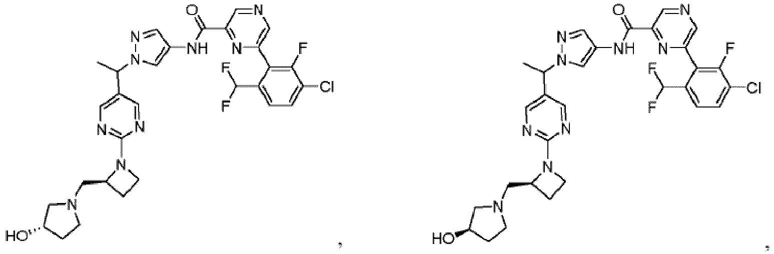 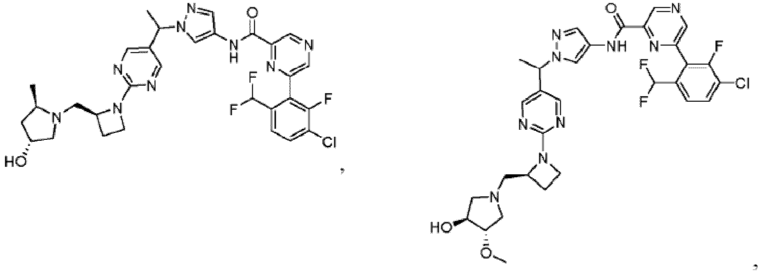 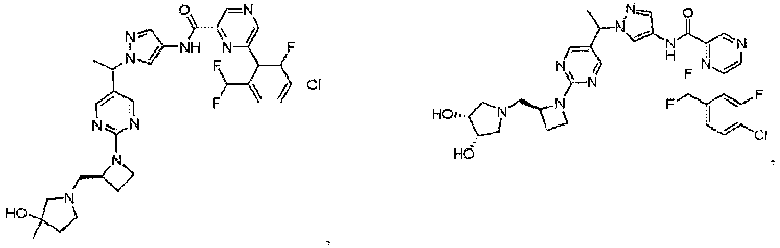 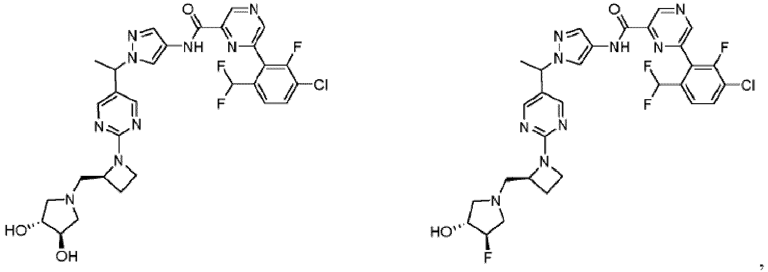 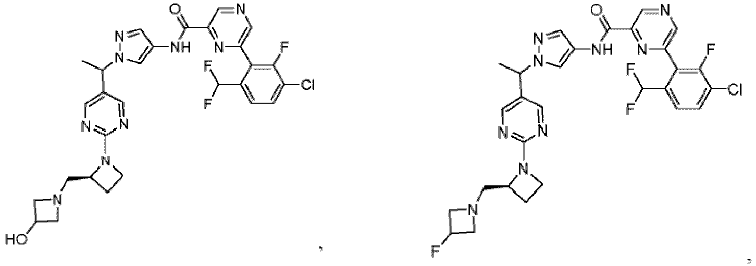 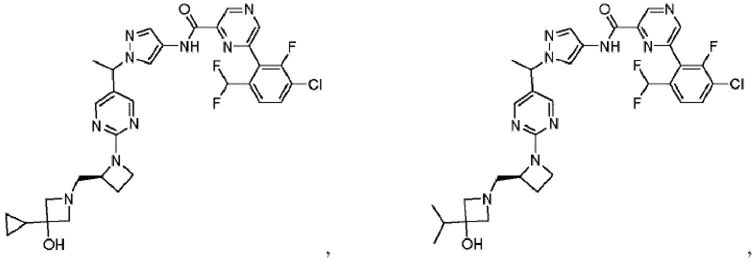 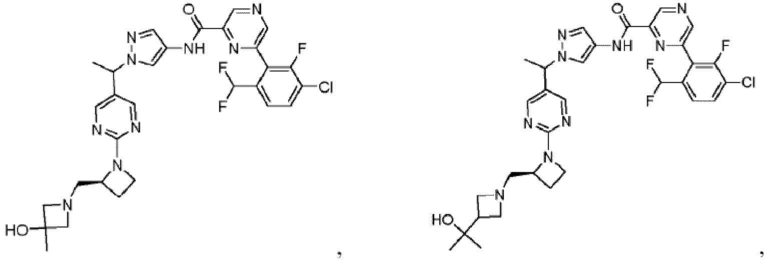 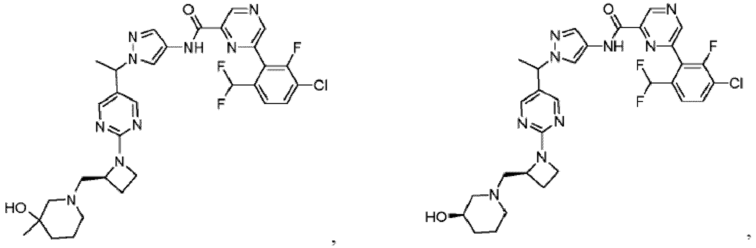 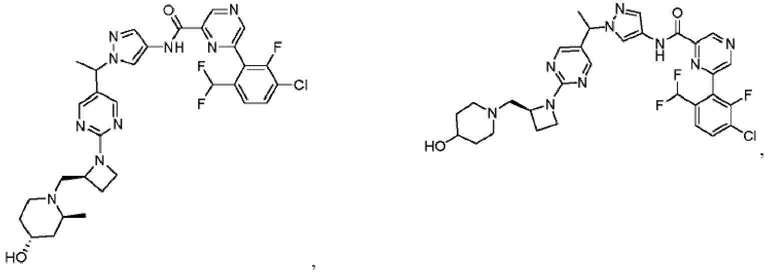 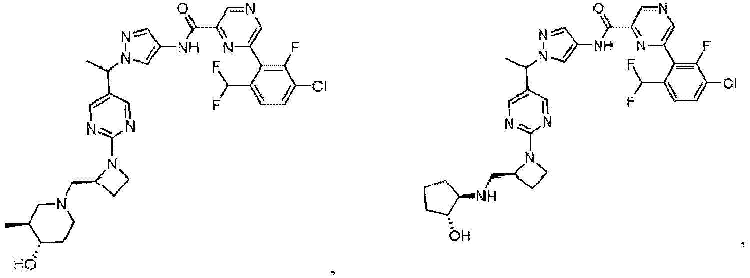 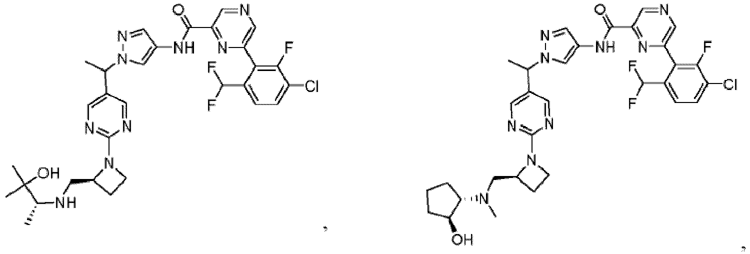 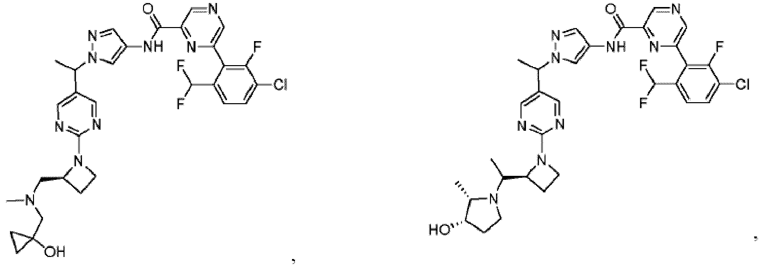 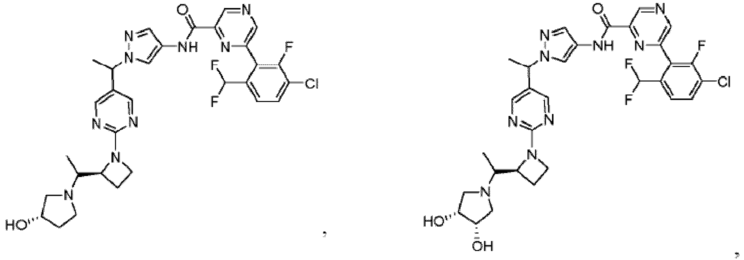 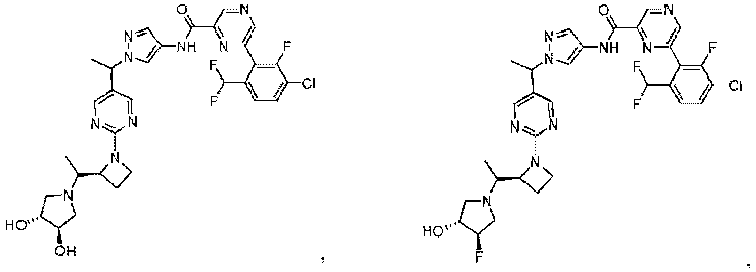 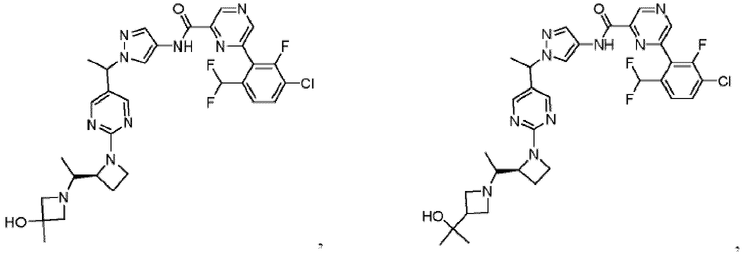 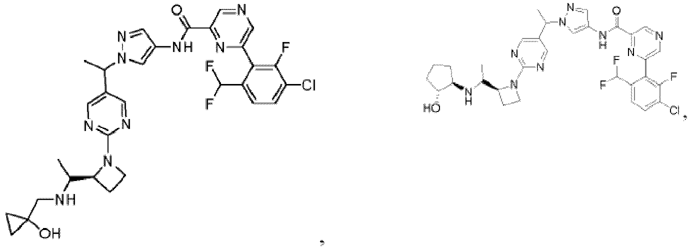 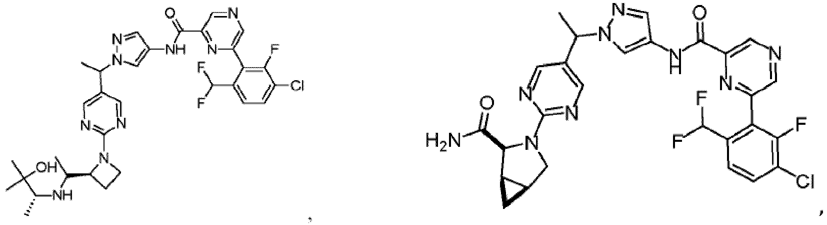 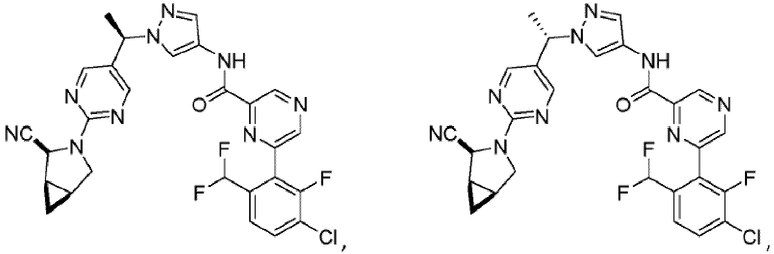 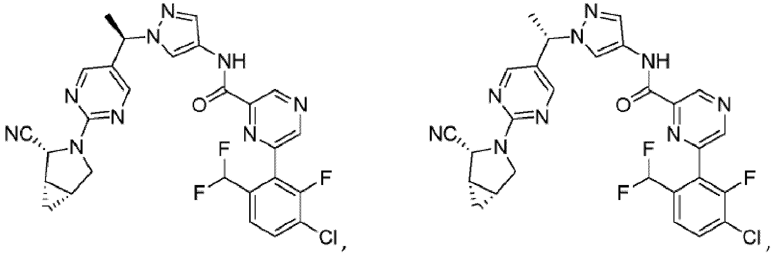 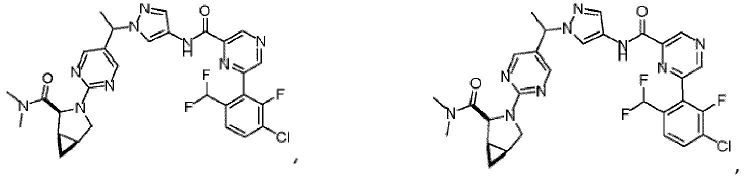 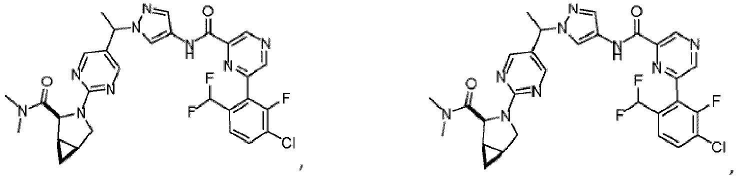 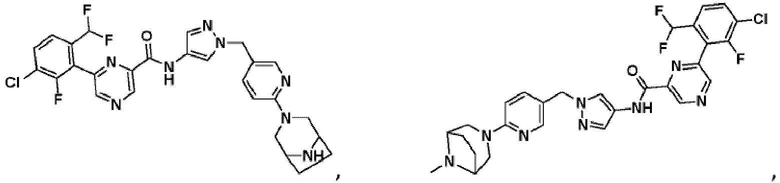 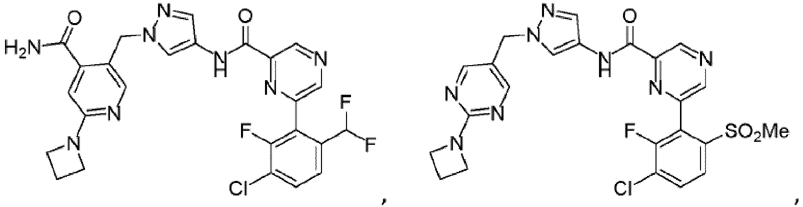 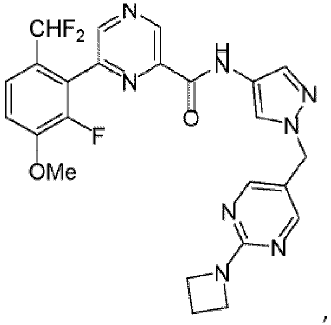 ou un sel pharmaceutiquement acceptable de ceux-ci.

7. Composition pharmaceutique comprenant un composé selon l'une quelconque des revendications 1-6 ou un sel pharmaceutiquement acceptable de celui-ci et un véhicule pharmaceutiquement acceptable.

8. Composé selon l'une quelconque des revendications 1-6 ou un sel pharmaceutiquement acceptable de celui-ci ou composition pharmaceutique selon la revendication 7, pour utilisation dans une méthode pour le traitement de : activité visuelle détériorée, rétinopathie diabétique, œdème maculaire diabétique, occlusion veineuse rétinienne, œdème de Quincke héréditaire, diabète, pancréatite, hémorragie cérébrale, néphropathie, cardiomyopathie, neuropathie, maladie intestinale inflammatoire, arthrite, inflammation, choc septique, hypotension, cancer, syndrome de détresse respiratoire chez l'adulte, coagulation intravasculaire disséminée, coagulation sanguine pendant une chirurgie de dérivation cardio-pulmonaire, saignement à partir d'une chirurgie post-opérative, uvéite, uvéite postérieure, œdème humide maculaire lié à l'âge.

9. Composé selon l'une quelconque des revendications 1-6, ou un sel pharmaceutiquement acceptable de celui-ci, pour utilisation en thérapie.

10. Combinaison comprenant :
un composé selon l'une quelconque des revendications 1-6 ou un sel pharmaceutiquement acceptable de celui-ci ou une composition pharmaceutique selon la revendication 7 ; et
un autre agent choisi dans le groupe constitué de : agents anti-inflammatoires, agents anti-VEGF, agents immunosuppresseurs, anticoagulants, agents antiplaquettaires et agents thrombolytiques.

11. Combinaison selon la revendication 10, pour utilisation en thérapie.
